(19) Europäisches Patentamt
European Patent Office
Office européen des brevets

(11) **EP 4 741 397 A1**

(12) **EUROPEAN PATENT APPLICATION**
published in accordance with Art. 153(4) EPC

(43) Date of publication:
**13.05.2026 Bulletin 2026/20**

(21) Application number: **24838710.2**

(22) Date of filing: **05.07.2024**

(51) International Patent Classification (IPC):
***C07D 491/14*** *(2006.01)* ***A61K 31/395*** *(2006.01)*
***A61P 25/00*** *(2006.01)*

(52) Cooperative Patent Classification (CPC):
**A61K 31/395; A61P 25/00; C07D 491/14**

(86) International application number:
**PCT/CN2024/103839**

(87) International publication number:
**WO 2025/011457 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.07.2023 CN 202310834844**
**20.10.2023 CN 202311370973**

(71) Applicant: **Shenzhen Zhongge Biological Technology Co., Ltd.**
**Futian District**
**Shenzhen, Guangdong 518017 (CN)**

(72) Inventors:
- **XUN, Guoliang**
  **Shenzhen, Guangdong 518017 (CN)**
- **E, Jingwen**
  **Shenzhen, Guangdong 518017 (CN)**
- **DU, Lifei**
  **Shenzhen, Guangdong 518017 (CN)**
- **PAN, Wei**
  **Shenzhen, Guangdong 518017 (CN)**
- **ZHOU, Jianlai**
  **Shenzhen, Guangdong 518017 (CN)**
- **MA, Hongyan**
  **Shenzhen, Guangdong 518017 (CN)**
- **CHEN, Jijun**
  **Shenzhen, Guangdong 518017 (CN)**
- **CAO, Bin**
  **Shenzhen, Guangdong 518017 (CN)**
- **CHEN, Bin**
  **Shenzhen, Guangdong 518017 (CN)**
- **WANG, Shaohui**
  **Shenzhen, Guangdong 518017 (CN)**
- **ZHANG, Peiyu**
  **Shenzhen, Guangdong 518017 (CN)**

(74) Representative: **Johnson, Stephen William et al**
**Venner Shipley LLP**
**406 Cambridge Science Park**
**Milton Road**
**Cambridge CB4 0WW (GB)**

# (54) FUSED TRICYCLIC COMPOUND, AND PREPARATION METHOD THEREFOR AND USE THEREOF

(57) The present invention relates to a fused tricyclic compound, and a preparation method therefor and a use thereof. The compound has a structure shown in a formula (I), has an SARM1 inhibition effect, and can be used for treating neurodegenerative diseases.

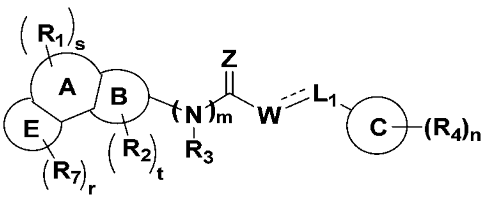

(I)

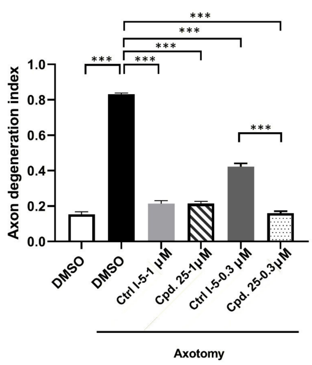


Figure 1

EP 4 741 397 A1

## Description

## Cross-Reference to Related Applications

The present application claims the priorities of Chinese Patent Applications 202310834844.5, filed on July 7, 2023, and 202311370973.X, filed on October 20, 2023, the contents of which are hereby incorporated by reference into the application in their entirety and for all purposes.

## Technical Field

The present invention relates to the field of medicine, specifically to a fused tricyclic compound and a preparation method therefor and a use thereof.

## Background

Axon degeneration is closely related to the occurrence and development of neurological diseases. Wallerian degeneration is the main pathway of axon degeneration. Wallerian degeneration, also known as secondary degeneration, refers to a process in which the distal nerve fibers of axons begin to degrade after the axons are broken due to trauma. Two key proteins are involved in this process: nicotinamide adenine dinucleotide ($NAD^+$) synthase NMNAT2 and $NAD^+$ hydrolase SARM1. SARM1 (Sterile alpha and Toll/interleukin-1 receptor motif-containing 1) is the main executive molecule of this neurodegradation process, and the regulation of its activity plays an important role in the treatment of corresponding neurological diseases.

In healthy neurons, NMNAT2 is transported along axons, maintaining high levels of $NAD^+$ and low levels of nicotinamide mononucleotide (NMN), and SARM1 is maintained at a low activity level. When neurons are damaged, the transport of NMNAT2 stops along axons, and NMNAT2 rapidly decomposes, causing NMN levels to begin to rise and $NAD^+$ levels to begin to decrease. This period is called the axonal degeneration latency period. Changes in NMN and $NAD^+$ lead to the activation of SARM1, which in turn causes $Ca^{2+}$ influx, calpain activation, and ATP depletion. With the accumulation of ROS, axonal degeneration begins to occur. At present, it is reported in many literature that knocking out or inhibiting SARM1 has a protective effect in various neurological disease models, such as peripheral neuropathy, traumatic brain injury, amyotrophic lateral sclerosis, Parkinson's disease, glaucoma, etc.

The treatment methods for neurological diseases such as Alzheimer's disease, Parkinson's disease, multiple sclerosis, amyotrophic sclerosis, Huntington's disease, etc., are very limited, and there is no effective treatment available. At present, most of the treatment methods are symptomatic treatments to alleviate symptoms, which cannot prevent the development of the disease, let alone cure it. In addition, the incidence of neurodegenerative diseases is increasing as the population ages. Therefore, there is an urgent need to develop more effective drugs to help slow down disease progression, improve patients' quality of life, or even prevent disease development in early stages.

## Summary of the invention

The first aspect of the present invention provides a compound represented by formula (I) or a stereoisomer, tautomer, polymorph, solvate, hydrate, N-oxide, isotope-labeled compound, metabolite, ester, prodrug of the compound, or a pharmaceutically acceptable salt thereof,

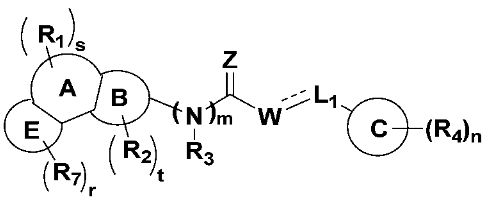

(I),

wherein,

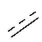

is selected from a single bond, a double bond, and a triple bond;

Z is selected from O and S;

$L_1$ is selected from $-C_{1-3}$ alkylene-, =CH-, -NH-, and -O-, or $L_1$ is absent;
ring A is selected from phenyl, 5-6 membered heteroaryl, $C_{4-8}$ cyclic groups, and 4-8 membered heterocyclyl; ring B is selected from phenyl, 5-6 membered heteroaryl, and 5-6 membered heterocyclyl;
ring C is selected from $C_{6-10}$ aryl, 5-12 membered heteroaryl, $C_{3-12}$ cyclic groups, and 4-10 membered heterocyclyl;
ring E is selected from 5-6 membered heteroaryl and phenyl;
$R_1$ is selected from H, -OH, halogen, $-NH_2$, -CN, $-NO_2$, -COOH, $C_{1-6}$ alkyl, $C_{1-6}$ haloalkyl, $C_{1-6}$ alkoxy, $C_{1-6}$ alkylene-OH, $-O-C_{1-6}$ haloalkyl, $-C_{3-7}$ cycloalkyl, and 3-7 membered heterocycloalkyl;
$R_2$ is selected from H, -OH, halogen, -CN, $-NH_2$, $-NO_2$, -COOH, $C_{1-6}$ alkyl, $C_{1-6}$ haloalkyl, $C_{1-6}$ alkoxy, $C_{1-6}$ alkylene-OH, $-O-C_{1-6}$ haloalkyl, $-C_{1-6}$ alkylene-$C_{1-6}$ alkoxy, $-C_{3-7}$ cycloalkyl, and 3-7 membered heterocycloalkyl; $R_3$ is selected from H, $C_{1-6}$ alkyl, and $-C(=O)C_{1-6}$ alkyl, wherein the $C_{1-6}$ alkyl and $-C(=O)C_{1-6}$ alkyl are optionally substituted with 1, 2, 3, 4 or 5 substituents selected from halogen, -OH, $-NH_2$, -CN, $-NO_2$, and -COOH;
W is selected from $-C(R_5R_6)-$, $-CR_5=$, $-C(=CH_2)-$, and $-NR_5-$, wherein $R_5$ is selected from H, -OH, $C_{1-6}$ alkyl, $C_{1-6}$ alkoxy, $C_{1-6}$ haloalkyl, and $C_{1-6}$ haloalkoxy; or
W and $R_3$ together with the atoms to which they are attached form a 4-6 membered heterocyclyl, wherein the 4-6 membered heterocyclyl is optionally substituted with 1, 2, 3, 4 or 5 R; or
W and ring B together with the atoms to which they are attached form a 6-10 membered heterocyclyl, wherein the 6-10 membered heterocyclyl is optionally substituted with 1, 2, 3, 4 or 5 R;
$R_4$ is selected from halogen, -OH, -CN, $-NH_2$, $C_{1-6}$ alkyl, $C_{1-6}$ alkoxy, $C_{1-6}$ haloalkyl, and $C_{1-6}$ haloalkoxy;
$R_6$ is selected from H, $C_{1-6}$ alkyl, $C_{1-6}$ alkoxy, $C_{1-6}$ haloalkyl, and $C_{1-6}$ haloalkoxy;
$R_7$ is selected from H, halogen, -OH, $-NO_2$, -CN, $-NH_2$, -COOH, $C_{1-6}$ alkyl, $C_{1-6}$ alkoxy, $C_{1-6}$ haloalkyl, $C_{1-6}$ alkylene-OH, $-O-C_{1-6}$ haloalkyl, $-C_{3-7}$ cycloalkyl, and 3-7 membered heterocycloalkyl;
R is selected from halogen, -OH, -CN, =O, $C_{1-6}$ alkyl, $C_{1-6}$ alkoxy, $C_{1-6}$ haloalkyl, and $C_{1-6}$ haloalkoxy;
m is selected from 0 and 1;
n is selected from 0, 1, 2, 3, 4, and 5;
r, s, and t are each independently 0, 1, 2, 3, 4, or 5.

The first aspect of the present invention provides a compound represented by formula (I) or a stereoisomer, tautomer, polymorph, solvate, hydrate, N-oxide, isotope-labeled compound, metabolite, ester, prodrug of the compound, or a pharmaceutically acceptable salt thereof,

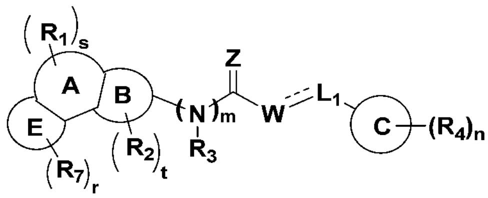

(I),

wherein,

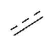

is selected from a single bond, a double bond, and a triple bond;
Z is selected from O and S;
$L_1$ is selected from $-C_{1-3}$ alkylene-, =CH-, -NH-, and -O-, or $L_1$ is absent;
ring A is selected from phenyl, 5-6 membered heteroaryl, $C_{4-8}$ cyclic groups, and 4-8 membered heterocyclyl; ring B is selected from phenyl, 5-6 membered heteroaryl, and 5-6 membered heterocyclyl;
ring C is selected from $C_{6-10}$ aryl, 5-12 membered heteroaryl, $C_{3-12}$ cyclic groups, and 4-10 membered heterocyclyl;
ring E is selected from 5-6 membered heteroaryl and phenyl;
$R_1$ is selected from H, -OH, halogen, $-NH_2$, -CN, $-NO_2$, -COOH, $C_{1-6}$ alkyl, $C_{1-6}$ haloalkyl, $C_{1-6}$ alkoxy, $C_{1-6}$ alkylene-OH, $-O-C_{1-6}$ haloalkyl, $-C_{3-7}$ cycloalkyl, and 3-7 membered heterocycloalkyl;
$R_2$ is selected from H, -OH, halogen, -CN, $-NH_2$, $-NO_2$, -COOH, $C_{1-6}$ alkyl, $C_{1-6}$ haloalkyl, $C_{1-6}$ alkoxy, $C_{1-6}$ alkylene-OH, $-O-C_{1-6}$ haloalkyl, $-C_{1-6}$ alkylene-$C_{1-6}$ alkoxy, $-C_{3-7}$ cycloalkyl, and 3-7 membered heterocycloalkyl; $R_3$ is selected from H, $C_{1-6}$ alkyl, and $-C(=O)C_{1-6}$ alkyl, wherein the $C_{1-6}$ alkyl and $-C(=O)C_{1-6}$ alkyl are optionally substituted with 1, 2, 3, 4 or 5 substituents selected from halogen, -OH, $-NH_2$, -CN, $-NO_2$, and -COOH;
W is selected from $-C(R_5R_6)-$, $-CR_5=$, $-C(=CH_2)-$, and $-NR_5-$, wherein $R_5$ is selected from H, -OH, $C_{1-6}$ alkyl, $C_{1-6}$ alkoxy, $C_{1-6}$ haloalkyl, and $C_{1-6}$ haloalkoxy; or
W and $R_3$ together with the atoms to which they are attached form a 4-6 membered heterocyclyl, wherein the 4-6

membered heterocyclyl is optionally substituted with 1, 2, 3, 4 or 5 R;
$R_4$ is selected from halogen, -OH, -CN, $-NH_2$, $C_{1-6}$ alkyl, $C_{1-6}$ alkoxy, $C_{1-6}$ haloalkyl, and $C_{1-6}$ haloalkoxy;
$R_6$ is selected from H, $C_{1-6}$ alkyl, $C_{1-6}$ alkoxy, $C_{1-6}$ haloalkyl, and $C_{1-6}$ haloalkoxy;
$R_7$ is selected from H, halogen, -OH, $-NO_2$, -CN, $-NH_2$, -COOH, $C_{1-6}$ alkyl, $C_{1-6}$ alkoxy, $C_{1-6}$ haloalkyl, $C_{1-6}$ alkylene-OH, $-O-C_{1-6}$ haloalkyl, $-C_{3-7}$ cycloalkyl, and 3-7 membered heterocycloalkyl;
R is selected from halogen, -OH, -CN, =O, $C_{1-6}$ alkyl, $C_{1-6}$ alkoxy, $C_{1-6}$ haloalkyl, and $C_{1-6}$ haloalkoxy;
m is selected from 0 and 1;
n is selected from 0, 1, 2, 3, 4, and 5;
r, s, and t are each independently 0, 1, 2, 3, 4, or 5.
In some embodiments, W is selected from $-C(R_5R_6)-$, $-CR_5=$, $-C(=CH_2)-$, and $-NR_5-$, or
W and $R_3$ together with the atoms to which they are attached form a 4-6 membered heterocyclyl, wherein the 4-6 membered heterocyclyl is optionally substituted with 1, 2, 3, 4 or 5 R.

In some embodiments, W is selected from $-C(R_5R_6)-$ and $-C(=CH_2)-$, or
W and $R_3$ together with the atoms to which they are attached form a 4-6 membered heterocyclyl, wherein the 4-6 membered heterocyclyl is optionally substituted with 1, 2, 3, 4 or 5 R.

In some embodiments, W is selected from $-C(R_5R_6)-$ and $-C(=CH_2)-$, or
W and $R_3$ together with the atoms to which they are attached form a 5-6 membered heterocyclyl, wherein the 5-6 membered heterocyclyl is optionally substituted with 1, 2, 3, 4 or 5 R.

In some embodiments, W is $-C(R_5R_6)-$, or

W and $R_3$ together with the atoms to which they are attached form a 5-6 membered heterocyclyl, wherein the 5-6 membered heterocyclyl is optionally substituted with 1, 2, 3, 4 or 5 R.

In some embodiments, m is 1.

In some embodiments, ring A is selected from phenyl, dihydropyranyl, pyridyl, dihydropyridyl, and oxepanyl.

In some embodiments, n is 0, 1, 2, or 3.

In some embodiments, r, s, and t are each independently 0, 1, or 2.

In some embodiments, r, s, and t are each independently 0 or 1.

In some embodiments, $R_1$ is selected from H, -OH, halogen, -CN, $-NH_2$, $-NO_2$, -COOH, $C_{1-4}$ alkyl, $C_{1-4}$ haloalkyl, $C_{1-4}$ alkoxy, $C_{1-4}$ alkylene-OH, $-O-C_{1-4}$ haloalkyl, $-C_{3-6}$ cycloalkyl, and 5-6 membered heterocycloalkyl.

In some embodiments, $R_1$ is selected from H, -OH, F, Cl, Br, I, $-NH_2$, -CN, $-NO_2$, -COOH, methyl, ethyl, *n*-propyl, isopropyl, *n*-butyl, isobutyl, *sec*-butyl, *tert-butyl,* halomethyl, haloethyl, halopropyl, halobutyl, methoxy, ethoxy, propoxy, butoxy, halomethoxy, haloethoxy, halopropoxy, halobutoxy, hydroxymethyl, hydroxyethyl, hydroxypropyl, cyclopropyl, cyclobutyl, cyclopentyl, and cyclohexyl.

In some embodiments, $R_1$ is selected from H, -OH, F, Cl, Br, $-NH_2$, -CN, $-NO_2$, -COOH, methyl, ethyl, n-propyl, methoxy, ethoxy, monofluoromethyl, difluoromethyl, trifluoromethyl, hydroxymethyl, and hydroxyethyl.

In some embodiments, $R_1$ is selected from F, Cl, Br, and I.

In some embodiments, $R_1$ is selected from $-NH_2$, -CN, $-NO_2$, and -COOH.

In some embodiments, $R_1$ is selected from methyl, methoxy, monofluoromethyl, difluoromethyl, trifluoromethyl, hydroxymethyl and hydroxyethyl.

In some embodiments, $R_1$ is selected from H and -OH

In some embodiments, $R_1$ is H.

In some embodiments, $R_2$ is selected from H, -OH, halogen, -CN, $-NH_2$, $-NO_2$, -COOH, $C_{1-4}$ alkyl, $C_{1-4}$ haloalkyl, $C_{1-4}$ alkoxy, $C_{1-4}$ alkylene-OH, $-O-C_{1-4}$ haloalkyl, $-C_{1-4}$ alkylene-$C_{1-4}$ alkoxy, $-C_{3-6}$ cycloalkyl, and 4-6 membered heterocycloalkyl.

In some embodiments, $R_2$ is selected from H, -OH, F, Cl, Br, I, -CN, $-NH_2$, $-NO_2$, -COOH, methyl, ethyl, *n*-propyl, isopropyl, *n*-butyl, isobutyl, *sec*-butyl, *tert*-butyl, halomethyl, haloethyl, halopropyl, halobutyl, methoxy, ethoxy, propoxy, butoxy, hydroxymethyl, hydroxyethyl, hydroxypropyl, halomethoxy, haloethoxy, halopropoxy, halobutoxy, $-CH_2OCH_3$, $-(CH_2)_2OCH_3$, $-(CH_2)_3OCH_3$, $-CH_2OCH_2CH_3$, $-CH_2O(CH_2)_2CH_3$, cyclopropyl, cyclobutyl, cyclopentyl, and cyclohexyl.

In some embodiments, $R_2$ is selected from H, -OH, F, Cl, Br, $-NH_2$, -CN, $-NO_2$, -COOH, methyl, ethyl, *n*-propyl, methoxy, ethoxy, monofluoromethyl, difluoromethyl, trifluoromethyl, hydroxymethyl, hydroxyethyl, $-CH_2OCH_3$, $-(CH_2)_2OCH_3$, and $-CH_2OCH_2CH_3$.

In some embodiments, $R_2$ is selected from H, Cl, -CN, $-NH_2$, $-CH_3$, and $-CH_2OCH_3$.

In some embodiments, $R_2$ is selected from H, $-NH_2$, $-CH_3$, and $-CH_2OCH_3$.

In some embodiments, $R_2$ is selected from H, $-CH_3$, and $-CH_2OCH_3$.

In some embodiments, $R_2$ is selected from H and $-NH_2$.

In some embodiments, $R_2$ is H.

In some embodiments, $R_7$ is selected from H, halogen, -OH, $-NO_2$, -CN, $-NH_2$, -COOH, $C_{1-4}$ alkyl, $C_{1-4}$ alkoxy, $C_{1-4}$ haloalkyl, $-C_{1-4}$ alkylene-OH, $-O-C_{1-4}$ haloalkyl, $-C_{3-6}$ cycloalkyl, and 4-6 membered heterocycloalkyl.

In some embodiments, $R_7$ is selected from H, F, Cl, Br, I, -OH, -$NO_2$, -CN, -$NH_2$, -COOH, methyl, ethyl, *n*-propyl, isopropyl, *n*-butyl, isobutyl, *sec*-butyl, *tert*-butyl, methoxy, ethoxy, propoxy, butoxy, halomethoxy, haloethoxy, halopropoxy, halobutoxy, hydroxymethyl, hydroxyethyl, hydroxypropyl, halomethoxy, haloethoxy, halopropoxy, halobutoxy, cyclopropyl, cyclobutyl, cyclopentyl, and cyclohexyl.

In some embodiments, $R_7$ is selected from H, F, Cl, Br, I, -OH, -$NO_2$, -CN, -$NH_2$, -COOH, methyl, ethyl, methoxy, and ethoxy.

In some embodiments, $R_7$ is selected from H, F, Cl, Br, and -$CH_3$.

In some embodiments, $R_7$ is selected from H, F, Cl, and Br.

In some embodiments, $R_7$ is H.

In some embodiments, the structural unit

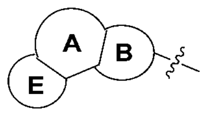

is selected from

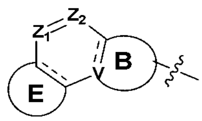

and

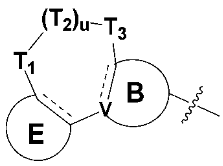

;

wherein,

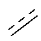

is selected from a single bond and a double bond;

$Z_1$ and $Z_2$ are each independently selected from CH and N;

V is selected from C, CH, and N; preferably, V is selected from C and N;

$T_1$, $T_2$, and $T_3$ are each independently selected from $CH_2$, O, S, and NH; preferably, $T_1$, $T_2$, and $T_3$ are each independently selected from $CH_2$, O, and NH;

u is selected from 0, 1, and 2; preferably, u is selected from 0 and 1.

In some embodiments, the structural unit

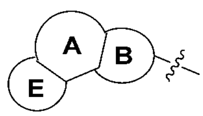

is selected from

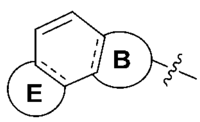

,

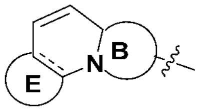, 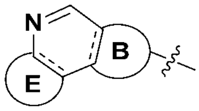, 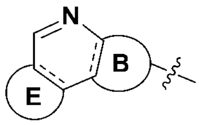, 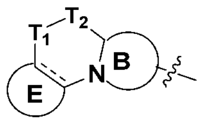, and 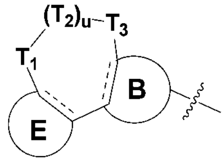.

In some embodiments, the structural unit

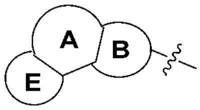

is selected from

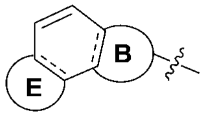,

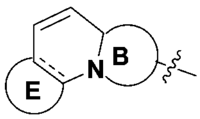, 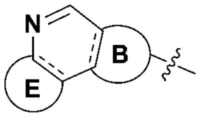, 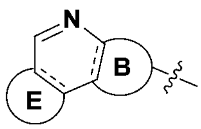, and 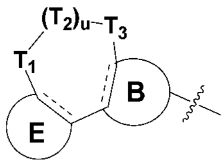,

wherein,

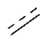

is selected from a single bond and a double bond;

$T_1$, $T_2$, and $T_3$ are each independently selected from $CH_2$, O, and N;

u is selected from 0, 1, and 2.

In some embodiments, the structural unit

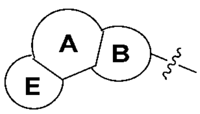

is selected from

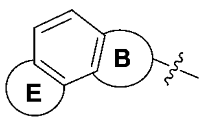,

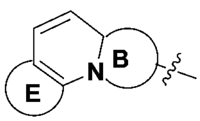, 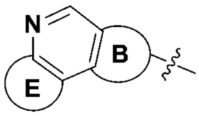, 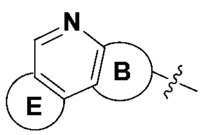, and 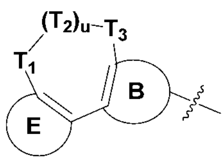,

wherein,

$T_1$, $T_2$, and $T_3$ are each independently selected from $CH_2$, O, and N;
u is selected from 0, 1, and 2.

In some embodiments, the structural unit

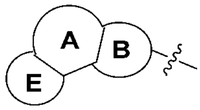

is selected from

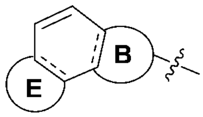,

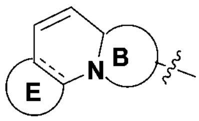, 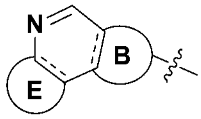, 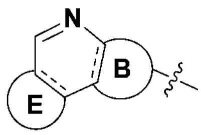, 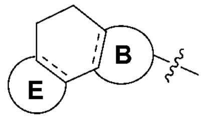, 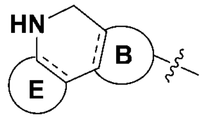,

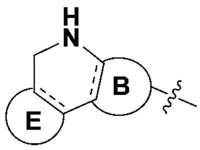, 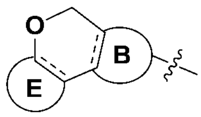, 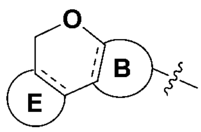, 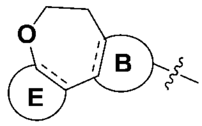, 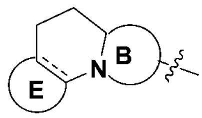,

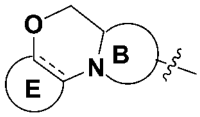, 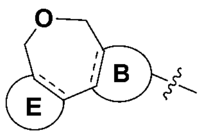, and 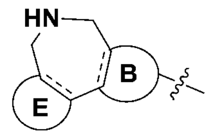;

wherein,

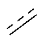

is selected from a single bond and a double bond.

In some embodiments, the structural unit

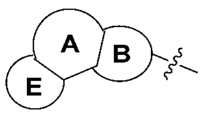

is selected from

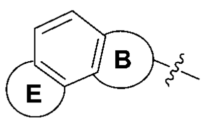,

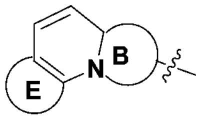, 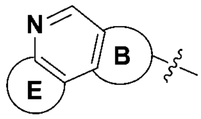, 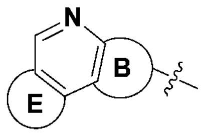, 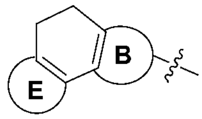, 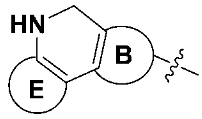, 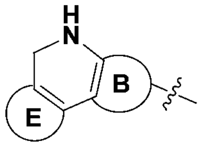, 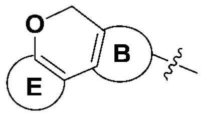, 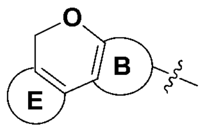, 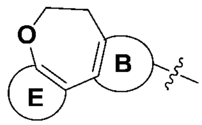, 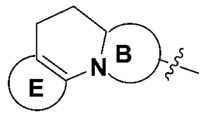, 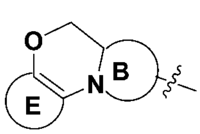, 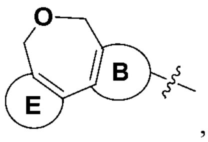, and 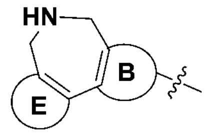;

wherein, rings E and B are as defined herein.

In some embodiments, the structural unit

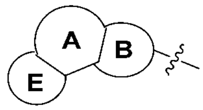

is selected from

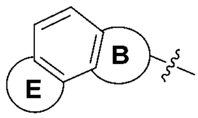,

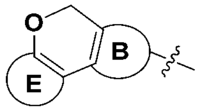, and 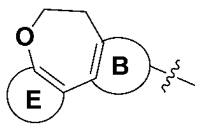.

In some embodiments, ring B is selected from 5-membered nitrogen-containing heteroaryl and 5-membered nitrogen-containing heterocyclic alkenyl.

In some embodiments, ring B is selected from pyrrolyl, pyrazolyl, imidazolyl, oxazolyl and dihydrotriazolyl.

In some embodiments, ring B is selected from pyrrolyl, pyrazolyl, imidazolyl, and dihydrotriazolyl.

In some embodiments, ring B is selected from pyrrolyl, pyrazolyl, imidazolyl, and oxazolyl.

In some embodiments, ring B is selected from imidazolyl, pyrazolyl, and oxazolyl.

In some embodiments, ring E is 6-membered nitrogen-containing heteroaryl.

In some embodiments, ring E is selected from pyridyl and pyridazinyl.

In some embodiments, ring E is pyridyl.

In some embodiments, the structural unit

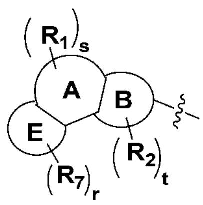

is selected from

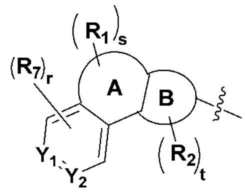

,

wherein, $Y_1$ and $Y_2$ are each independently selected from CH and N; preferably, $Y_1$ is N and $Y_2$ is CH, or $Y_1$ is CH and $Y_2$ is N, or $Y_1$ and $Y_2$ are N.

In some embodiments, the structural unit

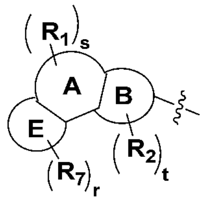

is selected from

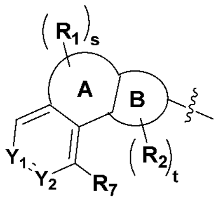

,

wherein, $Y_1$ and $Y_2$ are each independently selected from CH and N; ring A, ring B, $R_1$, $R_2$, $R_7$, r, s, and t are as defined herein. In some embodiments, $Y_1$ is N and $Y_2$ is CH.

In some embodiments, $Y_1$ is CH and $Y_2$ is N.

In some embodiments, the structural unit

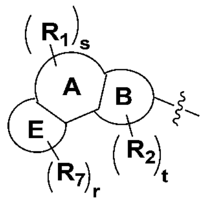

is selected from

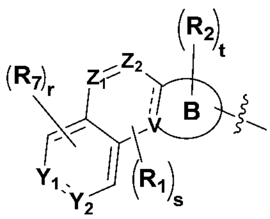

and

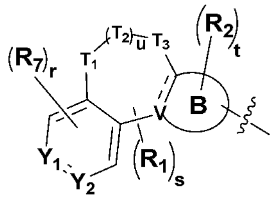

,

wherein,

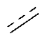

is selected from a single bond and a double bond;

$Z_1$ and $Z_2$ are each independently selected from CH and N;

V is selected from C, CH, and N; preferably, V is selected from C and N;

$T_1$, $T_2$, and $T_3$ are each independently selected from $CH_2$, O, and NH;

u is selected from 0, 1, and 2; preferably, u is selected from 0 and 1.

In some embodiments, the structural unit

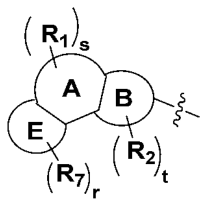

is selected from

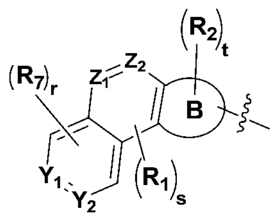

,

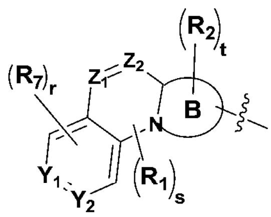

,

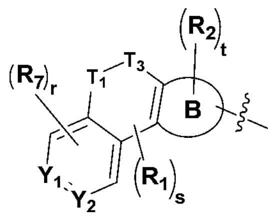

,

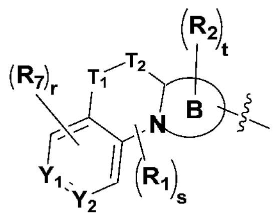

, and

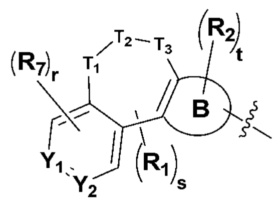

.

In some embodiments, the structural unit

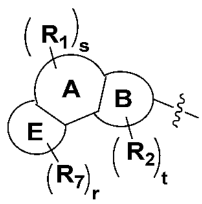

is selected from

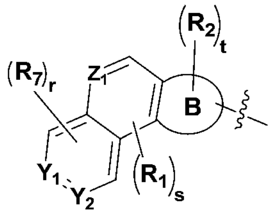

,

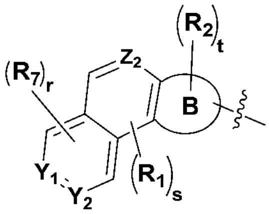

,

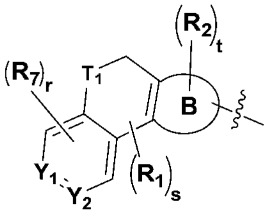

,

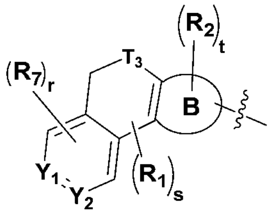

,

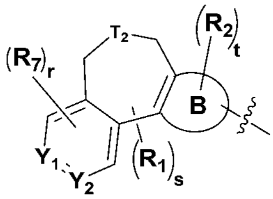

,

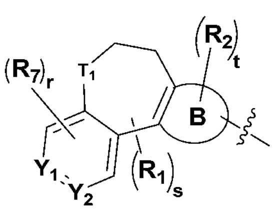

,

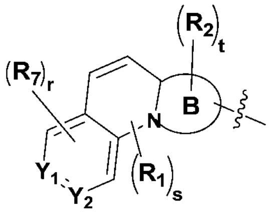

, and

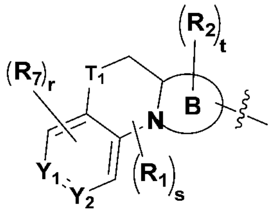

.

In some embodiments, the structural unit

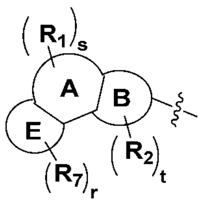

is selected from

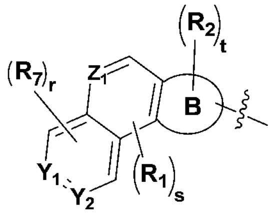

,

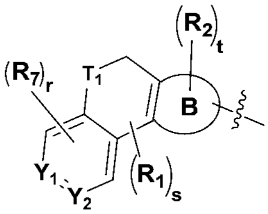

, and

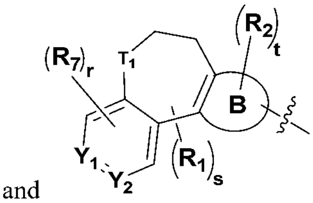

.

In some embodiments, the structural unit is selected from

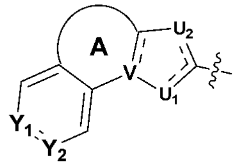,
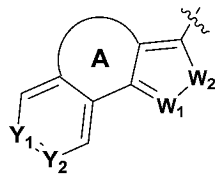,

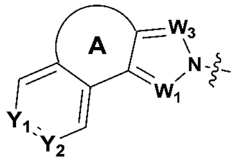, and
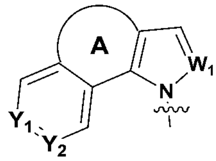,

wherein,

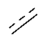

is selected from a single bond and a double bond;

$Y_1$ and $Y_2$ are each independently selected from CH and N;

V is selected from C, CH, and N; preferably, V is selected from C and N;

$U_1$ and $U_2$ are each independently selected from O, N, NH, and CH;

$W_1$ and $W_3$ are selected from CH and N;

$W_2$ is selected from NH and O;

ring A is selected from phenyl, 5-6 membered heteroaryl, $C_{4-8}$ cyclic groups, and 4-8 membered heterocyclyl; preferably, ring A is selected from phenyl, 6 membered heteroaryl, $C_{6-7}$ cyclic groups, and 6-7 membered heterocyclyl; more preferably, ring A is selected from phenyl and 6-7 membered heterocyclyl.

In some embodiments, the structural unit

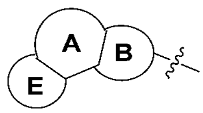

is selected from

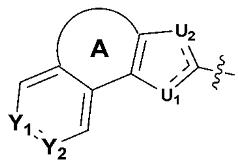,

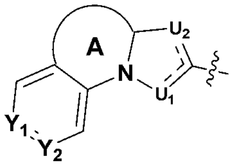,
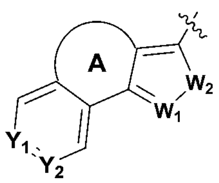,
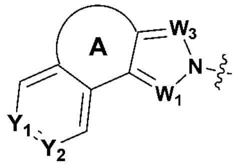, and
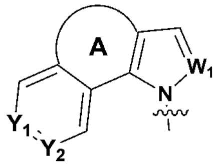.

In some embodiments, the structural unit

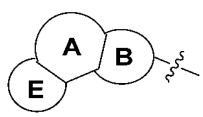

is selected from

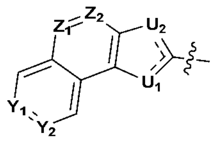

,

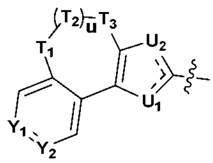

,

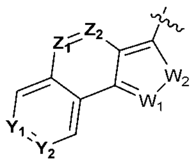

,

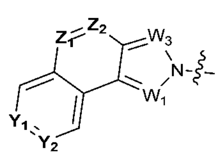

,

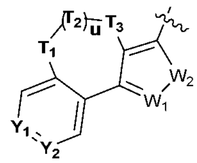

,

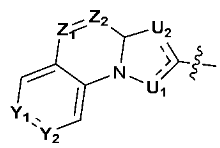

,

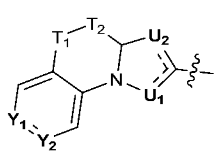

, and

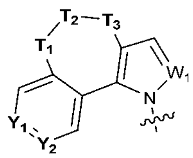

;

wherein,

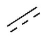

is selected from a single bond and a double bond, and when two

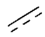

are attached to the same atom, one is a single bond, and the other is a double bond;

$Y_1$ and $Y_2$ are each independently selected from CH and N;

$Z_1$ and $Z_2$ are each independently selected from CH and N;

$U_1$ and $U_2$ are each independently selected from O, N, NH, and CH;

$T_1$, $T_2$, and $T_3$ are each independently selected from $CH_2$, O, and NH;

$W_1$ and $W_3$ are selected from CH and N;

$W_2$ is selected from NH and O;

u is selected from 0 and 1.

In some embodiments, the structural unit

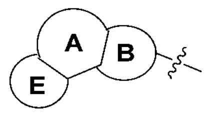

is selected from

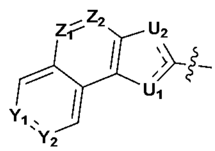

,

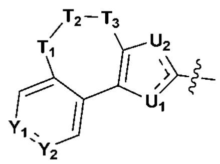, 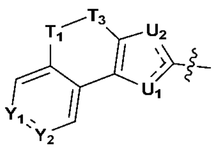, 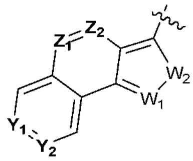, 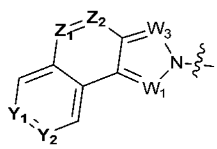, 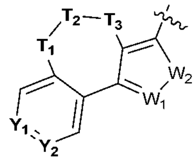,

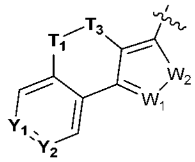, 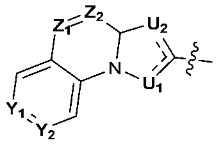, 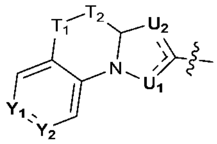, and 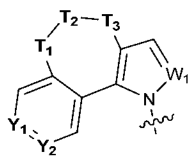.

In some embodiments, in the structural unit

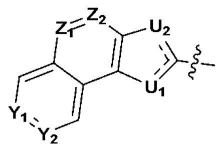,

preferably, $Y_1$ is N, $Y_2$ is CH, or $Y_1$, $Y_2$ are N; more preferably, $Y_1$ is N, $Y_2$ is CH;
preferably, $Z_1$ is CH, $Z_2$ is CH, or $Z_1$ is N, $Z_2$ is CH, or $Z_1$ is CH, $Z_2$ is N; more preferably, $Z_1$ is CH, $Z_2$ is CH;
preferably, $U_1$ is selected from N and CH, $U_2$ is selected from NH and O, or $U_1$ is selected from NH and O, $U_2$ is selected from N and CH; more preferably, $U_1$ is N, $U_2$ is NH, or $U_1$ is NH, $U_2$ is N, or $U_1$ is N, $U_2$ is O.

In some embodiments, in the structural unit

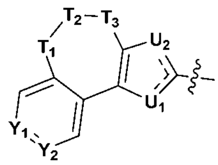,

preferably, $Y_1$ is N, $Y_2$ is CH;
preferably, $T_1$ is $CH_2$, $T_2$ is selected from O and NH, $T_3$ is $CH_2$; or $T_1$ is O, $T_1$ and $T_3$ are $CH_2$;
preferably, $U_1$ is N, $U_2$ is NH; or $U_1$ is NH, $U_2$ is N.

In some embodiments, in the structural unit

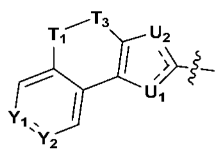,

preferably, $Y_1$ is N, $Y_2$ is CH;
preferably, $T_1$ is NH, $T_3$ is $CH_2$, or $T_1$ is $CH_2$, $T_3$ is NH, or $T_1$ is O, $T_3$ is $CH_2$, or $T_1$ is $CH_2$, $T_3$ is O, or $T_1$ is $CH_2$, $T_3$ is $CH_2$; more preferably, $T_1$ is O, $T_3$ is $CH_2$;
preferably, $U_1$ is selected from N and CH, $U_2$ is NH, or $U_1$ is NH, $U_2$ is selected from N and CH; more preferably, $U_1$ is N, $U_2$ is NH, or $U_1$ is NH, $U_2$ is N.

In some embodiments, in the structural unit

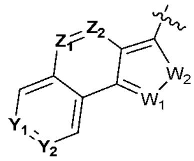

,

preferably, $Y_1$ is N, $Y_2$ is CH;
preferably, $Z_1$ is CH, $Z_2$ is CH;
preferably, $W_1$ is N, $W_2$ is NH.

In some embodiments, in the structural unit

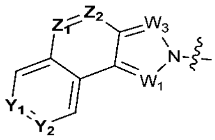

,

preferably, $Y_1$ is N, $Y_2$ is CH;
preferably, $Z_1$ is CH, $Z_2$ is CH;
preferably, $W_1$ is N, $W_3$ is CH.

In some embodiments, in the structural unit

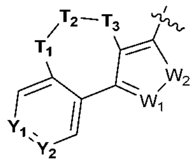

,

preferably, $Y_1$ is N, $Y_2$ is CH;
preferably, $T_1$ is O, $T_2$ and $T_3$ are $CH_2$, or $T_1$ is $CH_2$, $T_2$ is O, $T_3$ is $CH_2$; more preferably, $T_1$ is O, $T_2$ and $T_3$ are $CH_2$;
preferably, $W_1$ is N, $W_2$ is NH.

In some embodiments, in the structural unit

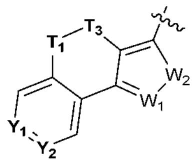

,

preferably, $Y_1$ is N, $Y_2$ is CH;
preferably, $T_1$ is O, $T_3$ is $CH_2$, or $T_1$ is $CH_2$, $T_3$ is O, or $T_1$ is NH, $T_3$ is $CH_2$, or $T_1$ is $CH_2$, $T_3$ is NH;
preferably, $W_1$ is N, $W_2$ is NH.

In some embodiments, in the structural unit

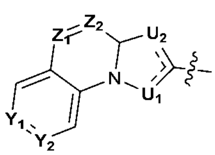

,

preferably, $Y_1$ is N, $Y_2$ is CH;

preferably, $Z_1$ is CH, $Z_2$ is CH;
preferably, $U_1$ is N, $U_2$ is NH, or $U_1$ is NH, $U_2$ is N; more preferably, $U_1$ is N, $U_2$ is NH.

In some embodiments, in the structural unit

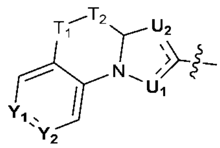,

preferably, $Y_1$ is N, $Y_2$ is CH;
preferably, $T_1$ is selected from $CH_2$ and O, $T_2$ is $CH_2$;
preferably, $U_1$ is N, $U_2$ is NH, or $U_1$ is NH, $U_2$ is N; more preferably, $U_1$ is N, $U_2$ is NH.

In some embodiments, in the structural unit

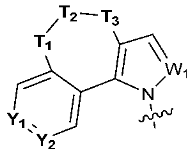,

preferably, $Y_1$ is N, $Y_2$ is CH;
preferably, $T_1$ is O, $T_2$ and $T_3$ are $CH_2$;
preferably, $W_1$ is N.

In some embodiments, the structural unit

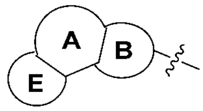

is selected from

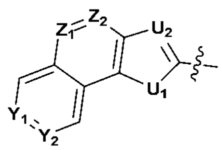,

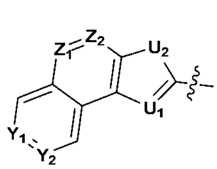, 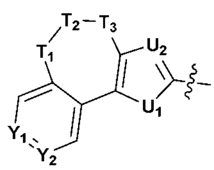, 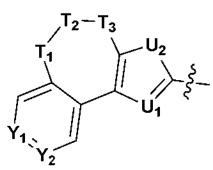, 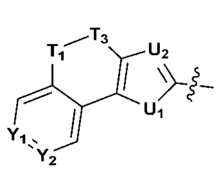, 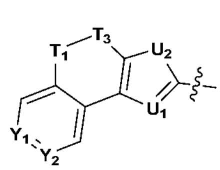,

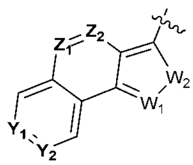, 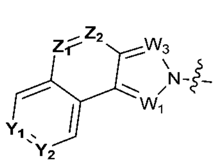, 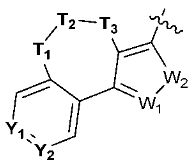, 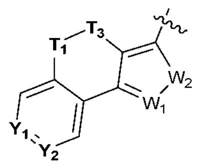, 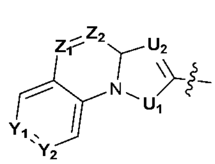,

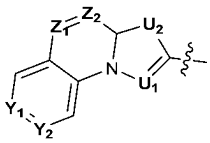, 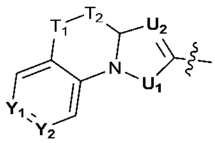, 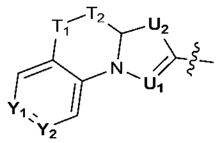, and 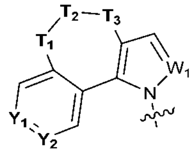.

In some embodiments, the structural unit

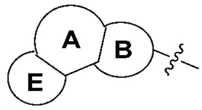

is selected from

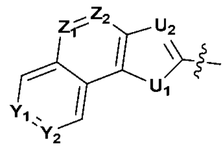,

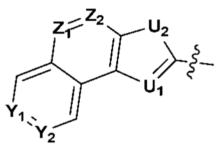, 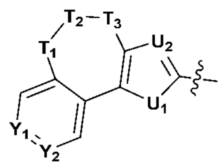, 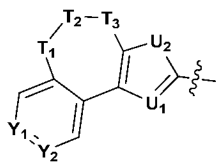, 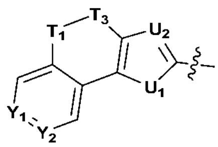, 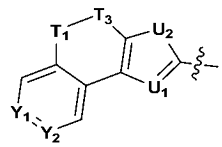,

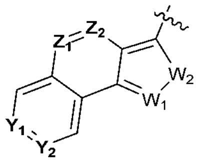, 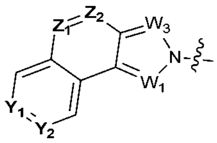, 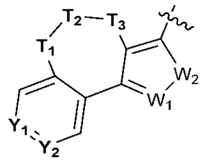, 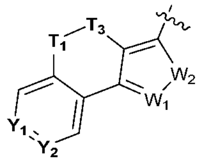, 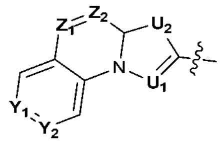,

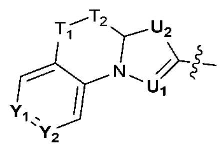, and 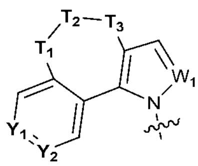.

In some embodiments, the structural unit

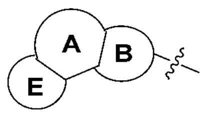

is selected from

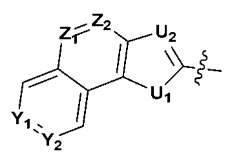,

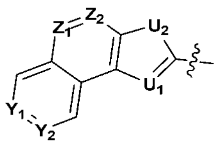, 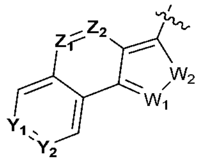, 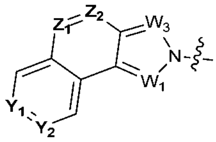, 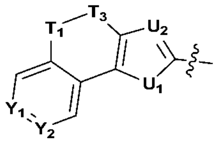, 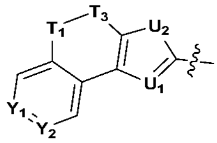,

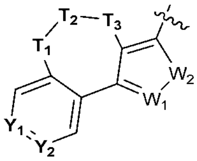, and 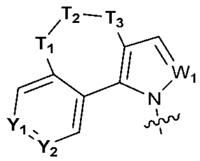.

In some embodiments, the structural unit

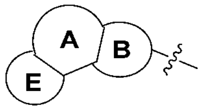

is selected from

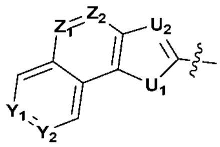,

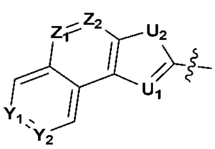, 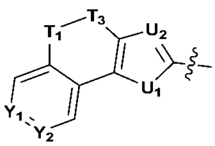, 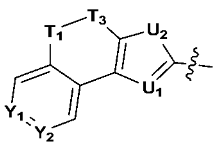, and 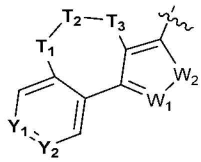.

In some embodiments, the structural unit

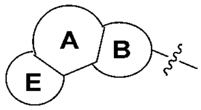

is selected from

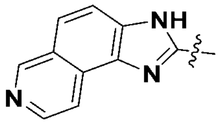,

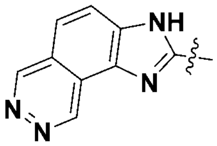, 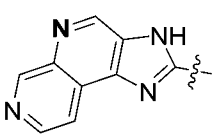, 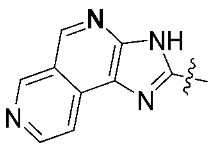, 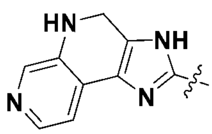, 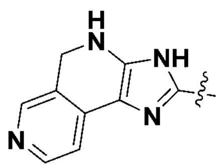,

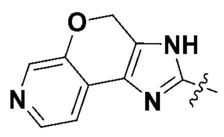, 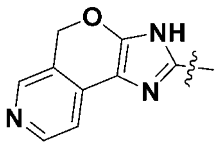, 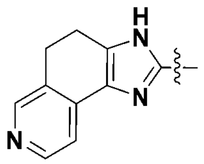, 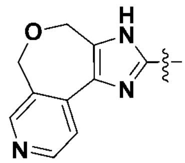, 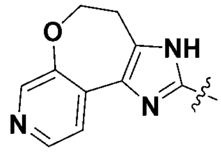,

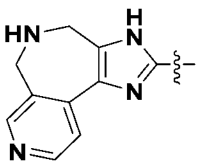, 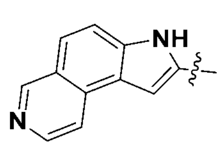, 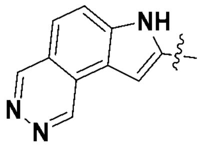, 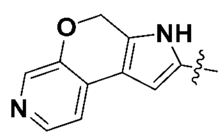, 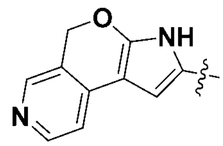,

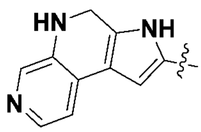, 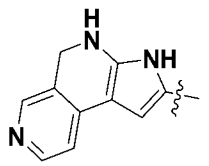, 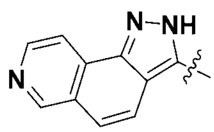, 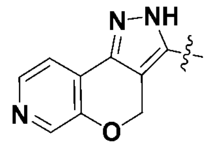, 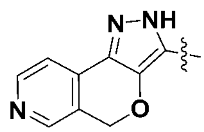,

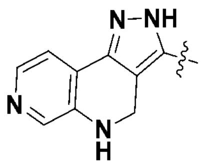, 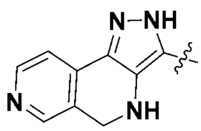, 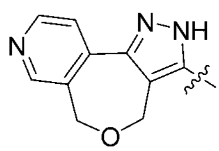, 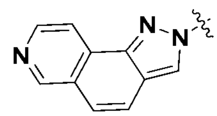, 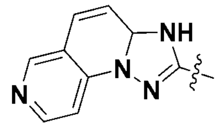,

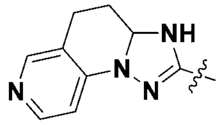, 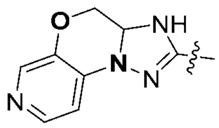, 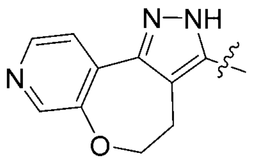, 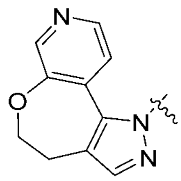,

and

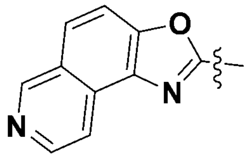.

In some embodiments, the structural unit

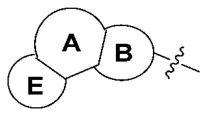

is selected from

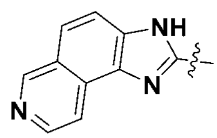,

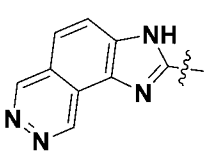, 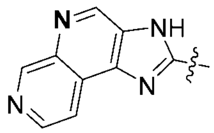, 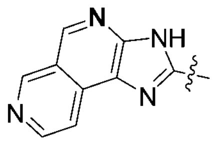, 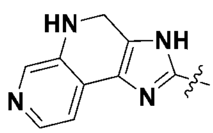, 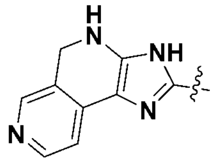,

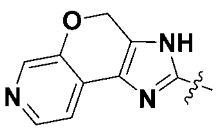, 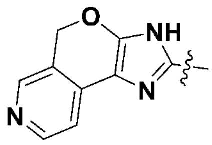, 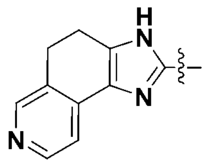, 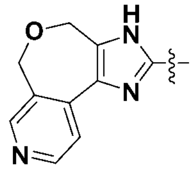, 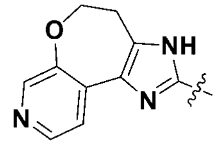,

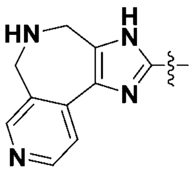, 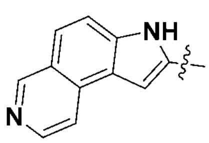, 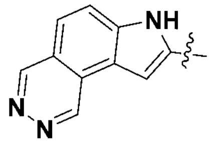, 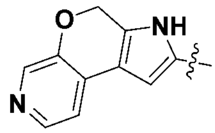, 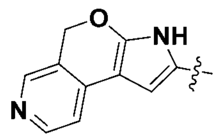,

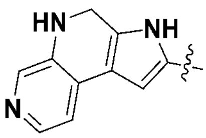, 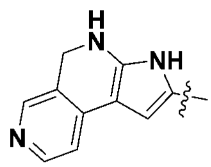, 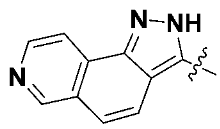, 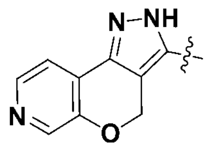, 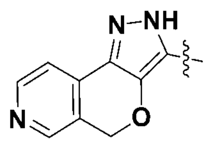,

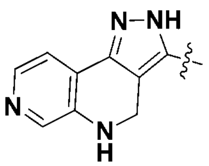, 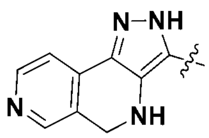, 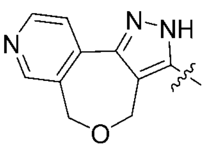, 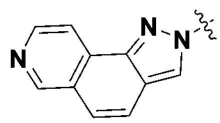, 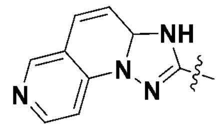,

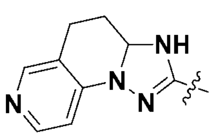, and 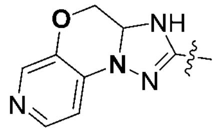.

In some embodiments, the structural unit

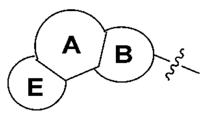

is selected from

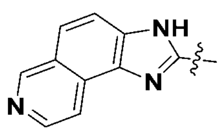,

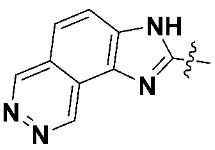, 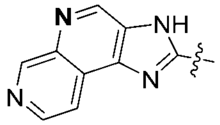, 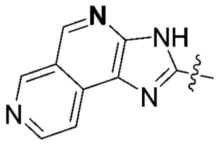, 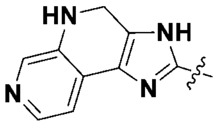, 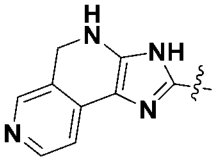,

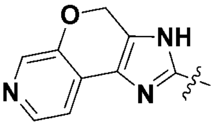, 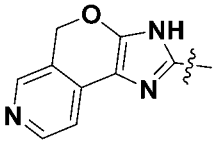, 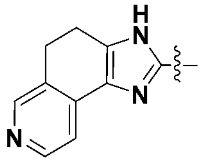, 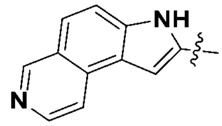, 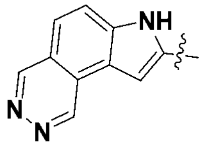,

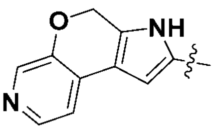, 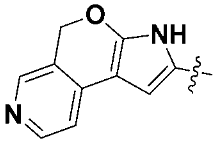, 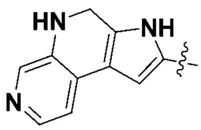, 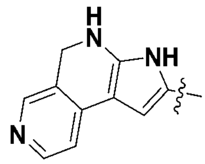, 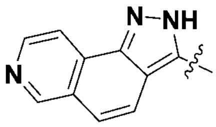,

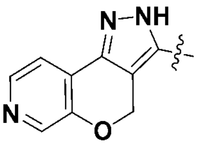, 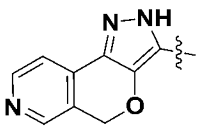, 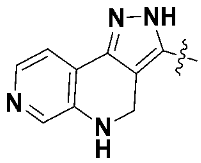, 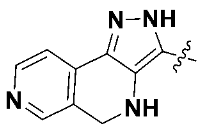, 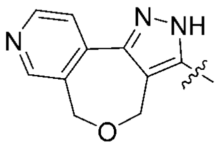,

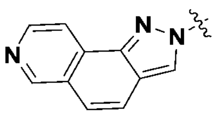, 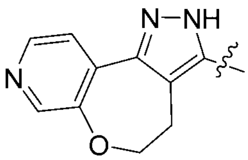, 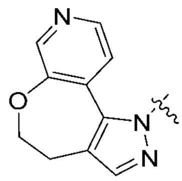, and 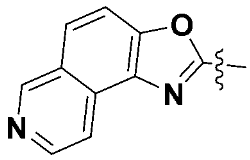.

In some embodiments, the structural unit

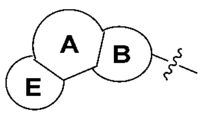

is selected from

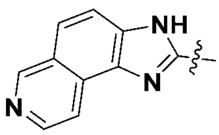,

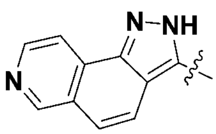, 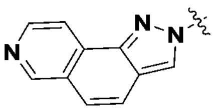, 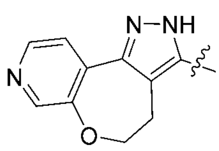, 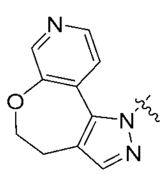, 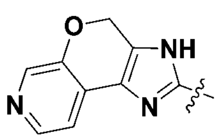,

and

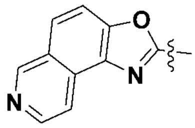

.

In some embodiments, the structural unit

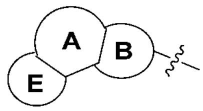

is selected from

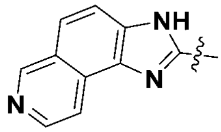

,

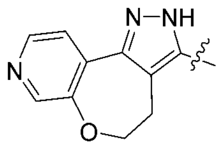

, 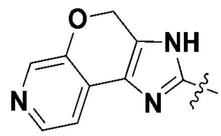

, and 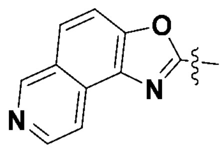

.

In some embodiments, the structural unit

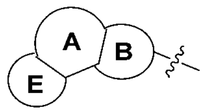

is

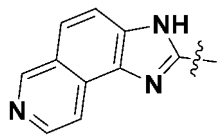

.

In some embodiments, the structural unit

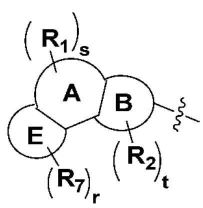

is selected from

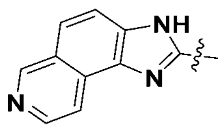

,

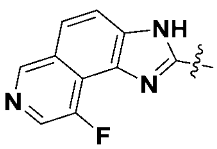, 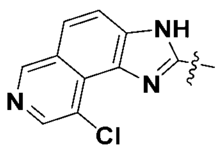, 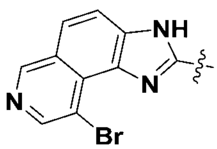, 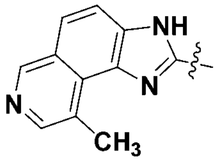, 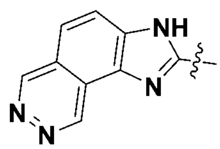,

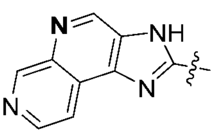, 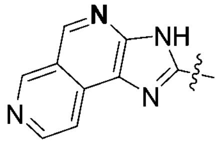, 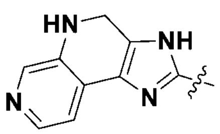, 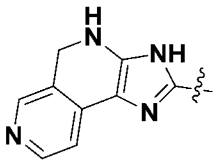, 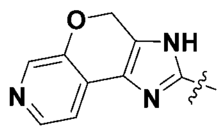,

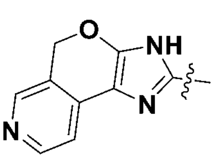, 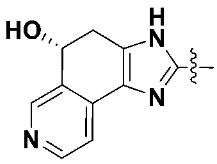, 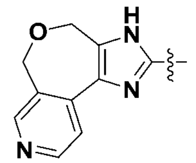, 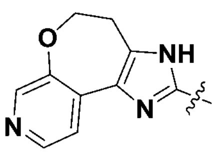, 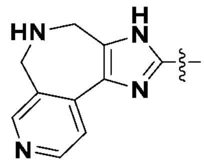,

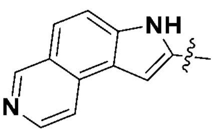, 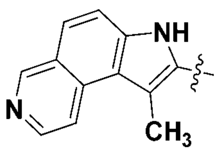, 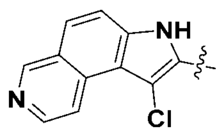, 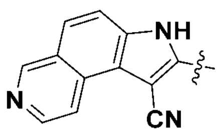, 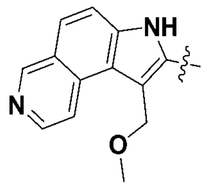,

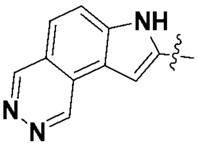, 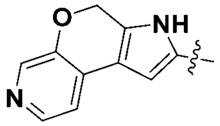, 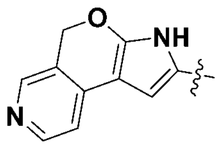, 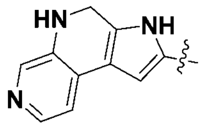, 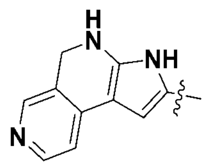,

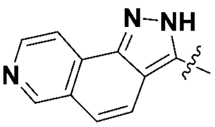, 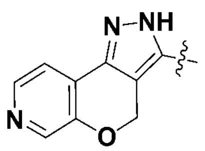, 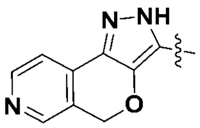, 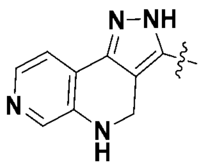, 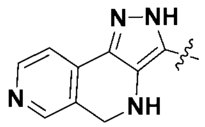,

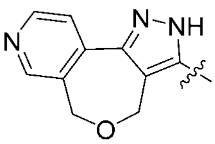, 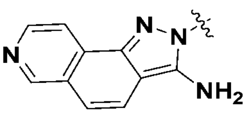, 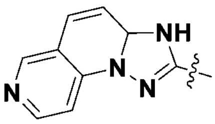, 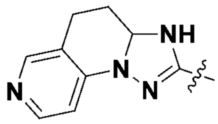, 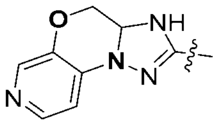,

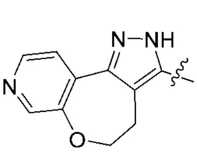, 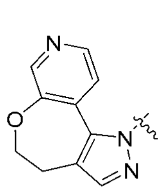, 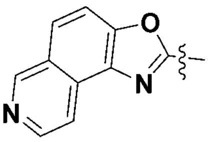, and 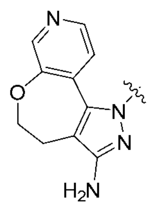.

In some embodiments, the structural unit

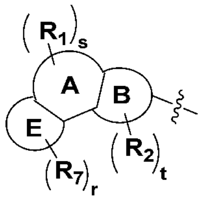

is selected from

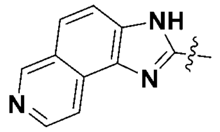 ,

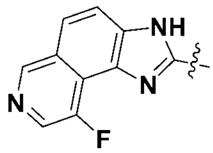 , 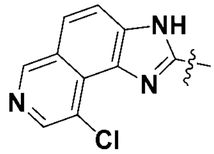 , 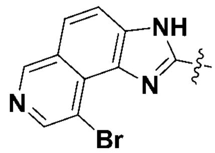 , 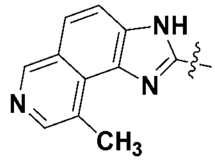 , 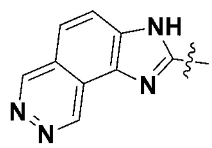 ,

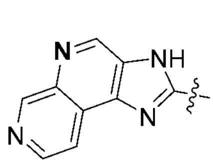 , 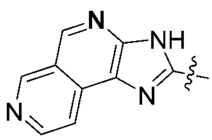 , 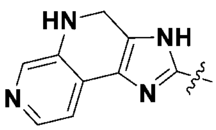 , 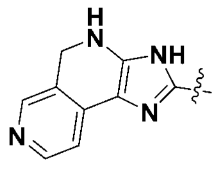 , 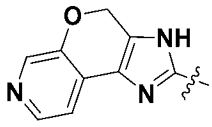 ,

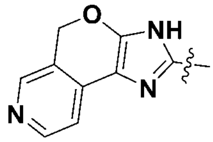 , 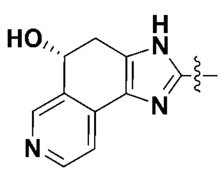 , 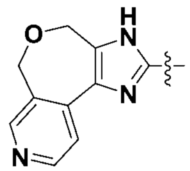 , 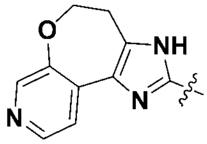 , 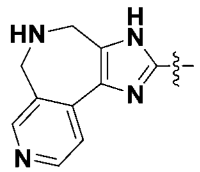 ,

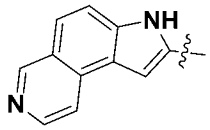 , 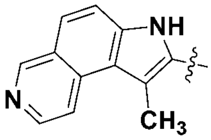 , 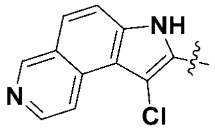 , 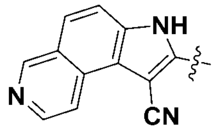 , 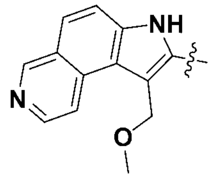 ,

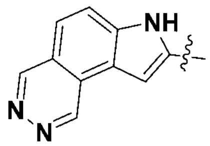 , 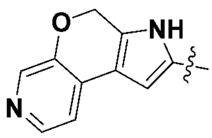 , 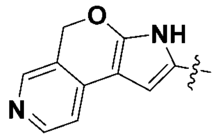 , 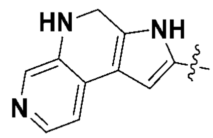 , 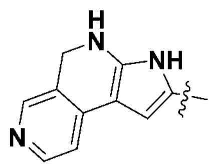 ,

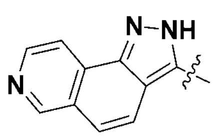 , 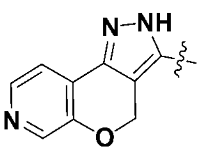 , 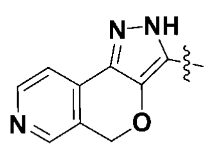 , 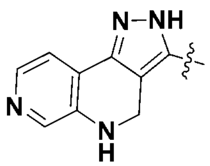 , 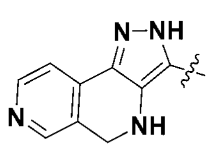 ,

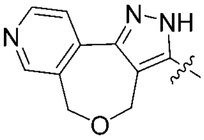 , 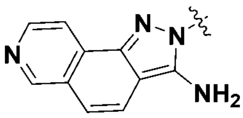 , 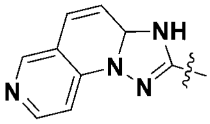 , 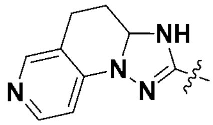 ,

and

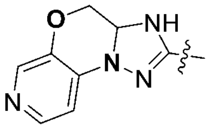 .

In some embodiments, the structural unit

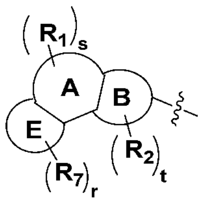

is selected from

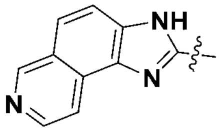 ,

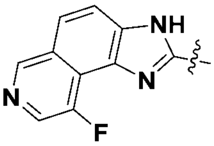 , 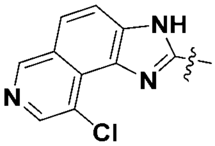 , 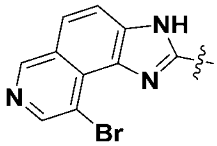 , 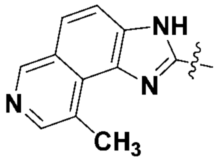 , 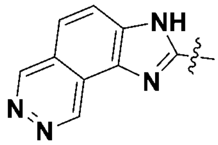 ,

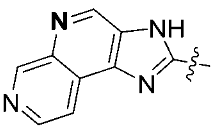 , 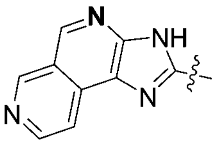 , 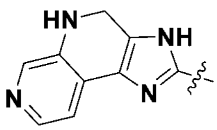 , 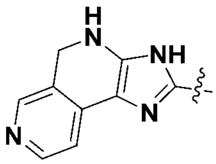 , 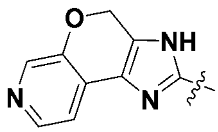 ,

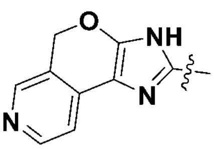 , 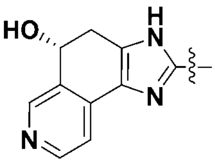 , 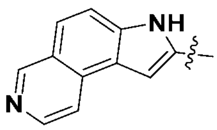 , 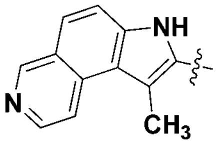 , 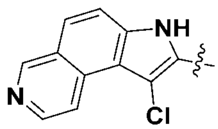 ,

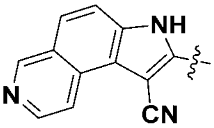 , 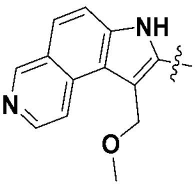 , 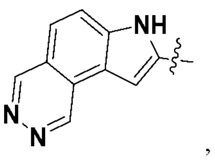 , 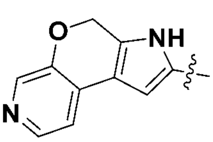 , 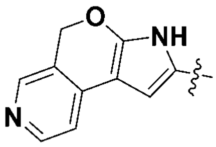 ,

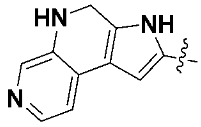, 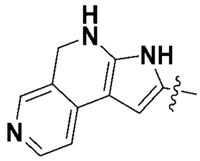, 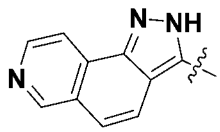, 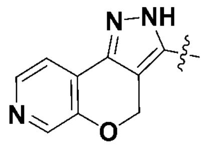, 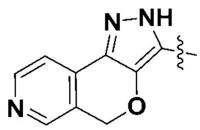, 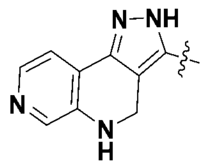, 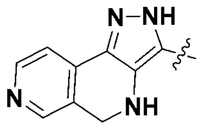, 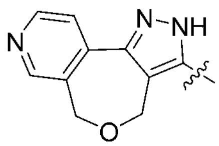, 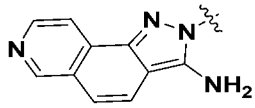, 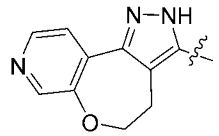,

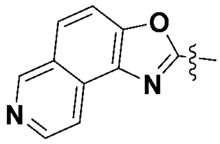, and 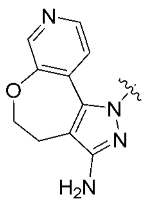.

In some embodiments, the structural unit

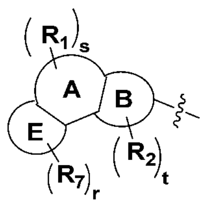

is selected from

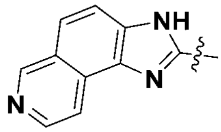,

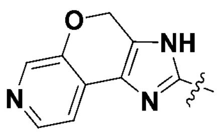, 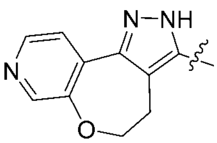, and 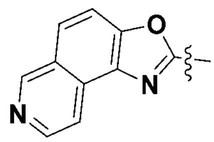.

In some embodiments, the structural unit

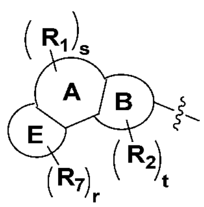

is selected from

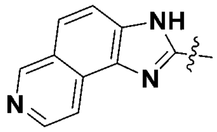

,

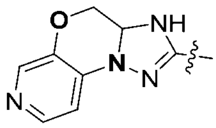

,

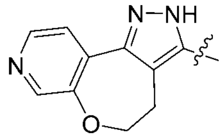

, and

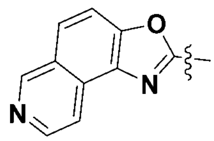

.

In some embodiments, the structural unit

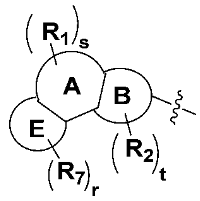

is

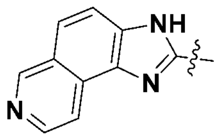

.

In some embodiments, W is selected from $-C(R_5R_6)-$, $-C(=CH_2)-$, and $-NR_5-$, wherein $R_5$, $R_6$ are as defined herein.

In some embodiments, $R_5$ is selected from H, -OH, $C_{1-4}$ alkyl, $C_{1-4}$ alkoxy, $C_{1-4}$ haloalkyl, and $C_{1-4}$ haloalkoxy.

In some embodiments, $R_6$ is selected from H, $C_{1-4}$ alkyl, $C_{1-4}$ alkoxy, $C_{1-4}$ haloalkyl, and $C_{1-4}$ haloalkoxy.

In some embodiments, $R_5$ is selected from H, -OH, methyl, ethyl, n-propyl, n-butyl, methoxy, ethoxy, propoxy, butoxy, halomethyl, haloethyl, halopropyl, halobutyl, halomethoxy, haloethoxy, halopropoxy, and halobutoxy.

In some embodiments, $R_6$ is selected from H, methyl, ethyl, n-propyl, n-butyl, methoxy, ethoxy, propoxy, butoxy, halomethyl, haloethyl, halopropyl, halobutyl, halomethoxy, haloethoxy, halopropoxy, and halobutoxy.

In some embodiments, $R_5$ is selected from H, -OH, methyl, methoxy, monofluoromethyl, difluoromethyl, and trifluoromethyl.

In some embodiments, $R_6$ is selected from H, methyl, ethyl, methoxy, monofluoromethyl, difluoromethyl, and trifluoromethyl.

In some embodiments, $R_5$ is selected from H and -OH.

In some embodiments, $R_6$ is H.

In some embodiments, $R_5$ is H.

In some embodiments, W is selected from $-CH_2-$, -CH(OH)-, $-C(=CH_2)-$, and -NH-.

In some embodiments, W is selected from $-CH_2-$ and -NH-.

In some embodiments, W is selected from $-CH_2-$ and $-C(=CH_2)-$.

In some embodiments, W is $-CH_2-$.

In some embodiments, $R_3$ is selected from H, $C_{1-4}$ alkyl, and $-C(=O)C_{1-4}$ alkyl, wherein the $C_{1-4}$ alkyl and $-C(=O)C_{1-4}$ alkyl are optionally substituted with 1, 2, 3, 4 or 5 substituents selected from halogen, -OH, $-NH_2$, -CN, $-NO_2$ and -COOH.

In some embodiments, $R_3$ is selected from H, methyl, ethyl, n-propyl, isopropyl, n-butyl, sec-butyl, *tert-butyl,* isobutyl, $-C(=O)CH_3$, $-C(=O)CH_2CH_3$, $-C(=O)(CH_2)_2CH_3$, and $-C(=O)(CH_2)_3CH_3$.

In some embodiments, $R_3$ is selected from H, $-CH_3$, and $-C(=O)CH_3$.

In some embodiments, $R_3$ is H.

In some embodiments, the structural unit

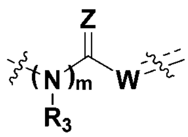

is selected from

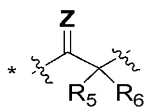, 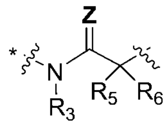,

and

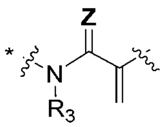,

wherein "*" indicates a connection to ring B.

In some embodiments, the structural unit

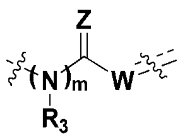

is selected from

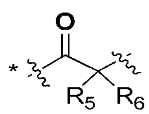, 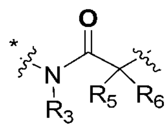,

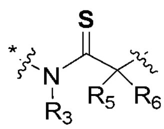, and 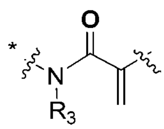.

In some embodiments, the structural unit

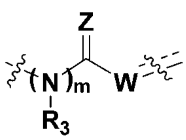

is selected from

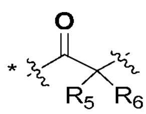, 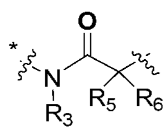,

and

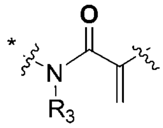

.

In some embodiments, the structural unit

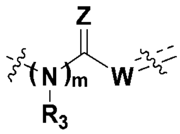

is

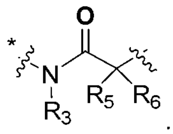

.

In some embodiments, the structural unit

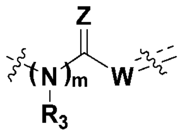

is selected from -C(=O)-$CH_2$-, -NH-C(=O)-$CH_2$-, - N($CH_3$)-C(=O)-$CH_2$-, -N(C(=O)$CH_3$)-C(=O)-$CH_2$-, -N($CH_3$)-C(=O)-CH(OH)-, -NH-C(=O)-C(=$CH_2$)-, and -NH-C(=S)-$CH_2$-.

In some embodiments, the structural unit

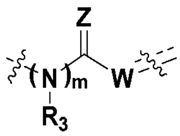

is -NH-C(=O)-$CH_2$-.

In some embodiments, the structural unit

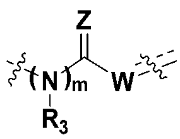

is

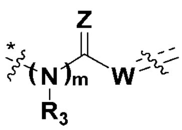

,

wherein "*" indicates a connection to ring B.

In some embodiments, the structural unit

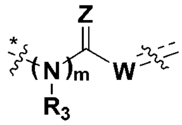

is selected from *-C(=O)-$CH_2$-, *-NH-C(=O)-$CH_2$-, *-N($CH_3$)-C(=O)-$CH_2$-, *-N(C(=O)$CH_3$)-C(=O)-$CH_2$-, *-N($CH_3$)-C(=O)-CH(OH)-, *-NH-C(=O)-C(=$CH_2$)-, and *-NH-C(=S)-$CH_2$-, wherein "*" indicates a connection to ring B.

In some embodiments, the structural unit

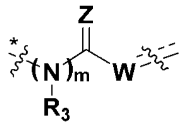

is *-NH-C(=O)-$CH_2$-, wherein "*" indicates a connection to ring B.

In some embodiments, R is selected from halogen, -OH, -CN, =O, $C_{1-4}$ alkyl, $C_{1-4}$ alkoxy, $C_{1-4}$ haloalkyl, and $C_{1-4}$ haloalkoxy.

In some embodiments, R is selected from F, Cl, Br, I, -OH, -CN, =O, methyl, ethyl, n-propyl, isopropyl, *n*-butyl, *sec*-butyl, isobutyl, *tert*-butyl, methoxy, ethoxy, propoxy, butoxy, halomethyl, haloethyl, halopropyl, halobutyl, halomethoxy, haloethoxy, halopropoxy, and halobutoxy.

In some embodiments, R is selected from F, Cl, Br, I, -OH, -CN, =O, methyl, ethyl, n-propyl, methoxy, ethoxy, monofluoromethyl, difluoromethyl, and trifluoromethyl.

In some embodiments, R is selected from F, Cl, Br, -OH, =O, methyl, ethyl, monofluoromethyl, difluoromethyl, and trifluoromethyl.

In some embodiments, R is selected from -OH, -$CH_3$, and =O.

In some embodiments, the structural unit

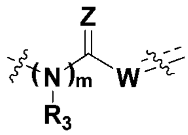

is selected from

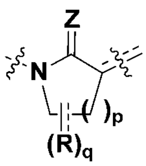

and

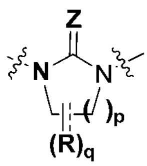

,

wherein p is selected from 0, 1, and 2; q is selected from 0, 1, 2, 3, 4, and 5;

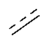

is selected from a single bond and a double bond.

In some embodiments, the structural unit

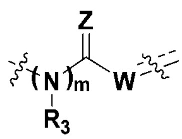

is selected from

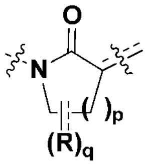 and 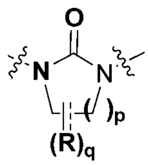 .

In some embodiments, the structural unit

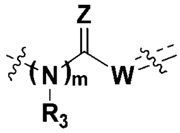

is

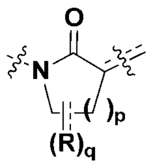 ,

wherein p is selected from 0, 1, and 2; q is selected from 0, 1, 2, 3, 4, and 5;

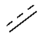

is selected from a single bond and a double bond; R is as defined herein.

In some embodiments, the structural unit

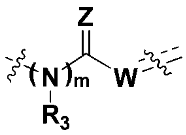

is selected from

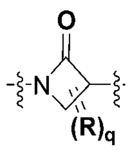 , 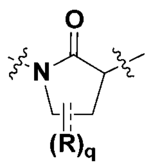 ,

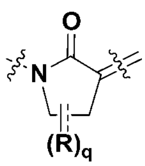 , 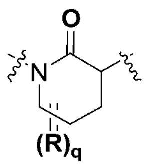 , and 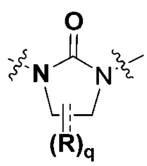 .

In some embodiments, the structural unit

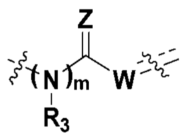

is selected from

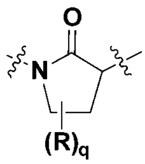 and 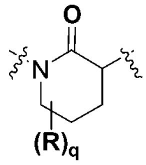 .

In some embodiments, the structural unit

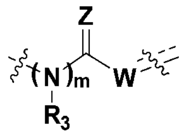

is selected from

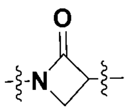 , 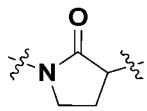 ,

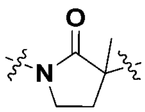 , 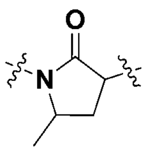 , 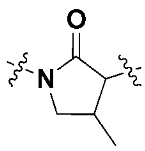 , 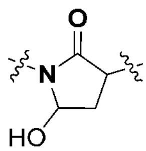 , 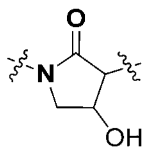 , 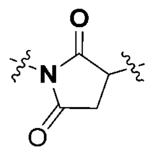 , 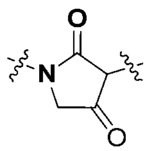 ,

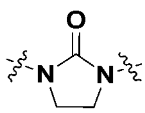 , 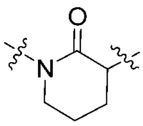 , and 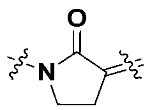 .

In some embodiments, the structural unit

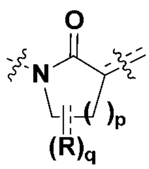

is selected from

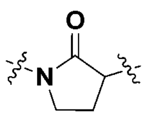 and 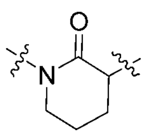 .

In some embodiments, the structural unit

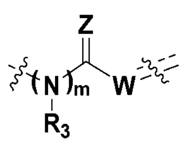

is

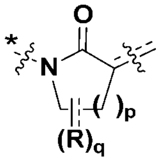 or 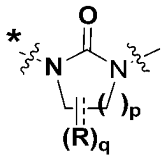 ,

wherein "*" indicates a connection to ring B.

In some embodiments, the structural unit

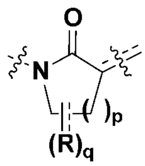

is

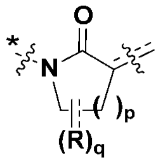 ,

wherein "*" indicates a connection to ring B.

In some embodiments, the structural unit

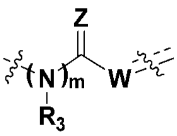

is selected from

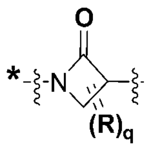 , 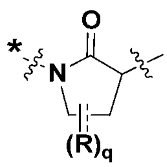 ,

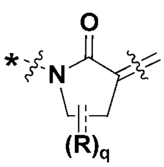 , 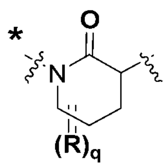 , and 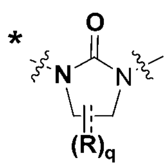 ,

wherein "*" indicates a connection to ring B.

In some embodiments, the structural unit

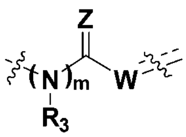

is selected from

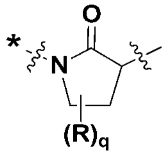

and

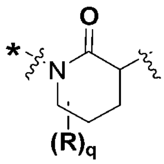
,

wherein "*" indicates a connection to ring B.

In some embodiments, the structural unit

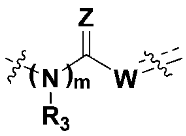

is selected from

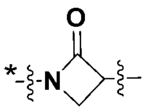, 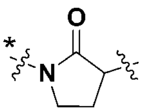,

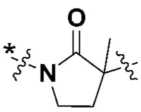, 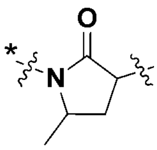, 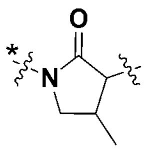, 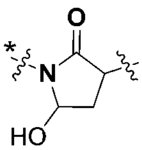, 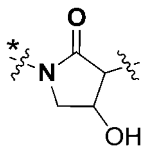, 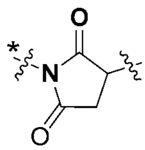, 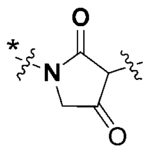,

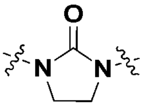, 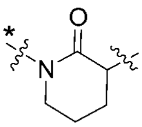, and 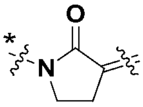,

wherein "*" indicates a connection to ring B.

In some embodiments, the structural unit

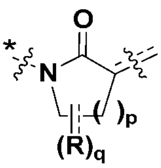

is selected from

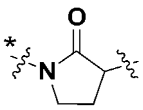 and 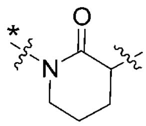 ,

wherein "*" indicates a connection to ring B.

In some embodiments, W and ring B together with the atoms to which they are attached form a 7-9 membered heterocyclyl.

In some embodiments, W and ring B together with the atoms to which they are attached form a 7 membered heterocyclyl;

preferably, the 7 membered heterocyclyl is

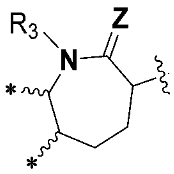

, wherein "*" indicates a fusion with ring B;

more preferably, the 7 membered heterocyclyl is

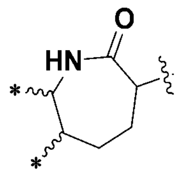

,

wherein "*" indicates a fusion with ring B.

In some embodiments, W, and ring B together with the atoms to which they are attached form a 8-9 membered heterocyclyl; preferably, the 8-9 membered heterocyclyl is

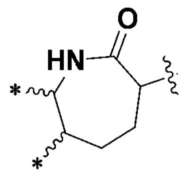

,

wherein "*" indicates a fusion with ring B.

In some embodiments, $L_1$ is absent.

In some embodiments, $L_1$ is selected from $-CH_2-$, $-CH_2CH_2-$, =CH-, -NH-, and -O-.

In some embodiments, $L_1$ is $-CH_2-$.

In some embodiments, $R_4$ is selected from halogen, -OH, -CN, $-NH_2$, $C_{1-4}$ alkyl, $C_{1-4}$ alkoxy, $C_{1-4}$ haloalkyl, and $C_{1-4}$ haloalkoxy.

In some embodiments, $R_4$ is selected from F, Cl, Br, I, -OH, -CN, $-NH_2$, methyl, ethyl, n-propyl, isopropyl, *n*-butyl, isobutyl, *sec*-butyl, *tert*-butyl, methoxy, ethoxy, propoxy, butoxy, halomethyl, haloethyl, halopropyl, halobutyl, halomethoxy, haloethoxy, halopropoxy, and halobutoxy.

In some embodiments, $R_4$ is selected from F, Cl, Br, -OH, -CN, $-NH_2$, methyl, ethyl, methoxy, ethoxy, monofluoromethyl, difluoromethyl and trifluoromethyl.

In some embodiments, $R_4$ is selected from F, Cl, -OH, -CN, $-NH_2$, methyl, and trifluoromethyl.

In some embodiments, $R_4$ is selected from F, Cl, and -CN.

In some embodiments, ring C is selected from $C_{6-10}$ aryl, 5-10 membered heteroaryl, $C_{3-8}$ cyclic groups, 4-6 membered heterocyclyl, and 5-8 membered heterocyclyl.

In some embodiments, ring C is selected from phenyl, 5-6 membered heteroaryl and 5-6 membered heterocyclyl. In some embodiments, ring C is selected from phenyl, thienyl, thiazolyl, pyridyl, 1,3-benzodioxolyl, and benzodioxolyl.
In some embodiments, ring C is selected from phenyl, thienyl, thiazolyl, pyridyl, and benzodioxolyl.
In some embodiments, ring C is selected from phenyl, thienyl, thiazolyl, and pyridyl.
In some embodiments, ring C is selected from phenyl and pyridyl.
In some embodiments, the structural unit

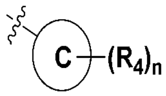

is selected from

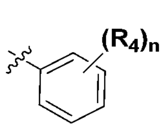 , 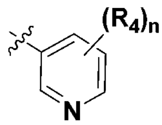 ,

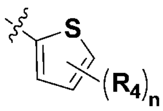 , 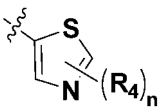 , and 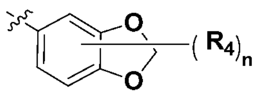 .

In some embodiments, the structural unit

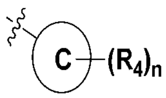

is selected from

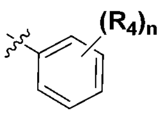 and 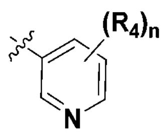 .

In some embodiments, the structural unit

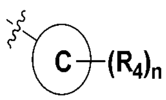

is selected from

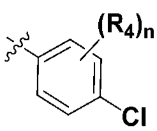 and 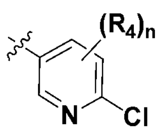 ,

wherein n and $R_4$ are as defined herein.
In some embodiments, the structural unit

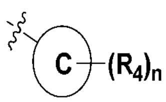

is selected from

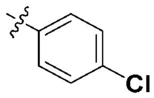, 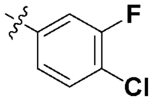,

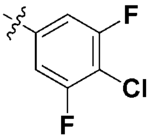, 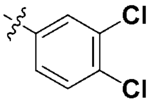, 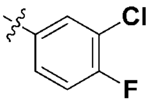, 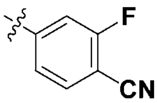, 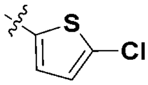, 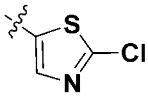, 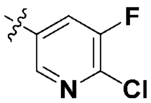,

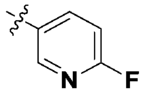, and 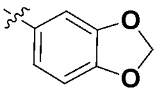.

In some embodiments, the structural unit

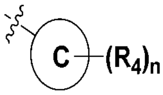

is selected from

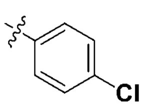, 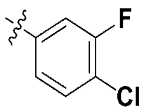,

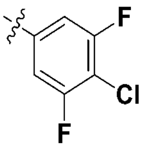, 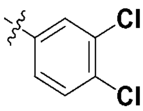, 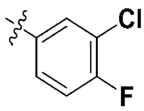, and 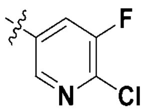.

In some embodiments, the structural unit

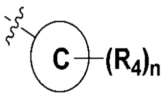

is selected from

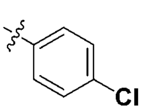, 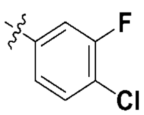,

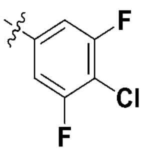, 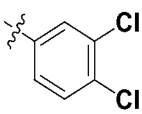, and 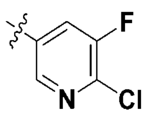.

In some embodiments, the structural unit

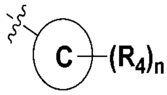

is selected from

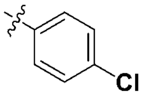, 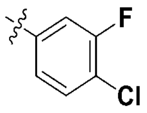,

and

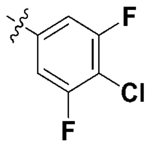.

In some embodiments, in the compound represented by Formula (I),

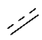

is selected from a single bond and a double bond;
Z is selected from O and S;
$L_1$ is selected from -$C_{1-3}$ alkylene-, =CH-, -NH-, and -O-, or $L_1$ is absent;
ring A is selected from phenyl, 5-6 membered heteroaryl, $C_{4-8}$ cyclic groups, and 4-8 membered heterocyclyl;
ring B is selected from 5-6 membered heteroaryl and 5-6 membered heterocyclyl;
ring C is selected from $C_{6-10}$ aryl, 5-12 membered heteroaryl, $C_{3-12}$ cyclic groups, and 4-10 membered heterocyclyl;
ring E is selected from 5-6 membered heteroaryl;
$R_1$ is selected from H, -OH, halogen, $C_{1-3}$ alkyl, and $C_{1-3}$ haloalkyl;
$R_2$ is selected from H, -OH, halogen, -CN, -$NH_2$, $C_{1-6}$ alkyl, $C_{1-6}$ haloalkyl, $C_{1-6}$ alkoxy, and -O-$C_{1-6}$ haloalkyl;
$R_3$ is selected from H, $C_{1-6}$ alkyl, and -C(=O)$C_{1-6}$ alkyl;
W is selected from -C($R_5R_6$)-, -$CR_5$=, -C(=$CH_2$)-, and -$NR_5$-, wherein $R_5$ is selected from H and -OH; or
W and $R_3$ together with the atoms to which they are attached form a 4-6 membered heterocyclyl, wherein the 4-6 membered heterocyclyl is optionally substituted with 1, 2, 3, 4 or 5 R; or
W and ring B together with the atoms to which they are attached form a 6-10 membered heterocyclyl, wherein the 6-10 membered heterocyclyl is optionally substituted with 1, 2, 3, 4 or 5 R;
$R_4$ is selected from halogen and -CN; preferably halogen;
$R_6$ is selected from H and $C_{1-6}$ alkyl;
$R_7$ is selected from H, halogen, and $C_{1-6}$ alkyl;
R is selected from halogen, -OH, and =O;
m is selected from 0 and 1;
n is selected from 0, 1, 2, and 3;
r, s, and t are each independently 0, 1, or 2; preferably 0 or 1.

In some embodiments, in the compound represented by Formula (I),

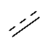

is selected from a single bond and a double bond;
Z is selected from O and S;
$L_1$ is selected from -$C_{1-3}$ alkylene-, =CH-, -NH-, and -O-, or $L_1$ is absent;
ring A is selected from phenyl, 5-6 membered heteroaryl, $C_{4-8}$ cyclic groups, and 4-8 membered heterocyclyl;
ring B is selected from 5-6 membered heteroaryl and 5-6 membered heterocyclyl;
ring C is selected from $C_{6-10}$ aryl, 5-12 membered heteroaryl, $C_{3-12}$ cyclic groups, and 4-10 membered heterocyclyl;

ring E is selected from 5-6 membered heteroaryl;
$R_1$ is selected from H, -OH, halogen, $C_{1-3}$ alkyl, and $C_{1-3}$ haloalkyl;
$R_2$ is selected from H, -OH, halogen, -CN, $-NH_2$, $C_{1-6}$ alkyl, $C_{1-6}$ haloalkyl, $C_{1-6}$ alkoxy, and $-O-C_{1-6}$ haloalkyl
$R_3$ is selected from H, $C_{1-6}$ alkyl, and $-C(=O)C_{1-6}$ alkyl;
W is selected from $-C(R_5R_6)-$, $-CR_5=$, $-C(=CH_2)-$, and $-NR_5-$, wherein $R_5$ is selected from H and -OH; or
W and $R_3$ together with the atoms to which they are attached form a 4-6 membered heterocyclyl, wherein the 4-6 membered heterocyclyl is optionally substituted with 1, 2, 3, 4 or 5 R;
$R_4$ is selected from halogen and -CN; preferably halogen;
$R_6$ is selected from H and $C_{1-6}$ alkyl;
$R_7$ is selected from H, halogen, and $C_{1-6}$ alkyl;
R is selected from halogen, -OH, and =O;
m is selected from 0 and 1;
n is selected from 0, 1, 2, and 3;
r, s, and t are each independently 0, 1, or 2; preferably 0 or 1.

In some embodiments,

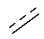

is a single bond.

In some embodiments, Z is O.

In some embodiments, $L_1$ is selected from $-C_{1-3}$ alkylene-, =CH-, -NH-, and -O-.

In some embodiments, $L_1$ is $-C_{1-3}$ alkylene-.

In some embodiments, ring A is selected from phenyl, 6 membered heteroaryl, $C_6$ cyclic groups, and 6-7 membered heterocyclyl.

In some embodiments, ring A is selected from phenyl and 6-7 membered heterocyclyl.

In some embodiments, ring B is selected from 5 membered heteroaryl and 5 membered heterocyclyl.

In some embodiments, ring B is 5 membered heteroaryl.

In some embodiments, ring C is selected from $C_{6-10}$ aryl, 5-12 membered heteroaryl, and 4-10 membered heterocyclyl.

In some embodiments, ring C is selected from phenyl, 5-6 membered heteroaryl, 5-10 membered heterocyclyl (such as 9-10 membered heterocyclyl).

In some embodiments, ring C is selected from phenyl and 6 membered heteroaryl.

In some embodiments, ring C is selected from phenyl and pyridyl.

In some embodiments, ring E is 6 membered heteroaryl.

In some embodiments, $R_1$ is selected from H and -OH.

In some embodiments, $R_1$ is H.

In some embodiments, $R_2$ is selected from H, -OH, halogen, -CN, $-NH_2$, $C_{1-6}$ alkyl, $C_{1-6}$ haloalkyl, $C_{1-6}$ alkoxy, $-O-C_{1-6}$ haloalkyl, and $-C_{1-6}$ alkylene-$C_{1-6}$ alkoxy.

In some embodiments, $R_2$ is selected from H, halogen, -CN, $-NH_2$, $C_{1-6}$ alkyl, and $-C_{1-6}$ alkylene-$C_{1-6}$ alkoxy.

In some embodiments, $R_2$ is selected from H and $-NH_2$.

In some embodiments, $R_2$ is selected from H, halogen, $-NH_2$, and $C_{1-6}$ alkyl.

In some embodiments, $R_3$ is H.

In some embodiments, W is selected from $-C(R_5R_6)-$ and $-C(=CH_2)-$, wherein $R_5$ is selected from H and -OH; or W and $R_3$ together with the atoms to which they are attached form a 4-6 membered heterocyclyl, wherein the 4-6 membered heterocyclyl is optionally substituted with 1, 2, 3, 4 or 5 R; or
W and ring B together with the atoms to which they are attached form a 7-9 membered heterocyclyl, wherein the 7-9 membered heterocyclyl is optionally substituted with 1, 2, 3, 4 or 5 R.

In some embodiments, W is selected from $-C(R_5R_6)-$ and $-C(=CH_2)-$, wherein $R_5$ is H; or

W and $R_3$ together with the atoms to which they are attached form a 4-6 membered heterocyclyl, wherein the 4-6 membered heterocyclyl is optionally substituted with 1, 2, 3, 4 or 5 R.

In some embodiments, W is selected from $-C(R_5R_6)-$, wherein $R_5$ is H; or
W and $R_3$ together with the atoms to which they are attached form a 5-6 membered heterocyclyl, wherein the 5-6 membered heterocyclyl is optionally substituted with 1, 2, 3, 4 or 5 R.

In some embodiments, $R_6$ is H.

In some embodiments, $R_7$ is H.

In some embodiments, R is selected from halogen, -OH, =O, and $C_{1-6}$ alkyl.

In some embodiments, R is selected from -OH, =O, and $C_{1-6}$ alkyl.

In some embodiments, m is 1.

In some embodiments, n is selected from 1, 2, and 3.

In some embodiments, r, s, and t are each independently 0.

In some embodiments, the compound represented by Formula (I) is selected from the compounds represented by Formula (I-A),

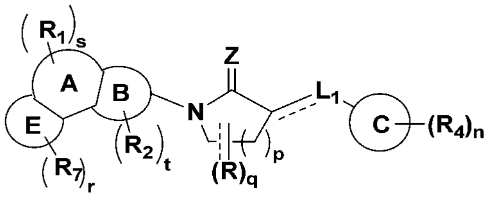

(I-A)

wherein,

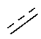

is selected from a single bond and a double bond;

p is selected from 0, 1, and 2;

q is selected from 0, 1, 2, 3, 4, and 5;

Z, $L_1$, ring A, ring B, ring C, ring E, $R_1$, $R_2$, $R_4$, $R_7$, R, n, r, s, and t are as defined herein.

In some embodiments, in the compound represented by Formula (I-A), the structural unit

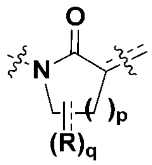

is as defined herein.

In some embodiments, the compound represented by Formula (I) is selected from the compounds represented by Formula (I-B),

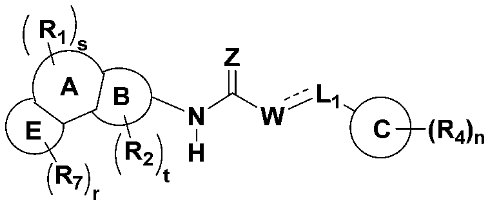

(I-B)

wherein,

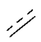

is selected from a single bond and a double bond;

Z, $L_1$, ring A, ring B, ring C, ring E, $R_1$, $R_2$, $R_4$, $R_7$, W, n, r, s, and t are as defined herein.

In some embodiments, the compound represented by Formula (I) is selected from the compounds represented by Formula (1-1),

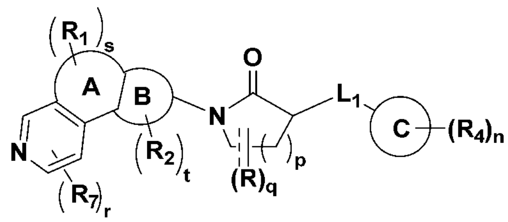

(I-1)

wherein,

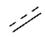

is selected from a single bond and a double bond;
p is selected from 0, 1, and 2;
q is selected from 0, 1, 2, 3, 4, and 5;
$L_1$, ring A, ring B, ring C, $R_1$, $R_2$, $R_4$, $R_7$, R, n, r, s, and t are as defined herein.

In some embodiments, in the compound represented by Formula (I-1), the structural unit

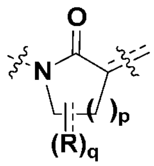

is as defined herein.

In some embodiments, the compound represented by Formula (I) is selected from the compounds represented by Formula (I-2),

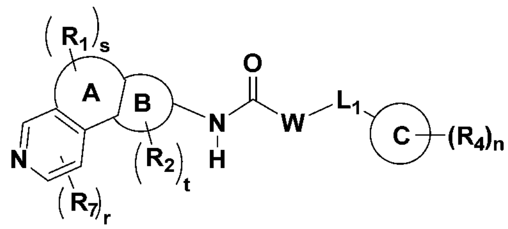

(I-2)

wherein,
$L_1$, ring A, ring B, ring C, $R_1$, $R_2$, $R_4$, $R_7$, W, n, r, s, and t are as defined herein.

In some embodiments, the compound represented by Formula (I) is selected from the compounds represented by Formula (I-3),

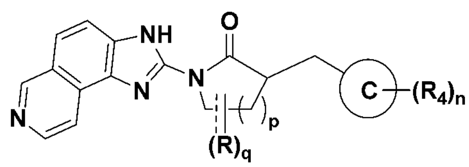

(I-3)

wherein,

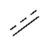

is selected from a single bond and a double bond;
p is selected from 0, 1, and 2;
q is selected from 0, 1, 2, 3, 4, and 5;

ring C, $R_4$, R, and n are as defined herein.

In some embodiments, in the compound represented by Formula (I-3), the structural unit

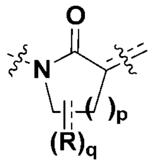

is as defined herein.

In some embodiments, the compound represented by Formula (I) is selected from the compounds represented by Formula (I-4),

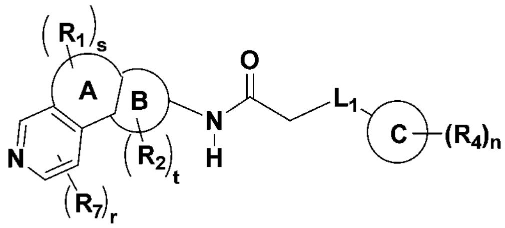

(I-4)

wherein,

$L_1$, ring A, ring B, ring C, $R_1$, $R_2$, $R_4$, $R_7$, n, r, s, and t are as defined herein.

In some embodiments, the compound represented by Formula (I) is selected from the compounds represented by Formula (I-5),

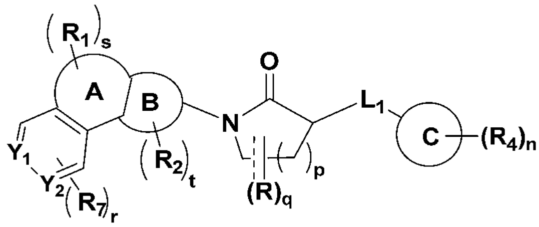

(I-5)

wherein,

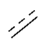

is selected from a single bond and a double bond;

p is selected from 0, 1, and 2;

q is selected from 0, 1, 2, 3, 4, and 5;

$Y_1$ and $Y_2$ are each independent selected from CH and N; preferably, $Y_1$ is N, $Y_2$ is CH, or $Y_1$ is CH, $Y_2$ is N;

$L_1$, ring A, ring B, ring C, $R_1$, $R_2$, $R_4$, $R_7$, R, n, r, s, and t are as defined herein.

In some embodiments, in the compound represented by Formula (I-5), the structural unit

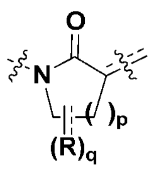

is as defined herein.

In some embodiments, in the compound represented by Formula (I-5), ring C is phenyl or pyridyl.

In some embodiments, the compound represented by Formula (I) is selected from the compounds represented by Formula (I-6),

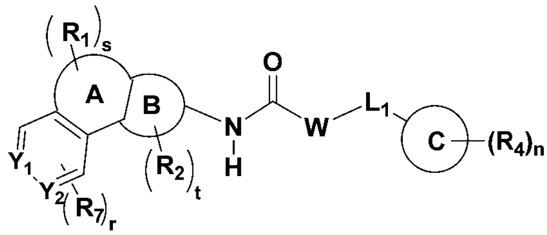

(I-6)

wherein,

$Y_1$ and $Y_2$ are each independent selected from CH and N;
$L_1$, ring A, ring B, ring C, $R_1$, $R_2$, $R_4$, $R_7$, n, r, s, and t are as defined herein.

In some embodiments, in the compound represented by Formula (I-6), $Y_1$ is N, $Y_2$ is CH.

In some embodiments, in the compound represented by Formula (I-6), $Y_1$ is CH, $Y_2$ is N. In some embodiments, the compound represented by Formula (I) is selected from the compounds represented by Formula (I-7),

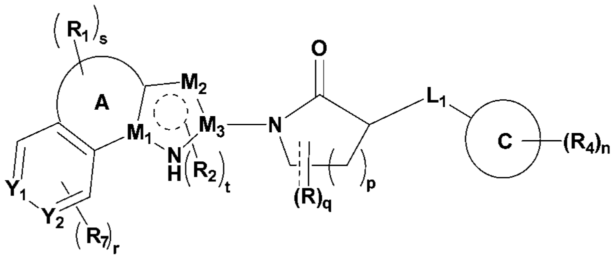

(I-7)

wherein,

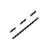

is selected from a single bond and a double bond;
p is selected from 0, 1, and 2;
q is selected from 0, 1, 2, 3, 4, and 5;
$M_1$, $M_2$, and $M_3$ are each independent C, CH, or N; preferably, $M_1$, $M_2$, and $M_3$ are each independent CH or N;
$Y_1$ and $Y_2$ are each independent selected from CH and N;
$L_1$, ring A, ring C, $R_1$, $R_2$, $R_4$, $R_7$, R, n, r, s, and t are as defined herein.

In some embodiments, in the compound represented by Formula (I-7), the structural unit

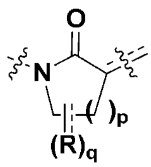

is as defined herein.

In some embodiments, in the compound represented by Formula (I-7), $Y_1$ is N, $Y_2$ is CH.

In some embodiments, in the compound represented by Formula (I-7), $Y_1$ is CH, $Y_2$ is N.

In some embodiments, the compound represented by Formula (I) is selected from the compounds represented by Formula (I-8),

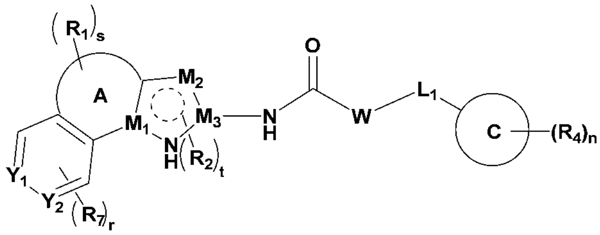

(I-8)

wherein,

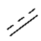

is selected from a single bond and a double bond;
$M_1$, $M_2$, and $M_3$ are each independent C, CH, or N; preferably, $M_1$, $M_2$, and $M_3$ are each independent CH or N;
$Y_1$ and $Y_2$ are each independent selected from CH and N;
$L_1$, ring A, ring C, $R_1$, $R_2$, $R_4$, $R_7$, W, n, r, s, and t are as defined herein.

In some embodiments, in the compound represented by Formula (I-8), $Y_1$ is N, $Y_2$ is CH.
In some embodiments, in the compound represented by Formula (I-8), $Y_1$ is CH, $Y_2$ is N.
In some embodiments, the compound represented by Formula (I) is selected from the compounds represented by Formula (I-9),

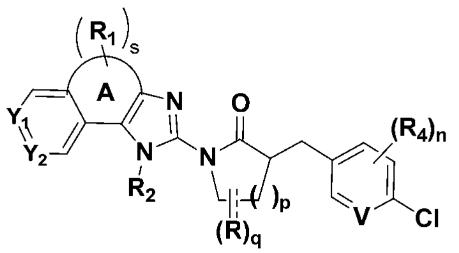

(I-9)

wherein,

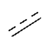

is selected from a single bond and a double bond;
p is selected from 1, and 2;
q is selected from 0, 1, 2, 3, 4, and 5;
V is selected from CH, $CR_4$, and N;
$Y_1$ and $Y_2$ are each independent selected from CH and N;
ring A, $R_1$, $R_2$, $R_4$, R, n, and s are as defined herein.

In some embodiments, in the compound represented by Formula (I-9), the structural unit

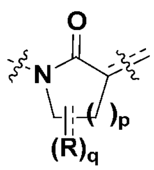

is as defined herein.
In some embodiments, in the compound represented by Formula (I-9), $Y_1$ is N, $Y_2$ is CH.
In some embodiments, in the compound represented by Formula (I-9), $Y_1$ is CH, $Y_2$ is N.
In some embodiments, the compound represented by Formula (I) is selected from the compounds represented by Formula (1-10),

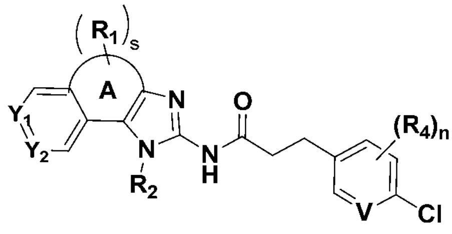

(I-10)

wherein,

V is selected from CH, $CR_4$, and N;
$Y_1$ and $Y_2$ are each independent selected from CH and N;
ring A, $R_1$, $R_2$, $R_4$, R, n, and s are as defined herein.

In some embodiments, in the compound represented by Formula (I-10), $Y_1$ is N, $Y_2$ is CH.

In some embodiments, in the compound represented by Formula (I-10), $Y_1$ is CH, $Y_2$ is N.

In some embodiments, the compound represented by Formula (I) is selected from the compounds represented by Formula (I-9'),

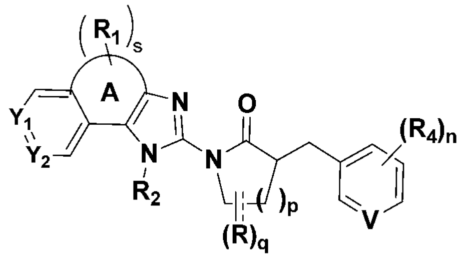

(I-9').

In some embodiments, the compound represented by Formula (I) is selected from the compounds represented by Formula (I-10'),

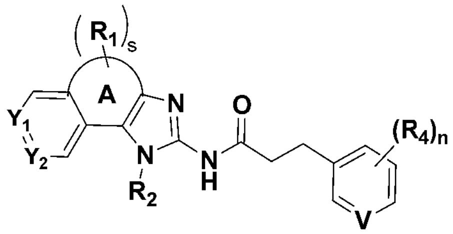

(I-10').

In some embodiments, the compound represented by Formula (I) is selected from the compounds represented by Formula (II-A) to Formula (II-P):

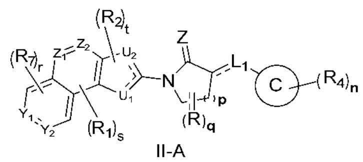

II-A ,

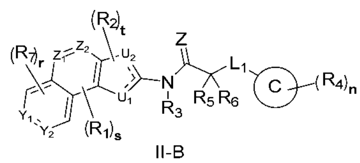

II-B ,

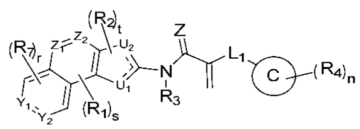

II-C ,

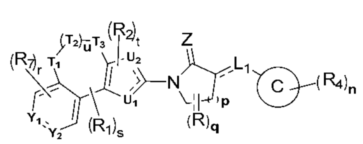

II-D ,

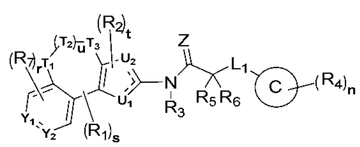

II-E ,

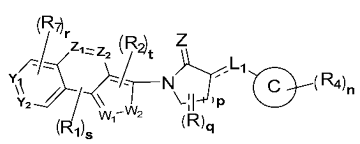

II-F ,

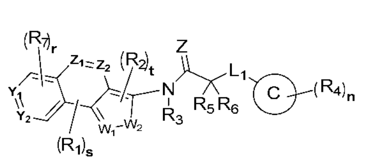
II-G ,
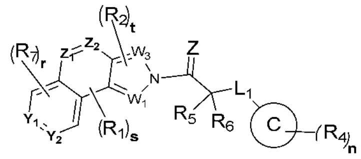
II-H ,
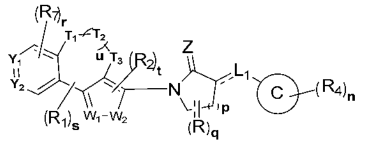
II-I ,

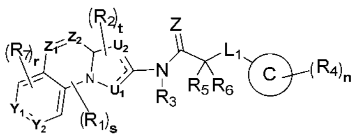
II-J ,
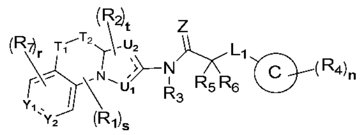
II-L ,
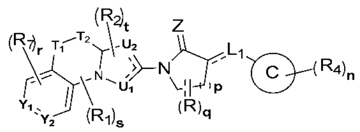
II-M ,

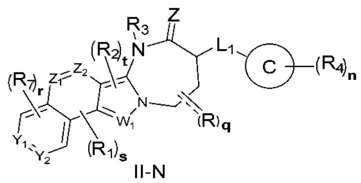
II-N ,
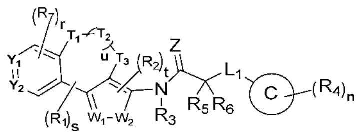
II-O , and
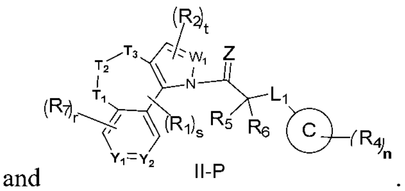
II-P .

In some embodiments, the compound represented by Formula (I) is selected from the compounds represented by Formula (II-1) to Formula (II-11):

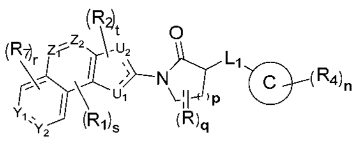
II-1 ,
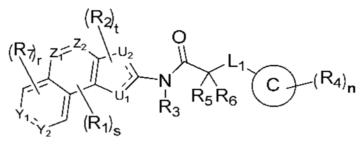
II-2 ,
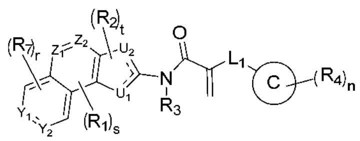
II-3 ,

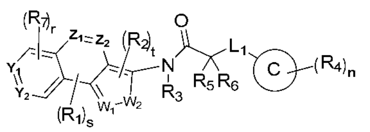
II-4 ,
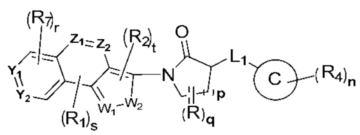
II-5 ,
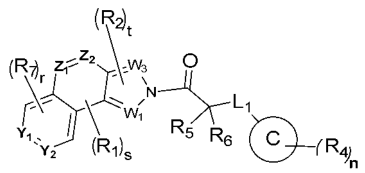
II-6 ,

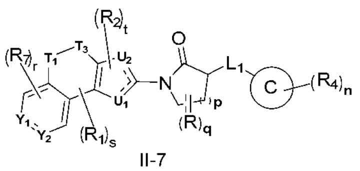
II-7 ,
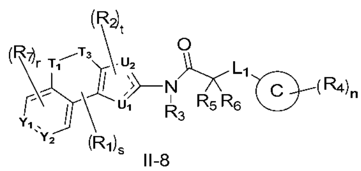
II-8 ,
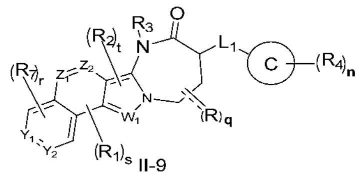
II-9 ,

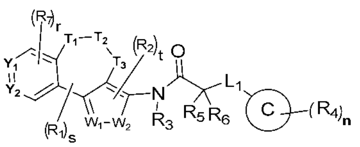
II-10 , and
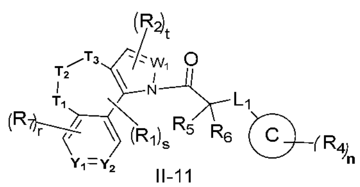
II-11 .

In some embodiments, the compound represented by Formula (I) is selected from the compounds represented by Formula (III-1) to Formula (III-5):

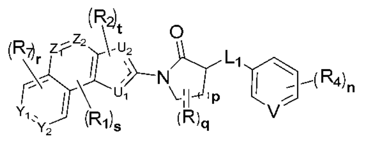
III-1
,
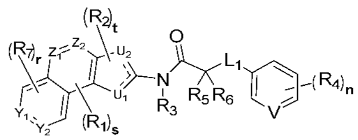
III-2
,
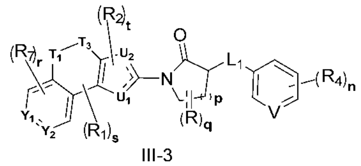
III-3
,
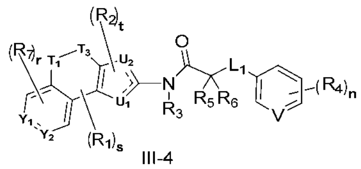
III-4
, and
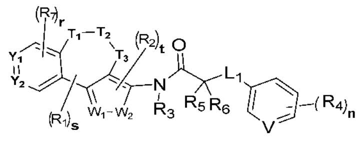
III-5
.

In some embodiments, the compound represented by Formula (I) is selected from:

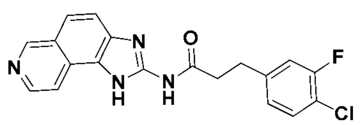
,
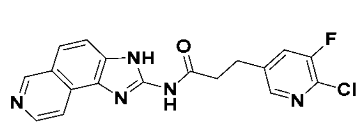
,
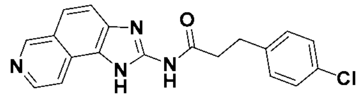
,
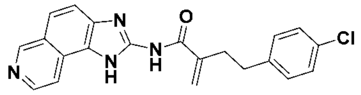
,
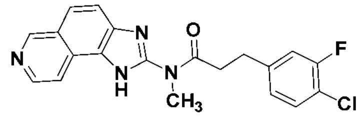
,
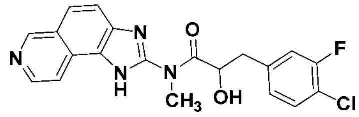
,
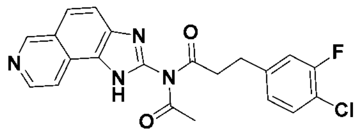
,
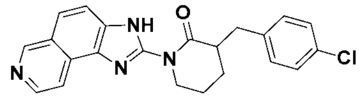
,
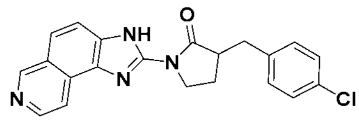
,
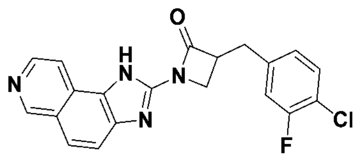
,
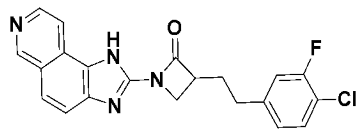
,
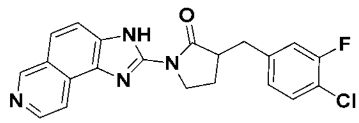
,
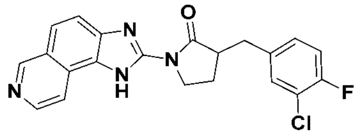
,
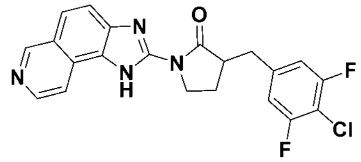
,
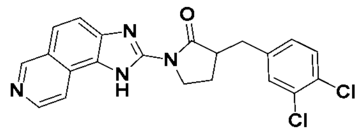
,
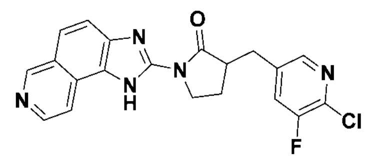
,
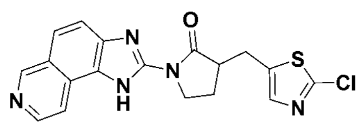
,
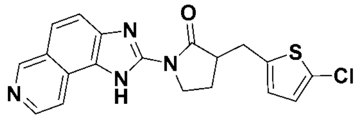
、
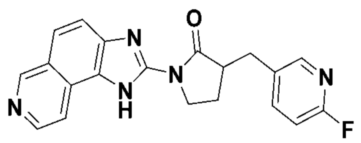
、
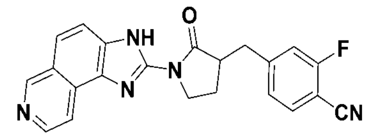
,
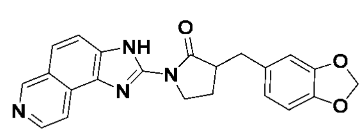
,

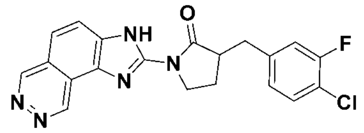, 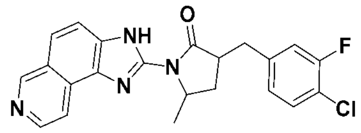, 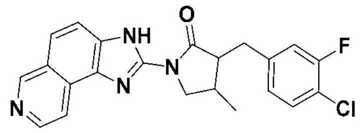,

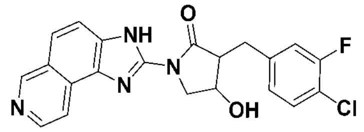, 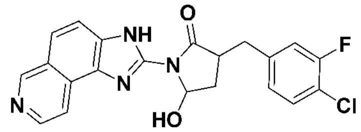, 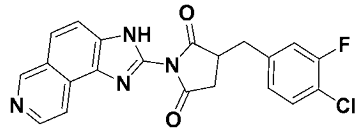,

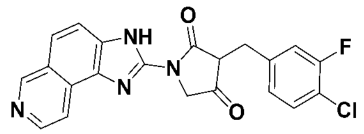, 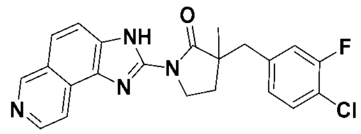, 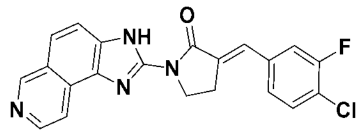,

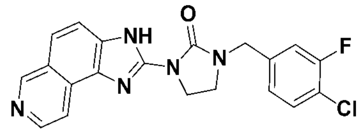, 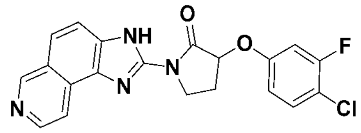, 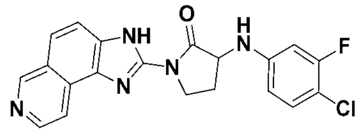,

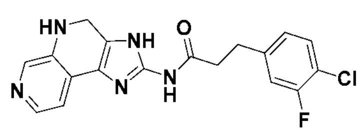, 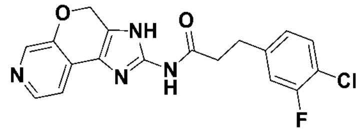, 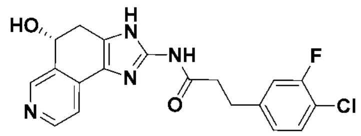,

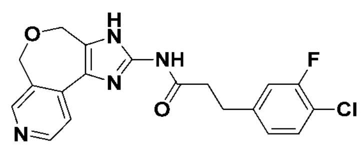, 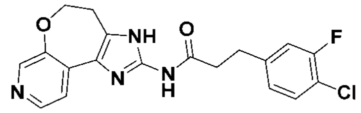, 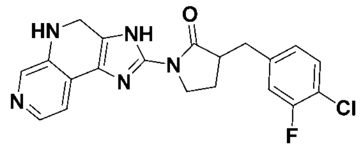,

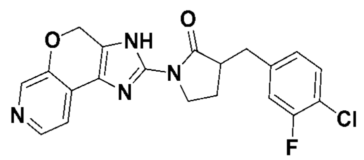, 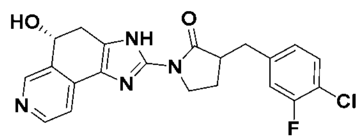, 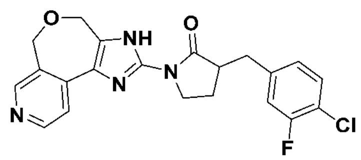,

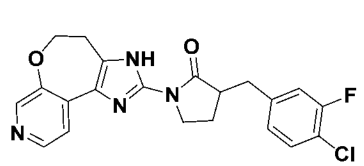, 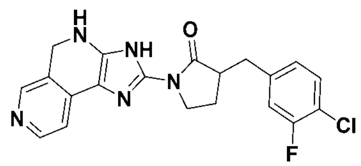, 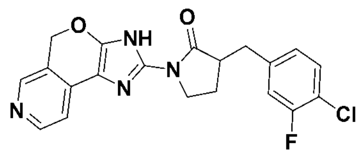,

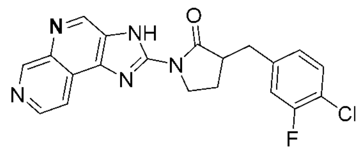, 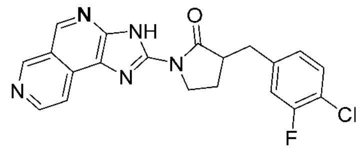, 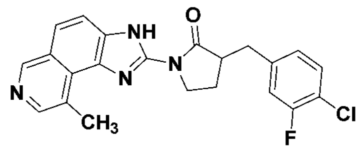,

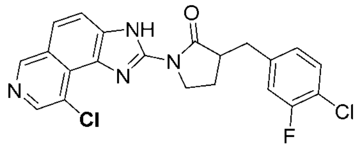 , 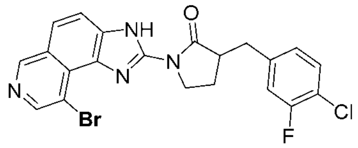 , 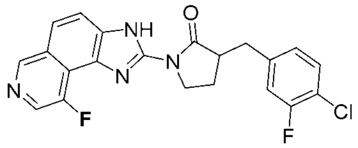 ,

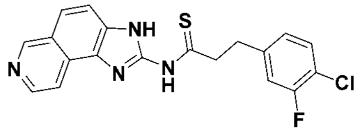 , 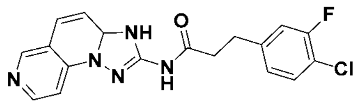 , 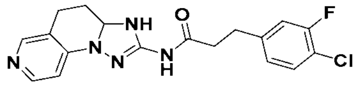 ,

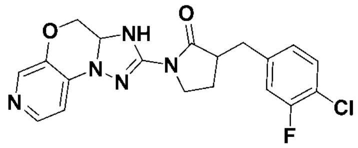 , 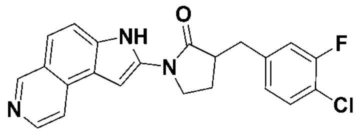 , 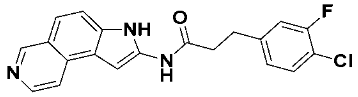 ,

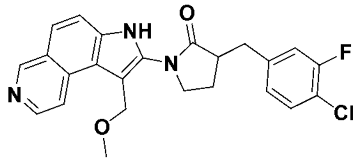 , 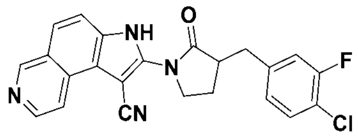 , 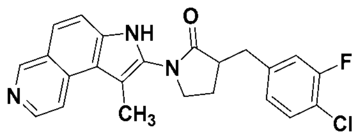 ,

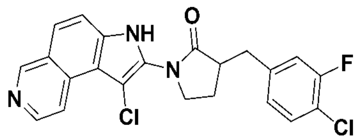 , 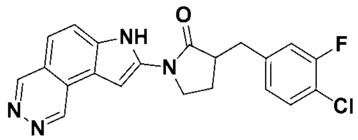 , 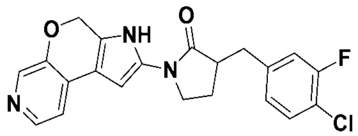 ,

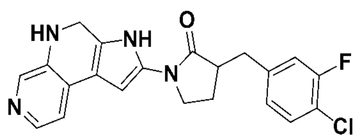 , 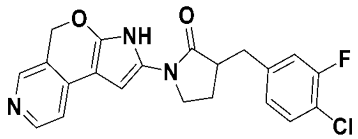 , 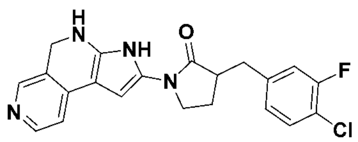 ,

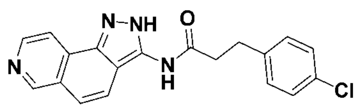 , 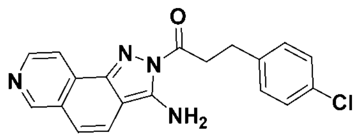 , 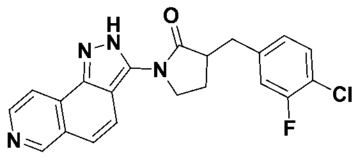 ,

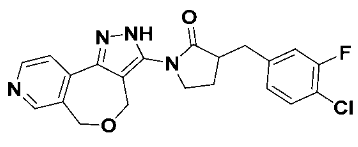 , 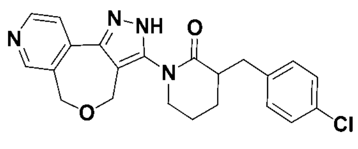 , 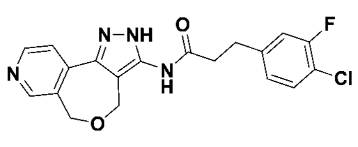 ,

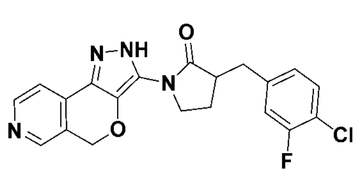 , 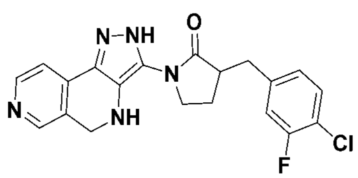 , 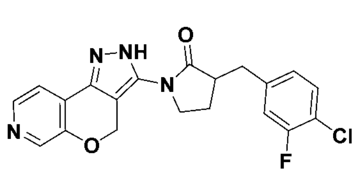 ,

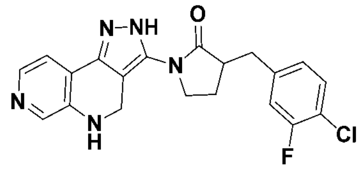, 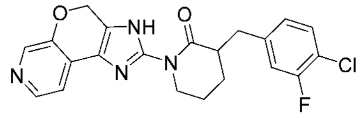, 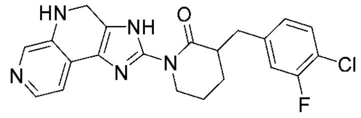,

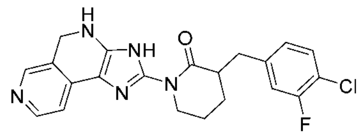, 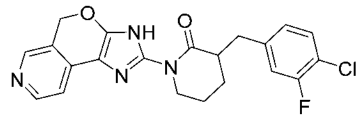, 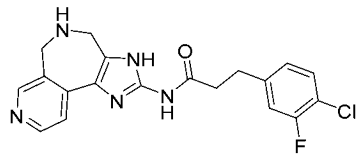,

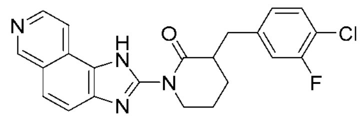, 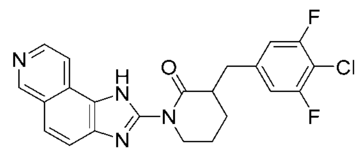, 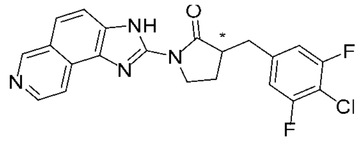,

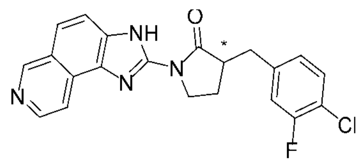, 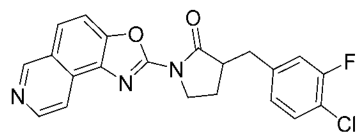, 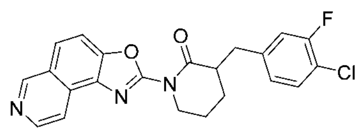,

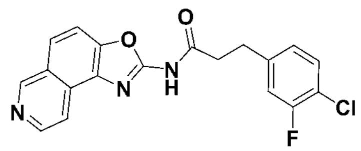, 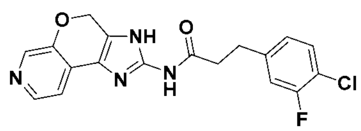, 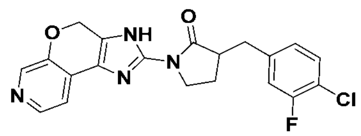,

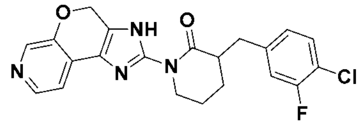, 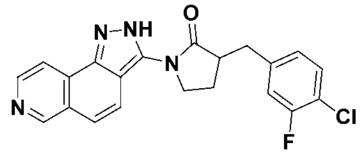, 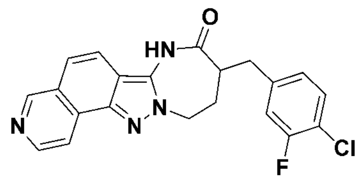,

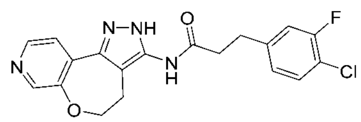, 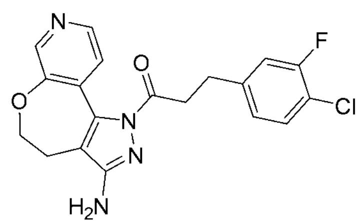, 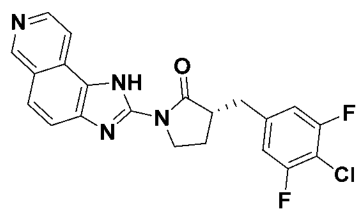,

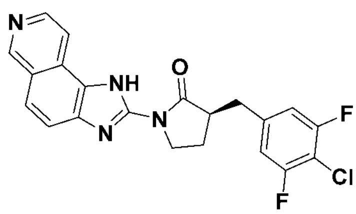, 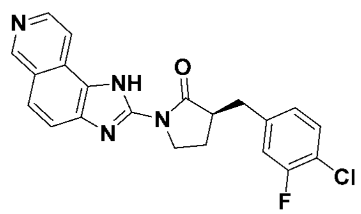, and 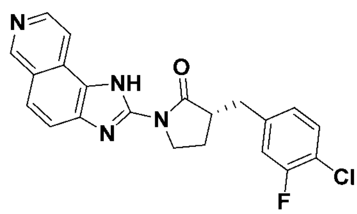.

The second aspect of the present invention provides a pharmaceutical composition, which comprises the

compound, or a stereoisomer, tautomer, polymorph, solvate, hydrate, N-oxide, isotope-labeled compound, metabolite, ester, prodrug of the compound, or a pharmaceutically acceptable salt thereof according to the present invention, and one or more pharmaceutically acceptable carriers or excipients.

In some embodiments, the compound, or a stereoisomer, tautomer, polymorph, solvate, hydrate, N-oxide, isotope-labeled compound, metabolite, ester, prodrug of the compound, or a pharmaceutically acceptable salt thereof is present in an effective amount.

The third aspect of the present invention provides a method for treating or preventing a disease comprising the following steps:

a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to the present invention, or a pharmaceutical composition according to the present invention, is administered to an individual who (i) suffers from a condition characterized by axonal degeneration or (ii) is at risk of suffering from a condition characterized by axonal degeneration.

The fourth aspect of the present invention provides a method for treating or preventing axonal degeneration, comprising administering to an individual in need thereof a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to the present invention, or a pharmaceutical composition according to the present invention.

The fifth aspect of the present invention provides a method for treating or preventing a disorder or condition characterized by axonal degeneration, comprising administering to an individual in need thereof a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to the present invention, or a pharmaceutical composition according to the present invention. The sixth aspect of the present invention provides use of the compound or a stereoisomer, tautomer, polymorph, solvate, hydrate, N-oxide, isotope-labeled compound, metabolite, ester, prodrug of the compound, or a pharmaceutically acceptable salt thereof according to the present invention, or the pharmaceutical composition according to the present invention, in the manufacture of a medicament for treating or preventing axonal degeneration.

The seventh aspect of the present invention provides use of the compound or a stereoisomer, tautomer, polymorph, solvate, hydrate, N-oxide, isotope-labeled compound, metabolite, ester, prodrug of the compound, or a pharmaceutically acceptable salt thereof according to the present invention, or the pharmaceutical composition according to the present invention, in the manufacture of a medicament for treating or preventing a disorder or condition characterized by axonal degeneration.

The eighth aspect of the present invention provides a compound or a stereoisomer, tautomer, polymorph, solvate, hydrate, N-oxide, isotope-labeled compound, metabolite, ester, prodrug of the compound, or a pharmaceutically acceptable salt thereof according to the present invention, or the pharmaceutical composition according to the present invention, for use in treating or preventing axonal degeneration, or treating or preventing a disorder or condition characterized by axonal degeneration.

In some embodiments, the disorder or condition characterized by axonal degeneration is a neurodegenerative disease or peripheral neuropathy.

In some embodiments, the neurodegenerative disease is Alzheimer's disease, Parkinson's disease, multiple sclerosis, amyotrophic sclerosis, or Huntington's disease.

The compounds of the present invention can be synthesized by common chemical synthesis knowledge familiar to those of ordinary skill in the art or by various methods similarly known in the art. Isolation and purification of the products can be achieved by standard procedures known to those of ordinary skill in the art.

## Definition of terms

The various terms and phrases used in the present invention have general meanings known to those skilled in the art. Even so, it is still desired to provide a more detailed description and explanation of these terms and phrases herein in the present application. If the mentioned terms and phrases are inconsistent with the known meanings, the meanings expressed in the present invention shall prevail.

When the name of the compound used in the present invention is inconsistent with the chemical structural formula, the chemical structural formula shall prevail.

In the structural formula of the present invention, the fragment containing imidazole can undergo tautomerism, for example:

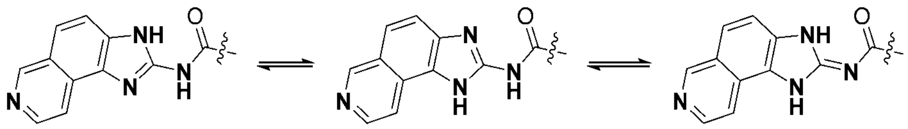

and

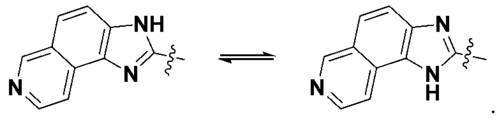

As used in the present invention, the term "pharmaceutically acceptable salt" refers to a salt of a compound of the present invention that is pharmaceutically acceptable and has the desired pharmacological activity of the parent compound. Such salts include: acid addition salts formed with inorganic acids or with organic acids, or coordination compounds formed by replacing acidic protons present on the parent compound with metal ions or with organic bases.

As used in the present invention, the term "stereoisomer" refers to an isomer formed due to at least one asymmetric center. In compounds with one or more (e.g., 1, 2, 3, or 4) asymmetric centers, it can produce racemic mixtures, single enantiomers, diastereomeric mixtures, and individual diastereomers. Specific individual molecules can also exist as geometric isomers (cis/trans). Similarly, the compounds of the present invention can exist as mixtures (commonly referred to as tautomers) of two or more structurally different forms in rapid equilibrium. Representative examples of tautomers include keto-enol tautomers, phenol-ketone tautomers, nitroso-oxime tautomers, imine-enamine tautomers, etc. It is to be understood that the scope of the present application covers all such isomers or mixtures thereof in any proportion (e.g., 60%, 65%, 70%, 75%, 80%, 85%, 90%, 95%, 96%, 97%, 98%, 99%).

Unless otherwise indicated, the compounds of the present invention are intended to exist in the form of stereoisomers, including cis and trans isomers, optical isomers (e.g., R and S enantiomers), diastereomers, geometric isomers, rotamers, conformational isomers, atropisomers, and mixtures thereof. The compounds of the present invention may exhibit more than one type of isomerism and consist of mixtures thereof (e.g., racemic mixtures and diastereomeric pairs.)

As used in the present invention, the term "isotope-labeled compound" refers to a compound in which one or more atoms have been replaced with atoms of the same atomic number, but of a different atomic mass or mass number than the dominant atomic mass or mass number in nature. Examples of isotopes suitable for inclusion in the compounds of the present invention include, but are not limited to, hydrogen isotopes such as $^{2}H$, $^{3}H$; carbon isotopes such as $^{11}C$, $^{13}C$ and $^{14}C$; chlorine isotopes such as $^{36}Cl$; fluorine isotopes such as $^{18}F$; iodine isotopes such as $^{123}I$ and $^{125}I$; nitrogen isotopes such as $^{13}N$ and $^{15}N$; oxygen isotopes such as $^{15}O$, $^{17}O$ and $^{18}O$; and sulfur isotopes such as $^{35}S$.

As used in the present invention, the term "prodrug" refers to a derivative that can be hydrolyzed, oxidized or otherwise reacted under biological conditions (*in vitro* or *in vivo*) to provide a compound disclosed herein. Prodrugs become active compounds only after the reaction under biological conditions, or they are not or only less active in their unreacted forms. Prodrugs can usually be prepared using known methods, such as those methods described in Burger's Medicinal Chemistry and Drug Discovery (1995) 172-178, 949-982 (Manfred E. Wolff, 5th edition). The term "ester" refers to esters that can be hydrolyzed *in vivo* and includes those that are easily decomposed in the human organism so as to separate from the parent compound. The hydroxyl group-containing compounds of the present invention can form esters with organic or inorganic acids, or the carboxyl group-containing compounds of the present invention can form esters with alcohols such as methanol, ethanol or propanol.

The compounds of the present invention may exist in the form of solvates (such as hydrates), wherein the compounds of the present application contain a solvent as a structural element of the lattice of the compound, such as water, methanol or ethanol. The amount of the solvent may be present in a stoichiometric ratio or a *non*-stoichiometric ratio.

The present invention encompasses all possible crystalline forms or polymorphs of the compound, which may be a single polymorph or a mixture of more than one polymorph in any ratio.

As used herein, the term "N-oxide" refers to a compound containing an amine oxide moiety (i.e., an oxide of a tertiary amine group) formed by oxidation of at least one nitrogen atom in the compound. Those skilled in the art will appreciate that not all nitrogen-containing heterocycles are capable of forming N-oxides, as nitrogen requires an available lone pair of electrons to oxidize to an oxide; those skilled in the art will recognize nitrogen-containing heterocycles that are capable of forming N-oxides. Those skilled in the art will also recognize that tertiary amines are capable of forming N-oxides. Synthetic methods for preparing N-oxides of nitrogen-containing heterocycles and tertiary amines are well known to those skilled in the art, for example, nitrogen atoms (e.g., trivalent nitrogen) can be converted to the corresponding N-oxide form by known methods such as treatment with an oxidizing agent. In the present invention, the purpose of the

pharmaceutical composition is to promote the administration of the organism, facilitate the absorption of the active ingredients and thus exert biological activity. The carriers include, but are not limited to: ion exchangers, alumina, aluminum stearate, lecithin, serum proteins such as human albumin, buffer substances such as phosphates, glycerol, sorbic acid, potassium sorbate, partial glyceride mixtures of saturated vegetable fatty acids, water, salts or electrolytes, such as protamine sulfate, dibasic sodium phosphate, potassium hydrogen phosphate, sodium chloride, zinc salts, colloidal silica, magnesium trisilicate, polyvinylpyrrolidone, cellulose substance, polyethylene glycol, carboxymethylcellulose sodium, polyacrylate, beeswax, lanolin. The excipient refers to an additive other than the main drug in the pharmaceutical preparation. It has stable properties, no incompatibility with the main drug, no side effects, no effect on the efficacy, is not easy to deform, crack, mildew, or be eaten by insects at room temperature, no harm to the human body, no physiological effects, no chemical or physical effects with the main drug, no effect on the content determination of the main drug, etc. For example, adhesives, fillers, disintegrants, and lubricants in tablets; wine, vinegar, and medicinal juice in traditional Chinese medicine pills; the matrix part in semisolid preparations ointments and creams; preservatives, antioxidants, flavoring agents, aromatics, cosolvents, emulsifiers, solubilizers, osmotic pressure regulators, colorants, etc. in liquid preparations can all be called excipients.

In the present invention, the pharmaceutical composition can be prepared into various suitable dosage forms according to the administration route, such as tablets, capsules, granules, oral solutions, oral suspensions, oral emulsions, powders, tinctures, syrups, injections, suppositories, ointments, creams, pastes, ophthalmic preparations, pills, implants, aerosols, powder sprays, sprays, etc. Wherein, the pharmaceutical composition or suitable dosage form can contain 0.01 mg to 1000 mg of the compound, or a stereoisomer, tautomer, polymorph, solvate, hydrate, N-oxide, isotope-labeled compound, metabolite, ester, prodrug of the compound, or a pharmaceutically acceptable salt of the present invention, preferably 0.1 mg to 800 mg, preferably 0.5-500 mg, preferably 0.5 to 350 mg, and particularly preferably 1-250 mg.

As used herein, the term "individual" includes humans or non-human animals. Exemplary human individuals include human individuals (referred to as patients) suffering from diseases (e.g., diseases described herein) or normal individuals. The term "non-human animal" in the present disclosure includes all vertebrates, such as non-mammals (e.g., birds, amphibians, reptiles) and mammals, such as non-human primates, livestock and/or domesticated animals (e.g., sheep, dogs, cats, cows, pigs, etc.).

As used herein, the term "treat", "treating" or "treatment" is intended to alleviate, mitigate, improve or eliminate the disease state or disorder being targeted. If a subject receives a therapeutic amount of the compound, or a stereoisomer thereof, or a tautomer thereof, or a polymorph thereof, or a solvate thereof, or a hydrate thereof, or an N-oxide thereof, or an isotope-labeled compound thereof, or a metabolite thereof, or an ester thereof, or a prodrug thereof, or a pharmaceutically acceptable salt thereof according to the methods described herein, and the subject shows an observable and/or detectable reduction or improvement in one or more signs and symptoms, then the subject is successfully "treated". It should also be understood that the treatment of the disease state or disorder includes not only complete treatment, but also less than complete treatment while achieving some biologically or medically relevant result.

As used in the present invention, the term "prevent", "preventing" or "prevention" aims to avoid, reduce, prevent or delay the occurrence of a disease or disease-related symptoms, and such disease or disease-related symptoms have not yet appeared before the administration of the relevant drug. "Prevention" does not require completely stopping the occurrence of a disease or disease-related symptoms. For example, after the administration of relevant drugs, the risk of a subject developing a specific disease or disease-related symptoms can be reduced, or the severity of related symptoms that appear later can be reduced, which can be considered as "preventing" the occurrence or development of the disease.

As used in the present invention, the term "effective amount" refers to an amount sufficient to achieve the desired therapeutic or preventive effect, for example, an amount that reduces the symptoms associated with the disease to be treated, or an amount that can effectively prevent, stop or delay the occurrence of the disease. The determination of such an effective amount is within the capabilities of those skilled in the art.

It should also be noted that the dosage and method of use of the compounds of the present invention depend on many factors, including the patient's age, weight, gender, natural health status, nutritional status, activity strength of the compound, time of administration, metabolic rate, severity of the disorder, and the subjective judgment of the treating physician. The preferred dosage is between 0.01-100 mg/kg body weight/day.

As used in the present invention, the term "optionally substituted" means that it may be substituted or unsubstituted. As used in the present invention, the term "halogen" refers to fluorine, chlorine, bromine, and iodine.

As used in the present invention, the term "$C_{1-6}$ alkyl" refers to a straight or branched-chain monovalent saturated hydrocarbon group having 1 to 6 carbon atoms, such as 1, 2, 3, 4, 5 or 6 carbon atoms. $C_{1-6}$ alkyl includes $C_{1-5}$ alkyl, $C_{1-4}$ alkyl, $C_{1-3}$ alkyl, etc., and specific examples include but are not limited to methyl, ethyl, propyl, butyl, pentyl, hexyl, etc.

As used in the present invention, the term "$C_{1-6}$ alkylene" refers to a straight or branched-chain divalent saturated hydrocarbon group having 1 to 6 carbon atoms, such as 1, 2, 3, 4, 5 or 6 carbon atoms. $C_{1-6}$ alkylene includes $C_{1-5}$ alkylene, $C_{1-4}$ alkylene, $C_{1-3}$ alkylene, etc., and specific examples include but are not limited to methylene, ethylene, propylene, isopropylene, etc.

As used in the present invention, the term "$C_{1-6}$ alkoxy" refers to -O-$C_{1-6}$ alkyl, that is, a group obtained by connecting the carbon atom on the $C_{1-6}$ alkyl group as defined above to an oxygen atom. The $C_{1-6}$ alkoxy includes $C_{1-5}$ alkoxy, $C_{1-4}$ alkoxy, $C_{1-3}$ alkoxy, etc. Specific examples include but are not limited to methoxy, ethoxy, propoxy, butoxy, etc.

As used in the present invention, the term "halo" means that its modified group is substituted by one or more halogens, for example, substituted with 1, 2, 3, 4, 5 or 6 halogens. For example, "$C_{1-6}$ haloalkyl" means that the $C_{1-6}$ alkyl as defined above is substituted by one or more halogens, and specific examples include but are not limited to $CF_3$, $CHF_2$, $CH_2F$, $CF_2CF_3$, etc.

As used in the present invention, the term "$C_6$-$C_{10}$ aryl" refers to an unsaturated aromatic carbocyclic group having a monocyclic ring or two or more fused rings consisting of 6 to 10 (e.g., 6, 7, 8, 9 or 10) carbon atoms with a conjugated $\pi$ electron system. Specific examples include, but are not limited to, phenyl, naphthyl, anthracenyl, and the like.

As used in the present invention, the term "5-12 membered heteroaryl" refers to a group having a conjugated $\pi$ electron system consisting of 5 to 12 (such as 5, 6, 7, 8, 9, 10, 11 or 12) ring atoms, including monocyclic heteroaromatic rings and polycyclic heteroaromatic rings, wherein 1, 2, 3 or 4 ring atoms are heteroatoms and the rest are carbon atoms; preferably, the heteroatoms are selected from N, O and S, wherein the nitrogen atom is optionally quaternized, and the nitrogen and sulfur heteroatoms are optionally oxidized. 5-12 membered heteroaryl includes 5-10 membered, 5-9 membered, 6-9 membered, 5-6 membered heteroaryl, etc. Specific examples include but are not limited to imidazolyl, thiazolyl, pyridyl, thienyl, furyl, etc. "5 membered nitrogen-containing heteroaryl" means that 1, 2, 3, or 4 ring atoms are N heteroatoms and the rest are carbon atoms, wherein the nitrogen atom is optionally quaternized or oxidized.

As used in the present invention, the term "$C_{3-12}$ cyclic groups" refers to a saturated or partially unsaturated cyclic hydrocarbon group consisting of 3 to 12 (such as 3, 4, 5, 6, 7, 8, 9, 10, 11, or 12) carbon atoms. The $C_{3-12}$ cyclic groups include $C_{3-10}$ cyclic groups, $C_{4-8}$ cyclic groups, $C_{4-6}$ cyclic groups, $C_{4-7}$ cyclic groups, $C_{5-6}$ cyclic groups, $C_{5-7}$ cyclic groups, $C_{6-7}$ cyclic groups, etc. The $C_{3-12}$ cyclic groups include monocyclic, bicyclic or polycyclic ring, including spirocyclic, fused ring, or bridged ring. Specific examples include, but are not limited to, cyclobutyl, cyclopentyl, cyclohexyl, cyclohexenyl, 1,3-cyclohexadienyl, 1,4-cyclohexadienyl, etc.

As used in the present invention, the term "$C_{3-7}$ cycloalkyl" refers to a saturated cyclic hydrocarbon group consisting of 3 to 7 (such as 3, 4, 5, 6 or 7) carbon atoms. The $C_{3-7}$ cycloalkyl includes $C_{4-6}$ cycloalkyl, $C_{4-7}$ cycloalkyl, $C_{5-6}$ cycloalkyl, $C_{5-7}$ cycloalkyl, $C_{6-7}$ cycloalkyl, etc. The $C_{3-7}$ cycloalkyl includes monocyclic, bicyclic or polycyclic ring, including spirocyclic, fused ring, or bridged ring. Specific examples of the $C_{3-7}$ cycloalkyl include, but are not limited to, cyclobutyl, cyclopentyl, cyclohexyl etc.

As used in the present invention, "4-10 membered heterocyclyl" refers to a saturated or partially unsaturated cyclic group consisting of 4 to 10 (such as 4, 5, 6, 7, 8, 9 or 10) ring atoms, wherein 1, 2, 3 or 4 ring atoms are heteroatoms and the rest are carbon atoms; preferably, the heteroatoms are selected from N, O or S, wherein the nitrogen atom is optionally quaternized, and the nitrogen and sulfur heteroatoms are optionally oxidized. The 4-10 membered heterocyclyl includes 4-8 membered, 4-6 membered, 5-6 membered, 8-10 membered, 9-10 membered heterocyclyl, etc. The 4-10 membered heterocyclyl includes monocyclic, bicyclic or polycyclic ring, including spirocyclic, fused ring, or bridged ring. The 4-10 membered heterocyclyl includes a monocyclic group in which the ring is a heterocyclyl and at least one of the fused rings containing a bicyclic or tricyclic group is a heterocyclyl, for example

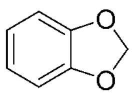 .

Specific examples of the 4-10 membered heterocyclyl include, but are not limited to, azetidinyl, oxetanyl, pyrrolidinyl, 2,3-dihydrofuranyl, 3,4-dihydro-2H-pyranyl, and the like.

As used in the present invention, the term "3-7 membered heterocycloalkyl" refers to a saturated cyclic hydrocarbon group consisting of 3 to 7 (e.g., 3, 4, 5, 6 or 7) ring atoms, of which 1, 2, 3, or 4 ring atoms are heteroatoms and the rest are carbon atoms; preferably, the heteroatoms are selected from N, O and S, wherein the nitrogen atom is optionally quaternized, and the nitrogen and sulfur heteroatoms are optionally oxidized. Specific examples of 3-7 membered heterocycloalkyl include, but are not limited to, azetidinyl, oxetanyl, pyrrolidinyl, etc. As used in the present invention, "*" indicates that the indicated C atom is a chiral carbon atom, unless otherwise specified.

Beneficial effects:

The compounds of the present invention can effectively inhibit SARM1 enzyme.

**Detailed descriptions**

The embodiments of the present invention will be described in detail below in conjunction with the examples, but

those skilled in the art will appreciate that the following examples are only used to illustrate the present invention and should not be considered to limit the scope of the present invention. If specific conditions are not indicated in the examples, they are carried out according to conventional conditions or the conditions recommended by the manufacturer. If the manufacturer is not indicated for the reagents or instruments used, they are all conventional products available commercially.

Instruments and Reagents

NMR was measured using a Bruker Avance III 400 NMR spectrometer, and the chemical shift (δ) was given in units of $10^{-6}$ (ppm). The solvents were deuterated methanol ($CD_3OD$), deuterated chloroform ($CDCl_3$), hexadeuterated dimethyl sulfoxide (DMSO-$d_6$), etc., and the internal standard was tetramethylsilane (TMS).
MS was measured using an Agilent (ESI) mass spectrometer (Agilent 1260, Agilent 6125B).
High performance liquid chromatography (HPLC) conditions: Gilson high pressure liquid chromatograph (Gilson GX-281), C18 column (10 μM, 19 mm x 250 mm), UV detection wavelength at 220 and 254 nm, elution conditions of 5-95% acetonitrile (containing 0.05% (v/v) formic acid or ammonium bicarbonate) with gradient elution for 15 minutes.
Reverse phase purification was performed using Biotage Isolera rapid purification system.
Thin layer chromatography separation and purification were performed using thin layer chromatography silica gel plates (aluminum plates (20 cm x 20 cm x 1 mm) produced by Meck, or GF 254 produced in Yantai).
Microwave reaction was performed using Biotage Initiator + (400 W, RT ~ 300°C) microwave reactor.
TLC or LC-MS was commonly used for reaction monitoring. Common developing solvent systems included: dichloromethane/methanol, n-hexane/ethyl acetate, petroleum ether/ethyl acetate. The volume ratio of the solvents was adjusted according to the polarity of the compound or by adding triethylamine.
The silica gel used for column chromatography was generally 100 ~ 200 mesh silica gel. Common eluent systems included: dichloromethane/methanol, petroleum ether/ethyl acetate. The volume ratio of the solvents was adjusted according to the polarity of the compound, and a small amount of triethylamine can also be added for adjustment. The reagents and solvents of the present invention were purchased from Aldrich Chemical Company, Energy Chemical, Adamas, Leyan, J&K Scientific, Bidepharm, PharmaBlock, Shanghai Titan Scientific Co.,Ltd., etc. Chiral HPLC analysis and determination were performed using Waters UPC2 analytical SFC (SFC-H) high performance liquid chromatograph.
Chiral preparation was performed using WATERS 150 preparative SFC (SFC-26) preparative chromatograph.
The reverse phase column model is SepaFlash® SW040/SW080 Bonded Series C18, Changzhou Santai.
In the conventional synthesis method as well as in the synthesis examples of the compounds and intermediates of the present invention, the meaning of each abbreviation was shown in Table 1 below.

Table 1

| Abbreviation | Meaning |
|---|---|
| $Et_3N$ | Triethylamine |
| DMF | N,N-Dimethylformamide |
| MeOH | Methanol |
| DIPEA or DIEA | N,N-Diisopropylethylamine |
| Pd(dppf)$Cl_2$ | [1,1'-Bis(diphenylphosphino)ferrocene]palladium(II)dichloride dichloromethane complex |
| Pd/C | Palladium/carbon |
| HATU | 2-(7-Azabenzotriazol-1-yl)-N,N,N',N'-tetramethyluronium hexafluorophosphate |
| Boc | *tert*-Butyloxycarbonyl |
| SEM | 2-(Trimethylsilyl)ethoxymethyl |
| THF | Tetrahydrofuran |
| SFC | supercritical fluid chromatography |
| DCM | Dichloromethane |
| EA | Ethyl acetate |
| OTf | Trifluoromethanesulfonyloxy |
| Me | Methyl |
| DMSO | Dimethyl sulfoxide |

(continued)

| Abbreviation | Meaning |
|---|---|
| EtOH | Ethanol |
| $NaBH(OAc)_3$ | Sodium triacetoxyborohydride |
| DCE | 1,2-Dichloroethane |
| iPrMgCl•LiCl | Isopropylmagnesium chloride lithium chloride |
| $NaBH_3CN$ | Sodium cyanoborohydride |
| HOAc | Acetic acid |
| DMP | Dess-Martin periodinane |
| $Boc_2O$ | Di-*tert*-butyl dicarbonate |
| $PPh_3$ | Triphenylphosphine |
| DMCDA | (1R,2R)-(-)-N,N-Dimethyl-1,2-cyclohexanediamine |
| FA | Formic acid |
| SEMC1 | 2-(Trimethylsilyl)ethoxymethyl chloride |
| LC-MS | Liquid Chromatograph Mass Spectrometer |
| NMR | Nuclear Magnetic Resonance Spectroscopy |
| HPLC | High Performance Liquid Chromatography |

Synthesis of intermediate a

Method 1

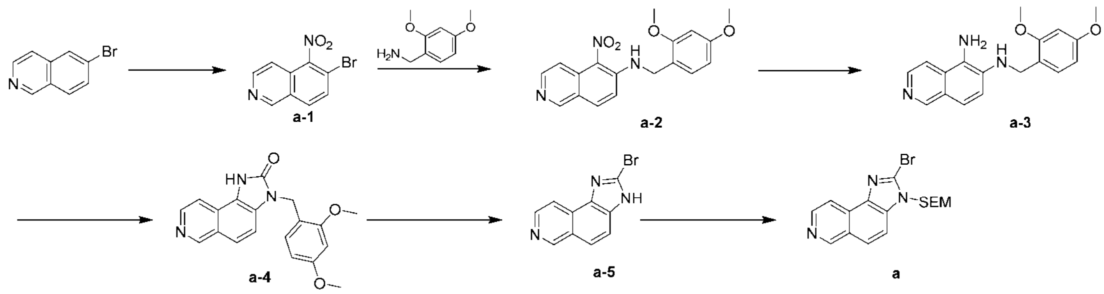

Step 1: 6-Bromoisoquinoline (25g, 120.16 mmol, Bidepharm, batch No. CPU905) and sodium nitrite (18.22g, 180.24 mmol) were added to a solution of concentrated sulfuric acid (60 mL) at 0 °C, warmed up naturally to room temperature, and stirred at room temperature for 16 h. LC-MS showed that the raw material was completely reacted. 500 mL of water was added to quench the reaction under ice bath condition and the pH was adjusted to 7 with 4 M NaOH solution, during which a large amount of solid precipitated. The solid was filtered and the filter cake was collected and slurried with water (500 mL × 2), followed by filtering and collecting the filter cake. The filter cake was dried under reduced pressure to obtain intermediate a-1. LC-MS (ESI): m/z =253.0/255.0$[M+H]^+$. $^1H$ NMR (400 MHz, $CDCl_3$) δ 9.28 (s, 1H), 8.65 (d, *J* = 6.0 Hz, 1H), 7.94 (d, *J* = 8.8 Hz, 1H), 7.77 (d, *J* = 8.8 Hz, 1H), 7.45 (d, *J* = 6.0 Hz, 1H).

Step 2: Intermediate a-1 (2.00 g, 7.90 mmol) was dissolved in N-methylpyrrolidone (40 mL) at room temperature and 2,4-dimethoxybenzylamine (2.64 g, 15.81 mmol, Energy Chemical, batch No.: 7MRKRRET) was added, and the reaction was subsequently warmed up to 120 °C and stirred for 3 h. LC-MS showed that the raw material was completely reacted. The reaction solution was cooled to room temperature, and water (50 mL) and ethyl acetate (50 mL × 2) were added for extraction. The organic phase was washed with saturated brine (50 mL × 5), dried over anhydrous sodium sulfate, and filtered, and the filtrate was concentrated and purified by silica gel column chromatography (ethyl acetate/petroleum ether (0/100 (v/v) to 40/60 (v/v))) to obtain intermediate a-2. $^1H$ NMR (400 MHz, $CDCl_3$) δ 9.48 (t, *J* = 6.0 Hz, 1H), 9.02 (s, 1H), 8.53 (d, *J* = 6.0 Hz, 1H), 8.34 (d, *J* = 6.0 Hz, 1H), 8.10 (d, *J* = 9.6 Hz, 1H), 7.40 (d, *J* = 9.6 Hz, 1H), 7.19 (d, *J* =

8.0 Hz, 1H), 6.62 (d, J= 2.4 Hz, 1H), 6.48 (dd, J = 8.8, 2.4 Hz, 1H), 4.66 (d, J = 6.0 Hz, 2H), 3.86 (s, 3H), 3.74 (s, 3H).

Step 3: Intermediate a-2 (2.4 g, 7.07 mmol) and 10% palladium/carbon (0.25 g, 2.35 mmol) were added to methanol (50 mL) at room temperature, purged with hydrogen (15 Psi) three times, and the reaction was carried out at room temperature for 3 h. The complete of the reaction was monitored by LC-MS, and the reaction solution was filtered and concentrated to obtain intermediate a-3. LC-MS (ESI): m/z = 310.2[M+H]$^+$.

Step 4: Intermediate a-3 (200 mg, 0.65 mmol) was dissolved in tetrahydrofuran (5 mL) at room temperature, followed by cooling to 0 °C and addition of triphosgene (289 mg, 0.98 mmol), and the reaction solution was warmed to room temperature and stirred overnight. LC-MS showed that the raw material was completely reacted. The reaction solution was extracted with water (20 mL) and ethyl acetate (20 mL×2), the organic phase was washed with saturated brine (20 mL×2), dried over anhydrous sodium sulfate, and filtered, and the filtrate was concentrated and purified by silica gel column chromatography (methanol/ dichloromethane (0/100 (v/v) to 10/90 (v/v))) to obtain intermediate a-4. LC-MS (ESI): m/z =336.0 [M+H]$^+$. $^1$H NMR (400 MHz, DMSO-$d_6$) δ 12.52 (s, 1H), 9.75 (s, 1H), 8.58 (d, J = 6.8 Hz, 1H), 8.42 (d, J = 6.7 Hz, 1H), 8.23 (d, J = 8.8 Hz, 1H), 7.89 (d, J= 8.8 Hz, 1H), 7.06 (d, J = 8.4 Hz, 1H), 6.59 (d, J = 2.3 Hz, 1H), 6.46 (dd, J = 8.4, 2.4 Hz, 1H), 5.09 (s, 2H), 3.81 (s, 3H), 3.73 (s, 3H).

Step 5: Intermediate a-4 (1.00 g, 2.98 mmol) and phosphorus oxybromide (4.27 g, 14.91 mmol) were added to the reaction flask at room temperature, and the mixture was heated to 110 °C and stirred for 3 h. LC-MS showed that the raw material was completely reacted. The mixture was cooled to room temperature and slowly added to saturated sodium bicarbonate solution. The solution was concentrated, then dissolved by DMF, and the insoluble substance was filtered off and the filtrate was purified by reversed-phase column (phase A: 0.5% aqueous ammonium bicarbonate solution, phase B: acetonitrile, gradient: 2% to 95% B) to obtain intermediate a-5. $^1$H NMR (400 MHz, DMSO-$d_6$) δ 9.35 (s, 1H), 8.60 (d, J = 5.7 Hz, 1H), 8.16 (d, J = 5.7 Hz, 1H), 7.91 (d, J = 8.8 Hz, 1H), 7.82 (d, J = 8.7 Hz, 1H).

Step 6: Intermediate a-5 (195 mg, 0.79 mmol) was dissolved in dichloromethane (5 mL) at room temperature, cooled to 0°C and triethylamine (0.27 mL, 0.98 mmol) and 2-(trimethylsilyl)ethoxymethyl chloride (0.17 mL, 0.94 mmol, Energy Chemical) were added, and the reaction solution was warmed to room temperature and stirred overnight. LC-MS showed that the raw material was completely reacted. Water (20 mL) and ethyl acetate (20 mL×2) were added to the reaction solution for extraction, and the organic phase was washed with saturated brine (20 mL×2), dried over anhydrous sodium sulfate, and filtered, the filtrate was concentrated and purified by silica gel column chromatography (methanol/ dichloromethane (0/100 (v/v) to 10/90 (v/v))) to obtain intermediate a. LC-MS (ESI): m/z =378.0, 380.0 [M+H]$^+$.

Method 2

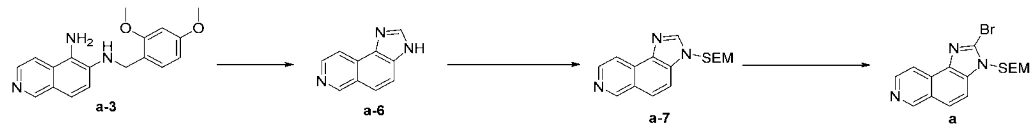

Step 1: Intermediate a-3 (6.40 g, 20.69 mmol) was dissolved in formic acid (60 mL) at room temperature, and the reaction solution was heated to 100°C and stirred for 2 h. LC-MS showed that the raw material was completely reacted. The reaction solution was cooled to room temperature and concentrated, and then was dissolved by adding dichloromethane after most of the formic acid was removed. Triethylamine was added to adjust the solution to alkalinity, then the solution was concentrated and purified by silica gel column chromatography (methanol/ dichloromethane (0/100 to 10/90)) to obtain intermediate a-6. LC-MS (ESI): m/z =170.0 [M+H]$^+$.

Step 2: Intermediate a-6 (2.68 g, 15.82 mmol) was dissolved in DMF (50 mL) at room temperature, followed by cooling to 0 °C and addition of N,N-diisopropylethylamine (5.11 g, 39.55 mmol) and 2-(trimethylsilyl)ethoxymethyl chloride (3.96 g, 23.73 mmol). The reaction solution was heated to 80 °C and stirred for 3 hours. LC-MS showed that the raw material was completely reacted. water (50 mL) and ethyl acetate (50 mL×2) were added to the reaction solution for extraction, and the organic phase was washed with saturated brine (50 mL×2), dried over anhydrous sodium sulfate, and filtered, and the filtrate was concentrated and purified by silica gel column chromatography (methanol/ dichloromethane (0/100 (v/v) to 5/95 (v/v))) to obtain intermediate a-7. LC-MS (ESI): m/z =300.2 [M+H]$^+$.

Step 3: Intermediate a-7 (2.12 g, 7.08 mmol) was dissolved in tetrahydrofuran (40 mL) at room temperature, followed by cooled to -78 °C and slowly addition of lithium diisopropylamide (7.08 mL, 14.16 mmol) dropwise. The reaction solution was stirred at -78 °C for 0.5 h, followed by addition of carbon tetrabromide (4.70 g, 14.16 mmol) in tetrahydrofuran (20 mL) dropwise, and the reaction solution was stirred at the same temperature for 1 h. LC-MS showed that the raw material was completely reacted. Saturated aqueous ammonium chloride solution was added to the reaction solution to quench, and water (50 mL) and ethyl acetate (50 mL × 2) were added for extraction. The organic phase was washed with saturated brine (50 mL × 2), dried over anhydrous sodium sulfate, and filtered, and the filtrate was concentrated and

purified by silica gel column chromatography (methanol/ dichloromethane (0/100 (v/v) to 3/97 (v/v))) to obtain intermediate a. LC-MS (ESI): m/z =378.0/380.0 $[M+H]^+$.

Synthesis of intermediate b

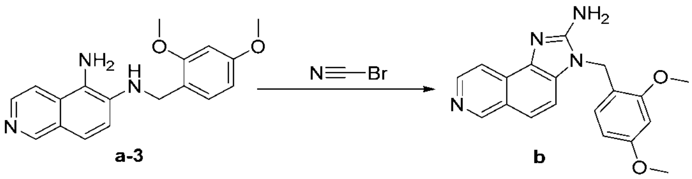

a-3 b

Intermediate a-3 (3.36 g, 10.86 mmol) was dissolved in methanol (40 mL) at room temperature, cyanogen bromide (1.73 g, 16.29 mmol) was added, and the reaction solution was heated to 60 °C and stirred for 3 h. LC-MS showed that the raw material was completely reacted. The reaction solution was cooled to room temperature and slowly added to 1 mol/L aqueous sodium hydroxide solution, the insoluble substance was filtered off and the filtrate was concentrated and purified by silica gel column chromatography (methanol/ dichloromethane (0/100 (v/v) to 5/95 (v/v))) to obtain intermediate b. LC-MS (ESI): m/z =335.1 $[M+H]^+$. $^1H$ NMR (400 MHz, DMSO-$d_6$) $\delta$ 9.17 (s, 1H), 8.40 (d, *J* = 5.7 Hz, 1H), 7.98 (d, *J* = 5.7 Hz, 1H), 7.56 (d, *J* = 8.6 Hz, 1H), 7.49 (d, *J* = 8.6 Hz, 1H), 6.75 - 6.67 (m, 3H), 6.64 - 6.57 (m, 1H), 6.46 - 6.36 (m, 1H), 5.26 (s, 2H), 3.85 (s, 3H), 3.71 (d, *J* = 6.2 Hz, 3H).

Synthesis of intermediate c

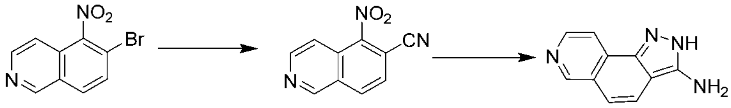

a-1 c-1 c

Step 1: Intermediate a-1 (1 g, 3.95 mmol), potassium ferrocyanide (2.91 g, 7.90 mmol) and cuprous iodide (0.75 g, 3.95 mmol) were added to N-methylpyrrolidone (20 mL) solution at room temperature, purged with nitrogen three times, and the reaction was stirred in a closed system for 16 h at 120 °C. LC-MS showed that the raw material was completely reacted. After the reaction was cooled to room temperature, ethyl acetate (100 mL) was added, and the solution was washed with water (100 mL×2) and washed with saturated brine (100 mL), and the organic phase was collected. The organic phase was concentrated under reduced pressure to obtain a crude product. The crude product was purified by silica gel column chromatography (ethyl acetate/petroleum ether (1/100 (v/v) to 1/1 (v/v))) to obtain intermediate c-1.

Step 2: Intermediate c-1 (130 mg, 0.65 mmol), hydrazine hydrochloride (134.13 mg, 1.96 mmol), and *N,N*-diisopropylethylamine (253.09 mg, 1.96 mmol) were added to N-methylpyrrolidone (3 mL) solution at room temperature. The reaction solution was heated to 130 °C and stirred for 30 min under microwave conditions. LC-MS showed that the raw material was completely reacted. The reaction was cooled to room temperature and water (20 mL) was added, extracted with ethyl acetate (20 mL×4), and the organic phase was washed with saturated brine (20 mL) and collected. The organic phase was concentrated under reduced pressure to obtain a crude product. The crude product was purified by silica gel column chromatography (methanol/dichloromethane (1/100 (v/v) to 1/15 (v/v))) to obtain intermediate c. LC-MS (ESI): m/z =185.0$[M+H]^+$.

Synthesis of intermediate d

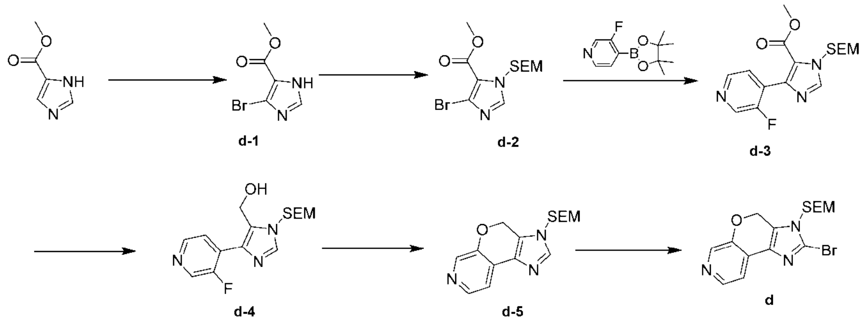

d-1 d-2 d-3 d-4 d-5 d

Step 1: Methyl 2-imidazolecarboxylate (5 g, 39.65 mmol) was added to acetonitrile (100 mL) solution in an ice bath, and then NBS dissolved in acetonitrile (100 mL) was added dropwise to the reaction solution, purged with nitrogen three times, and the reaction was warmed to room temperature and stirred for 16 h. LCMS showed that the raw material was completely consumed and intermediate d-1 was detected. The reaction solution was concentrated in vacuo to obtain a crude product, then the crude product was purified by silica gel chromatography column eluted with ethyl acetate/petroleum ether (1/100 to 1/1) to obtain intermediate d-1. LC-MS (ESI): m/z= 204.8[M+H]+.

Step 2: Intermediate d-1 (6.7 g, 32.68 mmol) and triethylamine (13.59 mL, 98.04 mmol) were added to anhydrous dichloromethane (100 mL) solution at room temperature, the reaction solution was cooled to 0°C with an ice-salt bath, and SEMCl (11.59 mL, 65.36 mmol) was added dropwise at 0°C, then warmed to room temperature and stirred for 16 hours. TLC (petroleum ether/ethyl acetate = 5/1) detected the disappearance of the raw material. The reaction solution was diluted with water (100 mL×3), the organic phase was separated, and the organic phase was dried over anhydrous sodium sulfate, filtered and concentrated under reduced pressure to obtain a crude product. The crude product was then purified by silica gel chromatography column eluted with ethyl acetate/petroleum ether (1/100-1/10) to obtain the intermediate d-2. LC-MS (ESI): m/z=335.0[M+H]+.

Step 3: Intermediate d-2, 3-fluoro-4-(4,4,5,5-tetramethyl-1,3,2-dioxaborolan-2-yl)pyridine (1.82 g, 8.17 mmol), Pd(dppf)$Cl_2$ (0.30 g, 0.41 mmol) and cesium carbonate (3.99 g, 12.26 mmol) were added to 1,2-dioxane (15 mL) and water (0.8 mL). The reaction solution was purged with nitrogen three times and stirred at 130 °C for 24 hours under nitrogen atmosphere. LC-MS showed the generation of product. The reaction solution was diluted with aqueous solution (30 mL) and extracted with ethyl acetate (50 mL × 2). The organic phases were combined, washed with saturated NaCl (30 mL), dried over anhydrous sodium sulfate, filtered, and concentrated under reduced pressure to obtain a crude product. The crude product was purified by silica gel chromatography column eluted with ethyl acetate/petroleum ether (1/100 to 1/1) to obtain intermediate d-3. LC-MS (ESI): m/z=352.1[M+H]+. Step 4: Intermediate d-3 (840 mg, 2.39 mmol) and lithium aluminum tetrahydride (136.06 mg, 3.59 mmol) were added to anhydrous tetrahydrofuran (15 mL) solution in an ice bath, and the reaction solution was stirred at 0°C for 1h. LC-MS showed the generation of product. The above solution was diluted with ethyl acetate (100 mL), and the organic layer was separated by extraction with water (100 mL) and ethyl acetate (100 mL× 2). The organic phase was concentrated in vacuo to obtain a crude product, and the crude product was purified by silica gel chromatography column eluted with dichloromethane/methanol (1/100 to 1/20) to obtain intermediate d-4. LC-MS (ESI): m/z=324.2[M+H]+.

Step 5: Intermediate d-4 (970 mg, 3.00 mmol) and potassium tert-butoxide (0.92 mL, 7.50 mmol) were added to anhydrous tetrahydrofuran (15 mL) solution at room temperature. The reaction solution was stirred at 40 °C for 16 hours under nitrogen atmosphere. LC-MS showed the generation of product. The reaction mixture was diluted with aqueous solution (30 mL) and extracted with ethyl acetate (50 mL × 2), and the organic phases were combined and washed with saturated NaCl (30 mL), dried over anhydrous sodium sulfate, filtered, and concentrated in vacuo to obtain a crude product, then the crude product was purified by silica gel chromatography column eluted with ethyl acetate/petroleum ether (1/100 to 1/1) to obtain intermediate d-5. LC-MS (ESI): m/z=304.0[M+H]+.

Step 6: Intermediate d-5 (540 mg, 1.78 mmol) was added to anhydrous tetrahydrofuran (20 mL) solution at room temperature, the reaction mixture was cooled to -50 °C, and LDA (1.78 mL, 3.56 mmol) was slowly added dropwise to the reaction solution. The reaction was carried out at -50°C for 1 hour, and carbon tetrabromide (1180.33 mg, 3.56 mmol) dissolved in tetrahydrofuran (1 mL) then was slowly added dropwise to the reaction solution, and the reaction was stirred and reacted at -50 °C for 1 h. LC-MS showed the generation of product. The reaction mixture was diluted with aqueous solution (30 mL), extracted with ethyl acetate (50 mL × 2), and the organic phases were combined and washed with saturated NaCl (50 mL), dried over anhydrous sodium sulfate, filtered, and concentrated in vacuo to obtain a crude product, then the crude product was purified by silica gel chromatography column eluted with ethyl acetate/petroleum ether (1/100 to 1/2) to obtain intermediate d. LC-MS (ESI): m/z=382.0[M+H]+.

Synthesis of intermediate e

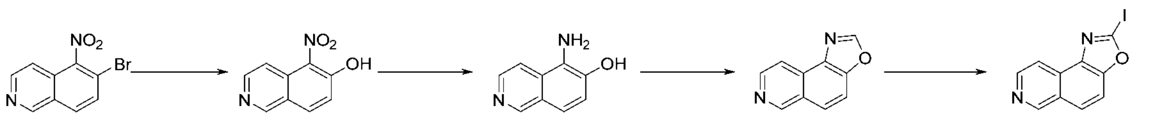

a-1 e-1 e-2 e-3 e

Step 1: A mixture of intermediate a-1 (2 g, 7.90 mmol), sodium hydroxide (3 g, 75.00 mmol) and water (50 mL) was stirred at 100 °C for 2 hours. After the reaction was completed, the reaction mixture was filtered through a Buchner funnel, and the filter cake was collected and dried to obtain a crude intermediate e-1. LC-MS (ESI): m/z=191.0[M+H] +.

Step 2: Intermediate e-1 (1.2 g, 6.31 mmol), palladium hydroxide (0.1 g, 0.71 mmol), ethanol (15 mL) and glacial acetic acid (15 mL) were stirred at 25 °C for 16 hours under hydrogen atmosphere. After the reaction, the reaction mixture was filtered through a Buchner funnel, and the filter cake was collected and rotary dried to obtain a crude intermediate e-2. LC-MS (ESI): m/z=161.0[M+H] +.

Step 3: A mixture of intermediate e-2 (1.5 g, 3.28 mmol), triethoxymethane (1.46 g, 9.83 mmol), 4-methylbenzenesulfonatopyridin-1-ium (0.82 g, 3.24 mmol) and toluene (20 mL) was stirred under nitrogen protection for 3 hours at 100 °C. After the reaction was completed, the reaction solution was concentrated under reduced pressure to obtain a crude product. The crude product was purified by silica gel column chromatography (petroleum ether/ethyl acetate = 7/3) to obtain intermediate e-3. LC-MS (ESI): m/z=170.9 [M+H] +.

Step 4: A mixture of intermediate e-3 (245 mg, 1.44 mmol), DMF (10 mL), sodium 2-methylpropan-2-ol (138.36 mg, 1.44 mmol) and 1,1,1,2,2,3,3,4,4-nonafluoro-4-iodobutane (996.10 mg, 2.88 mmol) was stirred at 25 °C under nitrogen protection for 2 hours. The reaction mixture was filtered through a Buchner funnel, and the filtrate was concentrated under reduced pressure to obtain intermediate e. LC-MS (ESI): m/z=296.9[M+H] +. $^{1}$H NMR (400 MHz, DMSO) δ 9.47 (d, J = 0.6 Hz, 1H), 8.70 (d, J = 5.7 Hz, 1H), 8.19 (d, J = 5.7 Hz, 1H), 8.17 - 8.10 (m, 2H).

Synthesis of intermediate f

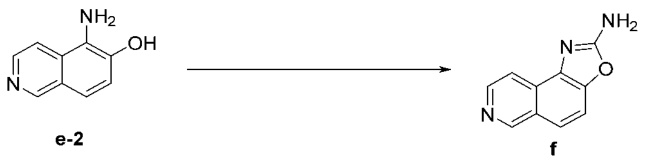

**e-2** **f**

A mixture of intermediate e-2 (1 g, 2.19 mmol), (imidazol-1-yl)(imidazol-3-yl)methanimine (0.70 g, 4.37 mmol), pyridin-1-ium 4-methylbenzenesulfonate (0.55 g, 2.19 mmol) and DMF (15 mL) was stirred under nitrogen protection for 3 h at 75 °C. After the reaction was completed, the solvent was concentrated under reduced pressure to obtain a crude product, then the crude product was purified by silica gel column chromatography (dichloromethane/methanol = 93/7) to obtain intermediate f. LC-MS (ESI): m/z=186.0[M+H]+.

Example 1 Preparation of Compound 1

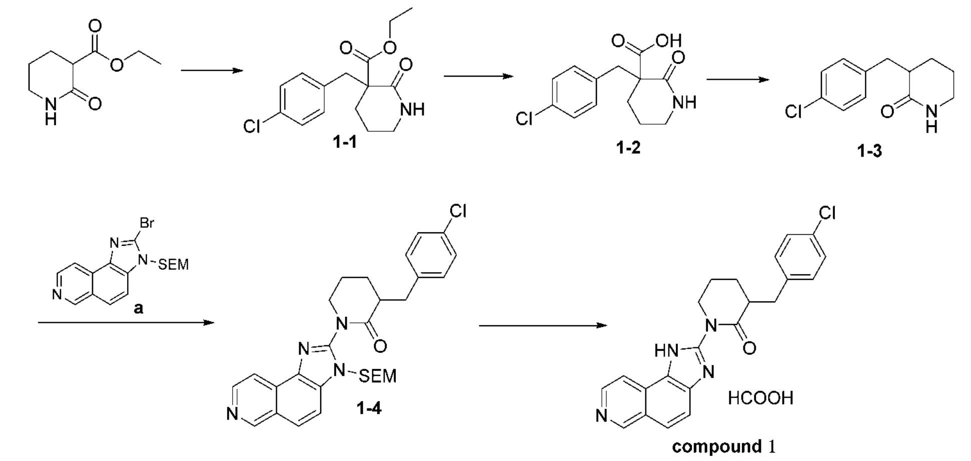

1-1 1-2 1-3

a

1-4

compound 1

Step 1: Ethyl 2-oxopiperidin-3-carboxylate (5 g, 29.21 mmol) was dissolved in ethanol (50 mL) at room temperature, and sodium ethoxide (11.42 mL, 29.21 mmol) was added under nitrogen protection and stirred at room temperature for 15 min, then 4-chlorobenzyl bromide (6.00 g, 29.21 mmol) was added, and the reaction solution was stirred at room temperature overnight. LC-MS showed that the raw material was completely reacted. The reaction solution was quenched by adding saturated aqueous ammonium chloride solution, then water (50 mL) and ethyl acetate (50 mL×2) were added for extration. The organic phase was washed with saturated brine (50 mL×2), dried over anhydrous sodium sulfate, and filtered, and the filtrate was concentrated and purified by silica gel column chromatography (ethyl acetate/petroleum ether (0/100 (v/v) to 30/70 (v/v))) to obtain intermediate 1-1. LC-MS (ESI): m/z =296.0 $[M+H]^+$. $^1H$ NMR (400 MHz, $CDCl_3$) δ 7.25 - 7.15 (m, 4H), 5.99 (s, 1H), 4.31 - 4.15 (m, 2H), 3.57 (d, *J*= 13.5 Hz, 1H), 3.30 - 3.16 (m, 1H), 3.12 - 2.94 (m, 2H), 2.13 - 2.03 (m, 1H), 1.88 - 1.76 (m, 1H), 1.73 (dd, *J* = 13.3, 2.8 Hz, 1H), 1.62 - 1.51 (m, 1H), 1.29 (t, *J* = 7.1 Hz, 3H).

Step 2: Intermediate 1-1 (3 g, 10.14 mmol) was dissolved in methanol (20 mL) and water (20 mL) at room temperature and cooled to 0°C, and sodium hydroxide (2.43 g, 60.84 mmol) was added, and the reaction solution was stirred at room temperature overnight. LC-MS showed that the raw material was completely reacted. The reaction solution was concentrated to remove methanol, and the pH was adjusted to 4 with 1 mol/L hydrochloric acid, then water (50 mL) and ethyl acetate (50 mL×2) were added for extraction, and the organic phase was washed with saturated brine (50 mL×2), dried over anhydrous sodium sulfate, and filtered, and the filtrate was concentrated to obtain intermediate 1-2. LC-MS (ESI): m/z =268.0 $[M+H]^+$.

Step 3: At room temperature, intermediate 1-2 (2.72 g, 10.16 mmol) was dissolved in ethyl acetate (50 mL), heated to 90 °C and refluxed with stirring for 3 h. LC-MS showed that the raw material was completely reacted. The reaction solution was cooled to room temperature and concentrated to obtain intermediate 1-3. LC-MS (ESI): m/z =224.0 $[M+H]^+$. $^1H$ NMR (400 MHz, $CDCl_3$) δ 7.18 (d, *J* = 8.4 Hz, 2H), 7.07 (d, *J* = 8.3 Hz, 2H), 6.52 (s, 1H), 3.30 - 3.11 (m, 3H), 2.63 (m, 1H), 2.51 - 2.38 (m, 1H), 1.81 - 1.63 (m, 2H), 1.63 - 1.53 (m, 1H), 1.41 - 1.28 (m, 1H).

Step 4: Intermediate a (55 mg, 0.15 mmol) and intermediate 1-3 (39 mg, 0.17 mmol) were placed in a microwave tube at room temperature, and dissolved by adding 1,4-dioxane (2 mL), and cesium carbonate (118 mg, 0.36 mmol), Xantphos (34 mg, 0.06 mmol) and tris(dibenzylideneacetone)dipalladium (27 mg, 0.03 mmol) were added, and after nitrogen displacement, the reaction solution was heated with a microwave synthesizer to 130 °C for 45 min. LC-MS showed that the raw material was completely reacted. The reaction solution was extracted with water (30 mL) and ethyl acetate (30 mL×2), and the organic phase was washed with saturated brine (30 mL×2), dried over anhydrous sodium sulfate, and filtered, and the filtrate was concentrated and purified by silica gel column chromatography (methanol/dichloromethane (0/100 (v/v) to 10/90 (v/v))) to obtain intermediate 1-4. LC-MS (ESI): m/z =521.2 $[M+H]^+$.

Step 5: Intermediate 1-4 (100 mg, 0.19 mmol) was dissolved in dichloromethane (2 mL) and trifluoroacetic acid (1 mL) at room temperature, and the reaction solution was stirred at room temperature for 3 h. LC-MS showed that the raw material was completely reacted. The reaction solution was concentrated, then dissolved in DMF and purified by HPLC (phase A: 0.1% FA in $H_2O$, phase B: ACN) to obtain compound 1. LC-MS (ESI): m/z =391.0 $[M+H]^+$. $^1H$ NMR (400 MHz, DMSO-$d_6$) δ 12.94 (s, 1H), 9.28 (s, 1H), 8.53 (d, *J* = 5.6 Hz, 1H), 8.32 (s, 1H), 8.15(brs, 1H), 7.93 - 7.85 (m, 1H), 7.84 -7.78 (m, 1H), 7.40 - 7.29 (m, 4H), 4.27 - 4.10 (m, 2H), 3.05 - 2.95 (m, 1H), 2.89 - 2.80 (m, 1H), 2.59 - 2.50 (m, 1H), 2.04 - 1.78 (m, 3H), 1.562- 1.48 (m, 1H).

Example 2 Preparation of Compound 2

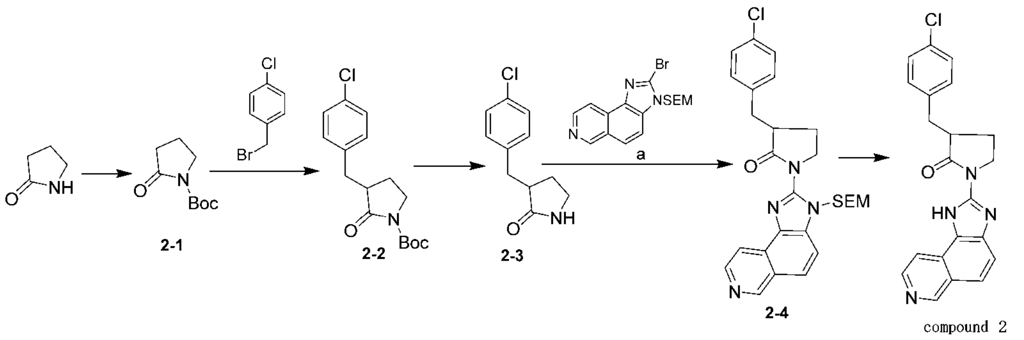


Step 1: Pyrrolidin-2-one (5 g, 58.8 mmol, Energy Chemical, batch No.: 0LGHRSDQ), *N,N*-diisopropylethylamine (30.4 g, 235 mmol), 4-dimethylaminopyridine (1.44 g, 11.8 mmol) and $Boc_2O$ (25.6 g, 118 mmol) were added to dichloromethane (50 mL) at room temperature and reacted at room temperature for 16 hours. TLC (petroleum ether/tetrahydrofuran = 3/1) showed that pyrrolidin-2-one was completely consumed and new spots with lower polarity were generated. The reaction solution was concentrated and purified by silica gel column chromatography (0-25% tetrahydrofuran/petroleum ether) to obtain intermediate 2-1. $^1$H NMR (400MHz, DMSO-$d_6$) δ: 3.64 (t, *J*=7.1 Hz, 2H), 2.40 (t, *J*=8.0 Hz, 2H), 1.90 (m, 2H), 1.45 (s, 9H).

Step 2: Intermediate 2-1 (5.00 g, 27.0 mmol) was added to tetrahydrofuran (50 mL) at room temperature and cooled to -78 °C under nitrogen protection. Lithium bis(trimethylsilyl)amide in 1.0 M tetrahydrofuran(29.7 mL, 29.7 mmol) was added and stirred for 1 h. P-chlorobenzyl bromide (5.82 g, 28.3 mmol) dissolved in tetrahydrofuran (25 mL) was added dropwise into the reaction system and reacted for 3 h at -78 °C. TLC (petroleum ether/tetrahydrofuran = 2/1) showed that intermediate 2-1 was completely consumed, and a new spot with greater polarity was generated. The reaction solution was quenched with saturated ammonium chloride (50 mL) at room temperature, diluted with water (50 mL), extracted with ethyl acetate (50 mL × 2), and the organic phase was dried over anhydrous sodium sulfate, filtered and concentrated. The residue was separated and purified by silica gel column chromatography (0-25% tetrahydrofuran/petroleum ether) to obtain intermediate 2-2. LC-MS (ESI): m/z = 254.1$[M+H-56]^+$.

Step 3: Intermediate 2-2 (400 mg, 1.29 mmol) was added to dioxane (10 mL) at room temperature, then dioxane hydrochloride (5 mL) was added, and was allowed to react at room temperature for 1 hour. LC-MS monitored that the reaction was completed, and the reaction solution was quenched with saturated sodium bicarbonate (15 mL) at room temperature, diluted with water (15 mL), and extracted with ethyl acetate (20 mL × 2), and the organic phase was dried over anhydrous sodium sulfate, filtered and concentrated to obtain intermediate 2-3.

Step 4: Intermediate a (36 mg, 0.10 mmol), intermediate 2-3 (29.9 mg, 0.14 mmol), cuprous iodide (1.81 mg, 0.01 mmol), (1R,2R)-(-)-N,N'-dimethyl-1,2-cyclohexanediamine (2.71 mg, 0.02 mmol), and cesium carbonate (93.0 mg, 0.29 mmol) were added to toluene (1 mL) at room temperature, and reaction solution was purged with nitrogen three times and heated to 110 °C for 3 h. LC-MS monitored that the reaction was completed. The reaction solution was naturally cooled, filtered and concentrated, and the residue was purified by thin layer chromatography (petroleum ether: tetrahydrofuran (0.5% triethylamine) = 2:3) to obtain intermediate 2-4. LC-MS (ESI): m/z = 507.2$[M+H]^+$.

Step 5: Intermediate 2-4 (58 mg, 0.11 mmol) and 70% pyridine in hydrofluoric acid (4 mL) were added to tetrahydrofuran (2 mL) at room temperature and was allowed to react at room temperature for 1 hour. LC-MS monitored that the reaction was completed, the reaction solution was concentrated to obtain a crude product. The crude product was purified by HPLC (column: Phenomenex C18 75*30mm*3μm; mobile phase: $H_2O$ (0.05% $NH_3.H_2O$ v/v)-ACN; B(ACN)%: 36%-76%, 9min) and lyophilized to obtain compound 2. LC-MS (ESI): m/z = 377.0 $[M+H]^+$. $^1$H NMR (400MHz, $CD_3OD$) δ: 9.27 (s, 1H), 8.51 (d, *J*=5.9 Hz, 1H), 8.41 (d, *J*=6.2 Hz, 1H), 7.92 (s, 2H), 7.32 (s, 4H), 4.15 - 3.98 (m, 2H), 3.32 - 3.26 (m, 1H), 3.23 - 3.13 (m, 1H), 2.95 - 2.86 (m, 1H), 2.40 - 2.30 (m, 1H), 2.10 - 1.99 (m, 1H).

Example 3 Preparation of Compound 3

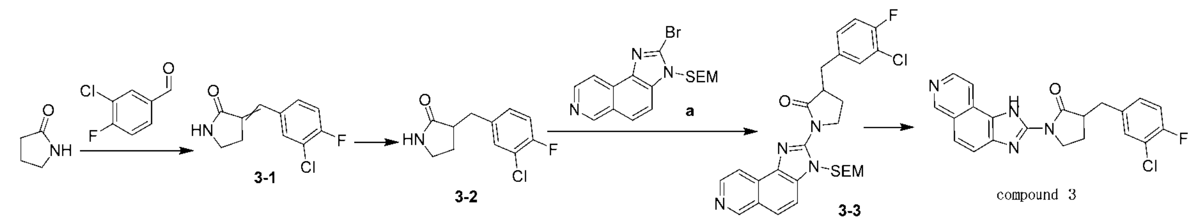

Step 1: Pyrrolidin-2-one (1.1 g, 6.94 mmol, Energy Chemical, Batch No.: 0LGHRSDQ) was dissolved in toluene (5 mL), and TFAA (1.05 mL, 7.57 mmol) was added at 0 °C. After 1 h, the reaction solution was concentrated, an appropriate amount of toluene was added to the residue, and re-concentrated, and the above operation was repeated twice, and the resulting colorless oily compound was re-dissolved in THF (3 mL) along with 3-chloro-4-fluorobenzaldehyde (1 g, 6.31 mmol, Bide Pharmatech), and 1 M t-BuOK (8.20 mL, 8.20 mmol) in THF was slowly added to the reaction system at 0 °C. After the addition was completed, the resulting mixture was heated to 55 °C in an oil bath and stirred for an additional 1 hour. LC-MS showed that the target product was generated. Water (30 mL) was added to the reaction system to quench the reaction. The mixture was extracted with ethyl acetate (20 mL×3). The mixed organic phase was washed once with saturated brine, dried over anhydrous sodium sulfate, and filtered, and the filtrate was concentrated to obtain intermediate 3-1. LC-MS (ESI): m/z =226.0$[M+H]^+$.

Step 2: Intermediate 3-1 (1.2 g, 5.32 mmol) was dissolved in ethyl acetate (20 mL), 10% palladium/carbon (280 mg, 2.66 mmol) was added, and the resulting reaction system was purged three times with hydrogen and stirred at room temperature for 3 hours. LC-MS monitored that the target product was generated, the reaction solution was filtered, concentrated, and purified by HPLC (ACN/$H_2O$/0.1% HCOOH) to obtain intermediate 3-2. LC-MS (ESI): m/z =228.0 $[M+H]^+$.

Step 3: Intermediate 3-2 (80 mg, 0.35 mmol), intermediate a (132.9 mg, 0.35 mmol), $Pd_2(dba)_3$ (32.18 mg, 0.035 mmol), XantPhos (40.67 mg, 0.07 mmol) and cesium carbonate (229 mg, 0.7 mmol) were dissolved in dioxane (10 mL), and the reaction system was purged with nitrogen three times and stirred at 100 °C for 18 hours. LC-MS showed that the reaction was completed, and the mixture was directly concentrated, and the residue was subjected to silica gel column chromatography (DCM: MeOH = 20: 1 (v/v)) to obtain intermediate 3-3. LC-MS (ESI): m/z =525.2$[M+H]^+$.

Step 4: Intermediate 3-3 (180 mg, 0.34 mmol) was dissolved in dioxane (5 mL), 4M HCl/EA (7 mL, 28.00 mmol) was added, and the resulting mixture was reacted under microwave at 80 °C for 1 hour. LC-MS monitored that the raw material was completely consumed, and the reaction solution was directly concentrated, and the residue was added to the water (10 mL) , and was adjusted to pH 7-8 with saturated $NaHCO_3$, and extracted with ethyl acetate (10 mL×3), and the organic phases were combined and concentrated to obtain a crude product, which was further purified by HPLC (ACN/$H_2O$/0.1%HCOOH) to obtain compound 3. LC-MS (ESI): m/z =395.2$[M+H]^+$. $^1H$ NMR (400 MHz, DMSO-$d_6$) δ 12.83 (s, 1H), 9.29 (s, 1H), 8.52 (d, J = 6.0 Hz, 1H), 8.26 - 8.06 (m, 1H), 7.94 - 7.78 (m, 2H), 7.62-7.53 (m, 1H), 7.45 - 7.27 (m, 2H), 4.18-4.06 (m, 1H), 4.00-3.89 (m, 1H), 3.25-3.12 (m, 2H), 2.90 - 2.78 (m, 1H), 2.25-2.14 (m, 1H), 1.99-1.86 (m, 1H).

Example 4 Preparation of Compound 4

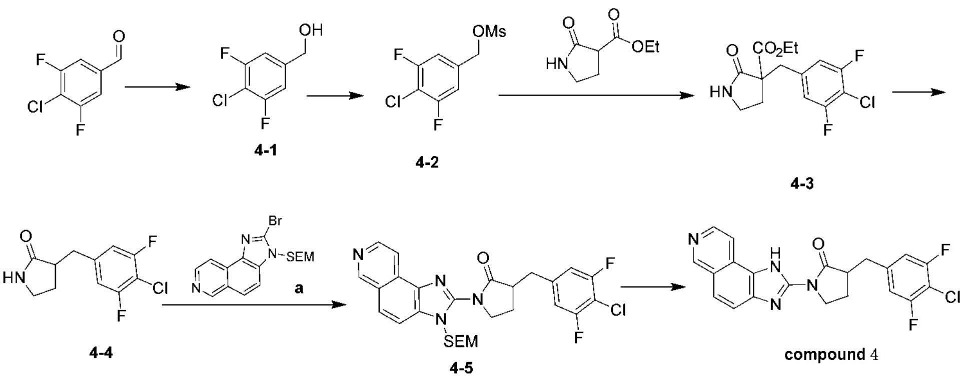


Step 1: 3,5-difluoro-4-chlorobenzaldehyde (2 g, 11.33 mmol, Bide Pharmatech, Batch No.: CNA149) was dissolved in methanol (50 mL) and $NaBH_4$ ( 430 mg, 11.33 mmol) was added in batch at 0°C. The resulting mixture was stirred at 0°C for 30 min. LC-MS monitored that the target product was generated, and the reaction was quenched by adding saturated ammonium chloride (20 mL) , and the excess methanol was removed by concentration. The residue was washed with ethyl acetate (30 mL×3), and the combined organic phase was washed once with saturated brine, dried over anhydrous sodium sulfate, and filtered, and the filtrate was concentrated to obtain a crude product intermediate 4-1. LC-MS (ESI): m/z =161.0$[M+H-18]^+$.

Step 2: Intermediate 4-1 (1.5 g, 8.4 mmol) was dissolved in DCM (35 mL), triethylamine (2.33 mL, 16.8 mmol) and MsCl (0.72 mL, 9.24 mmol) were added at 0 °C and the resulting mixture was stirred at 0 °C for 30 min. LC-MS monitored that the reaction was completed, and water (30 mL) was added to the reaction solution, the DCM phase was separated, and the aqueous phase was extracted with DCM (10 mL×3). The combined organic phase was washed once with saturated $NaHCO_3$ and once with brine, dried over anhydrous sodium sulfate, and filtered, and the filtrate was

concentrated to obtain a crude product intermediate 4-2. LC-MS (ESI): m/z =161.0[M+H-96]$^+$.

Step 3: Ethyl 2-oxopyrrolidin-3-carboxylate (900 mg, 5.73 mmol, Bide Pharmatech, Batch No.: CNZ287) was dissolved in ethanol (30 mL), 20 wt% NaOEt in ethanol (2.24 mL, 5.73 mmol) was added, and after 30 min, intermediate 4-2 (2.2 g, 8.59 mmol) in ethanol (3 mL) was added, and the resulting mixture was stirred for 3 hours at room temperature. LC-MS monitored that the reaction was completed, water (30 mL) was added to the reaction solution, the excess ethanol was removed by rotary evaporation, the residue was extracted with ethyl acetate (20 mL × 3), and the combined organic phases were concentrated and separated to obtain intermediate 4-3 by silica gel column chromatography (DCM : MeOH=20:1 (v/v)). LC-MS (ESI): m/z =318.0[M+H]$^+$.

Step 4: Intermediate 4-3 (500 mg, 1.57 mmol) and LiCl (113.41 mg, 2.68 mmol) were dissolved in DMSO (10 mL), water (0.09 mL, 5.19 mmol) was added, and the resulting mixture was stirred for 3 h at 180 °C. LC-MS monitored that the reaction was completed, the reaction system was cooled to room temperature and water (50 mL) was added to the mixture, extracted with ethyl acetate (20 mL × 3), and the combined organic phase was separated by silica gel column chromatography (DCM : MeOH=30:1 (v/v)) to obtain intermediate 4-4. LC-MS (ESI): m/z =246.0[M+H]$^+$.

Step 5: Intermediate 4-4 (84.4 mg, 0.34 mmol), intermediate a (130 mg, 0.34 mmol), $Pd_2(dba)_3$ (31.46 mg, 0.034 mmol), XantPhos (39.76 mg, 0.068 mmol) and cesium carbonate (223.9 mg, 0.68 mmol) were dissolved in dioxane (5 mL), and the reaction system was purged with nitrogen three times and stirred at 100°C for 18 hours. LC-MS monitored that the reaction was completed, and the mixture was directly concentrated. The residue was separated by silica gel column chromatography (DCM: MeOH = 30: 1 (v/v)) to obtain intermediate 4-5. LC-MS (ESI): m/z =543.2[M+H]$^+$.

Step 6: Intermediate 4-5 (170 mg, 0.31 mmol) was dissolved in dioxane (5 mL), 4M HCl/EA (7 mL, 28.00 mmol) was added, and the resulting mixture was reacted under microwave at 80 °C for 1 hour. LC-MS monitored that the raw material was completely consumed, the reaction solution was concentrated directly, water (10 mL) was added to the residue, the pH was adjusted to 7~8 with saturated $NaHCO_3$, and the residue was extracted with ethyl acetate (10 mL×3), and the combined organic phases were concentrated to obtain a crude product, which was further purified by HPLC (ACN/$H_2O$/0.1% HCOOH) to obtain compound 4. LC-MS (ESI): m/z =413.2[M+H]$^+$. $^1$H NMR (400 MHz, DMSO-$d_6$) δ 12.80 (s, 1H), 9.28 (s, 1H), 8.52 (d, J = 6.0 Hz, 1H), 8.18-8.10 (m, 1H), 7.94 - 7.77(m, 2H), 7.39-7.31 (m, 2H), 4.22-4.09 (m, 1H), 4.02-3.91(m, 1H), 3.30 - 3.16 (m, 2H), 2.93 - 2.81 (m, 1H), 2.30 - 2.18 (m, 1H), 2.0 -1.87 (m, 1H).

Example 5 Preparation of Compound 5

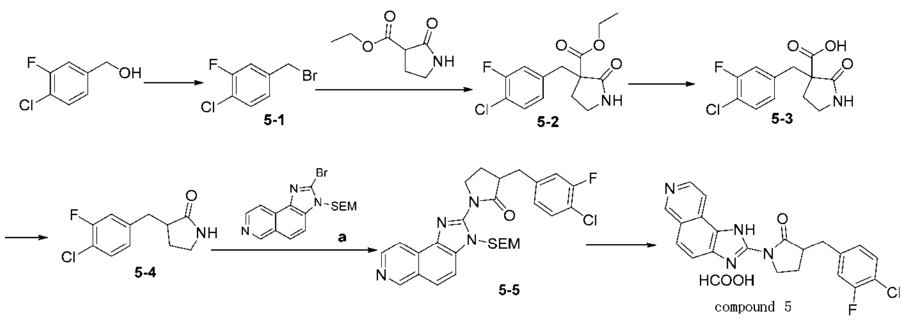


Step 1: 3-fluoro-4-chlorobenzyl alcohol (2.0 g, 12.46 mmol, Adamas, batch No. P2138624) was dissolved in dichloromethane (20 mL) at room temperature, and cooled in an ice-water bath, and then triphenylphosphine (3.92 g, 14.95 mmol) and carbon tetrabromide (4.96 g, 14.95 mmol) were slowly added and the reaction solution was stirred at room temperature for 3 h. TLC monitored that the raw material was completely reacted. The reaction solution was concentrated and purified by silica gel column chromatography (ethyl acetate/petroleum ether (0/100 (v/v) to 10/90 (v/v)) to obtain intermediate 5-1. $^1$H NMR (400 MHz, $CDCl_3$) δ 7.37 (t, *J* = 7.9 Hz, 1H), 7.19 (dd, *J* = 9.5, 2.0 Hz, 1H), 7.12 (dd, *J* = 8.2, 1.6 Hz, 1H), 4.42 (s, 2H).

Step 2: Ethyl 2-oxopyrrolidin-3-carboxylate (500 mg, 3.18 mmol, Bide Pharmatech, batch No. CNZ287) was dissolved in ethanol (10 mL) at room temperature, sodium ethoxide (1.24 mL, 3.18 mmol) was added, after stirred at room temperature for 15 minutes, intermediate 5-1 (711 mg, 3.18 mmol) was added, and the reaction solution was stirred at room temperature overnight. LC-MS monitored that the raw material was completely reacted. The reaction solution was quenched by adding saturated aqueous ammonium chloride solution, then was extracted with water (20 mL) and ethyl acetate (20 mL×2), and the organic phase was washed with saturated brine (20 mL×2), and then dried over anhydrous sodium sulfate, and filtered, and the filtrate was concentrated and purified by silica gel column chromatography (methanol/dichloromethane (0/100 (v/v) to 3/97 (v/v)) to obtain intermediate 5-2. LC-MS (ESI): m/z =300.2 [M+H]$^+$. $^1$H NMR (400 MHz, DMSO-$d_6$) δ 8.03 (s, 1H), 7.50 (t, *J* = 8.1 Hz, 1H), 7.26 (dd, *J* = 10.7, 1.8 Hz, 1H), 7.10 (dd, *J* = 8.3, 1.5 Hz,

1H), 4.21 - 4.02 (m, 2H), 3.20 - 3.08 (m, 2H), 3.08 - 2.95 (m, 1H), 2.89 - 2.70 (m, 1H), 2.36 - 2.26 (m, 1H), 2.07 - 1.98 (m, 1H), 1.25 - 1.09 (m, 3H).

Step 3: Intermediate 5-2 (800 mg, 2.67 mmol) was dissolved in ethanol (10 mL) and water (5 mL) at room temperature and cooled to 0°C, and sodium hydroxide (641 mg, 16.01 mmol) was added, and the reaction solution was stirred at room temperature for 3 h. LC-MS monitored that the raw material was completely reacted. The reaction solution was concentrated to remove ethanol, and the pH was adjusted to 4 with 1 mol/L hydrochloric acid, then water (30 mL) and ethyl acetate (30 mL×2) were added for extraction, and the organic phase was washed with saturated brine (30 mL), dried over anhydrous sodium sulfate, and filtered, and the filtrate was concentrated to obtain intermediate 5-3. LC-MS (ESI): m/z =272.0 $[M+H]^+$.

Step 4: Intermediate 5-3 (600 mg, 2.21 mmol) was dissolved in ethyl acetate (10 mL) at room temperature, heated to 90 °C, refluxed with stirring for 3 h. LC-MS monitored that the raw material was completed reacted. The reaction solution was cooled to room temperature and concentrated to obtain intermediate 5-4. LC-MS (ESI): m/z =228.0 $[M+H]^+$. $^1$H NMR (400 MHz, DMSO-$d_6$) δ 7.67 (s, 1H), 7.49 (t, *J* = 8.1 Hz, 1H), 7.32 (dd, *J* = 10.7, 1.7 Hz, 1H), 7.12 (dd, *J* = 8.2, 1.2 Hz, 1H), 3.14 - 3.04 (m, 2H), 3.03 - 2.94 (m, 1H), 2.62 - 2.53 (m, 2H), 2.04 - 1.91 (m, 1H), 1.70 - 1.54 (m, 1H).

Step 5: Intermediate 5-4 (57 mg, 0.25 mmol) and intermediate a (80 mg, 0.21 mmol) were placed in a microwave tube at room temperature and dissolved by adding 1,4-dioxane (2 mL), and cesium carbonate (172 mg, 0.53 mmol), Xantphos (49 mg, 0.08 mmol), and tris(dibenzylideneacetone)dipalladium (39 mg, 0.04 mmol) were added. After nitrogen displacement, the reaction solution was heated to 130 °C with a microwave synthesizer for 60 min. LC-MS monitored that the raw material was completely reacted. The reaction solution was extracted with water (30 mL) and ethyl acetate (30 mL×2), and the organic phase was washed with saturated brine (30 mL×2), dried over anhydrous sodium sulfate, and filtered, and the filtrate was concentrated and purified by silica gel column chromatography (methanol/ dichloromethane (0/100 (v/v) to 3/97 (v/v))) to obtain intermediate 5-5. LC-MS (ESI): m/z =525.2 $[M+H]^+$.

Step 6: Intermediate 5-5 (230 mg, 0.44 mmol) was dissolved in dichloromethane (2 mL) and trifluoroacetic acid (1 mL) at room temperature, and the reaction solution was stirred at room temperature for 1 h. LC-MS monitored that the raw material was completely reacted. The reaction solution was concentrated to obtain concentrated product A, which was then dissolved in DMF and purified by HPLC (Phase A: 0.1% FA in $H_2O$, Phase B: ACN) to obtain compound 5. LC-MS (ESI): m/z =395.0 $[M+H]^+$. $^1$H NMR (400 MHz, DMSO-$d_6$) δ 12.92 (s, 1H), 9.41 (s, 1H), 8.55 (d, *J* = 5.9 Hz, 1H), 8.26 (s, 1H), 8.00 - 7.90 (m, 2H), 7.56 - 7.50 (m, 1H), 7.44 - 7.38 (m, 1H), 7.20 (dd, *J* = 8.3, 2.0 Hz, 1H), 4.18 - 4.09 (m, 1H), 4.02-3.92 (m, 1H), 3.25 - 3.16 (m, 2H), 2.91 - 2.81 (m, 1H), 2.27 - 2.18 (m, 1H), 1.99 - 1.87 (m, 1H).

Example 6 Preparation of Compound 6

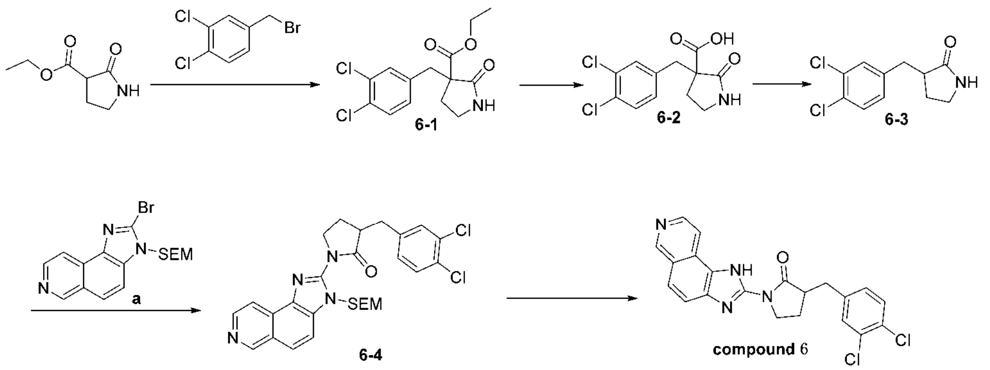

6-1 6-2 6-3 a 6-4 compound 6

Step 1: Ethyl 2-oxopyrrolidin-3-carboxylate (500 mg, 3.18 mmol, Bide Pharmatech, Batch No.: CNZ287) was dissolved in ethanol (10 mL), sodium ethoxide (1.24 mL, 3.18 mmol) was added, and the mixture was stirred at room temperature for 15 min, then 3,4-dichlorobenzyl bromide (763 mg, 3.18 mmol) was added. The reaction solution was stirred at room temperature overnight. LC-MS monitored that the raw material was completely reacted. The reaction solution was quenched by saturated aqueous ammonium chloride solution, and then was extracted by water (20 mL) and ethyl acetate (20 mL×2), the organic phase was washed with saturated brine (20 mL×2), and then dried over anhydrous sodium sulfate, and filtered, and the filtrate was concentrated and purified by silica gel column chromatography (methanol/dichloromethane (0/100 (v/v) to 3/97 (v/v)) to obtain intermediate 6-1. LC-MS (ESI): m/z =316.0 $[M+H]^+$. $^1$H NMR (400 MHz, $CDCl_3$) δ 7.34 (dd, *J* = 5.1, 3.1 Hz, 2H), 7.09 (dd, *J* = 8.3, 2.0 Hz, 1H), 6.27 (s, 1H), 4.23 (q, *J* = 7.1 Hz, 2H), 3.44 - 3.33 (m, 1H), 3.25 - 3.14 (m, 2H), 3.04 - 2.94 (m, 1H), 2.55 - 2.46 (m, 1H), 2.16 - 2.02 (m, 1H), 1.29 (t, *J* = 7.1 Hz, 3H).

Step 2: Intermediate 6-1 (820 mg, 2.59 mmol) was dissolved in ethanol (10 mL) and water (5 mL) at room

temperature and cooled to 0°C, and sodium hydroxide (519 mg, 12.97 mmol) was added, and the reaction solution was stirred at room temperature for 3 h. LC-MS monitored that the raw material was completely reacted. The reaction solution was concentrated to remove ethanol, and the pH was adjusted to 4 with 1 mol/L hydrochloric acid, then water (30 mL) and ethyl acetate (30 mL×2) were added for extraction, and the organic phase was washed with saturated brine (30 mL), dried over anhydrous sodium sulfate, and filtered, and the filtrate was concentrated to obtain the intermediate 6-2. LC-MS (ESI): m/z =288.0 $[M+H]^+$.

Step 3: Intermediate 6-2 (730 mg, 2.53 mmol) was dissolved in ethyl acetate (10 mL) at room temperature, heated to 90 °C, refluxed with stirring for 3 h. LC-MS monitored that the raw material was completely reacted. The reaction solution was cooled to room temperature and concentrated to obtain a crude intermediate 6-3. LC-MS (ESI): m/z =244.0 $[M+H]^+$. $^1$H NMR (400 MHz, DMSO-$d_6$) δ 7.67 (s, 1H), 7.54 (d, *J* = 8.4 Hz, 2H), 7.25 (dd, *J* = 8.2, 1.9 Hz, 1H), 3.13 - 3.01 (m, 2H), 3.01 - 2.92 (m, 1H), 2.64 - 2.53 (m, 2H), 2.02 - 1.90 (m, 1H), 1.69 - 1.55 (m, 1H).

Step 4: Intermediate a (100 mg, 0.26 mmol) and intermediate 6-3 (77 mg, 0.32 mmol) were placed in a microwave tube at room temperature and dissolved by adding 1,4-dioxane (2 mL), cesium carbonate (215 mg, 0.66 mmol), Xantphos (61 mg, 0.11 mmol), and tris(dibenzylideneacetone)dipalladium (48 mg, 0.05 mmol) were added. After nitrogen displacement, the reaction solution was heated to 130 °C with a microwave synthesizer for 60 min. LC-MS monitored that the raw material was completely reacted. The reaction solution was extracted with water (30 mL) and ethyl acetate (30 mL×2), and the organic phase was washed with saturated brine (30 mL×2), dried over anhydrous sodium sulfate, and filtered, and the filtrate was concentrated and purified by silica gel column chromatography (methanol/dichloromethane (0/100 (v/v) to 3/97 (v/v)) to obtain intermediate 6-4. LC-MS (ESI): m/z =541.2 $[M+H]^+$.

Step 5: Intermediate 6-4 (220 mg, 0.41 mmol) was dissolved in dichloromethane (2 mL) and trifluoroacetic acid (1 mL) at room temperature, and the reaction solution was stirred at room temperature for 1 h. LC-MS monitored that the raw material was completely reacted. The reaction solution was concentrated, then dissolved in DMF and purified by HPLC (phase A: 0.1% FA in $H_2O$, phase B: ACN) to obtain compound 6. LC-MS (ESI): m/z =411.0 $[M+H]^+$. $^1$H NMR (400 MHz, DMSO-$d_6$) δ 12.94 (br s, 1H), 9.42 (s, 1H), 8.55 (d, *J* = 5.9 Hz, 1H), 8.27 (s, 1H), 8.00 - 7.88 (m, 2H), 7.64 (d, *J* = 1.6 Hz, 1H), 7.59 (d, *J* = 8.2 Hz, 1H), 7.39 - 7.29 (m, 1H), 4.20 - 4.08 (m, 1H), 4.02 - 3.91 (m, 1H), 3.23 - 3.13 (m, 2H), 2.91 - 2.81 (m, 1H), 2.28 - 2.17 (m, 1H), 2.00 - 1.86 (m, 1H).

Example 7 Preparation of Compound 7

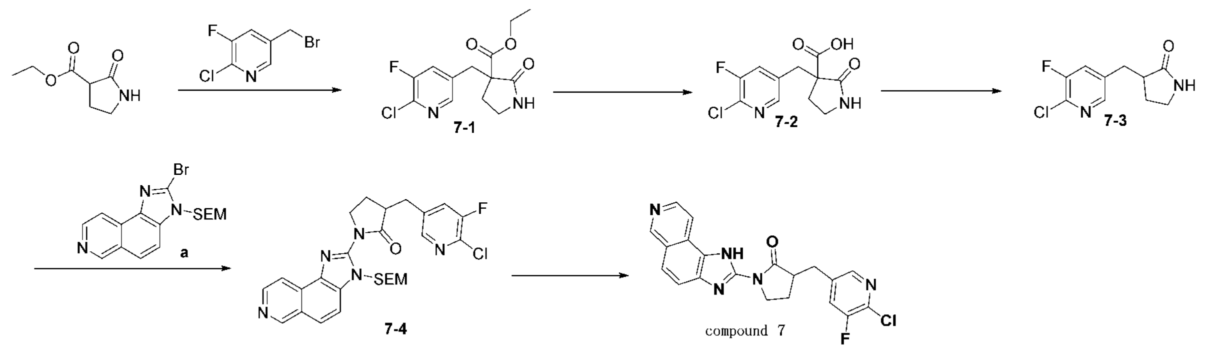


Step 1: Ethyl 2-oxopyrrolidin-3-carboxylate (500 mg, 3.18 mmol, Bide Pharmatech, Batch No.: CNZ287) was dissolved in ethanol (10 mL) at room temperature, sodium ethoxide (1.24 mL, 3.18 mmol) was added, and stirred at room temperature for 15 min, then 5-bromomethyl-2-chloro-3-fluoropyridine (714 mg, 3.18 mmol) was added, and the reaction solution was stirred at room temperature overnight. LC-MS monitored that the raw material was completely reacted. The reaction solution was quenched by adding saturated aqueous ammonium chloride solution, and then extracted by water (20 mL) and ethyl acetate (20 mL×2), the organic phase was washed with saturated brine (20 mL×2), and then dried over anhydrous sodium sulfate, and filtered, and the filtrate was concentrated and purified by silica gel column chromatography (methanol/dichloromethane (0/100 (v/v) to 3/97 (v/v)) to obtain intermediate 7-1. LC-MS (ESI): m/z =301.0 $[M+H]^+$.

Step 2: Intermediate 7-1 (580 mg, 1.93 mmol) was dissolved in ethanol (10 mL) and water (5 mL) at room temperature and cooled to 0°C, and sodium hydroxide (309 mg, 7.72 mmol) was added, and the reaction solution was stirred at room temperature for 3 h. LC-MS monitored that the raw material was completely reacted. The reaction solution was concentrated to remove ethanol, and the pH was adjusted to 4 with 1 mol/L hydrochloric acid, then water (30 mL) and ethyl acetate (30 mL×2) were added for extraction, and the organic phase was washed with saturated brine (30 mL), dried over anhydrous sodium sulfate, and filtered, and the filtrate was concentrated to obtain intermediate 7-2. LC-MS (ESI): m/z =273.0 $[M+H]^+$.

Step 3: Intermediate 7-2 (450 mg, 1.65 mmol) was dissolved in ethyl acetate (10 mL) at room temperature, heated to 100 °C, refluxed with stirring overnight. LC-MS monitored that the raw material was completely reacted. The reaction solution was cooled to room temperature, concentrated and purified by silica gel column chromatography (with methanol/dichloromethane (0/100 (v/v) to 3/97 (v/v))) to obtain intermediate 7-3. LC-MS (ESI): m/z =229.0 $[M+H]^+$. $^1H$ NMR (400 MHz, DMSO-$d_6$) δ 8.19 (s, 1H), 7.89 (dd, *J* = 9.8, 1.8 Hz, 1H), 7.68 (s, 1H), 3.13 - 3.07 (m, 2H), 3.07 - 2.97 (m, 1H), 2.69 - 2.57 (m, 2H), 2.07 - 1.96 (m, 1H), 1.71 - 1.59 (m, 1H). Step 4: Intermediate a (100 mg, 0.26 mmol) and intermediate 7-3 (73 mg, 0.32 mmol) were placed in a microwave tube at room temperature and dissolved by adding 1,4-dioxane (2 mL), and cesium carbonate (215 mg, 0.66 mmol), Xantphos (61 mg, 0.11 mmol), and tris(dibenzylideneacetone)dipalladium (48 mg, 0.05 mmol) were added. After nitrogen displacement, the reaction solution was heated to 130 °C with a microwave synthesizer for 60 min. LC-MS monitored that the raw material was completely reacted. The reaction solution was extracted with water (30 mL) and ethyl acetate (30 mL×2), and the organic phase was washed with saturated brine (30 mL×2), dried over anhydrous sodium sulfate, and filtered, and the filtrate was concentrated and purified by silica gel column chromatography (methanol/dichloromethane (0/100 (v/v) to 3/97 (v/v)) to obtain intermediate 7-4. LC-MS (ESI): m/z =526.2 $[M+H]^+$.

Step 5: Intermediate 7-4 (60 mg, 0.11 mmol) was dissolved in dichloromethane (2 mL) and trifluoroacetic acid (1 mL) at room temperature, and the reaction solution was stirred at room temperature for 1 h. LC-MS monitored that the raw material was completely reacted. The reaction solution was concentrated, then dissolved in DMF and purified by HPLC (phase A: 0.1% FA in $H_2O$, phase B: ACN) to obtain compound 7. LC-MS (ESI): m/z =396.0 $[M+H]^+$. $^1H$ NMR (400 MHz, DMSO-$d_6$) δ 12.81 (s, 1H), 9.28 (s, 1H), 8.52 (d, *J* = 5.6 Hz, 1H), 8.29 (s, 1H), 8.13 (d, *J* = 5.8 Hz, 1H), 8.00 (d, *J* = 8.4 Hz, 1H), 7.91 - 7.79 (m, 2H), 4.22 - 4.11 (m, 1H), 4.02-3.92 (m, 1H), 3.30 - 3.18 (m, 2H), 2.98-2.89 (m, 1H), 2.31 - 2.21 (m, 1H), 2.01 - 1.90 (m, 1H).

Example 8 Preparation of Compound 8

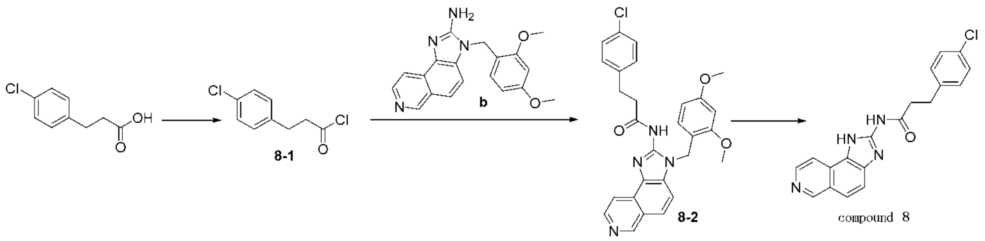

Step 1: 3-(4-chlorophenyl)propionic acid (500 mg, 2.71 mmol, Bide Pharmatech, batch No. CNZ584) was dissolved in dichloromethane (10 mL) at room temperature and cooled to 0 °C, and oxalyl chloride (1.16 mL, 13.54 mmol) was added, then was warmed to room temperature and stirred for 2 h. TLC monitored that the raw material was completely reacted. The reaction solution was concentrated to obtain intermediate 8-1.

Step 2: Intermediate b (100 mg, 0.30 mmol) was dissolved in DMF (3 mL) at room temperature and cooled to 0 °C, sodium hydride (18 mg, 0.45 mmol, 60% purity) was added and stirred for 30 min, intermediate 8-1 (73 mg, 0.36 mmol) was added, and then the reaction solution was warmed to room temperature and stirred for 3 hours. LC-MS monitored that the raw material was completely reacted. The reaction solution was quenched by adding saturated aqueous ammonium chloride solution, then extracted by water (20 mL) and ethyl acetate (20 mL × 2), the organic phase was washed with saturated brine (20 mL x 2), and then dried over anhydrous sodium sulfate, and filtered, and the filtrate was concentrated and purified by silica gel column chromatography (methanol/dichloromethane (0/100 (v/v) to 5/95 (v/v)) to obtain intermediate 8-2. LC-MS (ESI): m/z =501.0 $[M+H]^+$.

Step 3: Intermediate 8-2 (60 mg, 0.12 mmol) was dissolved in 1,2-dichloroethane (3 mL) and trifluoroacetic acid (1 mL) at room temperature, heated to 60 °C and stirred for 0.5 h. LC-MS monitored that the raw material was completely reacted. The reaction solution was concentrated, then dissolved in DMF and purified by HPLC (phase A: 0.1% FA in $H_2O$, phase B: ACN) to obtain compound 8. LC-MS (ESI): m/z =351.2 $[M+H]^+$. $^1H$ NMR (400 MHz, DMSO-$d_6$) δ 12.95 (s, 1H), 12.00 (s, 1H), 9.58 (s, 1H), 8.60 (d, *J* = 6.2 Hz, 1H), 8.33 (s, 1H), 8.02 (s, 2H), 7.40 - 7.30 (m, 4H), 2.98 (t, *J* = 7.4 Hz, 2H), 2.83 (t, *J* = 7.4 Hz, 2H).

Example 9 Preparation of Compound 9

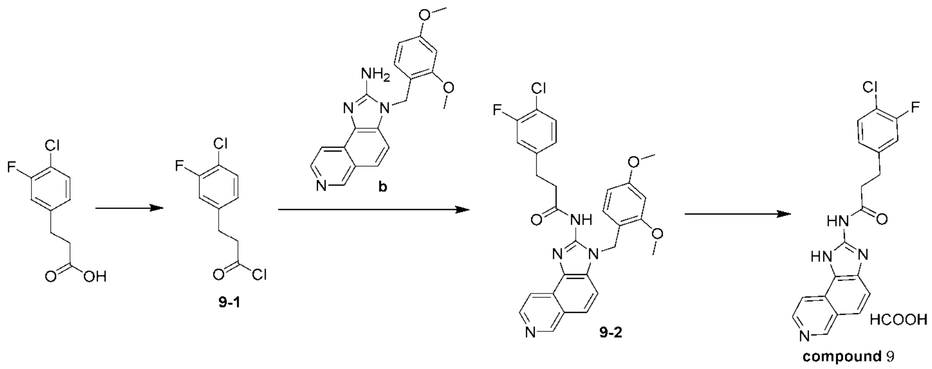


Step 1: 3-(4-chloro-3-fluorophenyl)propionic acid (200 mg, 0.99 mmol, Bide, batch No) CQY874) and oxalyl chloride (626.43 mg, 4.94 mmol) were added to a solution of dichloromethane (5 mL) at room temperature and stirred for 2 h. TLC (ethyl acetate/petroleum ether = 20/1) monitored that the raw material was completely reacted. The reaction solution was concentrated under reduced pressure to obtain intermediate 9-1.

Step 2: Intermediate b (100 mg, 0.30 mmol) was added to N,N-dimethylformamide (2 mL) solution in an ice bath, then sodium hydride (23.92 mg, 0.60 mmol, 60% purity) was added slowly, and the reaction solution was stirred at 0°C for 0.5 h. Then intermediate 9-1 was added slowly dropwise to the reaction system, and the final reaction solution was stirred at room temperature for 2 h. LC-MS monitored that the raw material was completely reacted. Water (10 mL) was added slowly in an ice bath, then extracted with ethyl acetate (20 mL×3), and the organic phase was concentrated in vacuo to obtain a crude product. The crude product was purified by silica gel column chromatography (methanol/dichloromethane (1/100 (v/v) to 1/10 (v/v)) to obtain intermediate 9-2. LC-MS (ESI): m/z =519.2$[M+H]^+$.

Step 3: Intermediate 9-2 (79.37 mg, 0.20 mmol) was added to a solution of 1,2-dichloroethane (2 mL) and TFA (1 mL) at room temperature, and the reaction solution was heated to 60°C and stirred for 1 h. LC-MS monitored that the raw material was completely reacted. The reaction solution was purified by HPLC under acidic conditions (Phase A: 0.1% FA/$H_2O$ Phase B: ACN) to obtain compound 9. LC-MS (ESI): m/z =369.0$[M+H]^+$. $^1$H NMR (400 MHz, DMSO-$d_6$) δ 12.66 (s, 1H), 11.90 (s, 1H), 9.28 (s, 1H), 8.52 (d, *J* = 5.7 Hz, 1H), 8.14 (s, 1H), 8.06 (d, *J* = 5.7 Hz, 1H), 7.88 - 7.76 (m, 2H), 7.54 -7.49 (m, 1H), 7.41 - 7.33 (m, 1H), 7.20 - 7.14 (m, 1H), 3.00 (t, *J* = 7.3 Hz, 2H), 2.83 (t, *J* = 7.4 Hz, 2H).

Example 10 Preparation of Compound 10

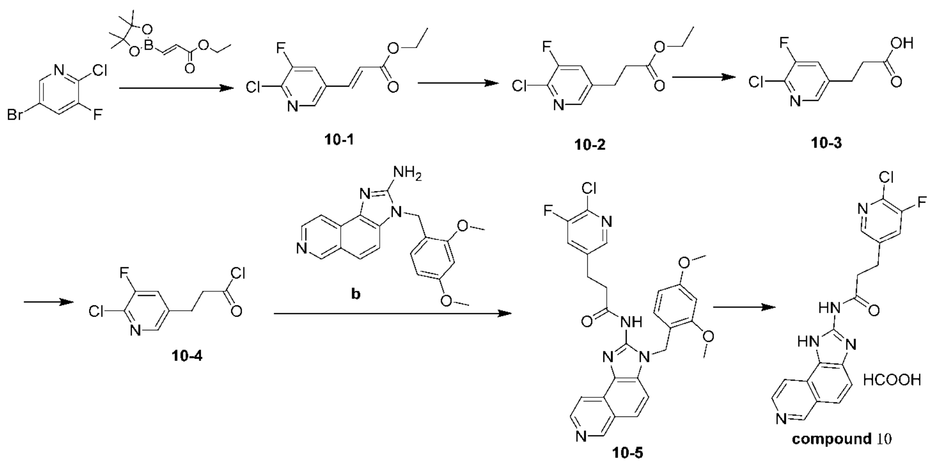


Step 1: 5-Bromo-2-chloro-3-fluoropyridine (200 mg, 0.95 mmol, Bide, batch No. DLY954), ethyl (E)-3-(4,4,5,5-tetramethyl-1,3,2-dioxaborolan-2-yl)acrylate (644.62 mg, 2.85 mmol, Bide, batch No. CGU883), [1,1'-bis(di-*tert*-butylphosphino)ferrocene]palladium dichloride (61.37 mg, 0.1 mmol) and potassium phosphate (605.22 mg, 2.85 mmol) were added to a solution of toluene (10 mL) and water (0.5 mL) at room temperature. The atmosphere was purged with nitrogen three times and the reaction was heated to 80°C under microwave and stirred for 40 min. LC-MS monitored that the raw material was completely reacted. After the reaction was cooled to room temperature, ethyl acetate (50 mL) was added, washed with water (50 mL×2), washed with saturated brine (100 mL), and the organic phase was collected. The organic

phase was concentrated under reduced pressure to obtain a crude product. The crude product was purified by silica gel column chromatography (ethyl acetate/petroleum ether (1/100 (v/v) to 1/8 (v/v))) to obtain intermediate 10-1. LC-MS (ESI): m/z =230.0[M+H]$^+$.

Step 2: Intermediate 10-1 (700 mg, 3.05 mmol) was added to anhydrous tetrahydrofuran (20 mL) solution at room temperature, then rhodium/carbon (31 mg, 9.15 mmol) was added, and the reaction was stirred under hydrogen atmosphere at room temperature for 2 h. LC-MS monitored that the raw material was completely reacted. The reaction solution was filtered and the filtrate was concentrated under vacuum to obtain a crude intermediate 10-2. LC-MS (ESI): m/z =232.0[M+H]$^+$.

Step 3: Intermediate 10-2 (500 mg, 2.16 mmol) and LiOH (271.70 mg, 6.48 mmol) were added to a solution of ethanol (5 mL), water (5 mL) and tetrahydrofuran (1.5 mL) at room temperature. The reaction mixture was stirred at room temperature for 1 h. LC-MS monitored that the raw material was completely reacted. The reaction solution was diluted with water (30 mL) and extracted with ethyl acetate (50 mL × 3), the organic layer was separated, and the organic phase was washed with saturated NaCl solution and then concentrated in vacuo, purified by silica gel column chromatography (ethyl acetate/petroleum ether (1/100 (v/v) to 1/1 (v/v)) to obtain intermediate 10-3. LC-MS (ESI): m/z =204.0[M+H]$^+$.

Step 4: Intermediate 10-3 (200 mg, 0.99 mmol) and oxalyl chloride (626.43 mg, 4.94 mmol) were added to dichloromethane (5 mL) solution at room temperature and stirred for 2 h. TLC (ethyl acetate/petroleum ether = 20/1 (v/v)) monitored that the raw material was completely reacted, and the reaction solution was directly concentrated under reduced pressure to obtain a crude intermediate 10-4.

Step 5: Intermediate b (100 mg, 0.30 mmol) was added to N,N-dimethylformamide (2 mL) solution in an ice bath, sodium hydride (23.92 mg, 0.60 mmol, 60% purity) was added slowly, and the reaction solution was stirred at 0°C for 0.5 h. Intermediate 10-4 was then added to the reaction system, and the final reaction solution was stirred at room temperature for 2 h. LC-MS monitored that the raw material was completely reacted. Water (10 mL) was added to the reaction solution slowly in an ice bath, then extracted with ethyl acetate (20 mL×3), and the organic phase was concentrated in vacuo to obtain a crude product. The crude product was purified by silica gel column chromatography (methanol/dichloromethane (1/100 (v/v) to 1/10 (v/v)) to obtain intermediate 10-5. LC-MS (ESI): m/z =520.0[M+H]$^+$.

Step 6: Intermediate 10-5 (79.37 mg, 0.20 mmol) was added to a solution of 1,2-dichloroethane (2 mL) and TFA (1 mL) at room temperature, and the reaction solution was heated to 60°C and stirred for 1 h. LC-MS monitored that the raw material was completely reacted. The reaction solution was purified by HPLC under acidic conditions (Phase A: 0.1% FA/$H_2O$ Phase B: ACN) to obtain compound 10. LC-MS (ESI): m/z =370.1[M+H]$^+$. $^1H$ NMR (400 MHz, DMSO-$d_6$) δ 12.64 (br s, 1H), 11.89 (br s, 1H), 9.27 (s, 1H), 8.52 (d, *J* = 5.7 Hz, 1H), 8.30 - 8.18 (m, 2H), 8.08 (brs, 1H), 7.95 (dd, *J* = 9.7, 1.7 Hz, 1H), 7.87 - 7.75 (m, 2H), 3.06 (t, *J* = 7.4 Hz, 2H), 2.88 (t, *J* = 7.3 Hz, 2H).

Example 11 Preparation of Compound 11

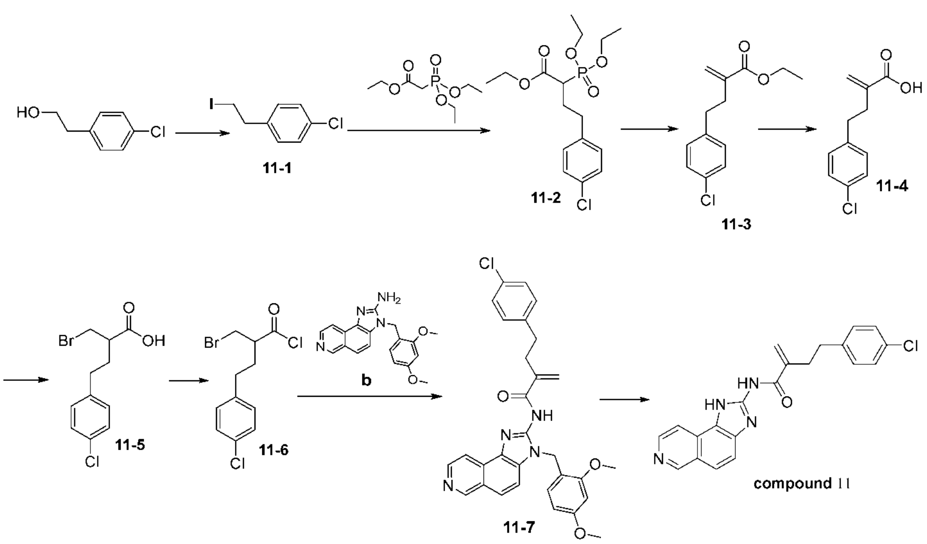

Step 1: 2-(4-Chlorophenyl)ethan-1-ol (5.00 g, 31.93 mmol, Bide, batch No. CMZ071) was dissolved in dichloromethane (50 mL) at room temperature, and triphenylphosphine (9.21 g, 35.12 mmol), imidazole (2.61 g, 38.31 mmol), and iodine (8.91 g, 35.12 mmol) were added in an ice water bath, stirred at room temperature for 2 h. TLC monitored that the raw material was completely reacted. Water (50 mL) and dichloromethane (50 mL × 2) were added to the reaction

solution for extraction, and the organic phase was washed with aqueous sodium thiosulfate (50 mL × 2) and saturated brine (50 mL × 5), and then dried over anhydrous sodium sulfate and filtered. The filtrate was concentrated and purified by silica gel column chromatography (ethyl acetate/petroleum ether (0/100 (v/v)-10/90 (v/v)) to obtain intermediate 11-1. $^1$H NMR (400 MHz, DMSO-$d_6$) δ 7.37 (d, *J* = 8.3 Hz, 2H), 7.29 (d, *J* = 8.4 Hz, 2H), 3.46 (t, *J* = 7.3 Hz, 2H), 3.12 (t, *J* = 7.3 Hz, 2H).

Step 2: Triethyl phosphonoacetate (3.00 g, 13.38 mmol, Bide, batch No. DLY849) was dissolved in tetrahydrofuran (40 mL) at room temperature, cooled to 0 °C, potassium *tert*-butoxide (1.95 g, 17.40 mmol) was added and stirred for 0.5 h. Intermediate 11-1 (3.74 g, 14.05 mmol) was added. The reaction solution was then warmed to room temperature and stirred for 3 h. LC-MS monitored that the raw material was completely reacted. The reaction solution was quenched by adding saturated aqueous ammonium chloride, extracted with water (40 mL) and ethyl acetate (40 mL×2), the organic phase was washed with saturated brine (30 mL×2), dried over anhydrous sodium sulfate, and filtered, and the filtrate was concentrated and purified by silica gel column chromatography (ethyl acetate/petroleum ether (0/100 (v/v)-30/70 (v/v)) to obtain intermediate 11-2. LC-MS (ESI): m/z =363.1 [M+H]$^+$. $^1$H NMR (400 MHz, $CDCl_3$) δ 7.28 - 7.25 (m, 2H), 7.18 - 7.08 (m, 2H), 4.30 - 4.07 (m, 6H), 3.02 - 2.87 (m, 1H), 2.78 - 2.67 (m, 1H), 2.65 - 2.52 (m, 1H), 2.38 - 2.23 (m, 1H), 2.22 - 2.06 (m, 1H), 1.40 - 1.27 (m, 9H).

Step 3: Intermediate 11-2 (2.60 g, 7.17 mmol) was dissolved in tetrahydrofuran (20 mL) and water (20 mL) at room temperature, potassium carbonate (2.97 g, 21.50 mmol) and formaldehyde aqueous solution (5.82 g, 71.67 mmol) were added, and the reaction solution was heated to 70 °C and stirred for 3 h. LC-MS monitored that the raw material was completely reacted. The reaction solution was extracted by water (20 mL) and ethyl acetate (30 mL×2), and the organic phase was washed with saturated brine (20 mL×2), then dried over anhydrous sodium sulfate, and filtered, and the filtrate was concentrated and purified by silica gel column chromatography (ethyl acetate/petroleum ether (0/100 (v/v)-20/80 (v/v)) to obtain intermediate 11-3. LC-MS (ESI): m/z =239.2 [M+H]$^+$. $^1$H NMR (400 MHz, $CDCl_3$) δ 7.21 - 7.11 (m, 2H), 7.08 - 6.97 (m, 2H), 6.08 (d, *J* = 1.2 Hz, 1H), 5.40 (dd, *J* = 2.5, 1.2 Hz, 1H), 4.15 (q, *J* = 7.1 Hz, 2H), 2.69 (dd, *J* = 9.1, 6.5 Hz, 2H), 2.51 (dd, *J* = 9.2, 6.4 Hz, 2H), 1.24 (t, *J* = 7.1 Hz, 3H).

Step 4: Intermediate 11-3 (1.40 g, 5.86 mmol) was dissolved in ethanol (20 mL) and water (10 mL) at room temperature and cooled to 0 °C, and lithium hydroxide (0.70 g, 29.32 mmol) was added, and then the reaction solution was warmed to room temperature and stirred for 3 h. LC-MS monitored that the raw material was completely reacted. The reaction solution was concentrated to remove ethanol, and the pH was adjusted to 4 with 1 mol/L hydrochloric acid, then water (20 mL) and ethyl acetate (20 mL×2) were added for extraction, and the organic phase was washed with saturated brine (20 mL×2), dried over anhydrous sodium sulfate, and filtered, and the filtrate was concentrated to obtain the intermediate 11-4. LC-MS (ESI): m/z =209.0 [M-H]$^-$. $^1$H NMR (400 MHz, DMSO-$d_6$) δ 12.51 (s, 1H), 7.36 - 7.29 (m, 2H), 7.22 (d, *J* = 8.4 Hz, 2H), 6.03 (d, *J* = 1.4 Hz, 1H), 5.55 (d, *J* = 1.3 Hz, 1H), 2.82 - 2.65 (m, 2H), 2.52 - 2.48 (m, 2H).

Step 5: Intermediate 11-4 (500 mg, 2.37 mmol) was dissolved in hydrobromic acid (10 mL, 33%wt in AcOH) at room temperature and the reaction solution was stirred at room temperature for 3 h. LC-MS monitored that the raw material was completely reacted. The reaction solution was concentrated to obtain intermediate 11-5. $^1$H NMR (400 MHz, DMSO-$d_6$) δ 12.65 (s, 1H), 7.38 - 7.31 (m, 2H), 7.26 - 7.19 (m, 2H), 3.74 - 3.60 (m, 2H), 2.76 - 2.66 (m, 1H), 2.66 - 2.56 (m, 2H), 1.96 - 1.70 (m, 2H).

Step 6: Intermediate 11-5 (200 mg, 0.69 mmol) was dissolved in dichloromethane (5 mL) at room temperature and cooled in an ice-water bath, and oxalyl chloride (0.29 mL, 3.43 mmol) was added, then the reaction solution was warmed to room temperature and stirred for 3 h. TLC monitored that the raw material was completely reacted. The reaction solution was concentrated to obtain the intermediate 11-6.

Step 7: Intermediate b (100 mg, 0.30 mmol) was dissolved in DMF (3 mL) at room temperature and cooled to 0 °C, sodium hydride (18 mg, 0.45 mmol, 60% purity) was added, stirred for 30 min, then intermediate 11-6 (111 mg, 0.36 mmol) was added, and the reaction solution was heated to 90 °C and stirred for 3 h. LC-MS monitored the reaction was completed. The reaction solution was cooled to room temperature, quenched by adding saturated aqueous ammonium chloride solution, extracted with water (20 mL) and ethyl acetate (20 mL×2), and the organic phase was washed with saturated brine (20 mL×2), dried over anhydrous sodium sulfate, and filtered, and the filtrate was concentrated and purified by silica gel column chromatography (methanol/dichloromethane (0/100 (v/v) to 5/95 (v/v)) to obtain intermediate 11-7. LC-MS (ESI): m/z =527.2 [M+H]$^+$.

Step 8: Intermediate 11-7 (115 mg, 0.22 mmol) was dissolved in 1,2-dichloroethane (2 mL) and trifluoroacetic acid (1 mL) at room temperature, heated to 60 °C and stirred for 0.5 h. LC-MS monitored that the raw material was completely reacted. The reaction solution was concentrated, then dissolved in DMF and purified by HPLC (phase A: 0.1% FA in $H_2O$, phase B: ACN) to obtain compound 11. LC-MS (ESI): m/z =377.0 [M+H]$^+$. $^1$H NMR (400 MHz, DMSO-$d_6$) δ 12.81 (br s, 1H), 11.89 (br s, 1H), 9.29 (s, 1H), 8.53 (d, *J* = 5.7 Hz, 1H), 8.15 (s, 1H), 7.90 - 7.78 (m, 2H), 7.37 - 7.32 (m, 2H), 7.31 - 7.26 (m, 2H), 6.17 (s, 1H), 5.67 (s, 1H), 2.84 - 2.77 (m, 2H), 2.74 - 2.65 (m, 2H).

Examples 12 and 13 Preparation of Compounds 12 and 13

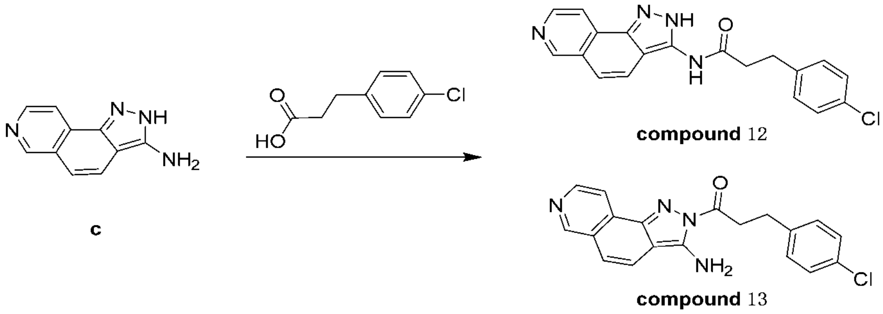

c

compound 12

compound 13

3-(4-chlorophenyl)propionic acid (40 mg, 0.22 mmol, Bide, batch No. CNZ584), intermediate c (48.11 mg, 0.26 mmol), triethylamine (65.92 mg, 0.65 mmol) and HATU (99.09 mg, 0.26 mmol) were added to N,N-dimethylformamide (0.5 mL) solution at room temperature. The reaction solution was stirred at room temperature for 1 hr. The reaction solution was purified by HPLC under alkaline conditions (phase A: 10mM $NH_4HCO_3/H_2O$ phase B: ACN) to obtain compound 12 and compound 13.

Compound 12: LC-MS (ESI): m/z =351.1[M+H]$^+$. $^1$H NMR (400 MHz, DMSO-$d_6$) δ 13.88 (s, 1H), 10.54 (s, 1H), 9.33 (s, 1H), 8.69 (d, $J$ = 5.6 Hz, 1H), 8.23 (d, $J$ = 5.3 Hz, 1H), 7.83 (d, $J$ = 8.8 Hz, 1H), 7.58 (d, $J$ = 8.9 Hz, 1H), 7.36 (m, 4H), 2.98 (t, $J$ = 7.9 Hz, 2H), 2.75 (t, $J$ = 7.5 Hz, 2H).

Compound 13: LC-MS (ESI): m/z =351.1[M+H]$^+$. $^1$H NMR (400 MHz, DMSO-$d_6$) δ 9.06 (s, 1H), 8.57 (d, $J$ = 5.3 Hz, 1H), 8.09 (d, $J$ = 5.3 Hz, 1H), 7.82 - 7.68 (m, 3H), 7.37(s, 4H), 7.08 (d, $J$ = 9.1 Hz, 1H), 3.61 (d, $J$ = 7.5 Hz, 2H), 3.05 (t, $J$ = 7.5 Hz, 2H).

Example 14 Preparation of Compound 14

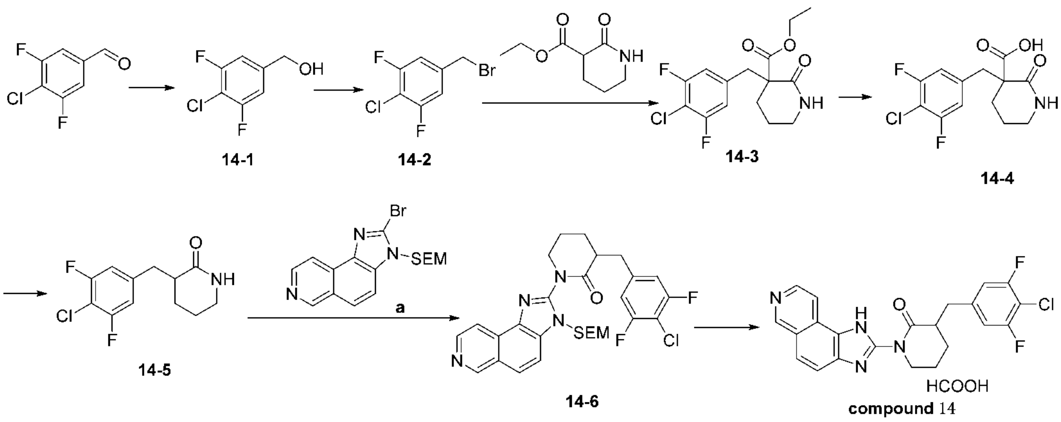

14-1

14-2

14-3

14-4

14-5

a

14-6

compound 14

Step 1: 3,5-difluoro-4-chlorobenzaldehyde (1.0 g, 5.66 mmol, Bide, DQU774) was dissolved in methanol (10 mL) at room temperature, and sodium borohydride (0.21 g, 5.66 mmol) was slowly added at 0~10°C and stirred at 0~10°C for 1 h. LC-MS monitored that the raw material was completely reacted. The reaction solution was extracted by water (30 mL) and ethyl acetate (30 mL×2), the organic phase was washed with saturated brine (30 mL), dried over anhydrous sodium sulfate, and filtered, the filtrate was concentrated to obtain a crude product, and the crude product was purified by silica gel column chromatography (0-20% ethyl acetate/petroleum ether) to obtain intermediate 14-1.

Step 2: Intermediate 14-1 (900 mg, 5.04 mmol) was dissolved in dichloromethane (20 mL) at room temperature and cooled in an ice-water bath and then phosphorus tribromide (2.04 g, 7.56 mmol, Titan, P2169731) was slowly added, stirred at room temperature for 1 h. TLC showed that the raw material was completely reacted. The reaction solution was concentrated to obtain a crude product, and the crude product was purified by silica gel column chromatography (0-5% ethyl acetate/petroleum ether) to obtain intermediate 14-2.

Step 3: Ethyl 2-oxopiperidin-3-carboxylate (921 mg, 3.82 mmol, Bide, CMY580) was dissolved in ethanol (10 mL) at room temperature, sodium ethoxide (432 mg, 6.36 mmol, Energy, WMEZRRRM) was added, and the reaction solution was stirred at room temperature for 15 min, then intermediate 14-2 (500 mg, 3.18 mmol) was added, and the reaction mixture was stirred at room temperature overnight. LC-MS showed that the raw material was completely reacted. The reaction solution was quenched by adding saturated aqueous ammonium chloride solution, and then extracted by water

(30 mL) and ethyl acetate (30 mL×2), the organic phase was washed with saturated brine (30 mL×2), dried over anhydrous sodium sulfate, and filtered. The filtrate was concentrated to obtain a crude product, and the crude product was purified by silica gel column chromatography (0-35% ethyl acetate/petroleum ether) to obtain intermediate 14-3. LC-MS (ESI): m/z =332.2 $[M+H]^+$.

Step 4: Intermediate 14-3 (550 mg, 1.88 mmol) was dissolved in ethanol (10 mL) and water (5 mL) at room temperature, and sodium hydroxide (199 mg, 4.97 mmol, Titan, P2468807) was added, and the reaction solution was stirred at room temperature for 3 h. LC-MS showed that the raw material was completely reacted. The reaction solution was concentrated to remove ethanol, and the pH was adjusted to 4 with 1 mol/L hydrochloric acid, then water (30 mL) and ethyl acetate (30 mL×2) were added for extraction, and the organic phase was washed with saturated brine (30 mL), dried over anhydrous sodium sulfate, and filtered. The filtrate was concentrated to obtain a crude intermediate 14-4. LC-MS (ESI): m/z =304.0 $[M+H]^+$.

Step 5: Intermediate 14-4 (480 mg, 1.58 mmol) was dissolved in ethyl acetate (20 mL) at room temperature, heated to 90 °C, refluxed with stirring for 3 h. LC-MS showed that the raw material was completely reacted. The reaction solution was cooled to room temperature and concentrated to obtain a crude intermediate 14-5. LC-MS (ESI): m/z =260.0 $[M+H]^+$.

Step 6: Intermediate 14-5 (76 mg, 0.29 mmol) and intermediate a (100 mg, 0.26 mmol) were placed in a microwave tube at room temperature and dissolved by adding 1,4-dioxane (4 mL), and cesium carbonate (258 mg, 0.79 mmol, Titan, P2299073), Xantphos (61 mg, 0.11 mmol, Titan, P2168097) and tris(dibenzylideneacetone)dipalladium (48 mg, 0.05 mmol, Titan, P2437515) were added. The reaction solution was purged with nitrogen and then heated to 130 °C with microwave synthesizer for 60 min. LC-MS showed that the raw material was completely reacted. The reaction solution was extracted with water (30 mL) and ethyl acetate (30 mL×2), the organic phase was washed with saturated brine (30 mL×2), dried over anhydrous sodium sulfate, and filtered. The filtrate was concentrated to obtain a crude product and the crude product was purified by silica gel column chromatography (0-60% ethyl acetate/petroleum ether) to obtain intermediate 14-6. LC-MS (ESI): m/z =557.2 $[M+H]^+$.

Step 7: Intermediate 14-6 (100 mg, 0.28 mmol) was dissolved in dichloromethane (2 mL) and trifluoroacetic acid (1 mL) at room temperature, and the reaction solution was stirred at room temperature for 1 h. LC-MS showed that the raw material was completely reacted. The reaction solution was concentrated and the residue was dissolved in DMF and purified by HPLC chromatography (Waters-CORTECS-C18-2.7μm-4.6*30mm, B: 30%-95%, 8 min, A: 0.1% FA in $H_2O$, B: ACN) to obtain compound 14. LC-MS (ESI): m/z =427.2 $[M+H]^+$. $^1H$ NMR (400 MHz, DMSO-$d_6$) δ 13.19-12.89 (m, 1H), 9.30 (s, 1H), 8.53 (d, J = 5.6 Hz, 1H), 8.16 (d, J = 5.7 Hz, 1H), 7.93 - 7.81 (m, 2H), 7.33 (d, J = 8.5 Hz, 2H), 4.30-4.10 (m, 2H), 3.33 - 3.26 (m, 1H), 3.20 - 3.05 (m, 1H), 2.86 (dd, J = 13.8, 8.4 Hz, 1H), 2.05 - 1.81 (m, 3H), 1.62 - 1.50 (m, 1H).

Example 15 Preparation of Compound 15

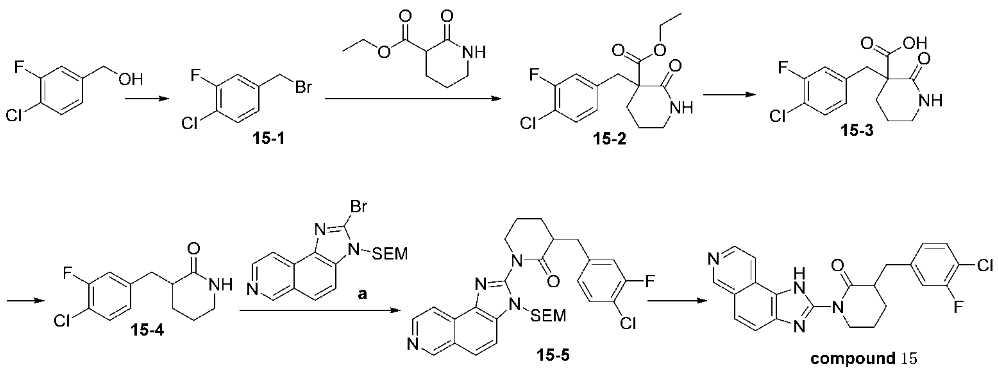
15-1
15-2
15-3
15-4
a
15-5
compound 15

Step 1: 3-Fluoro-4-chlorobenzyl alcohol (1.0 g, 6.23 mmol, Titan, P2138624) was dissolved in dichloromethane (20 mL) at room temperature and cooled in an ice-water bath, and triphenylphosphine (1.96 g, 7.48 mmol, Energy, 06EUGEPR) and carbon tetrabromide (4.96 g, 14.95 mmol, Titan, P2330286) were slowly added, and then the reaction solution was stirred at room temperature for 3 h. TLC showed that the raw material was completely reacted. The reaction solution was concentrated and purified by silica gel column chromatography (petroleum ether/ethyl acetate (100/0 (v/v))) to obtain intermediate 15-1.

Step 2: Ethyl 2-oxopiperidin-3-carboxylate (850 mg, 4.96 mmol, Bide, CMY580) was dissolved in ethanol (10 mL) at room temperature, sodium ethoxide (372 mg, 5.46 mmol, Energy, L0D8REUC) was added, and the reaction solution was stirred at room temperature for 15 min, then intermediate 15-1 (1.10 g, 4.96 mmol) was added, and the reaction

mixture was stirred at room temperature overnight. LC-MS showed that the raw material was completely reacted. The reaction solution was quenched by adding saturated aqueous ammonium chloride solution, then water (30 mL) and ethyl acetate (30 mL × 2) were added for extraction, and the organic phase was washed with saturated brine (30 mL × 2), dried over anhydrous sodium sulfate, and filtered. The filtrate was concentrated and purified by silica gel column chromatography (dichloromethane/methanol (100/0 (v/v) to 95/5 (v/v)) to obtain intermediate 15-2. LC-MS (ESI): m/z =314.0 $[M+H]^+$.

Step 3: Intermediate 15-2 (590 mg, 1.88 mmol) was dissolved in ethanol (10 mL) and water (5 mL) at room temperature and cooled to 0 °C, and then sodium hydroxide (451 mg, 11.28 mmol, Titan, P2468807) was added, the reaction solution was stirred at room temperature for 3 h. LC-MS showed that the raw material was completely reacted. The reaction solution was concentrated to remove ethanol, and the pH was adjusted to 4 with 1 mol/L hydrochloric acid, then water (30 mL) and ethyl acetate (30 mL×2) were added for extraction, and the organic phase was washed with saturated brine (30 mL), dried over anhydrous sodium sulfate, and filtered, and the filtrate was concentrated to obtain a crude intermediate 15-3. LC-MS (ESI): m/z =286.0 $[M+H]^+$.

Step 4: Intermediate 15-3 (490 mg, 1.72 mmol) was dissolved in ethyl acetate (10 mL) at room temperature, heated to 90 °C, refluxed with stirring for 3 h. LC-MS showed that the raw material was completely reacted. The reaction solution was cooled to room temperature and concentrated to obtain a crude intermediate 15-4. LC-MS (ESI): m/z =242.0 $[M+H]^+$.

Step 5: Intermediate 15-4 (77 mg, 0.32 mmol) and intermediate a (100 mg, 0.26 mmol) were placed in a microwave tube at room temperature and dissolved by adding 1,4-dioxane (2 mL), and cesium carbonate (258 mg, 0.79 mmol, Titan, P2299073), Xantphos (61 mg, 0.11 mmol, Titan, P2168097) and tris(dibenzylideneacetone)dipalladium (48 mg, 0.05 mmol, Titan, P2437515) were added. The reaction solution was purged with nitrogen and then heated to 130 °C with a microwave synthesizer for 60 min. LC-MS showed that the raw material was completely reacted. Water (30 mL) and ethyl acetate (30 mL×2) were added to the reaction solution for extraction, and the organic phase was washed with saturated brine (30 mL×2), dried over anhydrous sodium sulfate, and filtered, and the filtrate was concentrated and purified by silica gel column chromatography (dichloromethane/methanol (100/0 (v/v) to 97/3 (v/v)) to obtain intermediate 15-5. LC-MS (ESI): m/z =539.2 $[M+H]^+$.

Step 6: Intermediate 15-5 (150 mg, 0.28 mmol) was dissolved in dichloromethane (2 mL) and trifluoroacetic acid (1 mL) at room temperature, and the reaction solution was stirred at room temperature for 1 h. LC-MS showed that the raw material was completely reacted. The reaction solution was concentrated and the residue was dissolved in DMF and purified by HPLC (Waters-CORTECS-C18-2.7μm-4.6*30mm, B: 30%-95%, 8 min, A: 0.1% FA in $H_2O$, B: ACN) to obtain compound 15. LC-MS (ESI): m/z =409.2 $[M+H]^+$. $^1$H NMR (400 MHz, DMSO-$d_6$) δ 13.00 (s, 1H), 9.34 (s, 1H), 8.54 (d, J = 5.8 Hz, 1H), 8.20 (d, J = 5.6 Hz, 1H), 7.97-7.83 (m, 2H), 7.53 (t, J = 8.1 Hz, 1H), 7.40 (dd, J = 10.7, 1.7 Hz, 1H), 7.19 (dd, J = 8.2, 1.5 Hz, 1H), 4.28 - 4.12 (m, 2H), 3.32 - 3.27 (m, 1H), 3.14 - 3.02 (m, 1H), 2.86 (dd, J = 13.8, 8.6 Hz, 1H), 2.06 - 1.78 (m, 3H), 1.62 - 1.49 (m, 1H).

Example 16-17 Preparation of Compounds 16-17

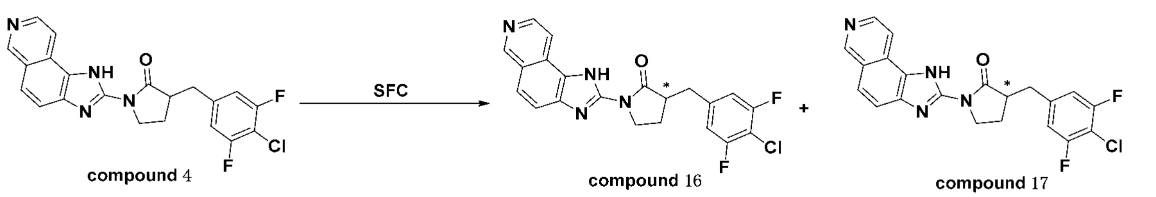


compound 4 compound 16 + compound 17

Step 1: Compound 4 (60 mg) was subjected to chiral resolution (resolution method: Waters UPC2 analytical SFC (SFC-H), ChiralCel OJ, 150×4.6mm I.D., 3μm, Mobile phase: A for $CO_2$ and B for Ethanol (0.05%DEA), Gradient: B 40%, Flow rate: 2.5 mL/min, Back pressure: 100 bar, Column temperature: 35°C, Wavelength: 220nm ,Cycle time: ~4min) to obtain compound 16 (28.54 mg) and compound 17 (18.62 mg).

Compound 16: LC-MS (ESI): m/z =413.0 [M+H] +. Retention time 2.240 min, $^1$H NMR (400 MHz, DMSO-d6) δ 12.84 (s, 1H), 9.28 (s, 1H), 8.52 (d, J = 5.7 Hz, 1H), 8.14 (s, 1H), 7.89-7.81 (m, 2H), 7.36 (d, J = 8.4 Hz, 2H), 4.21 - 4.11 (m, 1H), 3.96 (dd, J = 17.3, 9.2 Hz, 1H), 3.30 - 3.16 (m, 2H), 2.87 (dd, J = 13.6, 8.7 Hz, 1H), 2.30 - 2.17 (m, 1H), 2.00 - 1.87 (m, 1H).

Compound 17: LC-MS (ESI): m/z =413.0 [M+H] +. Retention time 2.875 min, $^1$H NMR (400 MHz, DMSO-d6) δ 12.86 (s, 1H), 9.28 (s, 1H), 8.52 (d, J = 5.7 Hz, 1H), 8.18 (s, 1H), 7.90 - 7.81 (m, 2H), 7.36 (d, J = 8.4 Hz, 2H), 4.16 (t, J = 8.7 Hz, 1H), 4.04 - 3.90 (m, 1H), 3.30 - 3.16 (m, 2H), 2.88 - 2.85 (m, 1H), 2.28 - 2.18 (m, 1H), 2.00 - 1.87 (m, 1H).

Example 18-19 Preparation of Compounds 18-19

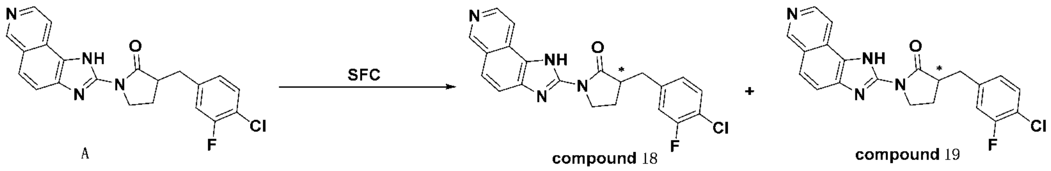


A compound 18 + compound 19

Step 1: The concentrated product A (64.5 mg) was subjected to chiral resolution (resolution method: WATERS 150 preparative SFC (SFC-26), ChiralCel OJ, 250×30mm I.D. 5μm, Mobile phase: A for $CO_2$ and B for Ethanol (0.1% $NH_3$ $H_2O$), Gradient: B 40%, Flow rate: 150mL /min, Back pressure: 100 bar, Column temperature: 38°C, Wavelength: 220nm ,Cycle time: ~5min) to obtain compound 18 (20.75 mg) and compound 19 (26.5 mg). Compound 18: LC-MS (ESI): m/z=395.2[M+H] +. Retention time 2.667 min, $^1$H NMR (400 MHz, DMSO) δ 12.83 (s, 1H), 9.29 (s, 1H), 8.52 (d, J = 4.9 Hz, 1H), 8.13 (d, J = 5.7 Hz, 1H), 7.91 - 7.81 (m, 2H), 7.54 (t, J = 8.1 Hz, 1H), 7.46 - 7.40 (m, 1H), 7.24 - 7.18 (m, 1H), 4.20 - 4.09 (m, 1H), 4.01 - 3.91 (m, 1H), 3.26 - 3.16 (m, 2H), 2.90 - 2.81 (m, 1H), 2.26 - 2.16 (m, 1H), 1.98 - 1.86 (m, 1H).

Compound 19: LC-MS (ESI): m/z=395.2[M+H] +. Retention time 4.007 min, $^1$H NMR (400 MHz, DMSO) δ 12.96 (s, 1H), 9.43 (s, 1H), 8.56 (d, J = 5.9 Hz, 1H), 8.27 (s, 1H), 8.00 - 7.91 (m, 2H), 7.54 (t, J = 8.1 Hz, 1H), 7.43 (dd, J = 10.6, 1.7 Hz, 1H), 7.21 (dd, J = 8.2, 1.5 Hz, 1H), 4.19 - 4.09 (m, 1H), 4.02-3.92 (m, 1H), 3.25 - 3.16 (m, 2H), 2.91-2.82 (m, 1H), 2.28-2.17 (m, 1H), 2.00-1.87 (m, 1H).

Example 20 Preparation of Compound 20

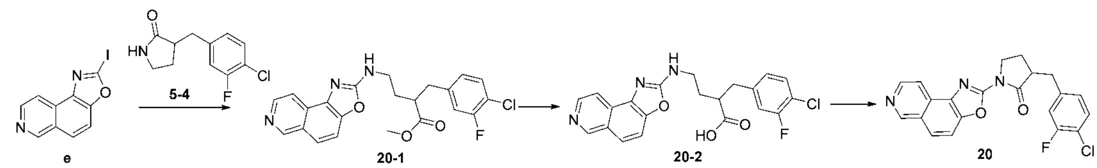


e 20-1 20-2 20

Step 1: Intermediate e (95 mg, 0.32 mmol), intermediate 5-4 (73.05 mg, 0.32 mmol), $Pd_2(dba)_3$ (58.77 mg, 0.06 mmol), Xantphos (55.70 mg, 0.10 mmol), $Cs_2CO_3$ (209.09 mg, 0.64 mmol) and 1,4-dioxane (12 mL) were stirred in a microwave at 130 °C under nitrogen protection for 1.5 hours. After the reaction was completed, the solvent was removed by concentrating under reduced pressure to obtain a crude product, and the crude product was purified by silica gel column chromatography (dichloromethane/methanol = 93/7) to obtain intermediate 20-1. LC-MS (ESI): m/z=428.1[M+H]$^+$.

Step 2: A mixture of intermediate 20-1 (150 mg, 0.35 mmol), LiOH (22.07 mg, 0.53 mmol), $H_2O$ (2 mL) and THF (4 mL) was stirred at 25 °C under nitrogen protection for 2 hours. After the reaction was completed, the solvent was removed in vacuo to obtain a crude product, and the crude product was purified by C18 (A: 10 mM $NH_4HCO_3/H_2O$ B: ACN, 2%-30%) to obtain intermediate 20-2. LC-MS (ESI): m/z=414.1[M+H]$^+$.

Step 3: A mixture of intermediate 20-2 (85 mg, 0.21 mmol), ethyl[di(propan-2-yl)]amine (132.74 mg, 1.03 mmol), $T_4P$ (50% ethyl acetate solution) (0.5 mL, 0.01 mmol) and tetrahydrofuran (10 mL) was stirred at 25°C under nitrogen protection for 16 hours. After the reaction was completed, the solvent was removed by concentration under reduced pressure to obtain a crude product, and the crude product was purified by preparative HPLC (Waters-Xbridge-C18-5μm-30*150mm, A: 10mM $NH_4HCO_3/H_2O$ B: ACN) to obtain compound 20. LC-MS (ESI): m/z=395.9[M+H]+, $^1$H NMR (400 MHz, DMSO) δ 9.45 (s, 1H), 8.64 (d, J = 5.8 Hz, 1H), 8.12 (d, J = 5.9 Hz, 1H), 8.10 (s, 2H), 7.54 (t, J = 8.1 Hz, 1H), 7.40 (dd, J = 10.6, 1.7 Hz, 1H), 7.19 (dd, J = 8.2, 1.4 Hz, 1H), 4.16 - 4.07 (m, 1H), 4.03-3.93 (m, 1H), 3.21 - 3.11 (m, 2H), 2.87 - 2.77 (m, 1H), 2.24-2.14 (m, 1H), 1.98-1.85 (m, 1H).

Example 21 Preparation of Compound 21

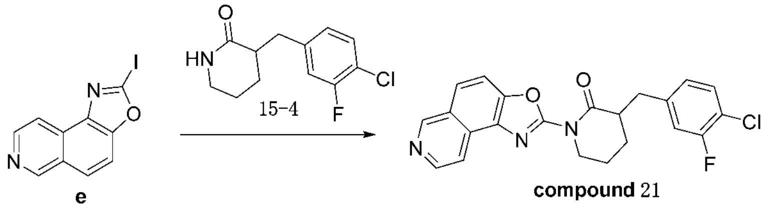


e compound 21

Step 1: Intermediate e (60 mg, 0.20 mmol), intermediate 15-4 (73.05 mg, 0.32 mmol), $Pd_2(dba)_3$ (37.12 mg, 0.04 mmol), Xantphos (35.18 mg, 0.06 mmol), $Cs_2CO_3$ (132.06 mg, 0.41 mmol) and 1,4-dioxane (10 mL) were stirred in microwave at 130 °C for 1.5 h under nitrogen protection. After the reaction was completed, a crude product was obtained by rotary evaporation under reduced pressure, and the crude product was purified by preparative HPLC (Waters-Xbridge-C18-10μm-19*250mm, A: 10mM $NH_4HCO_3$/ H2O B: ACN) to obtain compound 21. LC-MS (ESI): m/z=409.9 [M+H]+, $^1$H NMR (400 MHz, DMSO) δ 9.46 (s, 1H), 8.66 (d, J = 5.8 Hz, 1H), 8.16 - 8.11 (m, 2H), 8.08 (d, J = 8.9 Hz, 1H), 7.52 (t, J = 8.1 Hz, 1H), 7.38 (dd, J = 10.7, 1.8 Hz, 1H), 7.17 (dd, J = 8.2, 1.5 Hz, 1H), 4.09-4.03 (m, 2H), 3.29 - 3.23 (m, 1H), 3.07 - 2.97 (m, 1H), 2.83 - 2.76 (m, 1H), 2.04 - 1.80 (m, 3H), 1.65 - 1.53 (m, 1H).

Example 22 Preparation of Compound 22

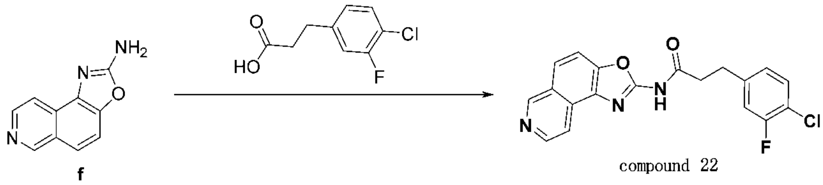

f compound 22

Step 1: Intermediate f (80 mg, 0.43 mmol), 3-(4-chloro-3-fluorophenyl)propionic acid (174.24 mg, 0.86 mmol), ethyldiisopropylamine (279.17 mg, 2.16 mmol), $T_4P$ (50% ethyl acetate solution) (1 mL, 0.05 mmol) and tetrahydrofuran (10 mL) were stirred at 25 °C under nitrogen protection for 16 hours. After the reaction was completed, the solvent was removed by rotary evaporation to obtain a crude product, and the crude product was purified by preparative HPLC (Waters-Xbridge-C18-10μm-19*250mm, A: 10mM NH4HCO3/H2O B: ACN) to obtain compound 22. LC-MS (ESI): m/z=370.0 [M+H]+, $^1$H NMR (400 MHz, DMSO) δ 11.96 (br s, 1H), 9.43 (s, 1H), 8.63 (d, J = 5.7 Hz, 1H), 8.10 - 8.01 (m, 3H), 7.51 (t, J = 8.1 Hz, 1H), 7.37 (dd, J = 10.6, 1.8 Hz, 1H), 7.17 (dd, J = 8.4, 1.8 Hz, 1H), 2.99-2.94 (m, 2H), 2.91-2.84 (m, 2H).

Example 23 Preparation of Compound 23

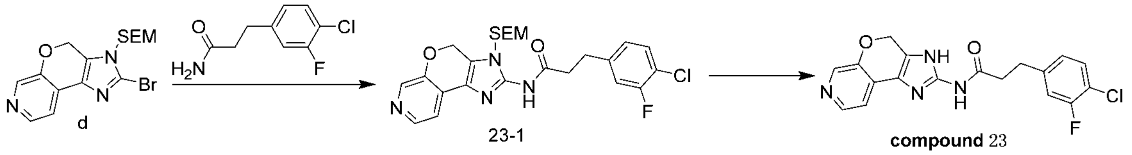

d 23-1 compound 23

Step 1: Intermediate d (150 mg, 0.39 mmol), 3-chloro-4-fluorophenylpropanamide (89.32 mg, 0.59 mmol), cesium carbonate (170.44 mg, 0.78 mmol), $Pd_2(dba)_3$ (47.90 mg, 0.08mmol) and XantPhos (60.54 mg, 0.16 mmol) were added to 1,2-dioxane (3 mL) solution. The reaction solution was purged with nitrogen three times and heated to 130°C by microwave under nitrogen protection for 45 minutes. LCMS showed that the product was generated. The reaction mixture was diluted with water (10 mL) and extracted with ethyl acetate (20 mL × 2), and the combined organic phases were washed with saturated NaCl (30 mL), the organic phases were dried and concentrated to obtain a crude product, then the crude product was purified by silica gel chromatography column eluting with dichloromethane/methanol (1/10) to obtain intermediate 23-1. LC-MS (ESI): m/z= 503.2[M+H]+.

Step 2: Intermediate 23-1 (61 mg, 0.12 mmol) was added to a solution of dichloromethane (2 mL) and trifluoroacetic acid (1 mL) at room temperature. The reaction solution was stirred at 25 °C for 2 h. LCMS showed that the product was generated. The reaction solution was concentrated directly in vacuum to obtain a crude product, and the crude product was purified by preparative liquid phase separation (A: 10mmoL/L $NaHCO_3$ in $H_2O$ B: ACN) to obtain

compound 23. LC-MS (ESI): m/z=373.1[M+H]+. $^1$HNMR (DMSO-d6) δ: 9.18 (s, 2H), 8.12 - 7.99 (m, 2H), 7.54 - 7.31 (m, 3H), 7.17 - 7.10 (m, 1H), 5.39 (s, 2H), 2.95 (t, J = 7.3 Hz, 2H), 2.73 (t, J = 7.3 Hz, 2H).

Example 24 Preparation of Compound 24

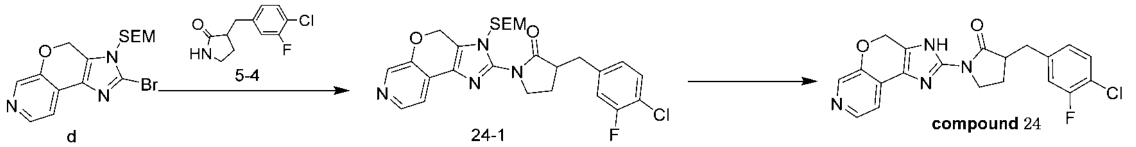


Step 1: Intermediate d (100 mg, 0.26 mmol), intermediate 5-4 (89.32 mg, 0.39 mmol), cesium carbonate (170.44 mg, 0.52 mmol), $Pd_2(dba)_3$ (47.90 mg, 0.05mmol) and XantPhos (60.54 mg, 0.10 mmol) were added to 1,4-dioxane (3 mL) solution at room temperature. The reaction solution was purged with nitrogen three times and heated to 130°C by microwave under nitrogen protection for 45 minutes. LCMS showed that the product was generated. The reaction mixture was diluted with water (10 mL) and extracted with ethyl acetate (20 mL × 2). The organic phases were combined and washed with saturated NaCl (20 mL), dried over anhydrous sodium sulfate, filtered, and concentrated in vacuo to obtain a crude product, then the crude product was purified by silica gel chromatography with dichloromethane/methanol (1/10) to obtain intermediate 24-1. LC-MS (ESI): m/z= 529.2[M+H]+.

Step 2: Intermediate 24-1 (140 mg, 0.26 mmol) was added to a solution of dichloromethane (2 mL) and trifluoroacetic acid (1 mL) at room temperature. The reaction solution was stirred at 25 °C for 2 h. LCMS showed that the product was generated. The reaction solution was concentrated directly in vacuum to obtain a crude product, then the crude product was purified by reversed-phase column preparation (3:2=0.1% formic acid dissolved in water:acetonitrile; 40 g C18 column) to obtain compound 24. LC-MS (ESI): m/z=399.1[M+H]+. $^1$HNMR (DMSO-d6) δ: 12.26 (s, 1H), 8.26 (s, 1H), 8.07 (d, J = 6.1 Hz, 2H), 7.52 (t, J = 8.1 Hz, 1H), 7.39 (dd, J = 10.6, 1.7 Hz, 1H), 7.17 (dd, J = 8.2, 1.4 Hz, 1H), 5.42 (s, 2H), 3.95 - 3.86 (m, 1H), 3.82 - 3.73 (m, 1H), 3.17 - 3.08 (m, 2H), 2.84 - 2.75 (m, 1H), 2.20 - 2.08 (m, 1H), 1.92 - 1.77 (m, 1H).

Example 25 Preparation of Compound 25

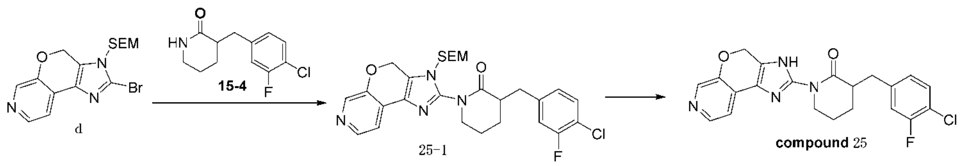


Step 1: Intermediate d (150 mg, 0.39 mmol) and Intermediate 15-4 (190 mg, 0.78 mmol) were placed in a microwave tube at room temperature and dissolved by adding 1,4-dioxane (2 mL), and cesium carbonate (383 mg, 1.18 mmol, Titan, P2299073), Xantphos (91 mg, 0.16 mmol, Titan, P2168097), and tris(dibenzylideneacetone)dipalladium (72 mg, 0.08 mmol, Titan, P2437515) were added. The reaction solution was purged with nitrogen and then heated to 130 °C with microwave synthesizer for 60 min. LC-MS showed that the raw material was completely reacted. The reaction solution was extracted with water (30 mL) and ethyl acetate (30 mL×2), and the organic phase was washed with saturated brine (30 mL×2), dried over anhydrous sodium sulfate, and filtered. The filtrate was concentrated and the residue was purified by silica gel column chromatography with a dichloromethane/methanol (100/0 to 97/3) system to obtain intermediate 25-1. LC-MS (ESI): m/z =543.2 [M+H]+.

Step 2: Intermediate 25-1 (75 mg, 0.14 mmol) was dissolved in dichloromethane (2 mL) and trifluoroacetic acid (1 mL) at room temperature, and the reaction solution was stirred at room temperature for 1 h. LC-MS showed that the raw material was completely reacted. The reaction solution was concentrated and the residue was dissolved in DMF and purified by prep-HPLC purification (Waters-CORTECS-C18-2.7μm-4.6*30mm, B: 0%-95%, 0.98 min, A: 0.1% FA in $H_2O$, B: ACN) to obtain compound 25. LC-MS (ESI): m/z =413.2 [M+H]+. $^1$H NMR (400 MHz, DMSO-d6) δ 12.89 - 12.18 (m, 1H), 8.15 - 8.07 (m, 2H), 7.88 - 7.68 (m, 1H), 7.51 (t, J = 8.1 Hz, 1H), 7.36 (dd, J = 10.7, 1.7 Hz, 1H), 7.15 (dd, J = 8.2, 1.4 Hz, 1H), 5.55 - 5.37 (m, 2H), 4.10 - 3.84 (m, 2H), 3.27 - 3.20 (m, 1H), 3.01 - 2.92 (m, 1H), 2.81 (dd, J = 13.7, 8.7 Hz, 1H), 1.98 - 1.87 (m, 1H), 1.87 - 1.71 (m, 2H), 1.57 - 1.43 (m, 1H).

Example 26-27 Preparation of Compounds 26-27

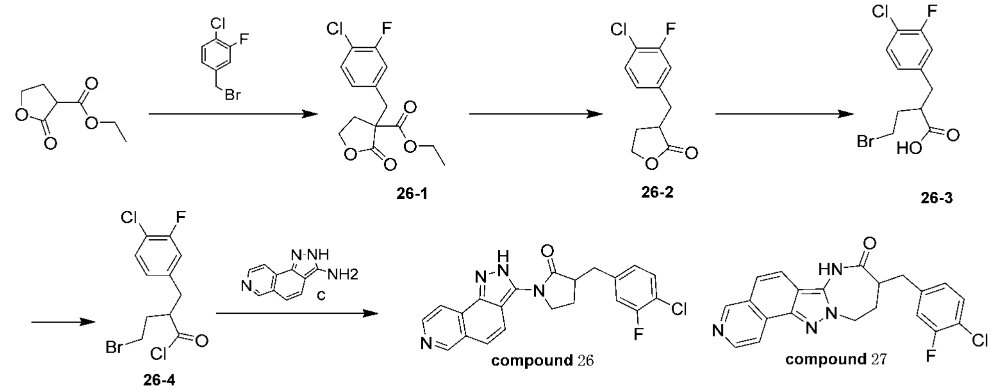


26-1 26-2 26-3

26-4 compound 26 compound 27

Step 1: Ethyl 2-oxotetrahydrofuran-3-carboxylate (5 g, 39.65 mmol) was added to tetrahydrofuran (20 mL) solution in an ice bath, then sodium hydride (395 mg, 9.89 mmol, 60% in oil) and 3-fluoro-4-chlorobenzyl bromide (1.62 g, 7.25 mmol) were added to the reaction solution, purged with nitrogen three times, the reaction was heated to 80 °C and stirred for 3 h. LCMS showed that the raw material was completely consumed. The reaction mixture was diluted with aqueous solution (30 mL) and extracted with ethyl acetate (50 mL × 2), and the organic phases were combined and washed with saturated NaCl (30 mL), and the organic phases were dried over anhydrous sodium sulfate, filtered and concentrated to obtain a crude product, then the crude product was purified by silica gel chromatography column eluting with ethyl acetate/petroleum ether (1/100 to 1/10) to obtain intermediate 26-1. LC-MS (ESI): m/z=287.0[M+H]+.

Step 2: Intermediate 26-1 (1.2 g, 4.19 mmol) and lithium chloride (1.77 g, 41.86 mmol) were added to DMSO (6 mL) at room temperature. The reaction solution was heated to 140 °C and stirred for 16 h. LCMS showed that the starting material was completely consumed. The above solution was then diluted with ethyl acetate (20 mL) and washed with water (10 mL × 3). The organic layer was separated and the organic phase was concentrated in vacuum to obtain a crude product, then the crude product was purified by silica gel chromatography column eluting with ethyl acetate/petroleum ether (1/100 to 1/10) to obtain intermediate 26-2. LC-MS (ESI): m/z=228.9[M+H]+.

Step 3: Intermediate 26-2 (450 mg, 1.97 mmol) was added to acetic acid (5 mL) solution and hydrogen bromide acetate (0.65 mL, 3.94 mmol) solution at room temperature and stirred at 50°C for 16 hours. LCMS showed that the raw material was completely consumed. The reaction solution was diluted with ethyl acetate (50 mL) and washed with water (50 mL× 3). The organic layer was separated and the organic phase was concentrated in vacuum to obtain a crude product, then the crude product was purified by silica gel chromatography column eluting with ethyl acetate/petroleum ether (1/100 to 1/2) to obtain intermediate 26-3. LC-MS (ESI): m/z=309.0[M+H]+.

Step 4: Intermediate 26-3 (100 mg, 0.32 mmol) and oxalyl chloride (0.28 mL, 3.23 mmol) were added to dichloromethane solution (2 mL) in an ice bath, and the reaction solution was stirred at 25 °C for 1 h. TLC (petroleum ether/ethyl acetate = 5/1) showed that the raw material was completely consumed. The reaction solution was concentrated directly to obtain a crude intermediate 26-4.

Step 5: Intermediate c (100 mg, 0.35 mmol) and sodium hydride (42.21 mg, 1.06 mmol, 60% in oil) were added to anhydrous DMF (2 mL) solution at room temperature. The reaction solution was stirred at 0 °C for 0.5 h under nitrogen atmosphere. Compound 26-4 was added to the reaction solution and stirred at 80 °C for 3 h. LCMS showed that the raw material was completely consumed. The reaction mixture was diluted with saturated aqueous ammonium chloride (10 mL) and extracted with ethyl acetate (50 mL × 2), and the organic phases were combined and then washed with saturated NaCl (30 mL), and the organic phase was dried over anhydrous sodium sulfate, filtered and concentrated to obtain a crude product. The crude product was purified by preparative liquid phase (A: 10mmoL/L $NaHCO_3$ in $H_2O$ B: ACN) to obtain compound 26 and compound 27.

Compound 26: LC-MS (ESI): m/z=395.0[M+H]+. $^1H$ NMR (DMSO-d6) δ: 14.07 (s, 1H), 9.36 (s, 1H), 8.71 (d, J = 5.6 Hz, 1H), 8.28 - 8.25 (m, 1H), 8.21-8.17 (m, 1H), 7.63 (d, J = 9.1 Hz, 1H), 7.54 (t, J = 8.1 Hz, 1H), 7.46 - 7.39 (m, 1H), 7.24-7.17 (m, 1H), 3.94 - 3.87 (m, 2H), 3.22 - 3.05 (m, 2H), 2.89-2.81 (m, 1H), 2.24-2.14 (m, 1H), 1.97-1.87 (m, 1H).

Compound 27: LC-MS (ESI): m/z=395.0[M+H]+. $^1H$ NMR (400 MHz, DMSO-d6) δ 10.72 (s, 1H), 9.20 (s, 1H), 8.64 (d, J = 5.4 Hz, 1H), 8.19 (d, J = 5.4 Hz, 1H), 7.63 (d, J = 8.9 Hz, 1H), 7.48 (d, J = 9.0 Hz, 1H), 7.41 (t, J = 8.1 Hz, 1H), 7.28 (dd, J = 10.8, 1.9 Hz, 1H), 7.07 (dd, J = 8.3, 1.9 Hz, 1H), 4.70-4.62 (m, 1H), 4.58-4.98 (m, 1H), 3.00 (dd, J = 13.3, 6.9 Hz, 1H), 2.78-2.71 (m, 1H), 2.66 - 2.62 (m, 1H), 2.47-2.40 (m, 1H), 2.18-2.08 (m, 1H).

Example 28-29 Preparation of Compounds 28-29

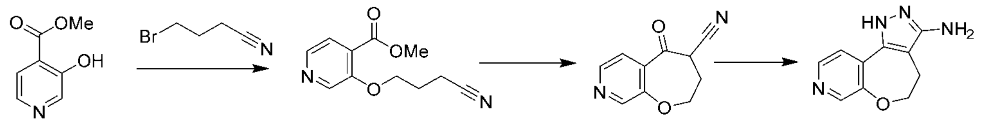

28-1 28-2 28-3

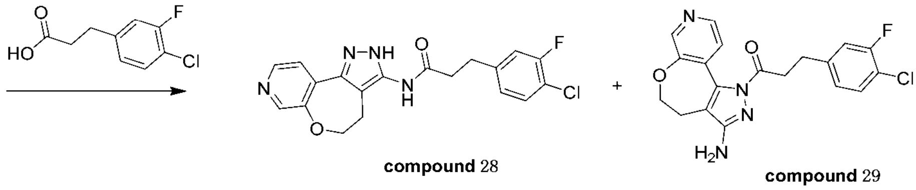

compound 28 compound 29

Step 1: 4-bromobutyronitrile (1.49 mL, 14.89 mmol) and potassium carbonate (4.29 g, 31.02 mmol) were added to methyl 3-hydroxyisonicotinate (1.9 g, 12.41 mmol) in DMF (50 mL) and the reaction was stirred for 18 h at 25 °C. LCMS showed that the raw material was consumed, and the target product was generated. The mixture was poured into water (60 mL) and extracted with ethyl acetate (40 mL ×3). The combined organic phases were washed with brine (100 mL), dried over anhydrous sodium sulfate, filtered and concentrated to dryness under reduced pressure to obtain a crude product. The crude product was purified by silica gel column chromatography (eluent: petroleum ether: ethyl acetate = 1:2) to obtain intermediate 28-1. LC-MS (ESI):m/z = 221.2 $[M+H]^+$.

Step 2: Intermediate 28-1 (500 mg, 2.27 mmol) was dissolved in DMF (20 mL) solution and cooled to 0 °C, potassium tert-butoxide (509.51 mg, 4.54 mmol) dissolved in DMF (10 mL) was added to the mixture and the reaction was stirred for 1 h at 0 °C. LCMS showed that the raw material was consumed, and the target product was generated. The reaction mixture was adjusted to pH=5 with saturated aqueous citric acid solution, the mixture was filtered, and the filtrate was concentrated to dryness under reduced pressure to obtain a crude product. The crude product was slurred with acetonitrile (20 mL), and the suspension was separated by filtration. The filter cake was washed with acetonitrile (20 mL×3) and then dried under reduced pressure to obtain intermediate 28-2. LC-MS (ESI):m/z = 189.1 $[M+H]^+$.

Step 3: Hydrazine hydrochloride (364 mg, 5.31 mmol) was added to intermediate 28-2 (200 mg, 1.06 mmol) in EtOH (10 mL), and the reaction was stirred at 80 °C for 18 h. LCMS showed that the raw material was consumed, and the target product was generated. The reaction solution was cooled to room temperature, the mixture was filtered, the filter cake was washed with MeOH (20 mL×3), and the filtrate was concentrated to dryness under reduced pressure to obtain a crude product, then the crude product was purified by reversed-phase (Phenomenex Gemini 150 mm * 25 mm * 10 um column (eluent: 30% to 60% (v/v) $CH_3CN$ and $H_2O$ with 0.1% $NH_4OH$ ) to obtain intermediate 28-3. LC-MS (ESI):m/z = 202.9 $[M+H]^+$.

Step 4: CDI (52.92 mg, 0.33 mmol) was added to 3-(4-chloro-3-fluorophenyl)propionic acid (60.12 mg, 0.30 mmol, Bide Pharmatech) in DMF (5 mL), and the mixture was stirred at room temperature for 1 hour, intermediate 28-3 (60 mg, 0.30 mmol) was added and stirred for additional 1 hour. LCMS showed the target product generated, and the reaction solution was added with water (50 mL), extracted with ethyl acetate (10 mL×3), and the mixed organic phase was concentrated to obtain a crude product, which was further purified by Prep-HPLC (CAN/Water/0.1%HCOOH) to obtain compound 28 and compound 29. LC-MS (ESI): m/z =387.0$[M+H]^+$.

Compound 28: $^1H$ NMR (400 MHz, DMSO-d6) δ 13.31 (br s, 1H), 9.98 (br s, 1H), 8.36-8.32 (m, 1H), 8.29-8.25 (m, 1H), 7.91-7.88 (m, 1H), 7.53-7.49 (m, 1H), 7.36-7.32 (m, 1H), 7.17-7.13 (m, 1H), 4.23 (t, J = 4.8 Hz, 2H), 2.96-2.92 (m, 2H), 2.76-2.72 (m, 2H), 2.68-2.64 (m, 2H).

Compound 29: $^1H$ NMR (400 MHz, DMSO-d6) δ 8.33 (s, 1H), 8.26-8.22 (m, 1H), 8.06-8.02 (m, 1H), 7.53-7.49 (m, 1H), 7.43-7.39 (m, 1H), 7.22-7.18 (m, 1H), 6.62 (s, 2H), 4.33-4.29 (m, 2H), 3.51-3.48 (m, 2H), 3.04-3.01 (m, 2H), 2.81-2.77 (m, 2H).

**Biological evaluation of compounds**

**Experimental Example 1: *In vitro* enzyme inhibition activity**

1. Reagents, consumables, and instruments are shown in Table 2.

Table 2

| Reagent | Supplier | Item No. |
|---|---|---|
| PBS,1×(pH7.2-7.4, 0.01M, cell culture) | Solarbio | P1020-500ml |
| PBS (containing protease inhibitors) | Roche | 11873580001 |
| NAD | Sigma | N8285 |
| DMSO | Sigma | D8418 |
| Trichloroacetic acid | Sigma | T6399-250G |
| X-tremeGENE 9DNA | Roche | 06365787001 |
| OptiMEM | Gibco | 31985062 |
| Nicotinamide riboside | Rhawn | R056456-1g |
| Consumable | Supplier | Item No. |
| OptiPlate-384 White Opaque | PerkinElmer | 6007290 |
| Culture dish | Corning | 430599 |
| Instrument | Supplier | |
| Shimadzu UHPLC-SCIEX Q-Trap 4500 LC/MS/MS | Sciex | |
| Centrifuge | Eppendorf | |
| Plate shaker | Thermo | |
| Echo | Labcyte | |

2. Preparation of SAM-TIR lysate

NRK1-HEK293T cells were seeded at approximately 10 × $10^6$ cells/plate into 150 mm culture dishes containing 25 mL of growth medium. Next day, the cells were transfected by first premixing 15 μg of human SARM1 expression plasmid (SARM1408-724 expression plasmid and vector-control customized by SinoBio) with 45 μL of X-tremeGENE 9 DNA transfection reagent and 750 μL of OptiMEM and then adding the mixture directly to the cells. At the time of transfection, 250 μL of nicotinamide riboside (100 mM) was added to each dish to minimize toxicity from SAM-TIR overexpression. 48 hours after the transfection, the cells were washed three to four times with cold PBS and collected. The cells were resuspended in 0.5 mL of PBS containing protease inhibitors. Cell lysates were prepared by sonication. The lysate was centrifuged at 12500 rpm for 10 min at 4°C to remove cell debris, and protein concentration was determined by bicinchoninic acid (BCA) assay and used to normalize lysate concentration. The aliquots of supernatant were stored at -80°C until use.

3. Experimental steps

1) Transfer the compound dilutions into an assay plate by Echo 650;
2) Add 10μl 2× SAM-TIR lysate to each well;
3) Seal the assay plate, centrifuge at 1000rpm for 1min, and incubate at 25°C for 30min;
4) Add 10μl 2× NAD to each well;
5) Centrifuge at 1000rpm for 1min, and incubate at 25°C for 3h;
6) Add 40μL trichloroacetic acid to each well to terminate the reaction, mix and shake for 30s, and centrifuge at 4000rpm for 10min;
7) Take 40μL supernatant from each well and add appropriate amount of ammonia water to adjust the pH;
8) Detect nicotinamide adenine dinucleotide (NAD) and adenosine diphosphate ribose (ADPR) by HPLC-MS/MS.

4. Data analysis

1) For each screening plate, calculate the average data and standard deviation (SD) of DMSO (as negative control (VC)) and 100 μM control I-5 (as positive control (PC))
2) Inhibition percentage of compound

NAD:

Inhibition rate = {(signal of compound - signal of negative control) / (signal of positive control - signal of negative control)} × 100%

ADPR:

Inhibition rate = {1-(signal of compound - signal of positive control) / (signal of negative control - signal of positive control)} × 100%

3) Calculate the $IC_{50}$ using the nonlinear regression equation of XLfit 5.3.1 with the following formula:

Inhibition rate = minimum value + (maximum value - minimum value) / (1 + 10^((Log$IC_{50}$-Log(compound concentration)) * slope coefficient))

Control I-5

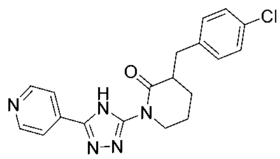

was prepared according to method C of WO2022046606A1.

Conclusion: At least some of the compounds of the present invention have better inhibitory effect on the SARM1 enzyme, and the activity data of some of the compounds at the enzymatic level of SARM1 are shown in Table 3.

Table 3

| Sample No. | SARM1 $IC_{50}$ | Sample No. | SARM1 $IC_{50}$ | Sample No. | SARM1 $IC_{50}$ |
|---|---|---|---|---|---|
| Cpd. 1 | A | Cpd. 11 | D | Cpd. 21 | B |
| Cpd. 2 | A | Cpd. 12 | C | Cpd. 22 | B |
| Cpd. 3 | C | Cpd. 13 | D | Cpd. 23 | A |
| Cpd. 4 | A | Cpd. 14 | A | Cpd. 24 | A |
| Cpd. 5 | A | Cpd. 15 | A | Cpd. 25 | A |
| Cpd. 6 | B | Cpd. 16 | A | Cpd. 26 | D |
| Cpd. 7 | B | Cpd. 17 | A | Cpd. 27 | D |
| Cpd. 8 | A | Cpd. 18 | A | Cpd. 28 | A |
| Cpd. 9 | A | Cpd. 19 | A | Cpd. 29 | D |
| Cpd. 10 | B | Cpd. 20 | A | | |
| Note: A < 150nM; 150nM ≤ B < 500nM; 500nM ≤ C < 1000nM; D ≥ 1000nM. | | | | | |

**Experimental Example 2: Kinetic Solubility Evaluation**

10 mM stock solution was prepared by dissolving the test compound in DMSO. 100 mM $K_2HPO_4$ solution was prepared by adding 8.71 g $K_2HPO_4$ to 500 mL deionized water. 100 mM $KH_2PO_4$ solution was prepared by adding 2.05 g $KH_2PO_4$ to 150 mL deionized water. 405 mL of 100 mM $K_2HPO_4$ and 95 mL of 100 mM $KH_2PO_4$ were mixed and adjusted to pH 7.4 with 100 mM $K_2HPO_4$/$KH_2PO_4$ solution.

16 μL of 10 mM compound stock solution was added to 784 μL of PBS buffer (n = 3) by 96 well plate, and the plate was sealed and shaken at 25°C, 1000 rpm for 1.5 h. After incubation, the solution was transferred to a filter plate. All samples were filtered. 5 μL of the filtrate was taken, added to 5 μL of DMSO and 490 μL acetonitrile aqueous solution containing internal standard (1:1) and mixed. Then the mixed solution was diluted with acetonitrile aqueous solution containing internal standard (1:1) according to the properties of the compound and its response in the mass spectrometer. The dilution factor was adjusted according to the solubility value and UPLC-MS/MS signal response.

Conclusion: At least some of the compounds of the present application have better solubility in PBS buffer, e.g., the solubility of compound 25 is >50 μg/mL.

**Experimental Example 3: Evaluation of liver microsomal stability *in vitro***

100 mM K-Mg-buffer containing 5 mM $MgCl_2$ was preheated. The spiking solution was prepared by adding 5 μL of 10 mM compound and reference stock solution to 95 μL of acetonitrile (ACN). 1.5 μL of 500 μM spiking solution and 18.75 μL of 20 mg/mL liver microsomes were added to 479.8 μL of K-Mg-buffer. NADPH stock solution (3 mM) was prepared by dissolving NADPH in K-Mg-buffer. 30 μL of 1.5 μM spiking solution containing microsomes was evenly dropped into the assay plate at different time points (0, 5, 15, 30, and 45 min), and pre-incubated at 37 °C for 5 min. At 0 min, 200 μL of ACN containing IS (internal standard, tolbutamid/terfenadine) was added to the wells, then 15 μL of NADPH stock solution (6 mM) was added. For the other time points, the reaction was initiated and timed by adding 15 μL of NADPH stock solution (6 mM) to the wells. The reaction was stopped by adding 200 μL of ACN containing IS to the corresponding plate wells at 5, 15, 30, and 45 min, respectively. After quenching, the plate was shaken at 600 rpm for 10 min and then centrifuged at 4000 rpm for 50 min. 80 μL of supernatant per well was pipetted to a 96-well sample plate containing 160 μL of pure water for UPLC/MS/MS analysis.

Conclusion: At least some of the compounds of the present application have good *in vitro* liver microsomal stability, and the test results of some of the compounds are shown in Table 4.

Table 4

| Sample No. | $T_{1/2}$ (minute) | Sample No. | $T_{1/2}$ (minute) | Sample No. | $T_{1/2}$ (minute) |
|---|---|---|---|---|---|
| 4 | 28.88 | 10 | 21.13 | 25 | 26.10 |
| 9 | 19.69 | 23 | 37.36 | 28 | 40.38 |

**Experimental Example 4: Evaluation of cell membrane permeability**

Test compounds were diluted from 10 mM stock solution to a concentration of 10 μM in transport buffer (HBSS + BSA) and applied to the apical or basolateral side of the cell monolayer. The permeability of the test compounds was determined in both direction of from A to B and B to A directions after 120 min incubation at 37°C, 5% CO2 and 95% relative humidity. In addition, the efflux ratio of each compound was determined. The analyte and reference compounds were quantified by LC-MS/MS based on the analyte/IS peak area ratio.

Conclusion: It is shown that at least some of the compounds of the present application have good cell membrane permeability and are not P-glycoprotein substrates, and the test results of some compounds are shown in Table 5.

Table 5

| Compound No. | Papp ($10^{-6}$ cm/s) | efflux ratio |
|---|---|---|
| | A to B | |
| 4 | 11 | 1.15 |
| 25 | 7.56 | 0.90 |
| 28 | 5.47 | 2.39 |

**Experimental Example 5: Pharmacokinetic Evaluation in Mice**

The test compound was dissolved in the vehicle to prepare a clear solution or a homogeneous suspension. Three mice (CD1 mice) per group were administered 2 mg/kg via the tail vein and 10 mg/kg orally (PO). Blood was collected at 0.083 h, 0.25 h, 0.5 h, 1 h, 2 h, 4 h, 8 h, and 24 h after intravenous administration, and at 0.083 h, 0.25 h, 0.5 h, 1 h, 2 h, 4 h, 8 h and 24 h after oral administration. Plasma samples were centrifuged, and the supernatant was collected to prepare

samples for quantitative analysis by LC/MS/MS. Conclusion: At least some of the compounds of the present application (e.g., compound 25) have excellent pharmacokinetic properties in mice (including but not limited to Cl (clearance rate), $T_{1/2}$ (half-life), $C_{max}$ (peak concentration), AUC (area under the drug-time curve), F (bioavailability), etc.). The test results of some compounds, such as the mouse PK properties of compound 25, are shown in Table 6.

Table 6 The mouse PK properties of compound 25

| | | |
|---|---|---|
| mouse IV (2 mg/kg) | $C_0$ (ng/mL) | 2113 |
| | $T_{1/2}$ (h) | 1.33 |
| | Cl (mL/h/kg) | 15.1 |
| | $V_{ss}$ (L/kg) | 1.31 |
| | AUC $_{(0-inf)}$ (ng*h/ml) | 2206 |
| mouse PO (10 mg/kg) | $C_{max}$ (ng/mL) | 3000 |
| | $T_{1/2}$ (h) | 1.84 |
| | $T_{max}$ (h) | 0.417 |
| | AUC $_{(0-inf)}$ (ng*h/ml) | 9818 |
| | F% | 89.0 |

**Experimental Example 6: Evaluation of *in vivo* brain permeability**

The test compound was dissolved in the vehicle to prepare a clear solution or a homogeneous suspension. Three mice (CD1 mice) per group were orally administered (PO) at 10 mg/kg. Plasma and cerebrospinal fluid were collected at 1 h, 4 h, and 8 h after oral administration and quantitatively analyzed by LC/MS/MS. Conclusion: At least some of the compounds of the present application have reached very high levels of Cmax and AUC in cerebrospinal fluid and have good *in vivo* brain permeability. For example, compound 25 has an $AUC_{brain}/AUC_{plasma}$ = 0.71, indicating that this compound has very good *in vivo* brain permeability. Table 7 shows the plasma and cerebrospinal fluid PK data for compound 25 of the present invention.

Table 7 Plasma and cerebrospinal fluid PK of compound 25

| | | |
|---|---|---|
| Plasma PK | $C_{max}$ (ng/mL) | 3090 |
| | $T_{1/2}$ (h) | 1.40 |
| | $T_{max}$ (h) | 1.00 |
| | AUC $_{(0-inf)}$ (ng*h/ml) | 9701 |
| Cerebrospinal fluid PK | $C_{max}$ (ng/mL) | 2197 |
| | $T_{1/2}$ (h) | 1.41 |
| | $T_{max}$ (h) | 1.00 |
| | $AUC_{(0-inf)}$ (ng*h/ml) | 6897 |

**Experimental Example 7: Neuronal injury model based on mouse primary neurons**

Methods: Neocortices were dissected from the brains of newborn mice at DIV 0 (Day In Vitro 0), digested with 0.05% (w/v) trypsin/EDTA, and incubated at 37°C for 10 min. Neuronal cells obtained after digestion were seeded in cell culture dishes preincubated with poly-D-lysine in Neurobasal/B-27 medium (Neurobasal medium supplemented with 1% (v/v) B-27, 2 mM glutamine, 100 U/ml penicillin, and 100 mg/ml streptomycin). Starting from DIV 3, a half of the medium was replaced with fresh Neurobasal/B-27 medium every two days. At DIV 10, the cultures were subjected to axotomy and compound administration. The cells were fixed with 4% paraformaldehyde the day after axotomy and subjected to cell immunofluorescence staining for TUJ1. Confocal imaging of axons was performed using a Zeiss LSM710 laser confocal microscope.

Conclusion: At least some of the compounds of the present application have stronger neuron-protective activity that can reduce axonal loss after axonal injury. For example, as shown in Figure 1, compared with the control I-5,

compound 25 shows stronger neuron-protecting activity at a lower concentration (0.3 μM) which could reduce axonal loss after axonal injury.

**Experimental Example 8: Mouse sciatic nerve axotomy (SNA) model**

Methods: Mice were anesthetized with isoflurane, and the skin of the right hind limb was shaved and sterilized with iodine. An incision was made between the knee and hip joints, and the gluteal muscle was carefully separated using a pair of sterile surgical scissors. The sciatic nerve was cut proximally to the thigh with a pair of sterile surgical scissors, and a 1-2 mm segment of the nerve was removed to prevent axonal regeneration into the distal stump. The gluteal muscle was then placed back to its original anatomical position, and the overlying skin was reattached with surgical staples or sutures.

To examine the denervation of the neuromuscular junction (NMJ), the tibialis anterior muscles were dissected from perfused animals and fixed overnight with 4% paraformaldehyde/PBS at 4°C. Immunohistochemical staining was subsequently performed with Neurofilament-L antibody and Synapsin-1 antibody as well as acetylcholine receptor (AchR) markers. Finally, imaging was performed by laser confocal microscope Zeiss LSM710.

Conclusion: At least some of the compounds of the present application can significantly protect axons from injury-induced axonal degeneration. For example, as shown in Figure 2, compound 25 can significantly protect axons from injury-induced axonal degeneration in mice.

Finally, it should be noted that the above embodiments are only used to illustrate the technical solution of the present invention rather than to limit it. Although the present invention has been described in detail with reference to the preferred embodiments, the person skilled in the art should understand that the specific embodiments of the present invention may still be modified or some technical features may be replaced by equivalents without departing from the spirit of the technical solution of the present invention, which should be included in the scope of the technical solution for protection of the present invention.

## Claims

**1.** A compound represented by Formula (I), or a stereoisomer, tautomer, polymorph, solvate, hydrate, N-oxide, isotope-labeled compound, metabolite, ester, prodrug of the compound, or a pharmaceutically acceptable salt thereof,

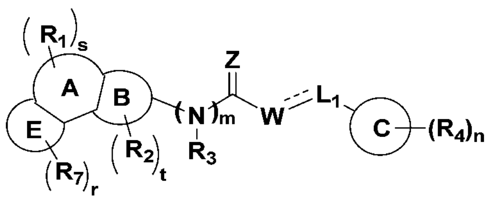

(I),

Wherein,

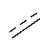

is selected from a single bond, a double bond, and a triple bond;

Z is selected from O and S;

$L_1$ is selected from $-C_{1-3}$ alkylene-, =CH-, -NH-, and -O-, or $L_1$ is absent;

ring A is selected from phenyl, 5-6 membered heteroaryl, $C_{4-8}$ cyclic groups, and 4-8 membered heterocyclyl; preferably, ring A is selected from phenyl, dihydropyranyl, pyridyl, dihydropyridyl, and oxepanyl;

ring B is selected from phenyl, 5-6 membered heteroaryl, and 5-6 membered heterocyclyl;

ring C is selected from $C_{6-10}$ aryl, 5-12 membered heteroaryl, $C_{3-12}$ cyclic groups, and 4-10 membered heterocyclyl;

ring E is selected from 5-6 membered heteroaryl and phenyl;

$R_1$ is selected from H, -OH, halogen, $-NH_2$, -CN, $-NO_2$, -COOH, $C_{1-6}$ alkyl, $C_{1-6}$ haloalkyl, $C_{1-6}$ alkoxy, $-C_{1-6}$ alkylene-OH, $-O-C_{1-6}$ haloalkyl, $-C_{3-7}$ cycloalkyl, and 3-7 membered heterocycloalkyl;

$R_2$ is selected from H, -OH, halogen, -CN, $-NH_2$, $-NO_2$, -COOH, $C_{1-6}$ alkyl, $C_{1-6}$ haloalkyl, $C_{1-6}$ alkoxy, $-C_{1-6}$ alkylene-OH, $-O-C_{1-6}$ haloalkyl, $-C_{1-6}$ alkylene-$C_{1-6}$ alkoxy, $-C_{3-7}$ cycloalkyl, and 3-7 membered heterocycloalkyl;

$R_3$ is selected from H, $C_{1-6}$ alkyl, and $-C(=O)-C_{1-6}$ alkyl, wherein the $C_{1-6}$ alkyl and $-C(=O)-C_{1-6}$ alkyl are optionally substituted with 1, 2, 3, 4 or 5 substituents selected from halogen, -OH, $-NH_2$, -CN, $-NO_2$, and -COOH;

W is selected from -C($R_5R_6$)-, -$CR_5$=, -C(=$CH_2$)-, and -$NR_5$-, wherein $R_5$ is selected from H, -OH, $C_{1-6}$ alkyl, $C_{1-6}$ alkoxy, $C_{1-6}$ haloalkyl, and $C_{1-6}$ haloalkoxy; or

W and $R_3$ together with the atoms to which they are attached form a 4-6 membered heterocyclyl, wherein the 4-6 membered heterocyclyl is optionally substituted with 1, 2, 3, 4 or 5 R; or

W and ring B together with the atoms to which they are attached form a 6-10 membered heterocyclyl, wherein the 6-10 membered heterocyclyl is optionally substituted with 1, 2, 3, 4 or 5 R;

$R_4$ is selected from halogen, -OH, -CN, -$NH_2$, $C_{1-6}$ alkyl, $C_{1-6}$ alkoxy, $C_{1-6}$ haloalkyl, and $C_{1-6}$ haloalkoxy;

$R_6$ is selected from H, $C_{1-6}$ alkyl, $C_{1-6}$ alkoxy, $C_{1-6}$ haloalkyl, and $C_{1-6}$ haloalkoxy;

$R_7$ is selected from H, halogen, -OH, -$NO_2$, -CN, -$NH_2$, -COOH, $C_{1-6}$ alkyl, $C_{1-6}$ alkoxy, $C_{1-6}$ haloalkyl, -$C_{1-6}$ alkylene-OH, -O-$C_{1-6}$ haloalkyl, -$C_{3-7}$ cycloalkyl, and 3-7 membered heterocycloalkyl;

R is selected from halogen, -OH, -CN, =O, $C_{1-6}$ alkyl, $C_{1-6}$ alkoxy, $C_{1-6}$ haloalkyl, and $C_{1-6}$ haloalkoxy;

m is selected from 0 and 1;

n is selected from 0, 1, 2, 3, 4, and 5; preferably, selected from 0, 1, 2, and 3;

r, s, and t are each independently 0, 1, 2, 3, 4, or 5; preferably 0, 1, or 2; more preferably 0 or 1.

**2.** The compound, or a stereoisomer, tautomer, polymorph, solvate, hydrate, N-oxide, isotope-labeled compound, metabolite, ester, prodrug of the compound, or a pharmaceutically acceptable salt thereof according to claim 1, wherein,

W is selected from -C($R_5R_6$)-, -$CR_5$=, -C(=$CH_2$)-, and -$NR_5$-, or

W and $R_3$ together with the atoms to which they are attached form a 4-6 membered heterocyclyl, wherein the 4-6 membered heterocyclyl is optionally substituted with 1, 2, 3, 4 or 5 R;

preferably, W is selected from -C($R_5R_6$)- and -C(=$CH_2$)-, or

W and $R_3$ together with the atoms to which they are attached form a 4-6 membered heterocyclyl, wherein the 4-6 membered heterocyclyl is optionally substituted with 1, 2, 3, 4 or 5 R;

preferably, W is selected from -C($R_5R_6$)- and -C(=$CH_2$)-, or

W and $R_3$ together with the atoms to which they are attached form a 5-6 membered heterocyclyl, wherein the 5-6 membered heterocyclyl is optionally substituted with 1, 2, 3, 4 or 5 R;

preferably, W is -C($R_5R_6$)-, or

W and $R_3$ together with the atoms to which they are attached form a 5-6 membered heterocyclyl, wherein the 5-6 membered heterocyclyl is optionally substituted with 1, 2, 3, 4 or 5 R.

**3.** The compound, or a stereoisomer, tautomer, polymorph, solvate, hydrate, N-oxide, isotope-labeled compound, metabolite, ester, prodrug of the compound, or a pharmaceutically acceptable salt thereof according to claim 1 or 2, wherein, m is 1.

**4.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-3, wherein,

$R_1$ is selected from H, -OH, halogen, -CN, -$NH_2$, -$NO_2$, -COOH, $C_{1-4}$ alkyl, $C_{1-4}$ haloalkyl, $C_{1-4}$ alkoxy, -$C_{1-4}$ alkylene-OH, -O-$C_{1-4}$ haloalkyl, -$C_{3-6}$ cycloalkyl, and 5-6 membered heterocycloalkyl;

preferably, $R_1$ is selected from H, -OH, F, Cl, Br, I, -$NH_2$, -CN, -$NO_2$, -COOH, methyl, ethyl, *n*-propyl, isopropyl, *n*-butyl, isobutyl, *sec*-butyl, *tert*-butyl, halomethyl, haloethyl, halopropyl, halobutyl, methoxy, ethoxy, propoxy, butoxy, halomethoxy, haloethoxy, halopropoxy, halobutoxy, hydroxymethyl, hydroxyethyl, hydroxypropyl, cyclopropyl, cyclobutyl, cyclopentyl, and cyclohexyl;

preferably, $R_1$ is selected from H, -OH, F, Cl, Br, -$NH_2$, -CN, -$NO_2$, -COOH, methyl, ethyl, n-propyl, methoxy, ethoxy, monofluoromethyl, difluoromethyl, trifluoromethyl, hydroxymethyl, and hydroxyethyl;

preferably, $R_1$ is selected from F, Cl, Br, and I;

preferably, $R_1$ is selected from -$NH_2$, -CN, -$NO_2$, and -COOH;

preferably, $R_1$ is selected from methyl, methoxy, monofluoromethyl, difluoromethyl, trifluoromethyl, hydroxymethyl, and hydroxyethyl;

preferably, $R_1$ is selected from H and -OH;

preferably, $R_1$ is H.

**5.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-4, wherein,

$R_2$ is selected from H, -OH, halogen, -CN, $-NH_2$, $-NO_2$, -COOH, $C_{1-4}$ alkyl, $C_{1-4}$ haloalkyl, $C_{1-4}$ alkoxy, $-C_{1-4}$ alkylene-OH, $-O-C_{1-4}$ haloalkyl, $-C_{1-4}$ alkylene-$C_{1-4}$ alkoxy, $-C_{3-6}$ cycloalkyl, and 4-6 membered heterocycloalkyl;
preferably, $R_2$ is selected from H, -OH, F, Cl, Br, I, -CN, $-NH_2$, $-NO_2$, -COOH, methyl, ethyl, *n*-propyl, isopropyl, *n*-butyl, isobutyl, *sec*-butyl, *tert*-butyl, halomethyl, haloethyl, halopropyl, halobutyl, methoxy, ethoxy, propoxy, butoxy, hydroxymethyl, hydroxyethyl, hydroxypropyl, halomethoxy, haloethoxy, halopropoxy, halobutoxy, $-CH_2OCH_3$, $-(CH_2)_2OCH_3$, $-(CH_2)_3OCH_3$, $-CH_2OCH_2CH_3$, $-CH_2O(CH_2)_2CH_3$, cyclopropyl, cyclobutyl, cyclopentyl, and cyclohexyl;
preferably, $R_2$ is selected from H, -OH, F, Cl, Br, $-NH_2$, -CN, $-NO_2$, -COOH, methyl, ethyl, *n*-propyl, methoxy, ethoxy, monofluoromethyl, difluoromethyl, trifluoromethyl, hydroxymethyl, hydroxyethyl, $-CH_2OCH_3$, $-(CH_2)_2OCH_3$, and $-CH_2OCH_2CH_3$;
preferably, $R_2$ is selected from H, Cl, -CN, $-NH_2$, $-CH_3$, and $-CH_2OCH_3$;
preferably, $R_2$ is selected from H, $-NH_2$, $-CH_3$, and $-CH_2OCH_3$;
preferably, $R_2$ is selected from H, $-CH_3$, and $-CH_2OCH_3$;
preferably, $R_2$ is selected from H and $-NH_2$;
preferably, $R_2$ is H.

**6.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-5, wherein,

$R_7$ is selected from H, halogen, -OH, $-NO_2$, -CN, $-NH_2$, -COOH, $C_{1-4}$ alkyl, $C_{1-4}$ alkoxy, $C_{1-4}$ haloalkyl, $-C_{1-4}$ alkylene-OH, $-O-C_{1-4}$ haloalkyl, $-C_{3-6}$ cycloalkyl, and 4-6 membered heterocycloalkyl;
preferably, $R_7$ is selected from H, F, Cl, Br, I, -OH, $-NO_2$, -CN, $-NH_2$, -COOH, methyl, ethyl, *n*-propyl, isopropyl, *n*-butyl, isobutyl, *sec*-butyl, *tert*-butyl, methoxy, ethoxy, propoxy, butoxy, halomethoxy, haloethoxy, halopropoxy, halobutoxy, hydroxymethyl, hydroxyethyl, hydroxypropyl, halomethoxy, haloethoxy, halopropoxy, halobutoxy, cyclopropyl, cyclobutyl, cyclopentyl, and cyclohexyl;
preferably, $R_7$ is selected from H, F, Cl, Br, I, -OH, $-NO_2$, -CN, $-NH_2$, -COOH, methyl, ethyl, methoxy, and ethoxy;
preferably, $R_7$ is selected from H, F, Cl, Br, and $-CH_3$;
preferably, $R_7$ is selected from H, F, Cl, and Br;
preferably, $R_7$ is H.

**7.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-6, wherein,

the structural unit

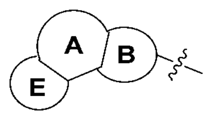

is selected from

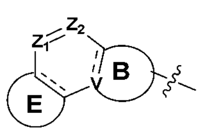

and

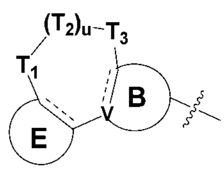

;

wherein,

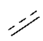

is selected from a single bond and a double bond;
$Z_1$ and $Z_2$ are each independently selected from CH and N;

V is selected from C, CH, and N; preferably, V is selected from C and N;

$T_1$, $T_2$, and $T_3$ are each independently selected from $CH_2$, O, S, and NH; preferably, $T_1$, $T_2$, and $T_3$ are each independently selected from $CH_2$, O, and NH;

u is selected from 0, 1, and 2; preferably, u is selected from 0 and 1;

preferably, the structural unit

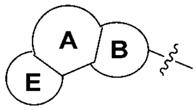

is selected from

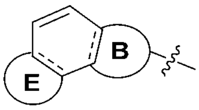
,
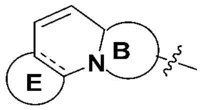
,

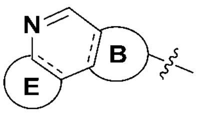
,
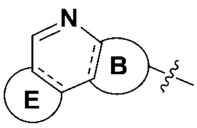
,
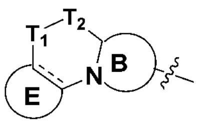
, and
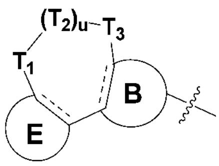

preferably, the structural unit

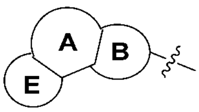

is selected from

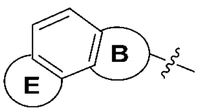
,
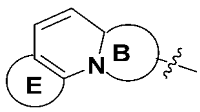
,

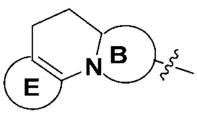
,
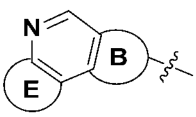
,
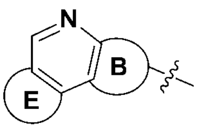
,
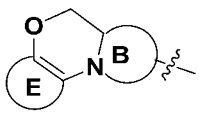
, and
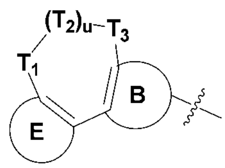
;

preferably, the structural unit

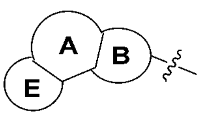

is selected from

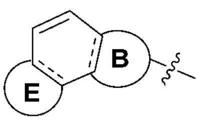
,
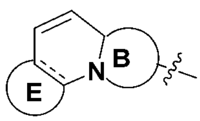
,

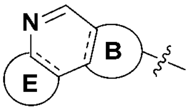, 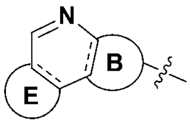, 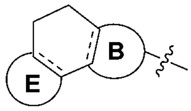, 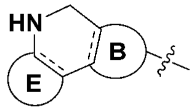, 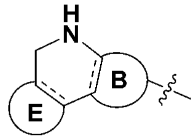,

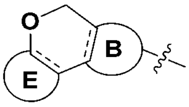, 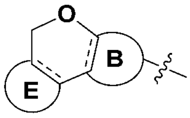, 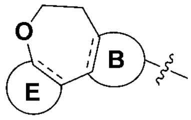, 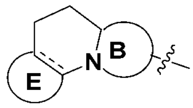, 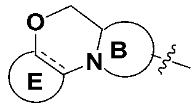,

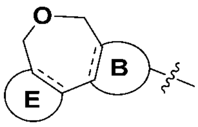, and 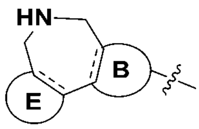;

more preferably, the structural unit

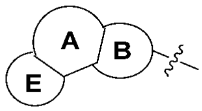

is selected from

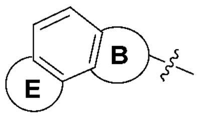, 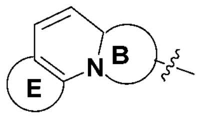,

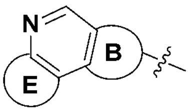, 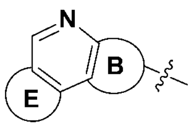, 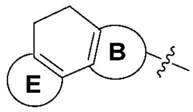, 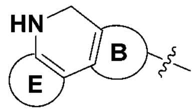, 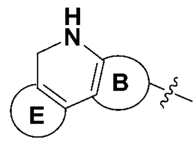,

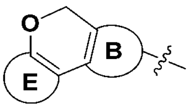, 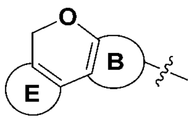, 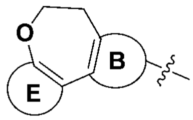, 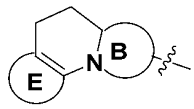, 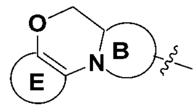,

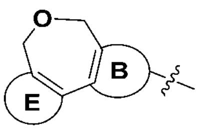, and 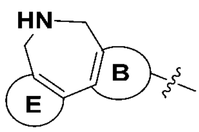;

more preferably, the structural unit

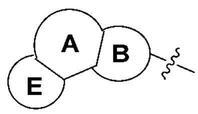

is selected from

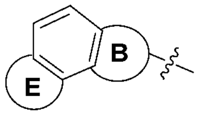 , 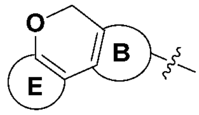 ,

and

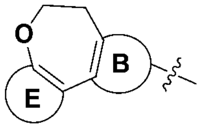 .

8. The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-7, wherein,

ring B is selected from 5-membered nitrogen-containing heteroaryl and 5-membered nitrogen-containing heterocyclic alkenyl;
preferably, ring B is selected from pyrrolyl, pyrazolyl, imidazolyl, oxazolyl and dihydrotriazolyl;
preferably, ring B is selected from pyrrolyl, pyrazolyl, imidazolyl, and oxazolyl;
more preferably, ring B is selected from imidazolyl, pyrazolyl, and oxazolyl.

9. The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-8, wherein,

ring E is 6-membered nitrogen-containing heteroaryl;
preferably, ring E is selected from pyridyl and pyridazinyl;
more preferably, ring E is pyridyl.

10. The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-9, wherein,

the structural unit

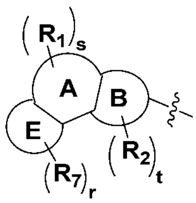

is selected from

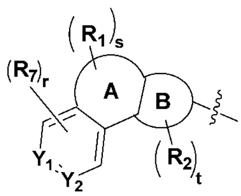

(preferably,

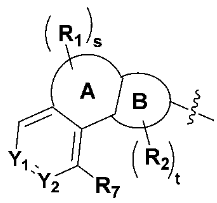

), wherein, $Y_1$, and $Y_2$ are each independently selected from CH and N; preferably, $Y_1$ is N and $Y_2$ is CH, or $Y_1$ is CH and $Y_2$ is N, or $Y_1$ is N, and $Y_2$ is N;
preferably,

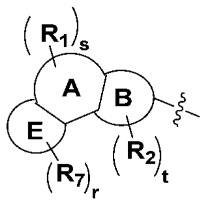

is selected from

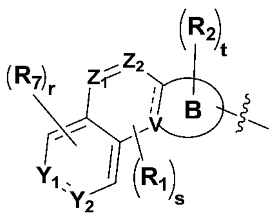

and

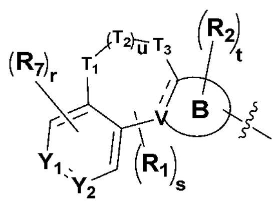

,

wherein,

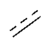

is selected from a single bond and a double bond;
$Z_1$ and $Z_2$ are each independently selected from CH and N;
V is selected from C, CH, and N; preferably, V is selected from C and N;
$T_1$, $T_2$, and $T_3$ are each independently selected from $CH_2$, O, and NH;
u is selected from 0, 1, and 2; preferably, u is selected from 0 and 1;
preferably,

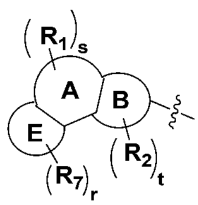

is selected from

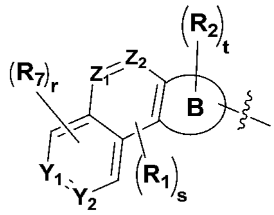 , 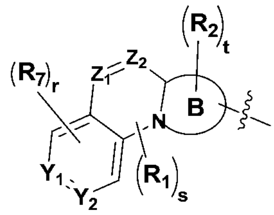 ,

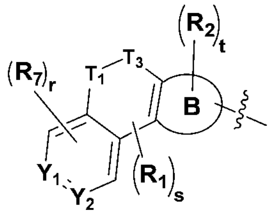 , 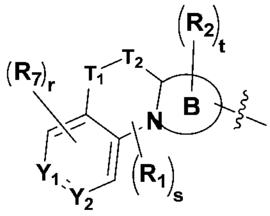 , and 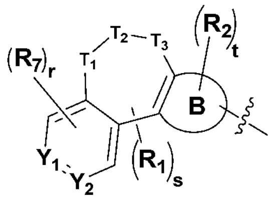 ;

preferably,

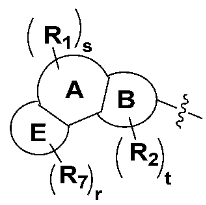

is selected from

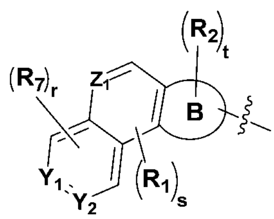 , 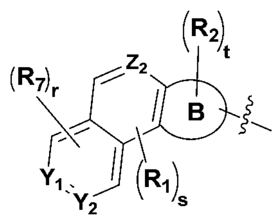 ,

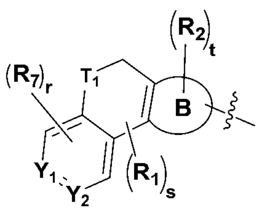 , 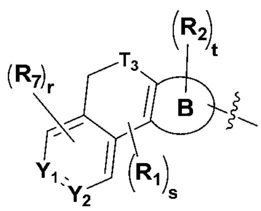 , 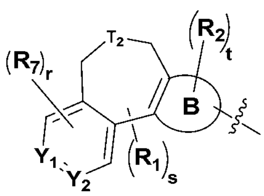 , 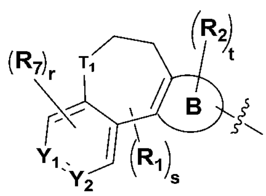 ,

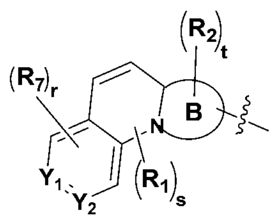 , and 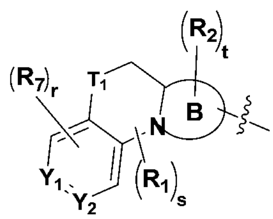 ;

preferably,

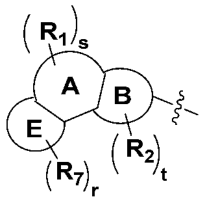

is selected from

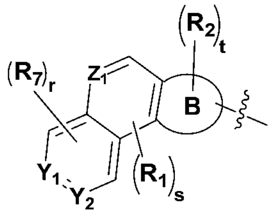,
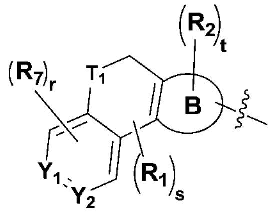,

and

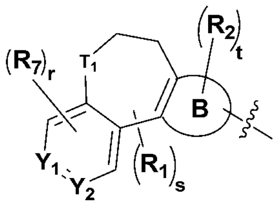.

**11.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-10, wherein,

the structural unit

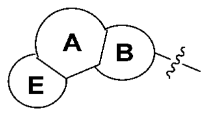

is selected from

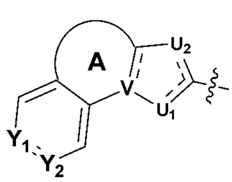,
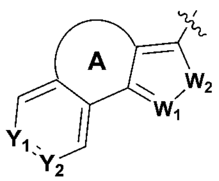,

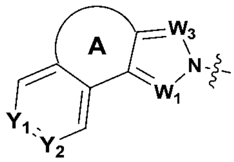, and
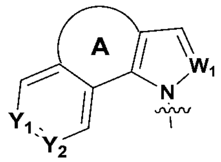,

wherein,

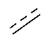

is selected from a single bond and a double bond;

$Y_1$ and $Y_2$ are each independently selected from CH and N;

V is selected from C, CH, and N; preferably, V is selected from C and N;

$U_1$ and $U_2$ are each independently selected from O, N, NH, and CH;

$W_1$ and $W_3$ are selected from CH and N;

$W_2$ is selected from NH and O;

ring A is selected from phenyl, 5-6 membered heteroaryl, $C_{4-8}$ cyclic groups, and 4-8 membered heterocyclyl; preferably, ring A is selected from phenyl, 6 membered heteroaryl, $C_{6-7}$ cyclic groups, and 6-7 membered heterocyclyl; more preferably, ring A is selected from phenyl and 6-7 membered heterocyclyl;

preferably, the structural unit

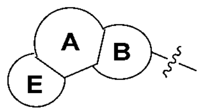

is selected from

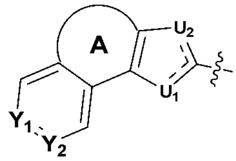 , 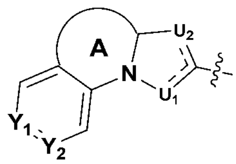 ,

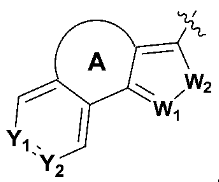 , 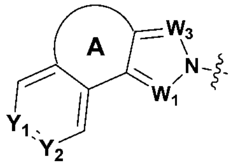 , and 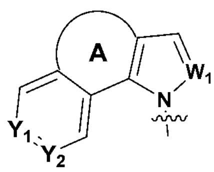 ;

preferably,

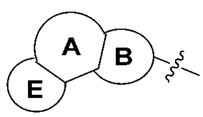

is selected from

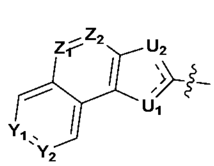 , 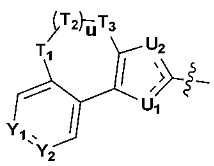 , 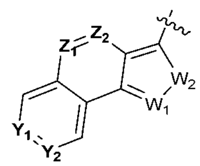 ,

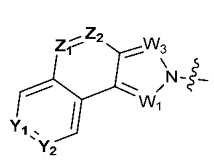 , 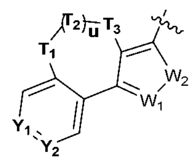 , 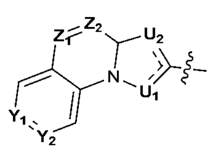 , 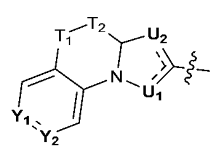 , and 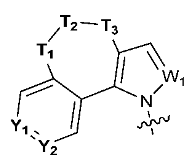 ;

wherein,

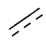

is selected from a single bond and a double bond, and when two

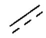

are attached to the same atom, one is a single bond, and the other is a double bond;
$Y_1$ and $Y_2$ are each independently selected from CH and N;
$Z_1$ and $Z_2$ are each independently selected from CH and N;
$U_1$ and $U_2$ are each independently selected from O, N, NH, and CH;
$T_1$, $T_2$, and $T_3$ are each independently selected from $CH_2$, O, and NH;
$W_1$ and $W_3$ are selected from CH and N;
$W_2$ is selected from NH and O;
u is selected from 0 and 1;
preferably, the structural unit

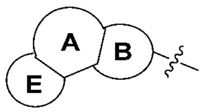

is selected from

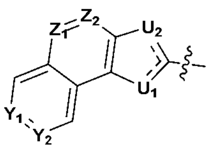 , 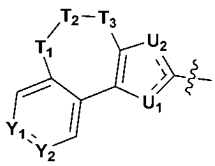 ,

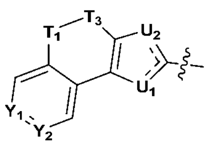 , 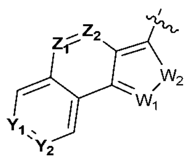 , 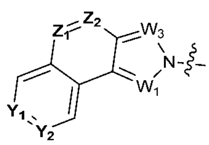 , 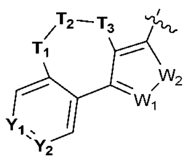 , 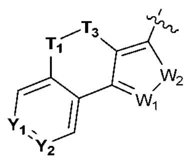 ,

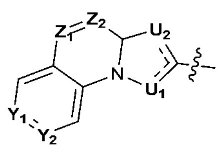 , 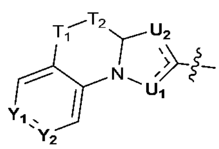 , and 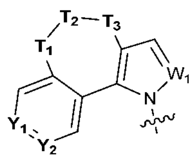 ;

wherein,
in the structural unit

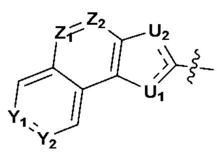 ,

preferably, $Y_1$ is N, and $Y_2$ is CH, or $Y_1$ is N, and $Y_2$ is N; more preferably, $Y_1$ is N, and $Y_2$ is CH;
preferably, $Z_1$ is CH, and $Z_2$ is CH, or $Z_1$ is N, and $Z_2$ is CH, or $Z_1$ is CH, and $Z_2$ is N; more preferably, $Z_1$ is CH, and $Z_2$ is CH;
preferably, $U_1$ is selected from N and CH, $U_2$ is selected from NH and O, or $U_1$ is selected from NH and O, $U_2$ is

selected from N and CH; more preferably, $U_1$ is N, and $U_2$ is NH, or $U_1$ is NH, and $U_2$ is N, or $U_1$ is N, and $U_2$ is O; in the structural unit

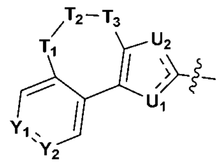

,

preferably, $Y_1$ is N, and $Y_2$ is CH;
preferably, $T_1$ is $CH_2$, $T_2$ is selected from O and NH, and $T_3$ is $CH_2$; or $T_1$ is O, and $T_1$ and $T_3$ are $CH_2$; preferably, $U_1$ is N, and $U_2$ is NH; or $U_1$ is NH, and $U_2$ is N;
in the structural unit

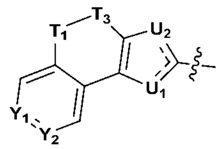

,

preferably, $Y_1$ is N, and $Y_2$ is CH;
preferably, $T_1$ is NH, and $T_3$ is $CH_2$, or $T_1$ is $CH_2$, and $T_3$ is NH, or $T_1$ is O, and $T_3$ is $CH_2$, or $T_1$ is $CH_2$, and $T_3$ is O, or $T_1$ is $CH_2$, and $T_3$ is $CH_2$; more preferably, $T_1$ is O, and $T_3$ is $CH_2$;
preferably, $U_1$ is selected from N and CH, and $U_2$ is NH, or $U_1$ is NH, and $U_2$ is selected from N and CH; more preferably, $U_1$ is N, and $U_2$ is NH, or $U_1$ is NH, and $U_2$ is N;
in the structural unit

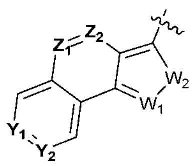

,

preferably, $Y_1$ is N, and $Y_2$ is CH; preferably, $Z_1$ is CH, and $Z_2$ is CH; preferably, $W_1$ is N, and $W_2$ is NH;
in the structural unit

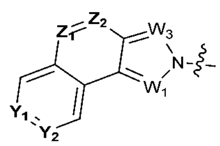

,

preferably, $Y_1$ is N, and $Y_2$ is CH; preferably, $Z_1$ is CH, and $Z_2$ is CH; preferably, $W_1$ is N, and $W_3$ is CH;
in the structural unit

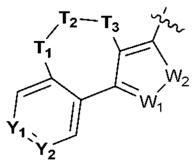

,

preferably, $Y_1$ is N, and $Y_2$ is CH;
preferably, $T_1$ is O, and $T_2$ and $T_3$ are $CH_2$, or $T_1$ is $CH_2$, $T_2$ is O, and $T_3$ is $CH_2$; more preferably, $T_1$ is O, and $T_2$ and $T_3$ are $CH_2$;
preferably, $W_1$ is N, and $W_2$ is NH;
in the structural unit

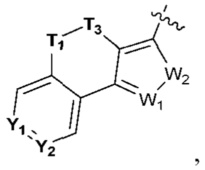

preferably, $Y_1$ is N, and $Y_2$ is CH;
preferably, $T_1$ is O, and $T_3$ is $CH_2$, or $T_1$ is $CH_2$, and $T_3$ is O, or $T_1$ is NH, and $T_3$ is $CH_2$, or $T_1$ is $CH_2$, and $T_3$ is NH;
preferably, $W_1$ is N, and $W_2$ is NH;
in the structural unit

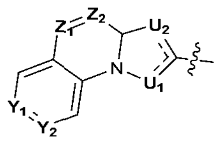

preferably, $Y_1$ is N, and $Y_2$ is CH;
preferably, $Z_1$ is CH, and $Z_2$ is CH;
preferably, $U_1$ is N, and $U_2$ is NH, or $U_1$ is NH, and $U_2$ is N; more preferably, $U_1$ is N, and $U_2$ is NH;
in the structural unit

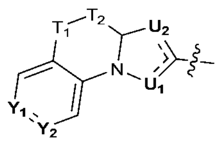

preferably, $Y_1$ is N, and $Y_2$ is CH;
preferably, $T_1$ is selected from $CH_2$ and O, and $T_2$ is $CH_2$;
preferably, $U_1$ is N, and $U_2$ is NH, or $U_1$ is NH, and $U_2$ is N; more preferably, $U_1$ is N, and $U_2$ is NH;
in the structural unit

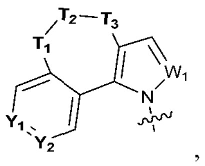

preferably, $Y_1$ is N, and $Y_2$ is CH;
preferably, $T_1$ is O, and $T_2$ and $T_3$ are $CH_2$;
preferably, $W_1$ is N;
more preferably, the structural unit

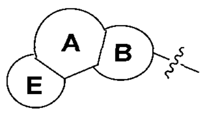

is selected from

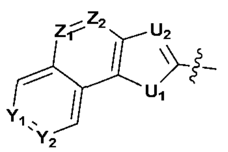

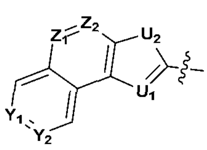, 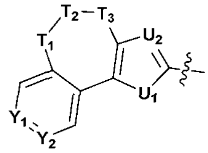, 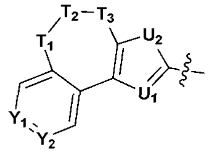, 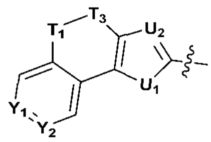, 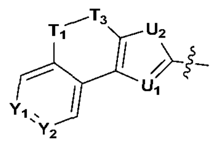,

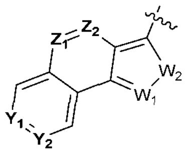, 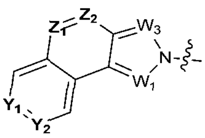, 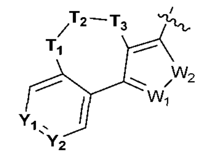, 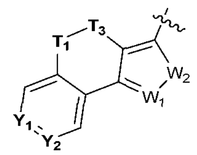, 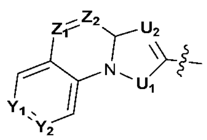,

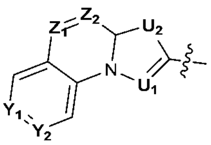, 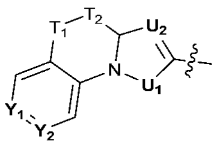, 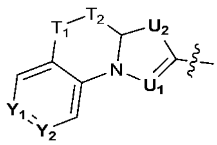, and 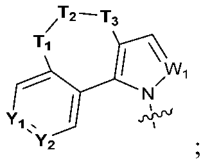;

further preferably,

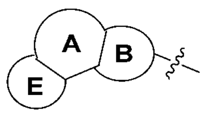

is selected from

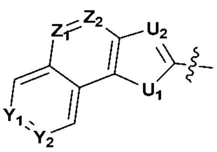, 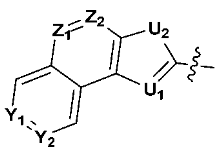,

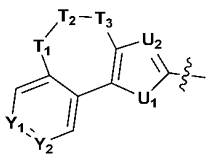, 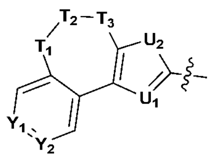, 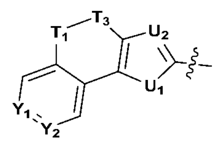, 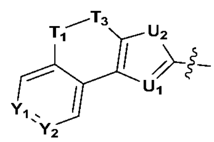, 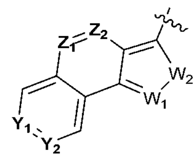,

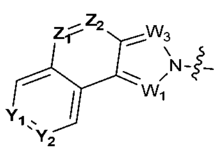, 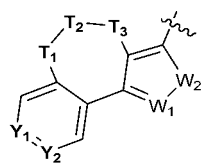, 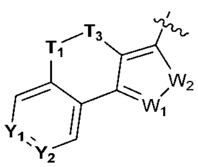, 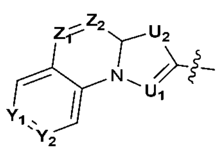, 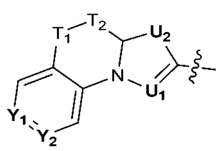,

and

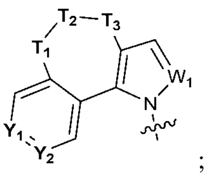;

further preferably, the structural unit

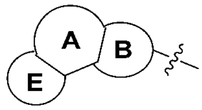

is selected from

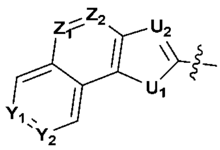

,

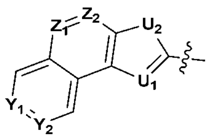 , 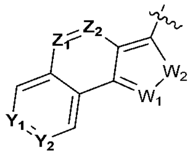 , 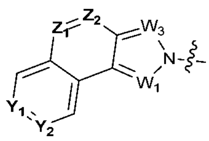 , 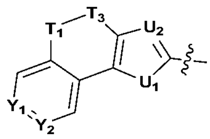 , 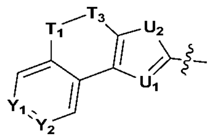 ,

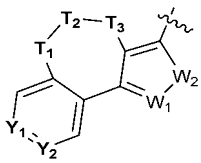 , and 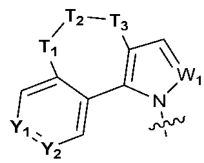 ;

further preferably, the structural unit

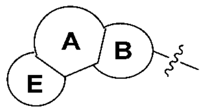

is selected from

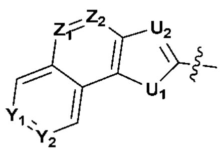

,

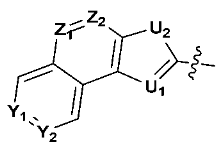 , 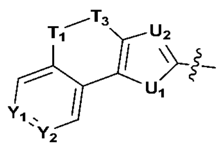 , 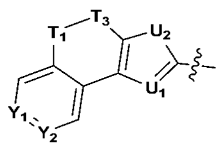 , and 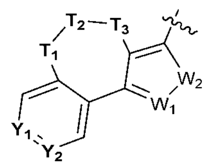 .

**12.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-11, wherein,

the structural unit

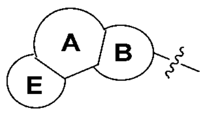

is selected from

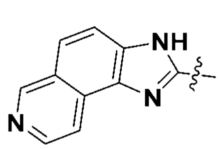, 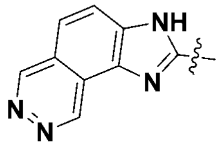,

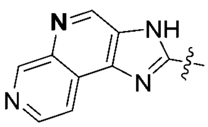, 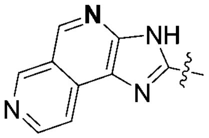, 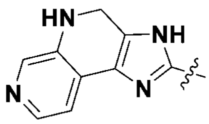, 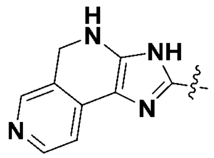, 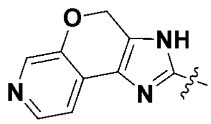,

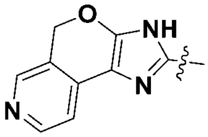, 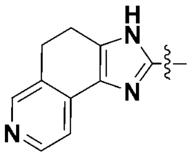, 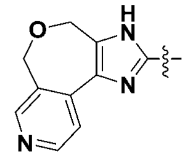, 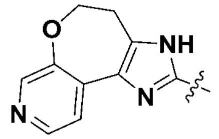, 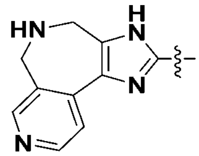,

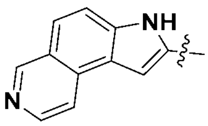, 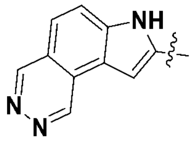, 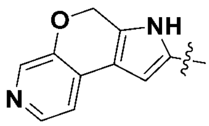, 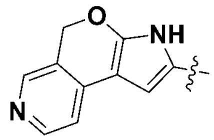, 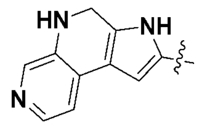,

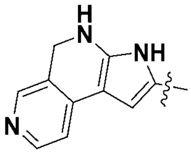, 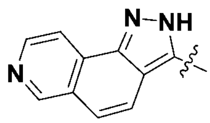, 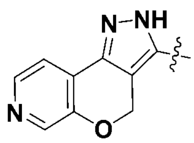, 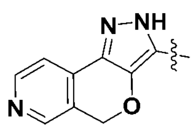, 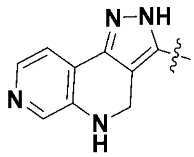,

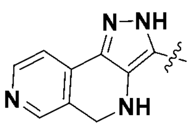, 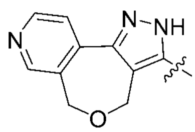, 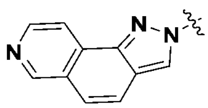, 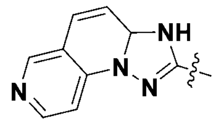, 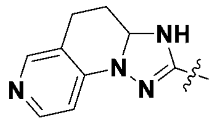,

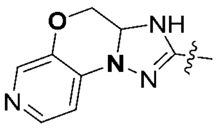, 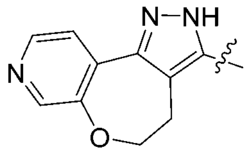, 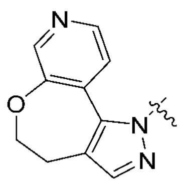, and 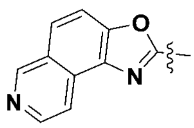;

preferably, the structural unit

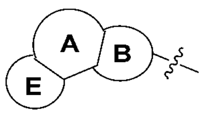

is selected from

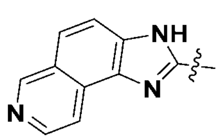, 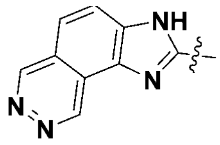,

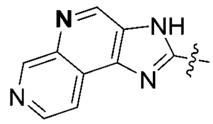, 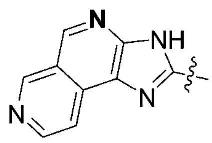, 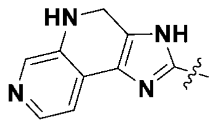, 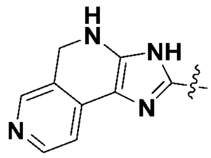, 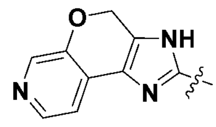,

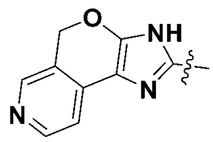, 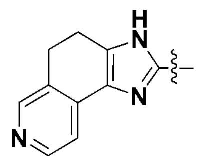, 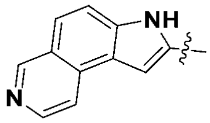, 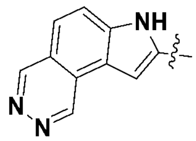, 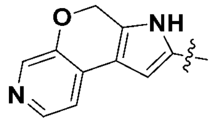,

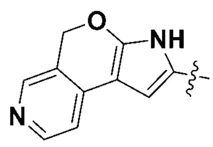, 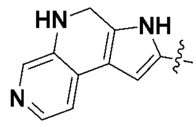, 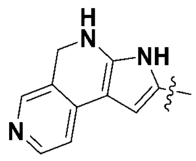, 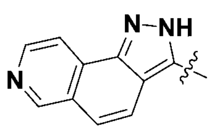, 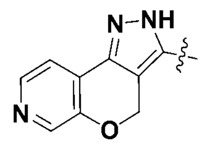,

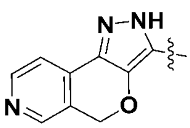, 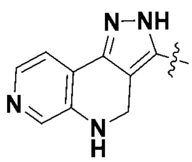, 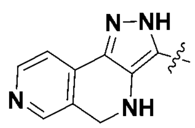, 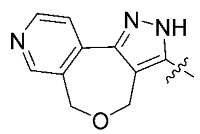, 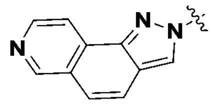,

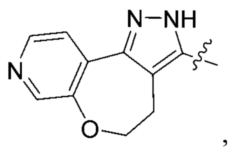, 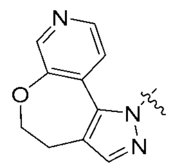, and 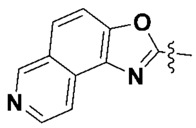;

preferably, the structural unit

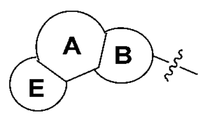

is selected from

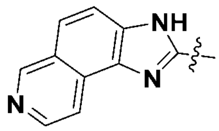, 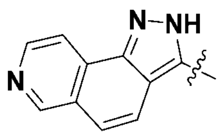,

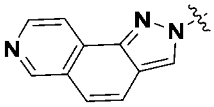, 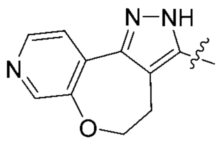, 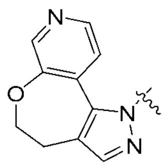, 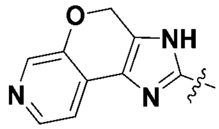, and 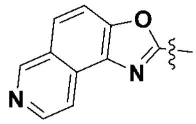;

preferably, the structural unit

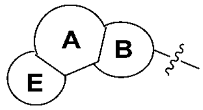

is selected from

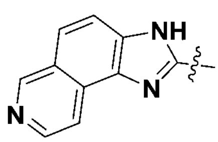, 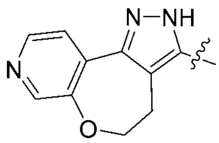,

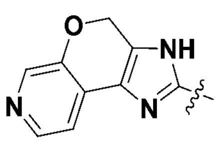, and 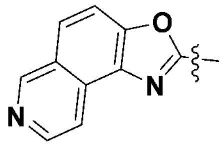;

preferably, the structural unit

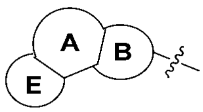

is

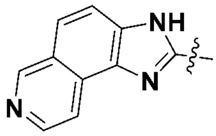.

**13.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-12, wherein,

the structural unit

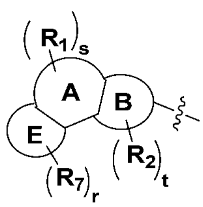

is selected from

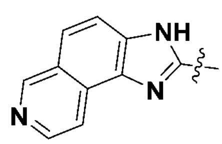,
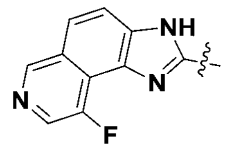,

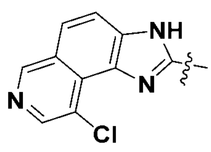,
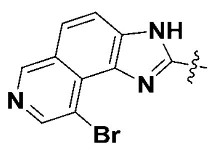,
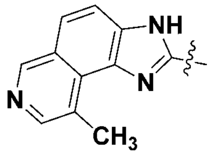,
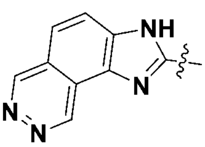,
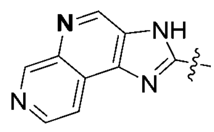,

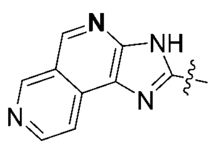,
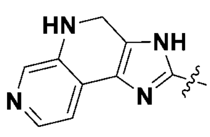,
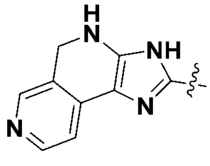,
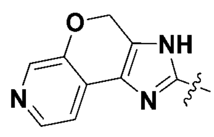,
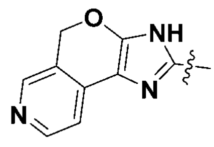,

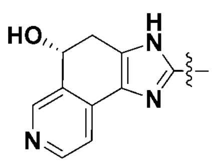,
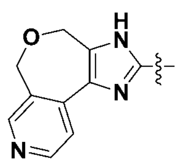,
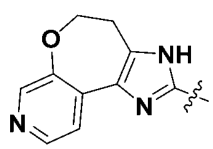,
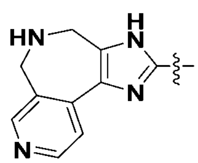,
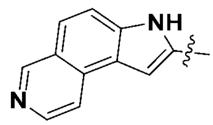,

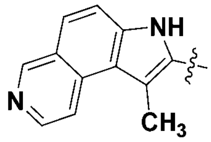,
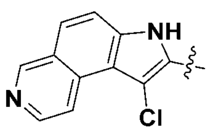,
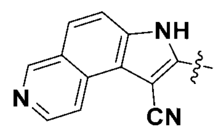,
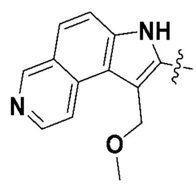,
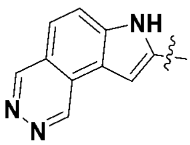,

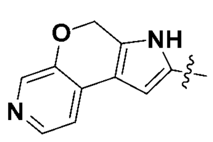,
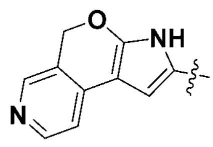,
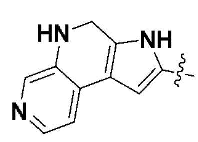,
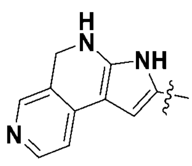,
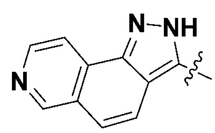,

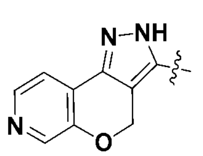,
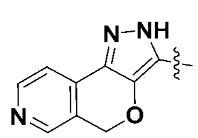,
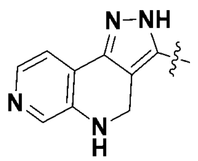,
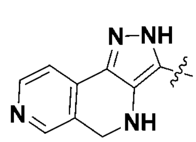,
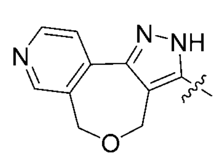,

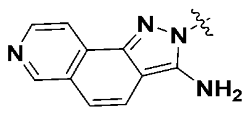,
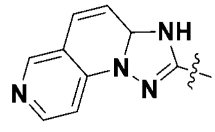,
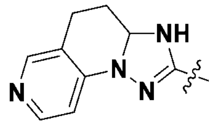,
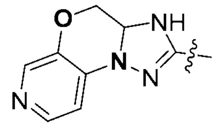,

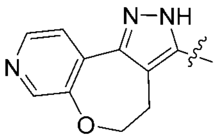, 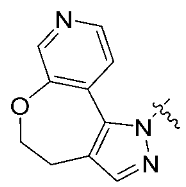, 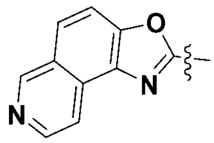, and 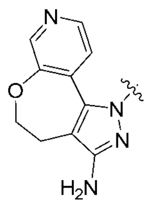;

preferably, the structural unit

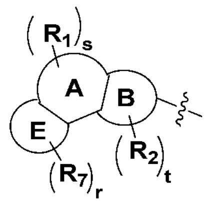

is selected from

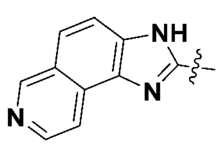, 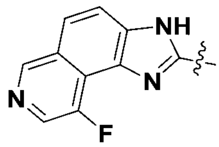,

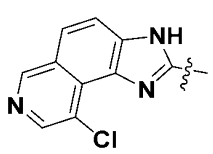, 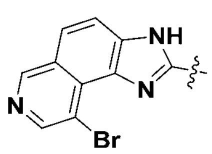, 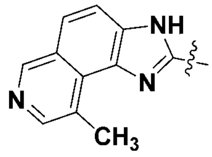, 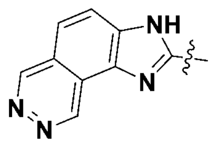, 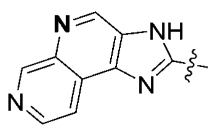,

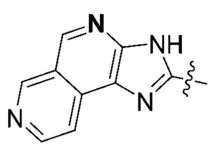, 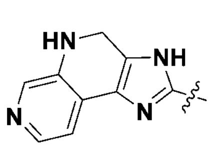, 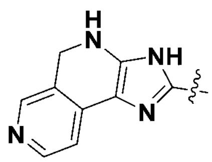, 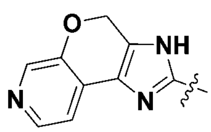, 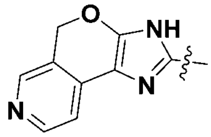,

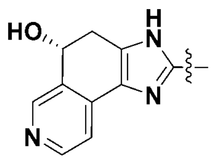, 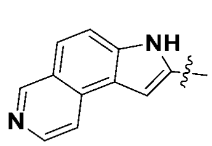, 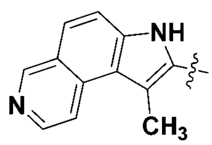, 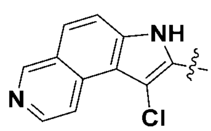, 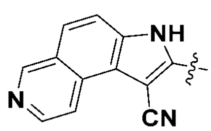,

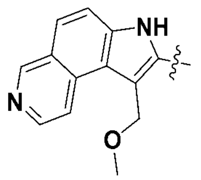, 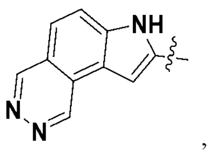, 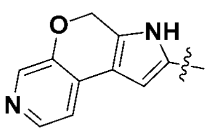, 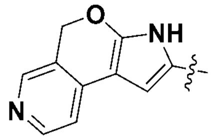, 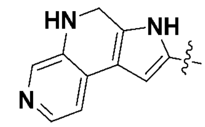,

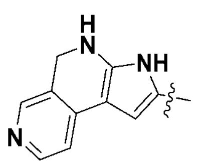, 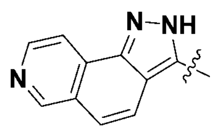, 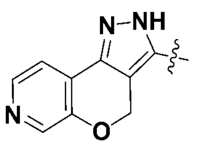, 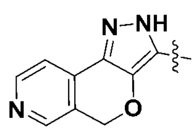, 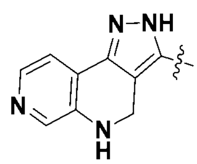,

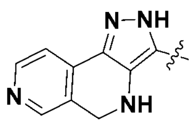 , 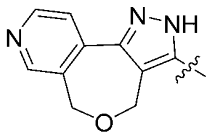 , 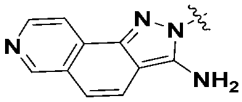 , 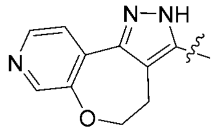 , 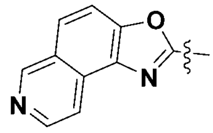 ,

and

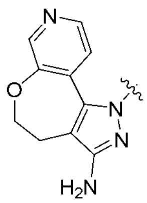 ;

preferably, the structural unit

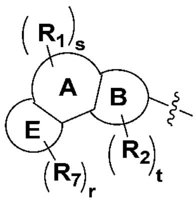

is

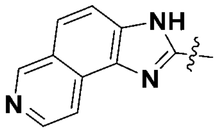 , 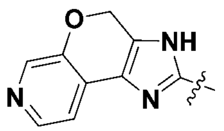 ,

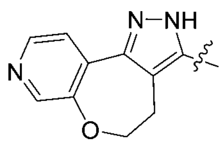 , and 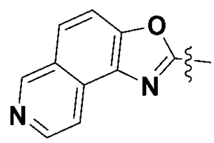 ;

preferably,

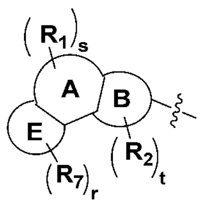

is

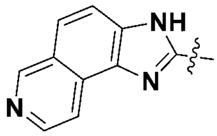 .

**14.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of

claims 1-13, wherein,

W is selected from $-C(R_5R_6)-$, $-C(=CH_2)-$, and $-NR_5-$, wherein $R_5$, $R_6$ are defined as claim 1;
preferably, $R_5$ is selected from H, -OH, $C_{1-4}$ alkyl, $C_{1-4}$ alkoxy, $C_{1-4}$ haloalkyl, and $C_{1-4}$ haloalkoxy; $R_6$ is selected from H, $C_{1-4}$ alkyl, $C_{1-4}$ alkoxy, $C_{1-4}$ haloalkyl, and $C_{1-4}$ haloalkoxy;
preferably, $R_5$ is selected from H, -OH, methyl, ethyl, n-propyl, n-butyl, methoxy, ethoxy, propoxy, butoxy, halomethyl, haloethyl, halopropyl, halobutyl, halomethoxy, haloethoxy, halopropoxy, and halobutoxy; $R_6$ is selected from H, methyl, ethyl, *n*-propyl, *n*-butyl, methoxy, ethoxy, propoxy, butoxy, halomethyl, haloethyl, halopropyl, halobutyl, halomethoxy, haloethoxy, halopropoxy, and halobutoxy;
preferably, $R_5$ is selected from H, -OH, methyl, methoxy, monofluoromethyl, difluoromethyl, and trifluoromethyl; $R_6$ is selected from H, methyl, ethyl, methoxy, monofluoromethyl, difluoromethyl, and trifluoromethyl;
preferably, $R_5$ is selected from H and -OH, and $R_6$ is H;
preferably, $R_5$ is H, and $R_6$ is H;
preferably, W is selected from $-CH_2-$, -CH(OH)-, $-C(=CH_2)-$, and -NH-;
preferably, W is selected from $-CH_2-$ and -NH-;
preferably, W is selected from $-CH_2-$ and $-C(=CH_2)-$;
preferably, W is $-CH_2-$.

**15.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-14, wherein,

$R_3$ is selected from H, $C_{1-4}$ alkyl, and $-C(=O)-C_{1-4}$ alkyl, wherein the $C_{1-4}$ alkyl and $-C(=O)-C_{1-4}$ alkyl are optionally substituted with 1, 2, 3, 4 or 5 substituents selected from halogen, -OH, $-NH_2$, -CN, $-NO_2$, and -COOH;
preferably, $R_3$ is selected from H, methyl, ethyl, *n*-propyl, isopropyl, *n*-butyl, *sec*-butyl, *tert*-butyl, isobutyl, - $C(=O)CH_3$, $-C(=O)CH_2CH_3$, $-C(=O)(CH_2)_2CH_3$, and $-C(=O)(CH_2)_3CH_3$;
preferably, $R_3$ is selected from H, $-CH_3$, and $-C(=O)CH_3$;
further preferably, $R_3$ is H.

**16.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-15, wherein,

the structural unit

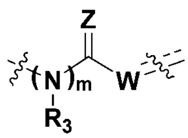

is selected from

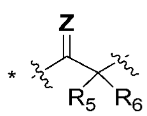 , 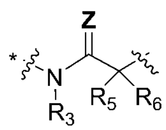 , and 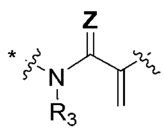 ,

wherein "*" indicates a connection to ring B;
preferably, the structural unit

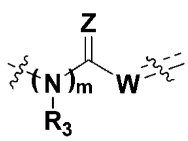

is selected from

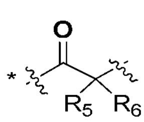, 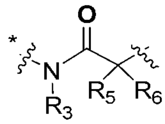, 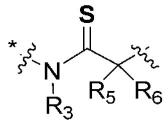,

and

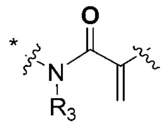;

preferably, the structural unit

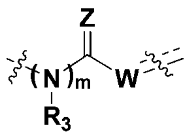

is selected from

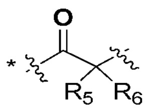, 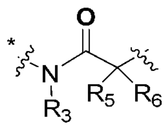,

and

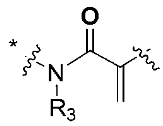;

preferably, the structural unit

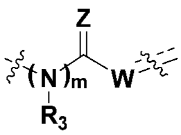 is 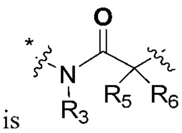;

preferably, the structural unit

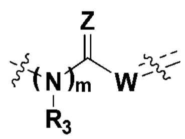

is selected from $-C(=O)-CH_2-$, $-NH-C(=O)-CH_2-$, $-N(CH_3)-C(=O)-CH_2-$, $-N(C(=O)CH_3)-C(=O)-CH_2-$, $-N(CH_3)-C(=O)-CH(OH)-$, $-NH-C(=O)-C(=CH_2)-$, and $-NH-C(=S)-CH_2-$;
preferably, the structural unit

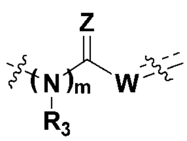

is -NH-C(=O)-$CH_2$-;
or,
the structural unit

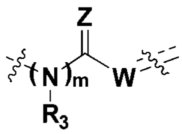 is 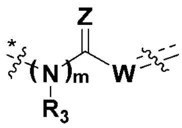,

wherein "*" indicates a connection to ring B;
preferably, the structural unit

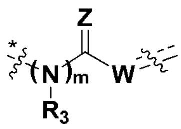

is selected from *-C(=O)-$CH_2$-, *-NH-C(=O)-$CH_2$-, *-N($CH_3$)-C(=O)-$CH_2$-, *-N(C(=O)$CH_3$)-C(=O)-$CH_2$-, *-N($CH_3$)-C(=O)-CH(OH)-, *-NH-C(=O)-C(=$CH_2$)-, and *-NH-C(=S)-$CH_2$-, wherein "*" indicates a connection to ring B;
further preferably, the structural unit

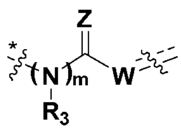

is *-NH-C(-O)-$CH_2$-, wherein "*" indicates a connection ring B.

**17.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-16, wherein,

R is selected from halogen, -OH, -CN, =O, $C_{1-4}$ alkyl, $C_{1-4}$ alkoxy, $C_{1-4}$ haloalkyl, and $C_{1-4}$ haloalkoxy;
preferably, R is selected from F, Cl, Br, I, -OH, -CN, =O, methyl, ethyl, *n*-propyl, isopropyl, *n*-butyl, *sec*-butyl, isobutyl, *tert*-butyl, methoxy, ethoxy, propoxy, butoxy, halomethyl, haloethyl, halopropyl, halobutyl, halomethoxy, haloethoxy, halopropoxy, and halobutoxy;
preferably, R is selected from F, Cl, Br, I, -OH, -CN, =O, methyl, ethyl, n-propyl, methoxy, ethoxy, monofluoromethyl, difluoromethyl, and trifluoromethyl;
preferably, R is selected from F, Cl, Br, -OH, =O, methyl, ethyl, monofluoromethyl, difluoromethyl, and trifluoromethyl;
preferably, R is selected from -OH, -$CH_3$, and =O.

**18.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-17, wherein,

the structural unit

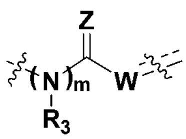

is selected from

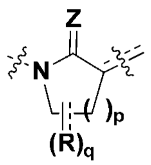 and 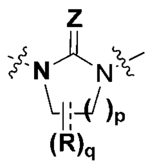 ,

wherein p is selected from 0, 1, and 2; q is selected from 0, 1, 2, 3, 4, and 5;

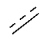

is selected from a single bond and a double bond;
preferably,

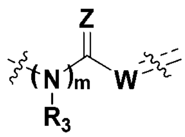

is selected from

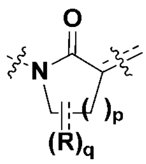 and 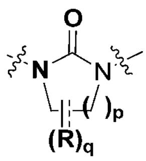 ; preferably, 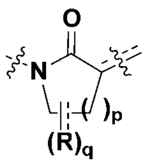 ;

preferably,

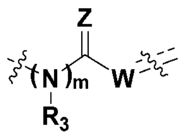

is selected from

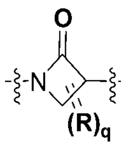 , 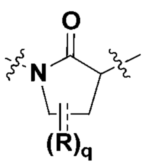 , 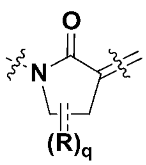 , 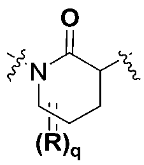 ,

and

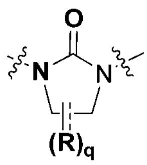 ;

preferably,

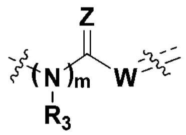

is selected from

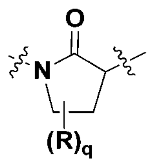 and 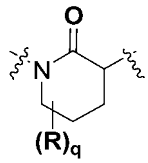 ;

preferably, the structural unit

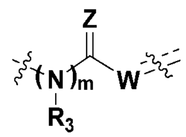

is selected from

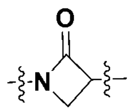 , 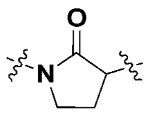 , 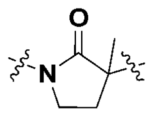 ,

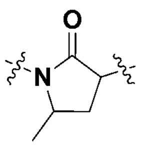 , 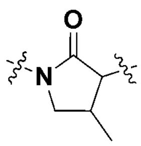 , 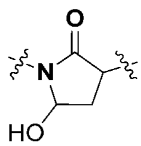 , 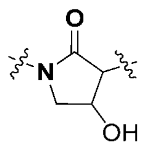 , 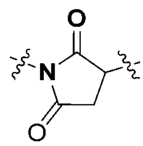 , 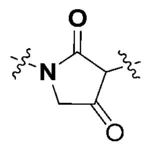 , 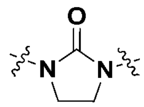 ,

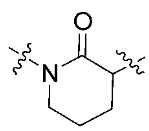 , and 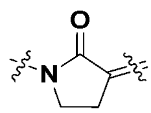 ;

further preferably, the structural unit

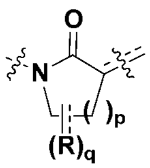

is selected from

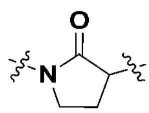 and 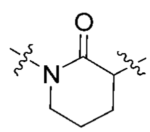 ;

or,
the structural unit

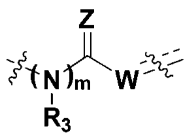

is

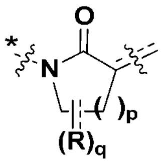 or 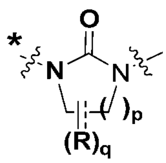 ,

wherein "*" indicates a connection to ring B;
preferably, the structural unit

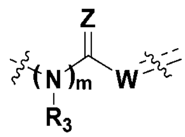

is selected from

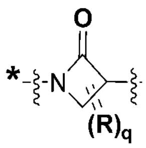 , 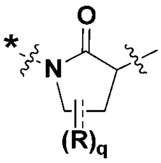 , 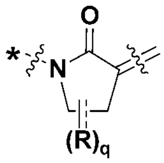 ,

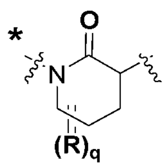 , and 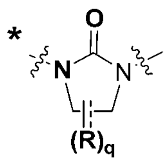 ,

wherein "*" indicates a connection to ring B;
preferably,

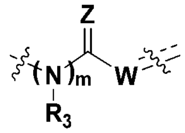

is selected from

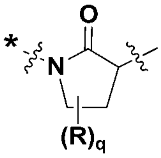 and 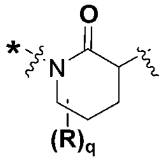 ,

wherein "*" indicates a connection to ring B;
preferably, the structural unit

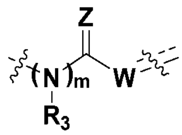

is selected from

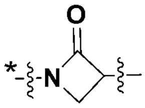, 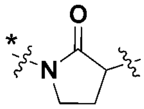, 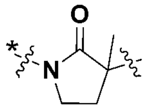,

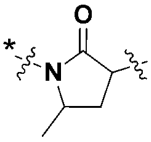, 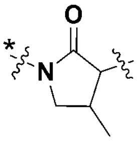, 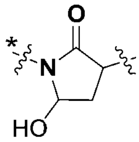, 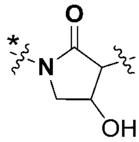, 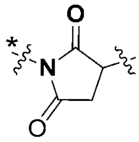, 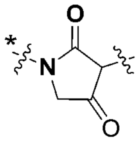, 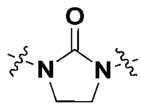,

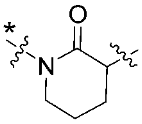, and 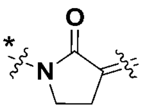,

wherein "*" indicates a connection to ring B;
further preferably, the structural unit

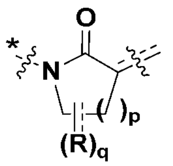

is selected from

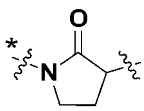 and 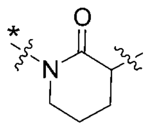,

wherein "*" indicates a connection to ring B.

**19.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-18, wherein,

W and ring B together with the atoms to which they are attached form a 7-9 membered heterocyclyl;
preferably, W and ring B together with the atoms to which they are attached form a 7 membered heterocyclyl;
preferably, the 7 membered heterocyclyl is selected from

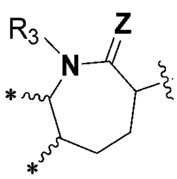

, wherein "*" indicates a fusion with ring B;
preferably, the 7 membered heterocyclyl is selected from

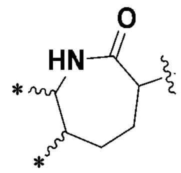

, wherein "*" indicates a fusion with ring B.

**20.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-19, wherein,

$L_1$ is absent, or $L_1$ is selected from $-CH_2-$, $-CH_2CH_2-$, =CH-, -NH-, and -O-;
preferably, $L_1$ is $-CH_2-$.

**21.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-20, wherein,

$R_4$ is selected from halogen, -OH, -CN, $-NH_2$, $C_{1-4}$ alkyl, $C_{1-4}$ alkoxy, $C_{1-4}$ haloalkyl, and $C_{1-4}$ haloalkoxy;
preferably, $R_4$ is selected from F, Cl, Br, I, -OH, -CN, $-NH_2$, methyl, ethyl, n-propyl, isopropyl, n-butyl, isobutyl, *sec*-butyl, *tert*-butyl, methoxy, ethoxy, propoxy, butoxy, halomethyl, haloethyl, halopropyl, halobutyl, halomethoxy, haloethoxy, halopropoxy, and halobutoxy;
preferably, $R_4$ is selected from F, Cl, Br, -OH, -CN, $-NH_2$, methyl, ethyl, methoxy, ethoxy, monofluoromethyl, difluoromethyl and trifluoromethyl;
preferably, $R_4$ is selected from F, Cl, -OH, -CN, $-NH_2$, methyl, and trifluoromethyl;
preferably, $R_4$ is selected from F, Cl, and -CN.

**22.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-21, wherein,

ring C is selected from $C_{6-10}$ aryl, 5-10 membered heteroaryl, $C_{3-8}$ cyclic groups, 4-6 membered heterocyclyl, and 5-8 membered heterocyclyl;
preferably, ring C is selected from phenyl, 5-6 membered heteroaryl, and 5-6 membered heterocyclyl;
further preferably, ring C is selected from phenyl, thienyl, thiazolyl, pyridyl, 1,3-benzodioxolyl and benzodioxolyl;
further preferably, ring C is selected from phenyl, thienyl, thiazolyl, and pyridyl;
further preferably, ring C is selected from phenyl and pyridyl.

**23.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-22, wherein,

the structural unit

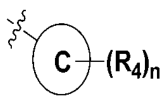

is selected from

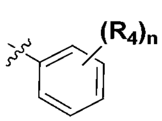 , 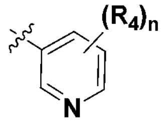 , 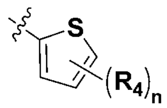 ,

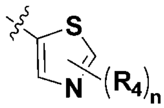 , and 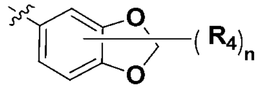 ;

preferably, the structural unit

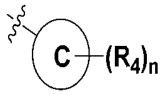

is selected from

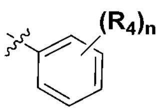 and 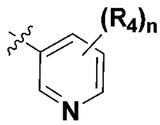 ;

preferably, the structural unit

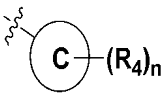

is selected from

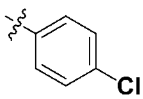 , 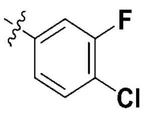 , 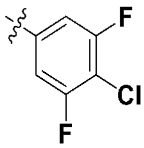 ,

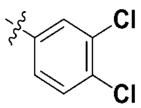 , 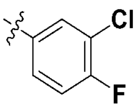 , 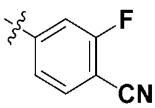 , 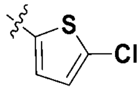 , 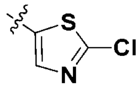 , 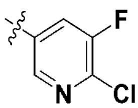 , 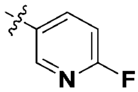 ,

and

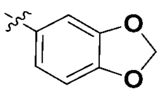 ;

preferably, the structural unit

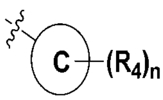

is selected from

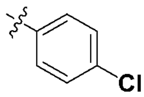, 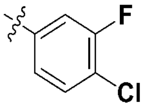, 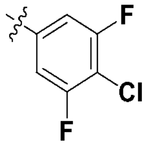,

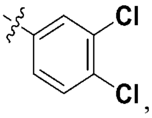, 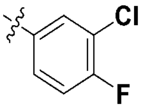, and 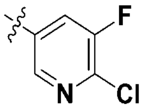;

further preferably, the structural unit

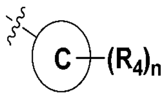

is selected from

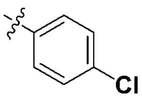, 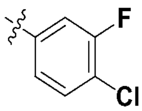,

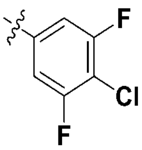, 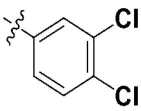, and 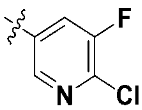;

further preferably, the structural unit

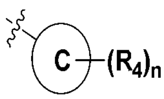

is selected from

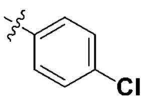, 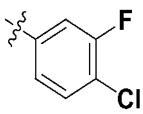,

and

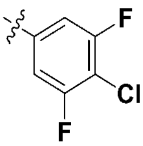.

**24.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-23, wherein,

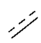

is selected from a single bond and a double bond; preferably,

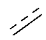

is a single bond;

Z is selected from O and S; preferably, Z is O;

$L_1$ is selected from -$C_{1-3}$ alkylene-, =CH-, -NH-, and -O-, or $L_1$ is absent; preferably, $L_1$ is selected from -$C_{1-3}$ alkylene-, =CH-, -NH-, and -O-; more preferably, $L_1$ is selected from -$C_{1-3}$ alkylene-;

ring A is selected from phenyl, 5-6 membered heteroaryl, $C_{4-8}$ cyclic groups, and 4-8 membered heterocyclyl; preferably, ring A is selected from phenyl, 6 membered heteroaryl, $C_6$ cyclic groups, and 6-7 membered heterocyclyl; more preferably, ring A is selected from phenyl and 6-7 membered heterocyclyl;

ring B is selected from 5-6 membered heteroaryl and 5-6 membered heterocyclyl; preferably, ring B is selected from 5 membered heteroaryl and 5 membered heterocyclyl; more preferably, ring B is 5 membered heteroaryl;

ring C is selected from $C_{6-10}$ aryl, 5-12 membered heteroaryl, $C_{3-12}$ cyclic groups, and 4-10 membered heterocyclyl; preferably, ring C is selected from $C_{6-10}$ aryl, 5-12 membered heteroaryl, and 4-10 membered heterocyclyl; more preferably, ring C is selected from phenyl, 5-6 membered heteroaryl, 5-10 membered heterocyclyl (such as 9-10 membered heterocyclyl); further preferably, ring C is selected from phenyl and 6 membered heteroaryl; more preferably, ring C is selected from phenyl and pyridyl;

ring E is selected from 5-6 membered heteroaryl; preferably, ring E is selected from 6 membered heteroaryl;

$R_1$ is selected from H, -OH, halogen, $C_{1-3}$ alkyl, and $C_{1-3}$ haloalkyl; preferably, H and -OH; more preferably H;

$R_2$ is selected from H, -OH, halogen, -CN, -$NH_2$, $C_{1-6}$ alkyl, $C_{1-6}$ haloalkyl, $C_{1-6}$ alkoxy, -O-$C_{1-6}$ haloalkyl, and -$C_{1-6}$ alkylene-$C_{1-6}$ alkoxy; preferably, H, halogen, -CN, -$NH_2$, $C_{1-6}$ alkyl, and -$C_{1-6}$ alkylene-$C_{1-6}$ alkoxy; more preferably H and -$NH_2$;

$R_3$ is selected from H, $C_{1-6}$ alkyl, and -C(=O)$C_{1-6}$ alkyl; preferably, H;

W is selected from -C($R_5R_6$)-, -$CR_5$=, -C(=$CH_2$)-, and -$NR_5$-, wherein $R_5$ is selected from H and -OH; or

W and $R_3$ together with the atoms to which they are attached form a 4-6 membered heterocyclyl, wherein the 4-6 membered heterocyclyl is optionally substituted with 1, 2, 3, 4 or 5 R; or

W and ring B together with the atoms to which they are attached form a 6-10 membered heterocyclyl, wherein the 6-10 membered heterocyclyl is optionally substituted with 1, 2, 3, 4 or 5 R;

preferably, W is selected from -C($R_5R_6$)- and -C(=$CH_2$)-, wherein $R_5$ is selected from H and -OH; or

W and $R_3$ together with the atoms to which they are attached form a 4-6 membered heterocyclyl, wherein the 4-6 membered heterocyclyl is optionally substituted with 1, 2, 3, 4 or 5 R; or

W and ring B together with the atoms to which they are attached form a 7-9 membered heterocyclyl, wherein the 7-9 membered heterocyclyl is optionally substituted with 1, 2, 3, 4 or 5 R;

more preferably, W is selected from -C($R_5R_6$)- and -C(=$CH_2$)-, wherein $R_5$ is H; or

W and $R_3$ together with the atoms to which they are attached form a 4-6 membered heterocyclyl, wherein the 4-6 membered heterocyclyl is optionally substituted with 1, 2, 3, 4 or 5 R;

further preferably, W is selected from -C($R_5R_6$)-, wherein $R_5$ is H; or

W and $R_3$ together with the atoms to which they are attached form a 5-6 membered heterocyclyl, wherein the 5-6 membered heterocyclyl is optionally substituted with 1, 2, 3, 4 or 5 R;

$R_4$ is selected from halogen and -CN; preferably halogen;

$R_6$ is selected from Hand $C_{1-6}$ alkyl; preferably H;

$R_7$ is selected from H, halogen, and $C_{1-6}$ alkyl; preferably H;

R is selected from halogen, -OH, =O, and $C_{1-6}$ alkyl; preferably R is selected from -OH, =O, and $C_{1-6}$ alkyl;

m is selected from 0 and 1; preferably 1;

n is selected from 0, 1, 2, and 3; preferably 1, 2, and 3;

r, s, and t are each independently 0, 1, or 2; preferably 0 or 1; more preferably 0.

**25.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-24, wherein,

the compound is selected from the compounds represented by Formula (I-A)

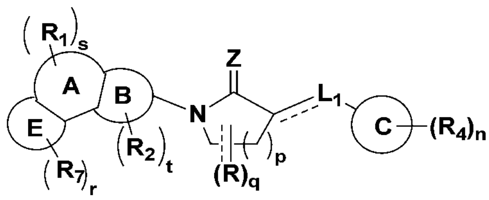

(I-A)

or

the compound is selected from the compounds represented by Formula (I-B)

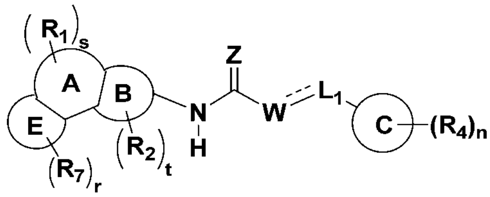

(I-B)

preferably,

(1) the compound is selected from the compounds represented by Formula (I-1)

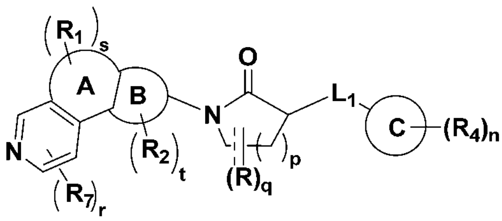

(I-1)

or

the compound is selected from the compounds represented by Formula (I-2)

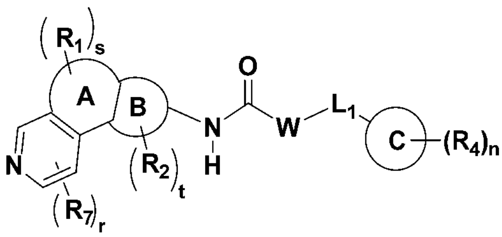

(I-2)

further preferably,

the compound is selected from the compounds represented by Formula (I-3)

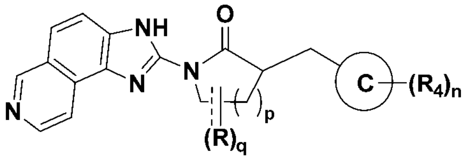

(I-3)

or

the compound is selected from the compounds represented by Formula (I-4)

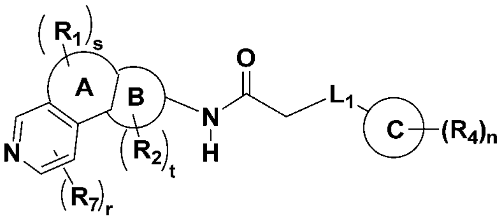

(I-4)

or

(2) the compound is selected from the compounds represented by Formula (I-5)

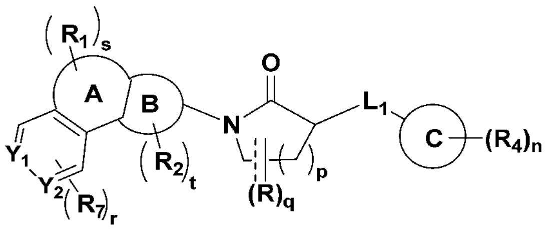

(I-5)

or

the compound is selected from the compounds represented by Formula (I-6)

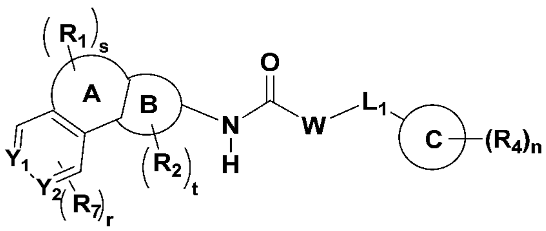

(I-6)

or

(3) the compound is selected from the compounds represented by Formula (I-7)

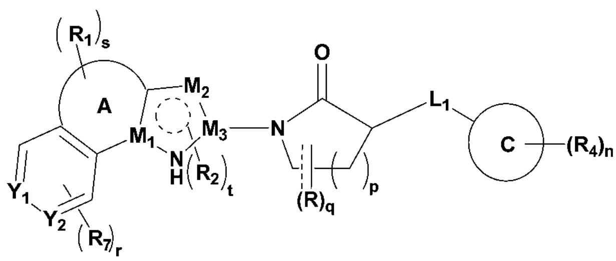

(I-7)

wherein,

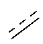

is selected from a single bond and a double bond; $M_1$, $M_2$, and $M_3$ are each independently C, CH or N; or the compound is selected from the compounds represented by Formula (I-8)

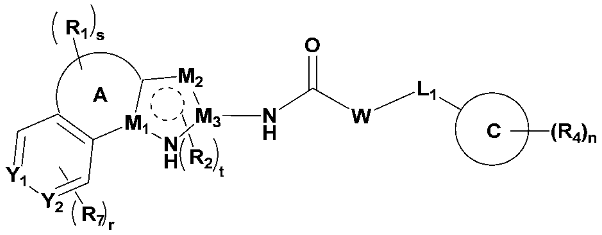

(I-8)

wherein,

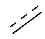

is selected from a single bond and a double bond; $M_1$, $M_2$, and $M_3$ are each independently C, CH, or N; or

(4) the compound is selected from the compounds represented by Formula (I-9')

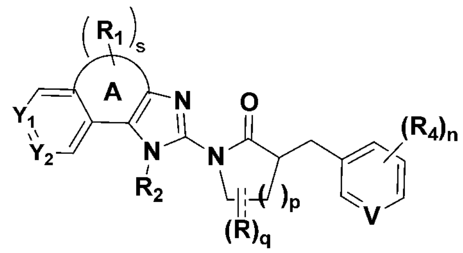

(I-9')

wherein, V is selected from CH and N; or
the compound is selected from the compounds represented by Formula (I-10')

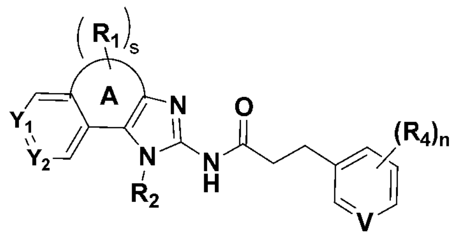

(I-10')

wherein, V is selected from CH and N.

**26.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-25, having a structure represented by Formula (II-A) to Formula (II-P):

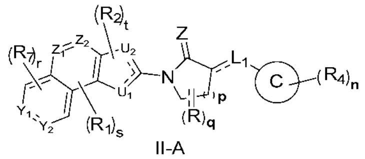

II-A ,

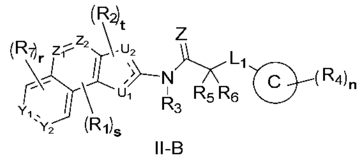

II-B ,

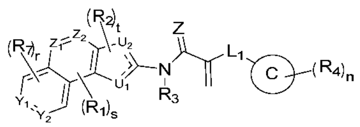

II-C ,

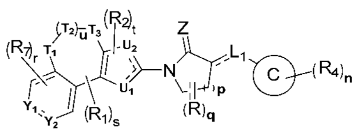
II-D ,
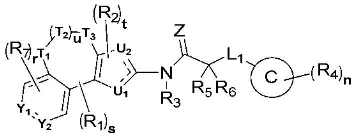
II-E ,
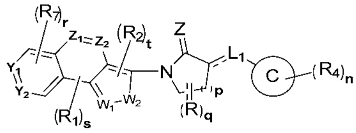
II-F ,

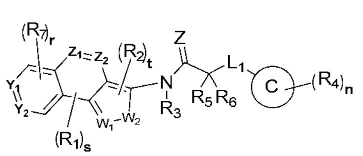
II-G ,
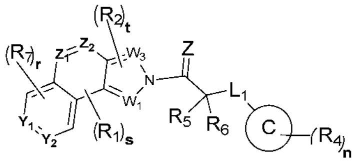
II-H ,
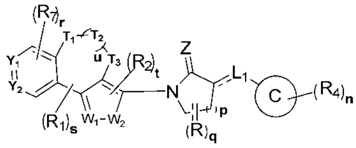
II-I ,

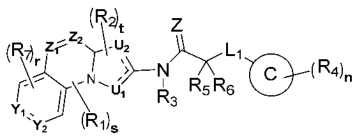
II-J ,
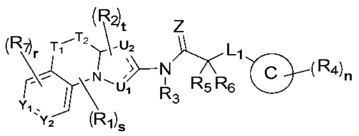
II-L ,
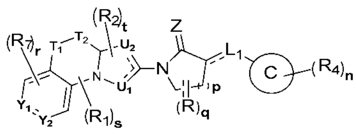
II-M ,

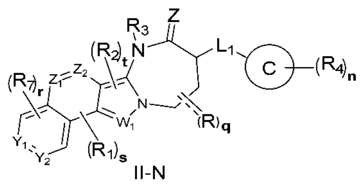
II-N ,
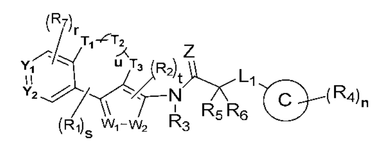
II-O , and
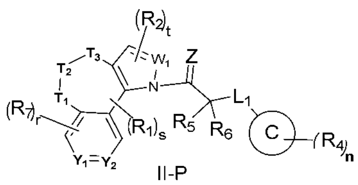
II-P ;

preferably, having a structure represented by Formula (II-1) to Formula (II-11):

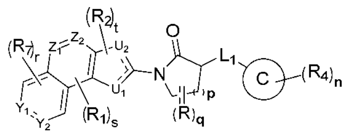
II-1 ,
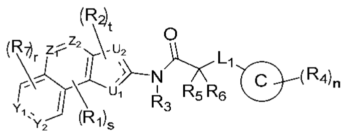
II-2 ,
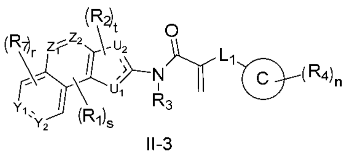
II-3 ,

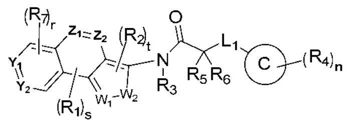
II-4 ,
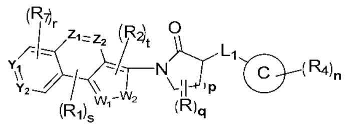
II-5 ,
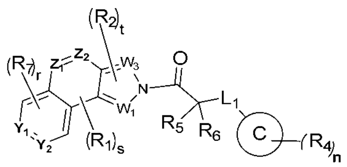
II-6 ,

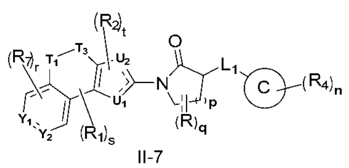
II-7 ,
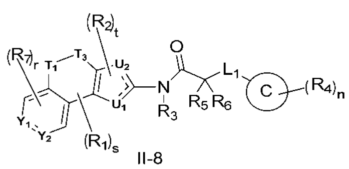
II-8 ,
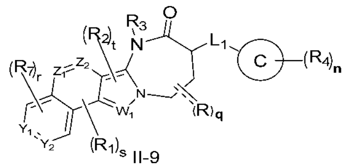
II-9 ,

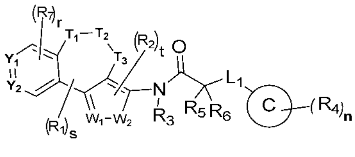

II-10

, and

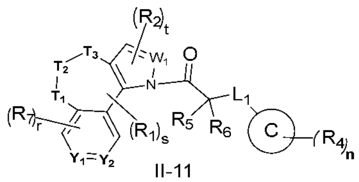

II-11

;

more preferably, having a structure represented by Formula (III-1) - Formula (III-5):

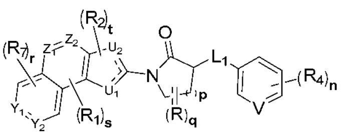

III-1

,

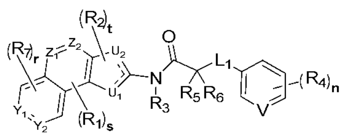

III-2

,

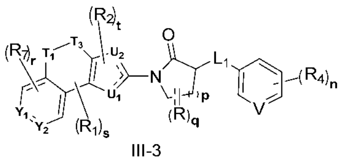

III-3

,

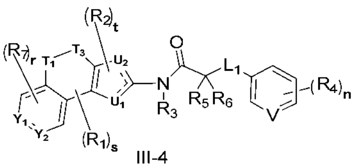

III-4

, and

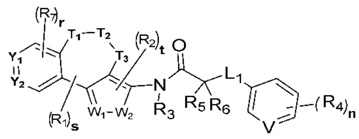

III-5

.

**27.** The compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-26, wherein, the compound is selected from:

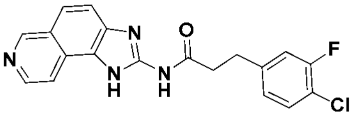

,

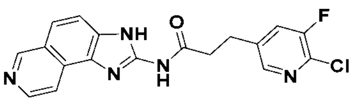

,

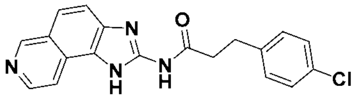

,

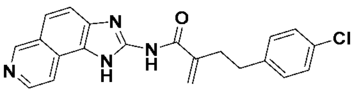

,

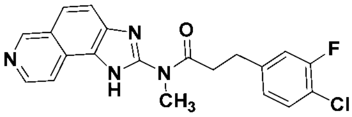

,

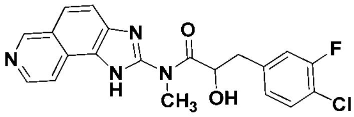

,

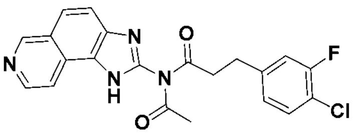

,

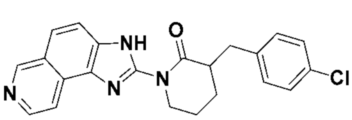

,

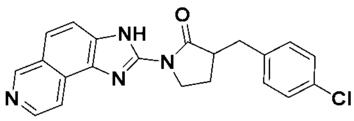

,

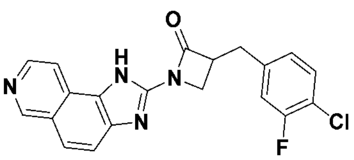

,

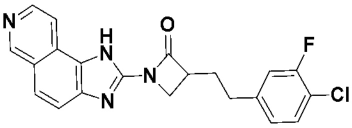

,

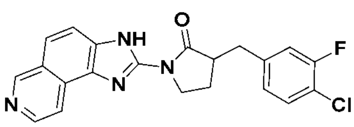

,

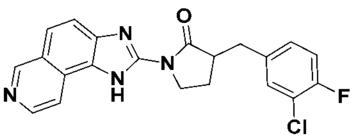

,

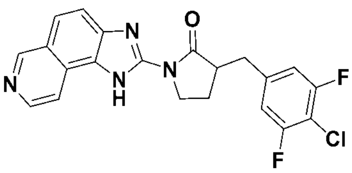

,

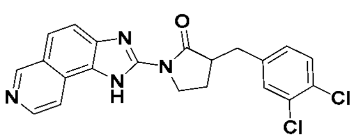

,

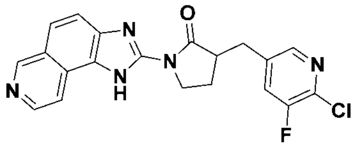 , 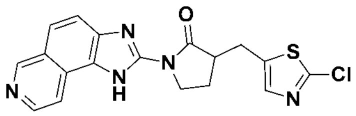 , 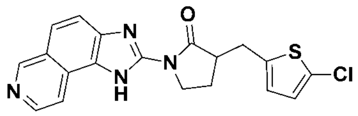 ,

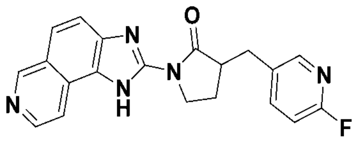 , 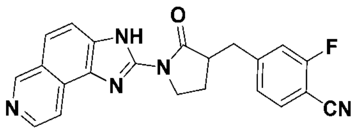 , 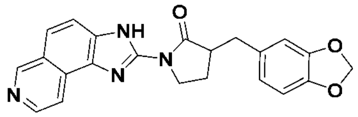 ,

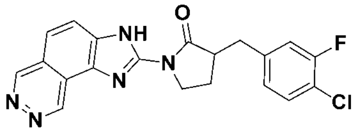 , 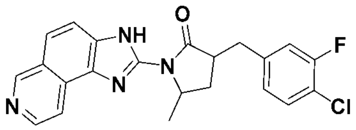 , 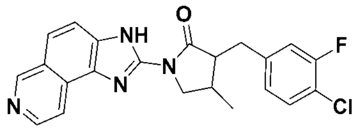 ,

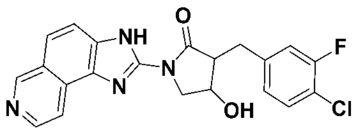 , 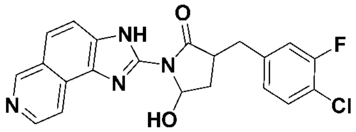 , 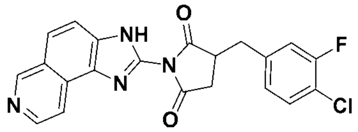 ,

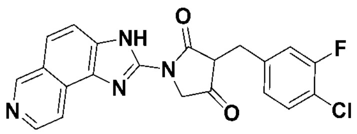 , 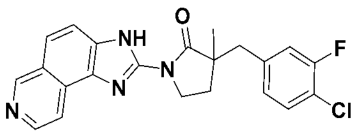 , 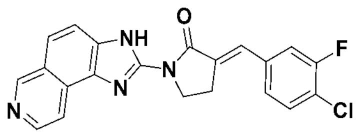 ,

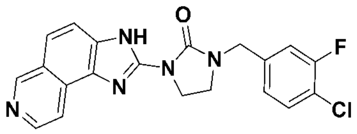 , 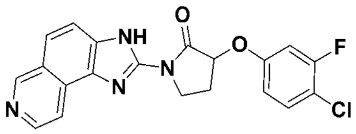 , 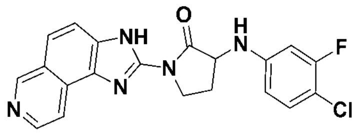 ,

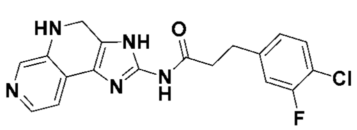 , 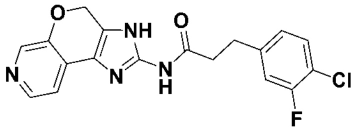 , 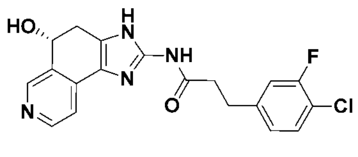 ,

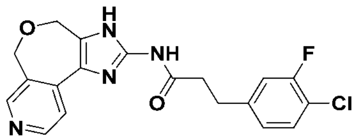 , 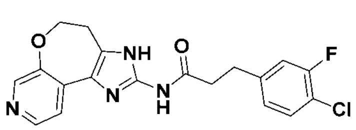 , 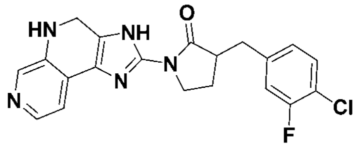 ,

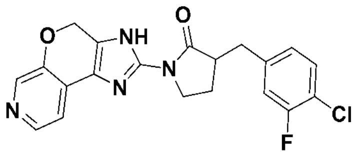 , 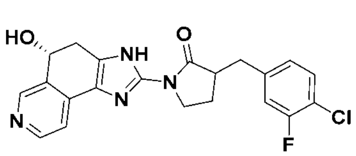 , 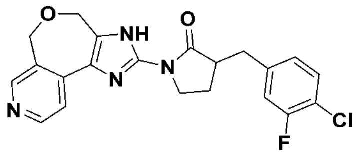 ,

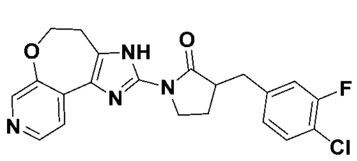, 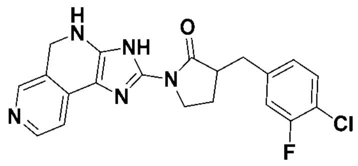, 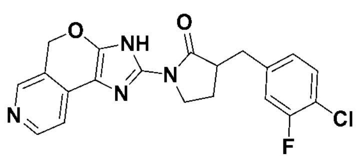,

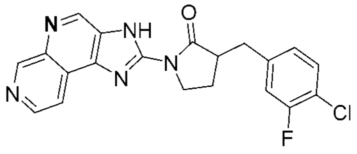, 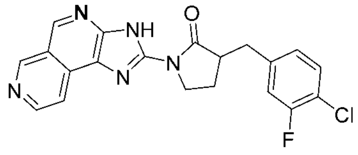, 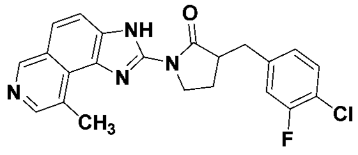,

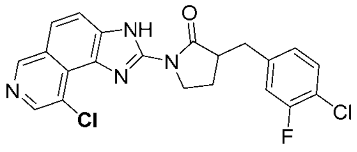, 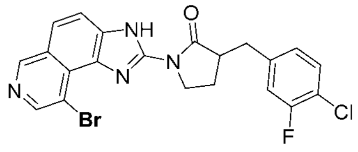, 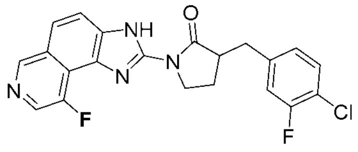,

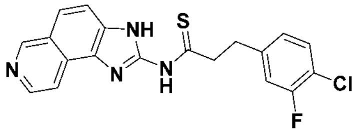, 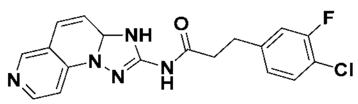, 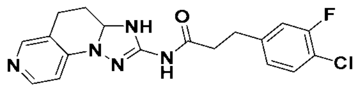,

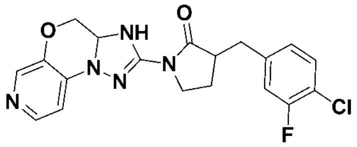, 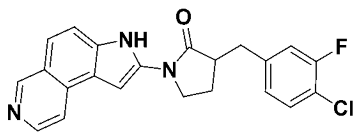, 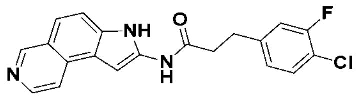,

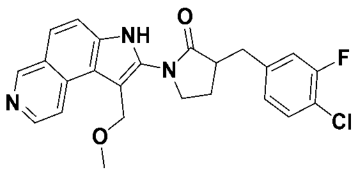, 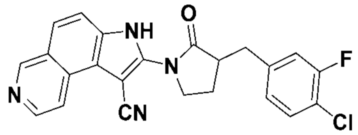, 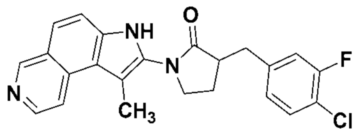,

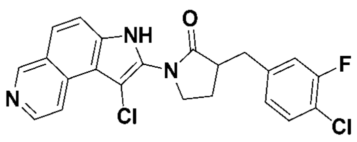, 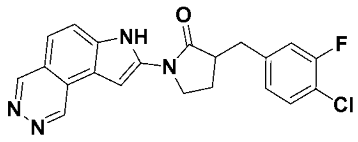, 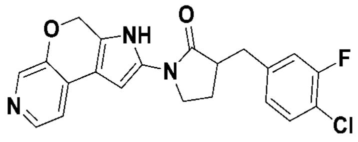,

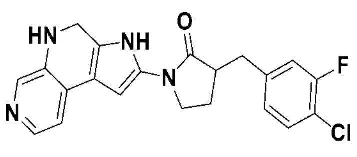, 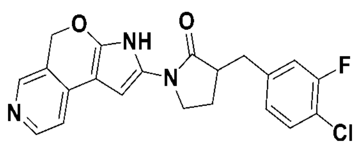, 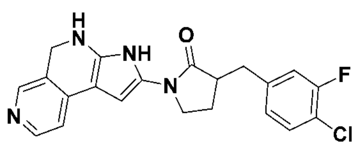,

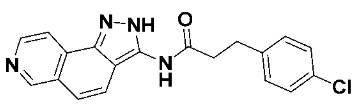, 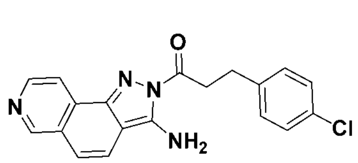, 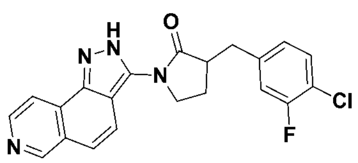,

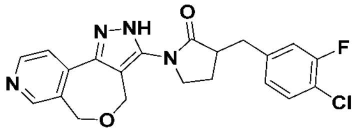, 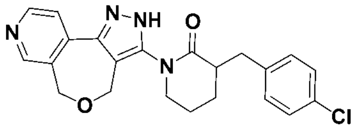, 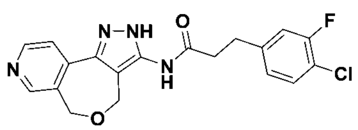,

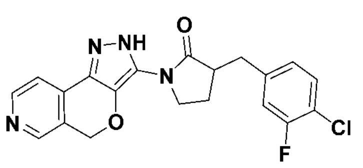, 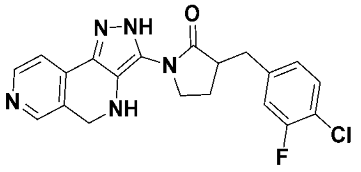, 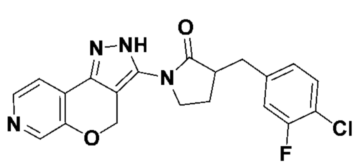,

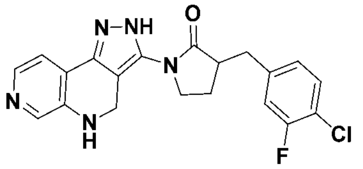, 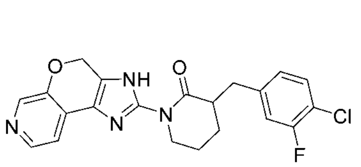, 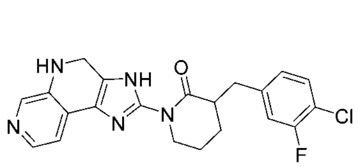,

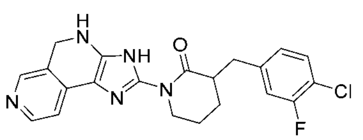, 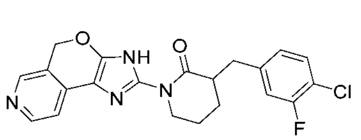, 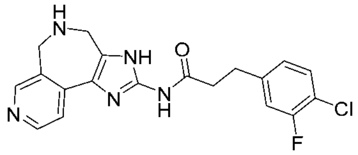,

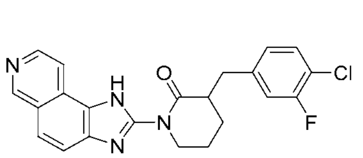, 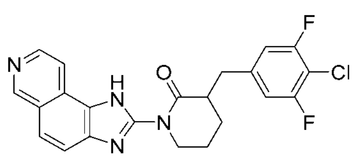, 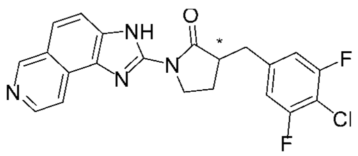,

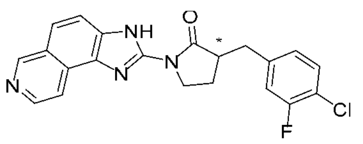, 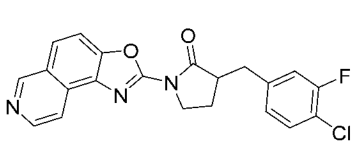, 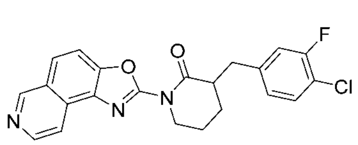,

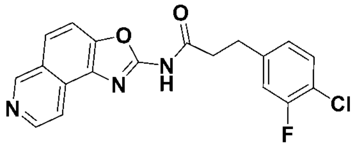, 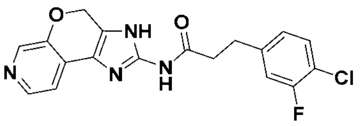, 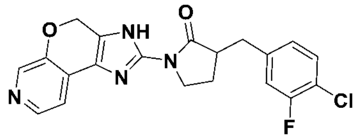,

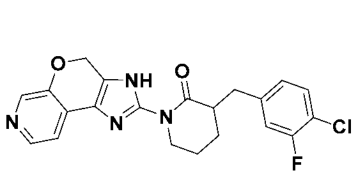, 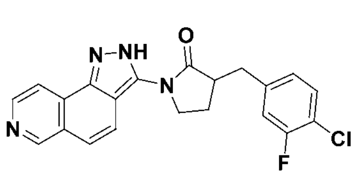, 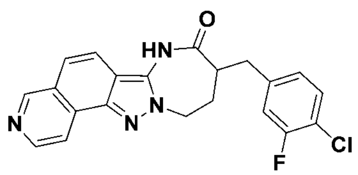,

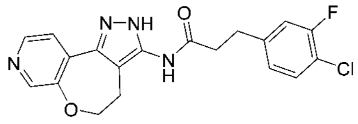, 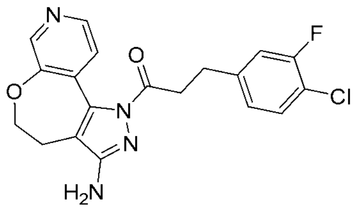, 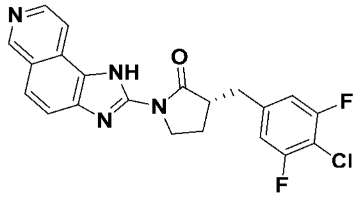,

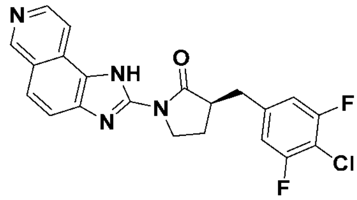, 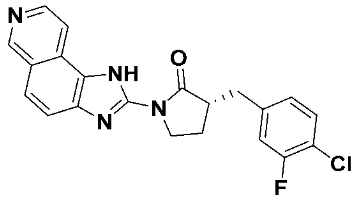, and 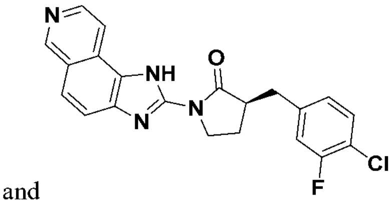.

**28.** A pharmaceutical composition, which comprises the compound, or a stereoisomer, tautomer, polymorph, solvate, hydrate, N-oxide, isotope-labeled compound, metabolite, ester, prodrug of the compound, or a pharmaceutically acceptable salt thereof according to any one of claims 1-27, and
one or more pharmaceutically acceptable carriers or excipients.

**29.** A method for treating or preventing a disease comprising the following steps:
administering a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to any one of claims 1-27, or the pharmaceutical composition according to claim 28, to an individual who (i) suffers from a condition **characterized by** axonal degeneration or (ii) is at risk of suffering from a condition **characterized by** axonal degeneration.

**30.** A method for treating or preventing axonal degeneration, comprising administering to an individual in need thereof a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to any one of claims 1-27, or the pharmaceutical composition according to claim 28.

**31.** A method for treating or preventing a disorder or condition **characterized by** axonal degeneration, comprising administering to an individual in need thereof a therapeutically or prophylactically effective amount of the compound, or a stereoisomer, tautomer, polymorph, solvate, hydrate, N-oxide, isotope-labeled compound, metabolite, ester, prodrug of the compound, or a pharmaceutically acceptable salt thereof, according to any one of claims 1-27, or the pharmaceutical composition according to claim 28;

preferably, the disorder or condition **characterized by** axonal degeneration is neurodegenerative disease or peripheral neuropathy;
the neurodegenerative disease is Alzheimer’s disease, Parkinson’s disease, multiple sclerosis, amyotrophic sclerosis, or Huntington’s disease.

**32.** Use of the compound or a stereoisomer, tautomer, polymorph, solvate, hydrate, N-oxide, isotope-labeled compound, metabolite, ester, prodrug of the compound, or a pharmaceutically acceptable salt thereof according to any one of claims 1-27, or the pharmaceutical composition according to claim 28, in the manufacture of a medicament for treating or preventing axonal degeneration, or in the manufacture of a medicament for treating or preventing a disorder or condition **characterized by** axonal degeneration;

preferably, the disease or condition **characterized by** axonal degeneration is neurodegenerative disease or peripheral neuropathy;
the neurodegenerative disease is Alzheimer’s disease, Parkinson’s disease, multiple sclerosis, amyotrophic sclerosis or Huntington’s disease.

**33.** The compound or a stereoisomer, tautomer, polymorph, solvate, hydrate, N-oxide, isotope-labeled compound, metabolite, ester, prodrug of the compound, or a pharmaceutically acceptable salt thereof according to any one of

claims 1-27, or the pharmaceutical composition according to claim 28, for use in treating or preventing axonal degeneration, or treating or preventing a disorder or condition **characterized by** axonal degeneration;

preferably, the disease or condition **characterized by** axonal degeneration is neurodegenerative disease or peripheral neuropathy;
the neurodegenerative disease is Alzheimer's disease, Parkinson's disease, multiple sclerosis, amyotrophic sclerosis or Huntington's disease.

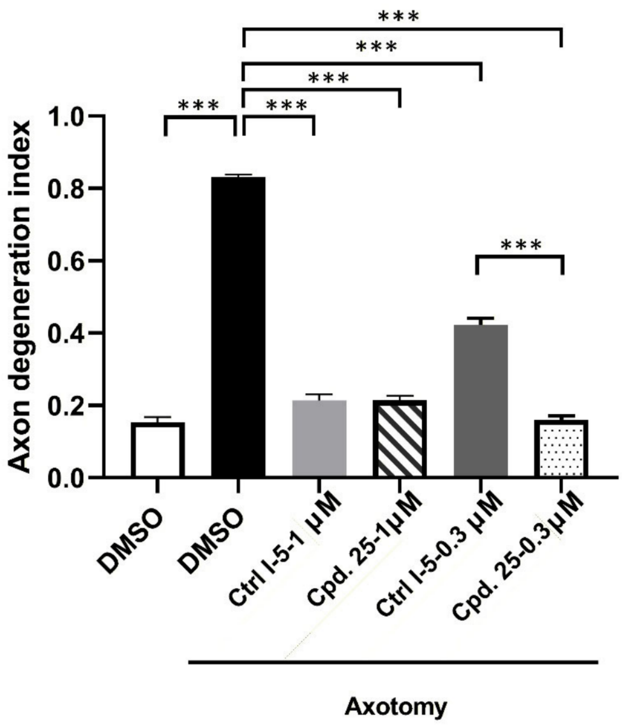


Figure 1

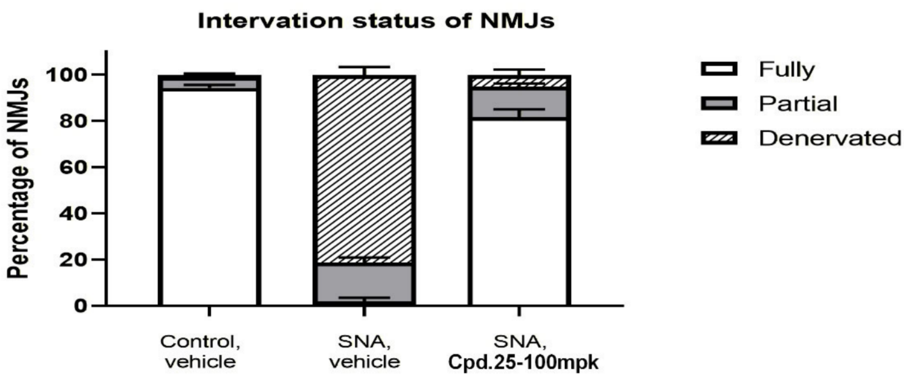


Figure 2

**INTERNATIONAL SEARCH REPORT**

International application No.
**PCT/CN2024/103839**

**A. CLASSIFICATION OF SUBJECT MATTER**

C07D491/14(2006.01)i; A61K31/395(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: C07D/-, A61K/-, A61P/-

Documentation searched other than minimum documentation to the extent that such documents are included in the fields searched

Electronic data base consulted during the international search (name of data base and, where practicable, search terms used)

CNTXT, ENTXT, CNKI, STN (CAPLUS, REGISTRY, MARPAT): 检索结构式, search for structural formula, 苯基, 二氢吡喃, 氧杂环庚烷, 吡啶, 稠环, 稠和, 酰胺, 神经轴突变性, 沃勒变性, structure, NAD+, NMNAT2, SARM1, phenyl, dihydropyran, oxepane, pyridine, fuse+ ring, amide, axon degeneration, wallerian degeneration

**C. DOCUMENTS CONSIDERED TO BE RELEVANT**

| Category* | Citation of document, with indication, where appropriate, of the relevant passages | Relevant to claim No. |
|---|---|---|
| X | Registry. "RN 2940663-16-9 etc." *STN*, 03 July 2023 (2023-07-03), pp. 1-7<br>pp. 1-7 | 1-7, 9-10, 14-17, 20-25, 33 |
| PX | WU, Jiawen et al. "Gold-Catalyzed Redox-Neutral Reaction of Nitriles with Quinoline N-Oxides"<br>*Advanced Synthesis & Catalysis*, Vol. 365, No. (19), 23 August 2023 (2023-08-23), pp. 3335-3341<br>compound 31 | 1-7, 10, 15-17, 20-24, 33 |
| PX | Registry. "RN 2973088-94-5 etc." *STN*, 08 September 2023 (2023-09-08), p. 1<br>page 1 | 1-7, 9-10, 15-17, 20-24, 33 |
| PX | Registry. "RN 2999193-43-8 etc." *STN*, 06 November 2023 (2023-11-06), p. 1<br>page 1 | 2-7, 10, 15-17, 20-22, 24, 33 |

☑ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 September 2024** | **25 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/103839**

**C. DOCUMENTS CONSIDERED TO BE RELEVANT**

| Category* | Citation of document, with indication, where appropriate, of the relevant passages | Relevant to claim No. |
|---|---|---|
| A | Registry. "RN 2940663-16-9 etc."<br>*STN*, 03 July 2023 (2023-07-03), pp. 1-7<br>pages 1-7 | 8, 11-13, 18-19, 26-32 |

Form PCT/ISA/210 (second sheet) (July 2022)

**INTERNATIONAL SEARCH REPORT**

International application No.
**PCT/CN2024/103839**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29-31**
because they relate to subject matter not required to be searched by this Authority, namely:

Claims 29-31 relate to a method for treating a disease. The present search report for said claims is provided on the basis of the corresponding pharmaceutical use of the compound set forth in the present application.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310834844
- CN 202311370973X
- WO 2022046606 A1